# EUROPÄISCHE PATENTSCHRIFT

(11) **EP 1 100 789 B1**
(45) Veröffentlichungstag und Bekanntmachung des Hinweises auf die Patenterteilung: **09.02.2005**
(21) Anmeldenummer: 99941423.8
(22) Anmeldetag: 13.07.1999
(51) Int. Cl.: C07D 249/12, C07D 239/10, C07D 231/20, C07D 253/08, C07D 285/12, C07D 263/58, C07D 233/30, C07D 237/32, A01N 43/653

(54) **SUBSTITUIERTE BENZOYLCYCLOHEXANDIONE**
SUBSTITUTED BENZOYLCYCLOHEXANDIONES
BENZOYLCYCLOHEXANEDIONES SUBSTITUEES

(30) Priorität: 24.07.1998 DE 19833360; 11.05.1999 DE 19921732
(43) Veröffentlichungstag der Anmeldung: 23.05.2001
(73) Patentinhaber: Bayer CropScience AG, 40789 Monheim (DE)
(72) Erfinder: SCHWARZ, Hans-Georg, D-40764 Langenfeld (DE); MÜLLER, Klaus-Helmut, D-40593 Düsseldorf (DE); LEHR, Stefan, D-40764 Langenfeld (DE); SCHALLNER, Otto, D-40789 Monheim (DE); DREWES, Mark, Wilhelm, D-40764 Langenfeld (DE); FEUCHT, Dieter, D-40789 Monheim (DE); PONTZEN, Rolf, D-42799 Leichlingen (DE); WETCHOLOWSKY, Ingo, CEP-13280-000 Vinhedo, SP (BR); WROBLOWSKY, Heinz-Jürgen, D-40764 Langenfeld (DE)
(74) Vertreter: Schwenk, Norbert, Dr.
(86) Internationale Anmeldenummer: PCT/EP1999/004929
(87) Internationale Veröffentlichungsnummer: WO 2000/005221

(56) Entgegenhaltungen:
- EP-A- 0 370 332
- EP-A- 0 597 360
- EP-A- 0 617 026
- WO-A-96/26200
- WO-A-97/46530
- WO-A-99/07688
- DE-A- 4 405 614
- CHEMICAL ABSTRACTS, vol. 100, no. 25, 18. Juni 1984 (1984-06-18) Columbus, Ohio, US; abstract no. 209881, NIHON NOHYAKU CO. LTD.: "1,2,4-Triazolin-5-one derivatives" XP002124210 in der Anmeldung erwähnt & JP 58 225070 A
- CHEMICAL ABSTRACTS, vol. 113, no. 3, 16. Juli 1990 (1990-07-16) Columbus, Ohio, US; abstract no. 23929, MURAI T. ET AL.: "Preparation of Delta 2-1,2,4-triazolin-5-one derivatives as antiinflammatory agents" XP002124211 in der Anmeldung erwähnt & JP 02 015069 A (KAKEN PHARMACEUTICAL CO., LTD.)

## Beschreibung

Die Erfindung betrifft neue substituierte Benzoylcyclohexandione. Verfahren zu ihrer Herstellung und ihre Verwendung als Herbizide.

Es ist bereits bekannt, daß bestimmte substituierte Benzoylcyclohexandione herbizide Eigenschaften aufweisen (vgl. EP-A-090262, EP-A-135191, EP-A-186118, EP-A-186119, EP-A-186120, EP-A-319075, WO-A-96/26200, WO-A-97/46530, WO-A-99/07688). Die Wirkung dieser Verbindungen ist jedoch nicht in allen Belangen zufriedenstellend.

Es wurden nun die neuen substituierten Benzoylcyclohexandione der allgemeinen Formel (I), 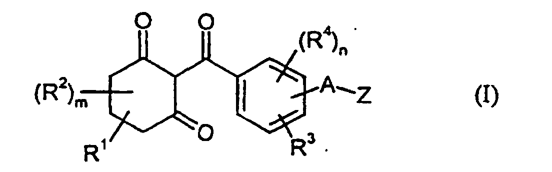 in welcher
- m: für die Zahlen 0, 1 oder 2;
- n: für die Zahlen 0, 1 oder 2;
- A: für Alkandiyl (Alkylen) mit 1 bis 4 Kohlenstoffatomen;
- R¹: für Wasserstoff, für gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl mit 1 bis 6 Kohlenstoffatomen oder für Alkoxycarbonyl mit bis zu 6 Kohlenstoffatomen;
- R²: für gegebenenfalls durch Halogen substituiertes Alkyl mit 1 bis 6 Kohlenstoffatomen, oder zusammen mit R¹ für Alkandiyl (Alkylen) mit 2 bis 5 Kohlenstoffatomen, wobei in diesem Fall m für 1 steht und R¹ und R² am gleichen Kohlenstoffatom ("geminal") oder an zwei benachbarten Kohlenstoffatomen ("vicinal") stehen;
- R³: für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen;
- R⁴: für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen;
- Z: für eine der nachstehenden heterocyclischen Gruppierungen 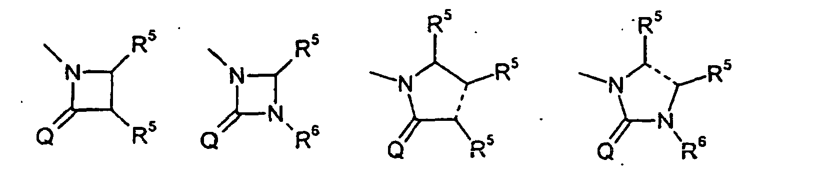 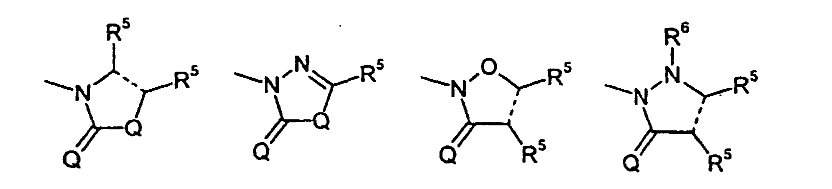 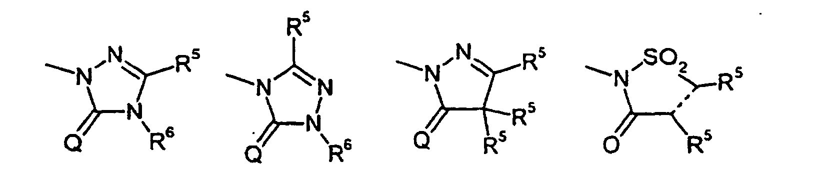 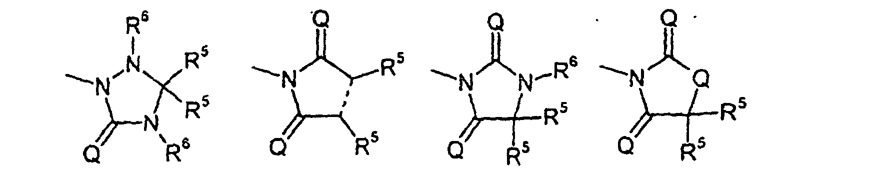 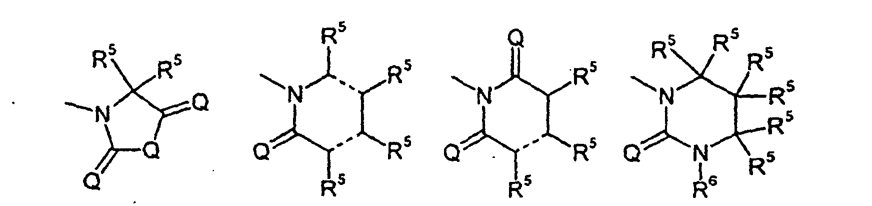 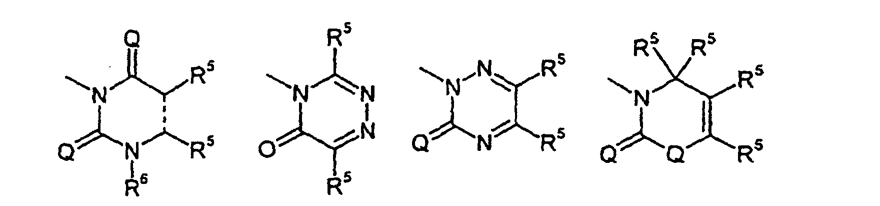 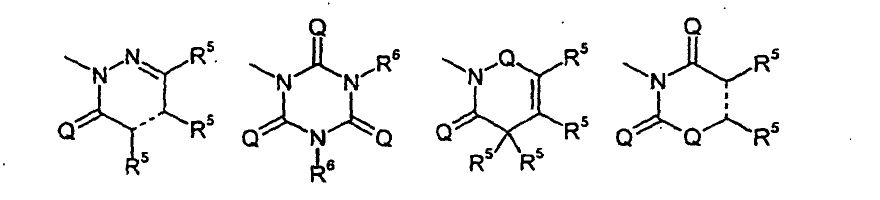 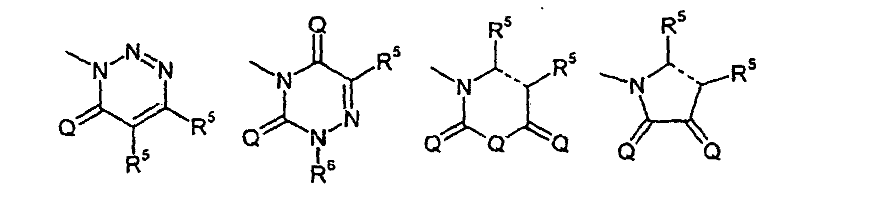 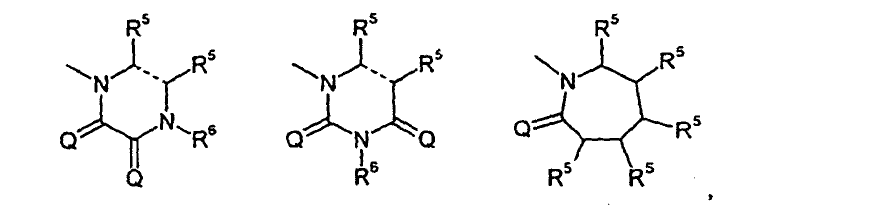
worin jeweils die gestrichelt gezeichnete Bindung eine Einfachbindung oder eine Doppelbindung ist;
- Q: für Sauerstoff oder Schwefel;
- R⁵: für Wasserstoff, Hydroxy, Mercapto, Cyano, Halogen, für jeweils gegebenenfalls durch Cyano, Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für Propadienylthio, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkyloxy, Cycloalkylthio, Cycloalkylamino, Cycloalkylalkyl, Cycloalkylalkoxy, Cycloalkylalkylthio oder Cycloalkylalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, C₁-C₄-Alkyl oder C₁-C₄-Alkoxy substituiertes Phenyl, Phenyloxy, Phenylthio, Phenylamino. Benzyl, Benzyloxy, Benzylthio oder Benzylamino steht, für Pyrrolidino, Piperidino oder Morpholino steht, oder - für den Fall, daß zwei benachbarte Reste R⁵ und R⁵ sich an einer Doppelbindung befindenzusammen mit dem benachbarten Rest R⁵ auch für eine Benzogruppierung und
- R⁶: für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, C₁-C₄-Alkyl oder C₁-C₄-Alkoxy substituiertes Phenyl oder Benzyl steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht;

- wobei die einzelnen Reste R⁵ und R⁶, soweit mehrere davon an gleiche heterocyclische Gruppierungen gebunden sind, gleiche oder verschiedene Bedeutungen im Rahmen der obigen Definition haben können;
- einschließlich aller möglichen tautomeren Formen der Verbindungen der allgemeinen Formel (I) und der möglichen Salze der Verbindungen der allgemeinen Formel (I) -gefunden.

In den Definitionen sind die Kohlenwasserstoffketten, wie Alkyl oder Alkandiyl - auch in Verbindung mit Heteroatomen, wie in Alkoxy - jeweils geradkettig oder verzweigt.

Neben den Verbindungen der allgemeinen Formel (I) - oben - können immer auch die entsprechenden tautomeren Formen - nachstehend beispielhaft dargestellt - vorliegen.

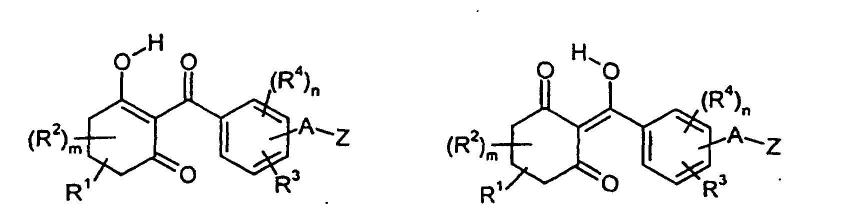

Bevorzugte Substituenten der in den vorstehend gezeigten Formeln aufgeführten Reste werden im folgenden erläutert:
- A: steht besonders bevorzugt für eine Einfachbindung, Methylen, Ethyliden (Ethan-1,1-diyl) oder Dimethylen (Ethan-1,2-diyl).
- R¹: steht besonders bevorzugt für Wasserstoff, für jeweils gegebenenfalls durch Fluor, Chlor, Methoxy, Ethoxy, n- oder i-Propoxy, Methylthio, Ethylthio, n- oder i-Propylthio, Methylsulfmyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i- oder s-Butyl, oder für Methoxycarbonyl, Ethoxycarbonyl, n- oder i-Propoxycarbonyl.
- R²: steht besonders bevorzugt für Methyl, Ethyl, n- oder i-Propyl, oder zusammen mit R¹ für Methylen, Ethan-1,1-diyl (Ethyliden, -CH(CH₃)-), Ethan-1,2-diyl (Dimethylen, -CH₂CH₂-), Propan-1,3-diyl (Trimethylen, -CH₂CH₂CH₂-), Butan-1,4-diyl (Tetramethylen, -CH₂CH₂CH₂CH₂-) oder Pentan-1,5-diyl (Pentamethylen, -CH₂CH₂CH₂CH₂CH₂-), wobei in diesem Fall m für 1 steht und R¹ und R² am gleichen Kohlenstoffatom ("geminal") oder an zwei benachbarten Kohlenstoffatomen ("vicinal") stehen.
- R³: steht besonders bevorzugt für Wasserstoff, Nitro, Cyano, Carboxy, Carbamoyl, Thiocarbamoyl, Fluor, Chlor, Brom, Iod, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s - oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i- Propylsulfonyl, oder für Methylamino, Ethylamino, n- oder i-Propylamino, Dimethylamino, Diethylamino, Dimethylaminosulfonyl oder Diethylaminosulfonyl.
- R⁴: steht besonders bevorzugt für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t- Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i- Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl.
- Z: steht besonders bevorzugt für die nachstehende heterocyclische Gruppierung 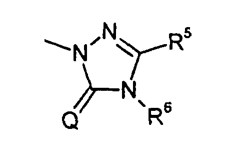
- R⁵: steht besonders bevorzugt für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n - oder i-Propylsulfinyl, Methylsulfonyl, Ethylsulfonyl, n- oder i-Propylsulfonyl substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino, Cyclohexylamino, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopropylmethylthio, Cyclobutylmethylthio, Cyclopentylmethylthio, Cyclohexylmethylthio, Cyclopropylmethylamino, Cyclobutylmethylamino, Cyclopentylmethylamino oder Cyclohexylmethylamino, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino, oder - für den Fall, daß zwei benachbarte Reste R⁵ und R⁵ sich an einer Doppelbindung befinden zusammen mit dem benachbarten Rest R⁵ auch für eine Benzogruppierung.
- R⁶: steht besonders bevorzugt für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl oder Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl, oder zusammen mit einem benachbarten Rest R⁵ oder R⁶ für jeweils gegebenenfalls durch Methyl und/oder Ethyl substituiertes Propan-1,3-diyl (Trimethylen), Butan-1,4-diyl (Tetramethylen) oder Pentan-1-5-diyl (Pentamethylen),
wobei die einzelnen Reste R⁵ und R⁶ - soweit mehrere davon an gleiche heterocyclische Gruppierungen gebunden sind, gleiche oder verschiedene Bedeutungen im Rahmen der obigen Definition haben können.
- A: steht ganz besonders bevorzugt für eine Einfachbindung oder für Methylen.
- R¹: steht ganz besonders bevorzugt für Wasserstoff, Methyl, Ethyl, n- oder i-Propyl.
- R²: steht ganz besonders bevorzugt für Methyl.
- R³: steht ganz besonders bevorzugt für Wasserstoff, Nitro, Cyano, Fluor, Chlor, Brom, Iod, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.
- R⁴: steht ganz besonders bevorzugt für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl.
- R⁵: steht ganz besonders bevorzugt für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy.
- R⁶: steht ganz besonders bevorzugt für Amino, Methyl, Ethyl, n- oder i-Propyl, n-, i-, s- oder t-Butyl, Methoxy, Ethoxy, Methylamino, Dimethylamino, Cycloproypyl oder Cyclopropylmethyl steht, oder zusammen mit R⁵ für Propan-1,3-diyl (Trimethylen), Butan-1,4-diyl (Tetramethylen) oder Pentan-1,5-diyl (Pentamethylen).
- A: steht am meisten bevorzugt für Methylen.

Gegenstand der Erfindung sind vorzugsweise die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzylammonium-Salze der Verbindungen der Formel (I), in welcher m, n, A, R¹, R², R³, R⁴ und Z die oben. angegebenen Bedeutungen haben.

Erfindungsgemäß bevorzugt sind die Verbindungen der Formel (I), in welchen eine Kombination der vorstehend als bevorzugt aufgeführten Bedeutungen vorliegt.

Erfindungsgemäß besonders bevorzugt sind die Verbindungen der Formel (I) in welchen eine Kombination der vorstehend als besonders bevorzugt aufgeführten Bedeutungen vorliegt.

Erfindungsgemäß ganz besonders bevorzugt sind die Verbindungen der Formel (I), in welchen eine Kombination der vorstehend als ganz besonders bevorzugt aufgeführten Bedeutungen vorliegt.

Verbindungen der folgenden allgemeinen Formeln (IA), (IB) und (IC) werden insbesondere als erfindungsgemäß hervorgehoben: 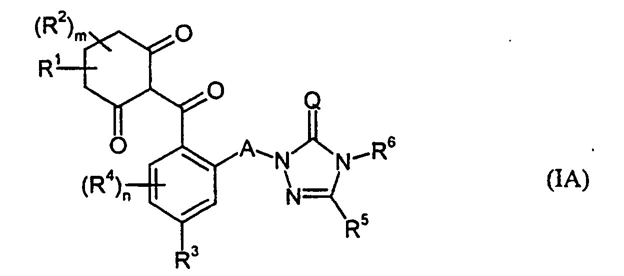 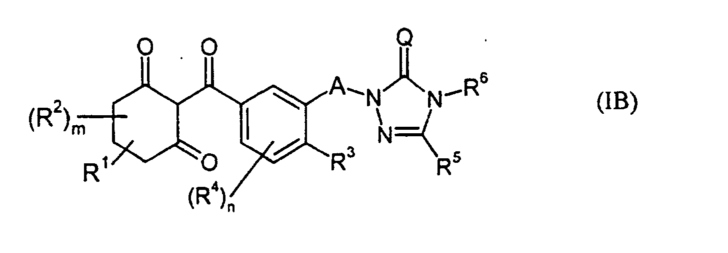 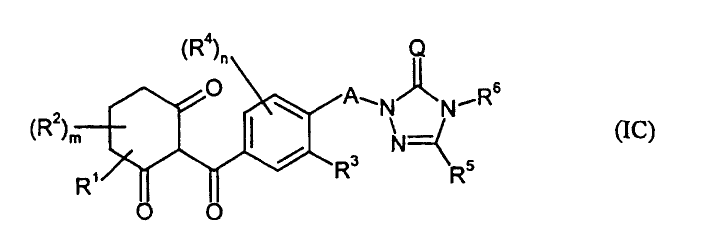 in welchen
- m: für die Zahlen 0, 1 oder 2 steht,
- n: für die Zahlen 0, 1 oder 2 steht,
- A: steht besonders bevorzugt für eine Einfachbindung oder für Methylen.
- Q: für Sauerstoff oder Schwefel steht,
- R¹: für Wasserstoff, Methyl, Ethyl, n- oder i-Propyl steht,
- R²: für Methyl steht,
- R³: für Wasserstoff, Nitro, Cyano, Fluor, Chlor, Brom, Iod, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
- R⁴: für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio. Ethylthio, Methylsulfinyl, Ethylsulfinyl, Methylsulfonyl, Ethylsulfonyl oder Dimethylaminosulfonyl steht,
- R⁵: für Wasserstoff, Hydroxy, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Fluorethyl, Chlorethyl, Difluorethyl, Dichlorethyl, Fluor-n-propyl, Fluor-i-propyl, Chlor-n-propyl, Chlor-i-propyl, Methoxymethyl, Ethoxymethyl, Methoxyethyl, Ethoxyethyl, Methoxy, Ethoxy, n- oder i-Propoxy, n-, i-, s- oder t-Butoxy, Fluorethoxy, Chlorethoxy, Difluorethoxy, Dichlorethoxy, Trifluorethoxy, Trichlorethoxy, Chlorfluorethoxy, Chlordifluorethoxy, Fluordichlorethoxy, Methylthio, Ethylthio, n- oder i-Propylthio, Fluorethylthio, Chlorethylthio, Difluorethylthio, Dichlorethylthio, Chlorfluorethylthio, Chlordifluorethylthio, Fluordichlorethylthio, Methylsulfinyl, Ethylsulfinyl, n- oder i-Propylsulfinyl, Methylsulfonyl, Ethylsulfonyl, n- oder i-Propylsulfonyl, Dimethylamino, Propenylthio, Butenylthio, Propinylthio, Butinylthio, Cyclopropyl, Cyclopropylmethyl, Cyclopropylmethoxy, Phenyl oder Phenoxy steht, und
- R⁶: für Amino, Methyl, Ethyl, n- oder i-Propyl, n-, i-, s- oder t-Butyl, Methoxy, Ethoxy, Methylamino, Dimethylamino, Cycloproypyl oder Cyclopropylmethyl steht, oder zusammen mit R⁵ für Propan-1,3-diyl (Trimethylen), Butan-1,4-diyl (Tetramethylen) oder Pentan-1,5-diyl (Pentamethylen) steht.

Die Verbindungen der Formel (IA), bei welchen A für Methylen steht, werden hierbei ganz besonders hervorgehoben.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Beispiele für die erfindungsgemäßen Verbindungen der allgemeinen Formel (I) sind in den nachstehenden Gruppen aufgeführt.

### Gruppe 1

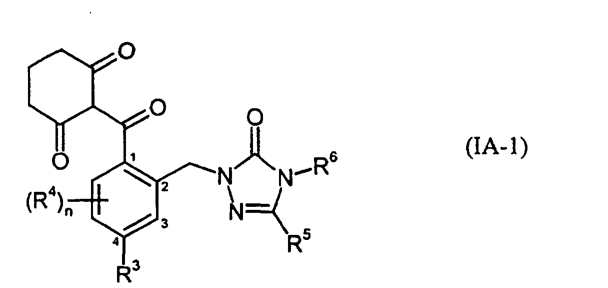

R³, (R⁴)ₙ, R⁵ und R⁶ haben hierbei beispielhaft die in der nachstehenden Tabelle angegebenen Bedeutungen:

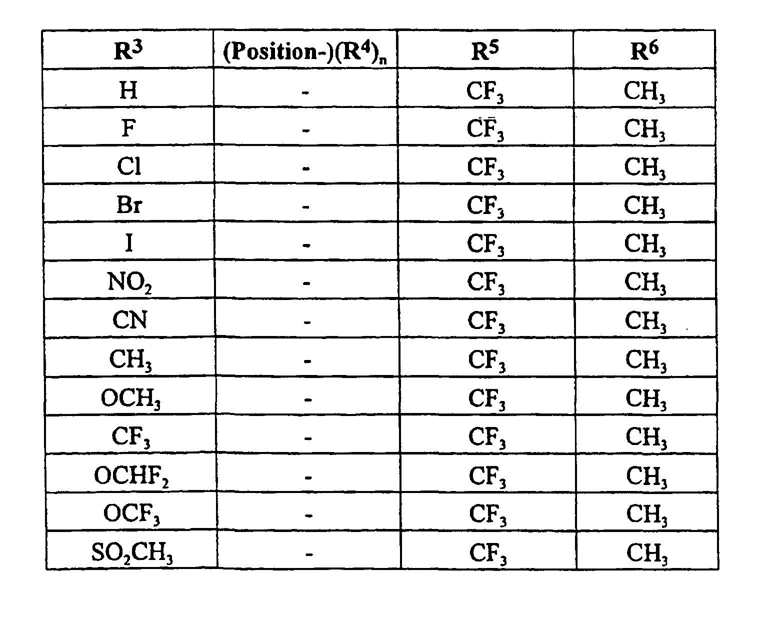

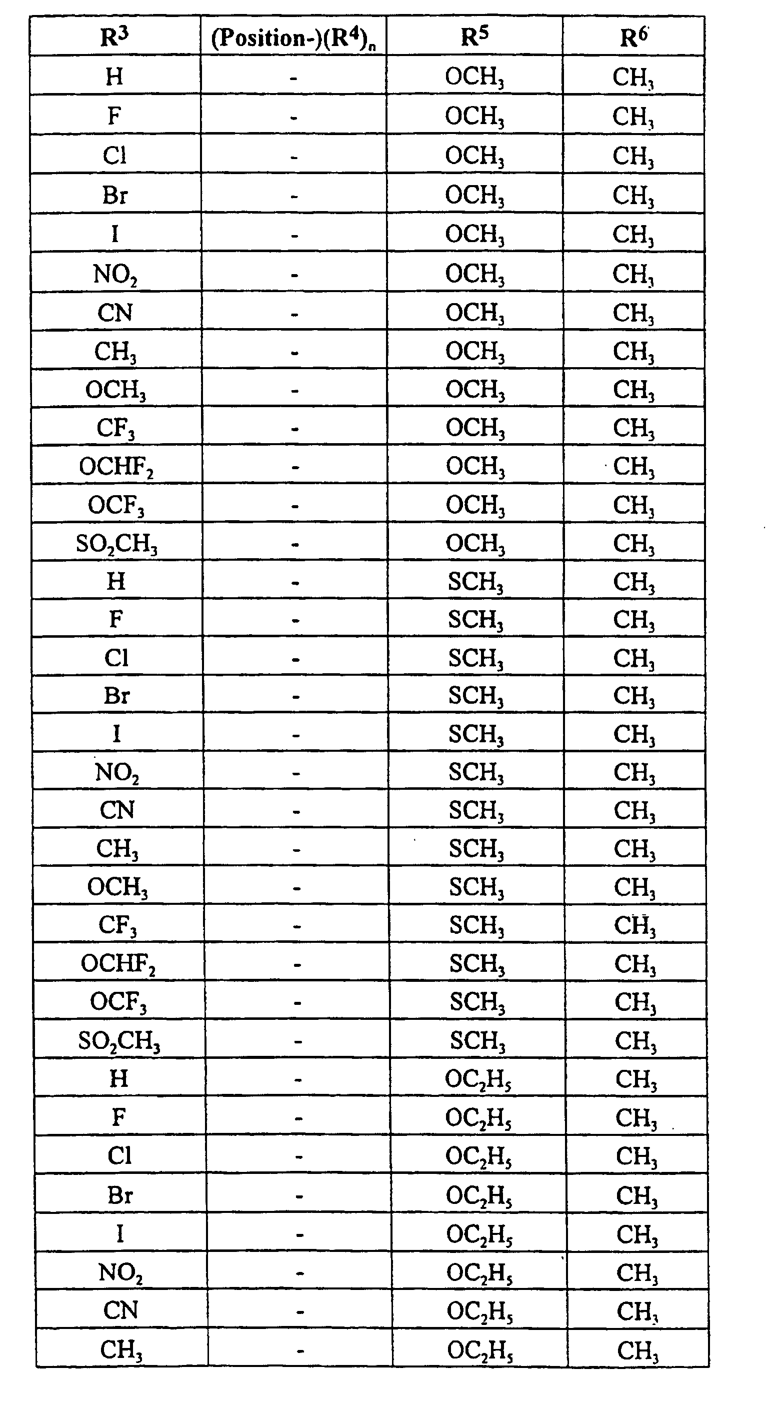

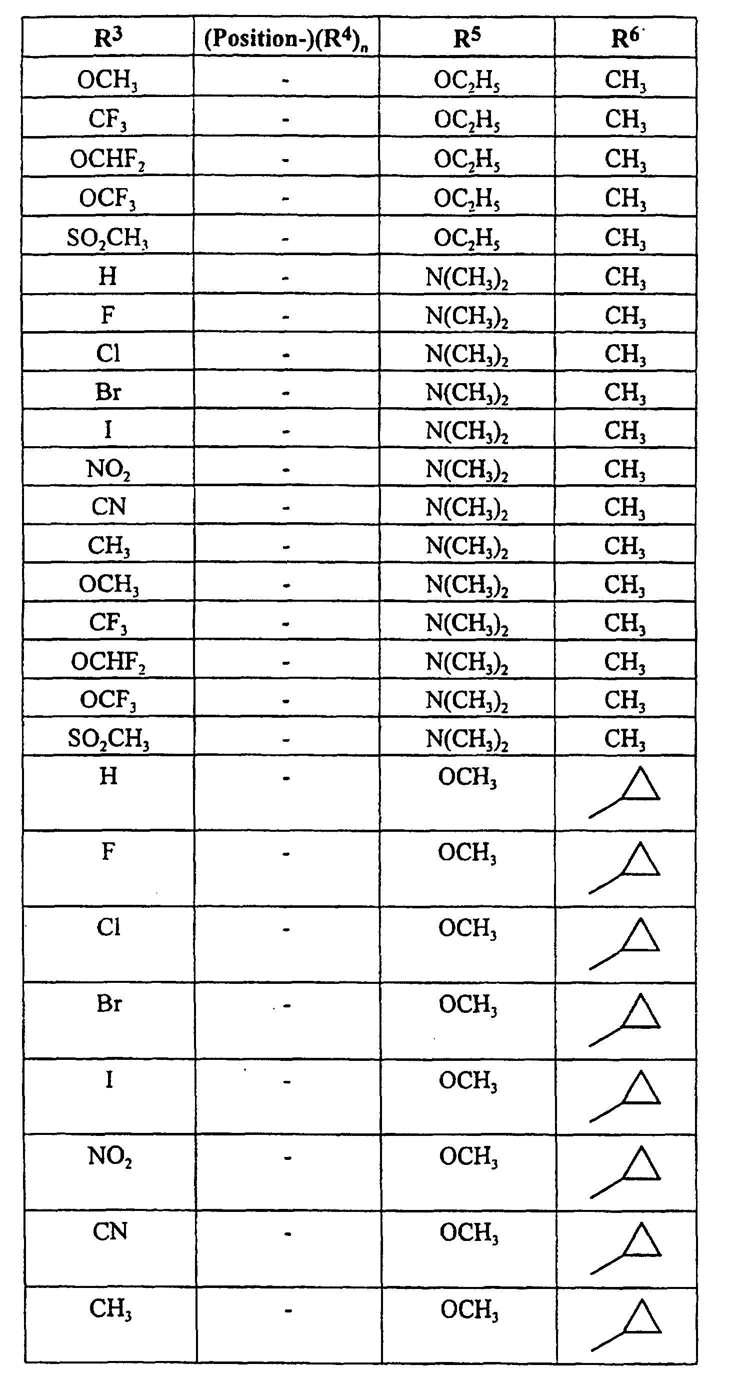

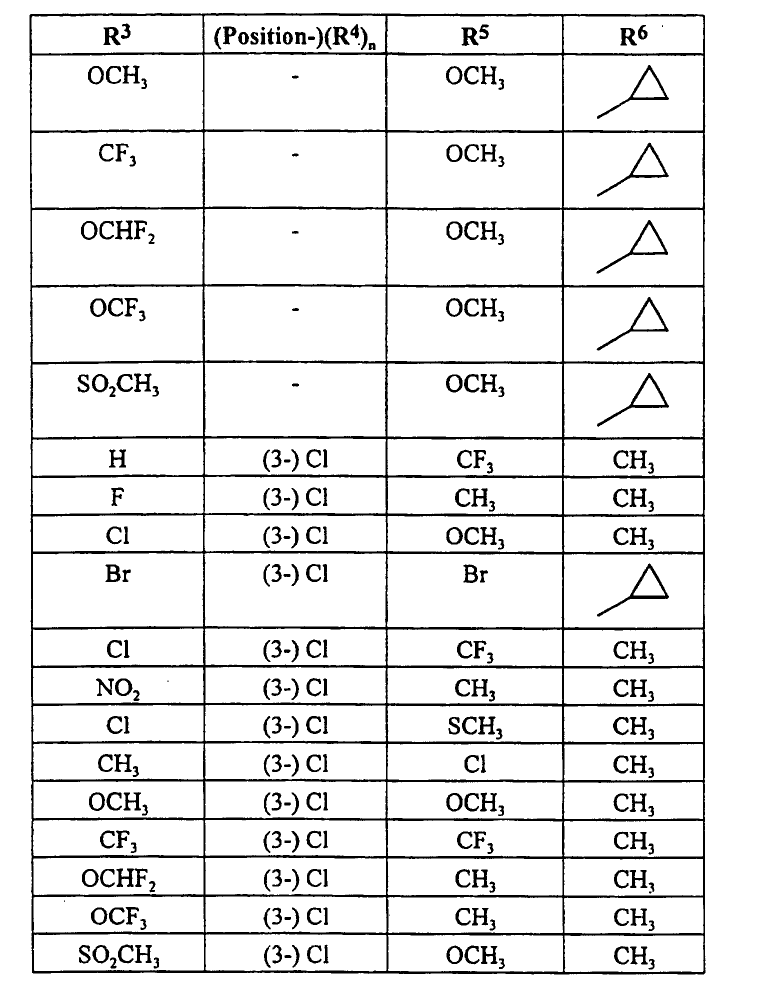

### Gruppe 2

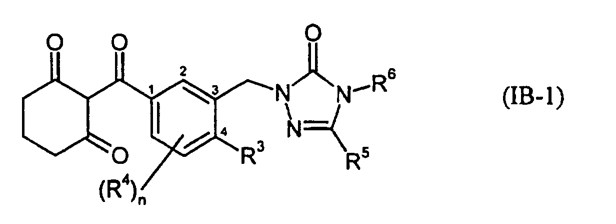

R³, (R⁴)ₙ, R⁵ und R⁶ haben hierbei beispielhaft die in der nachstehenden Tabelle angegebenen Bedeutungen:

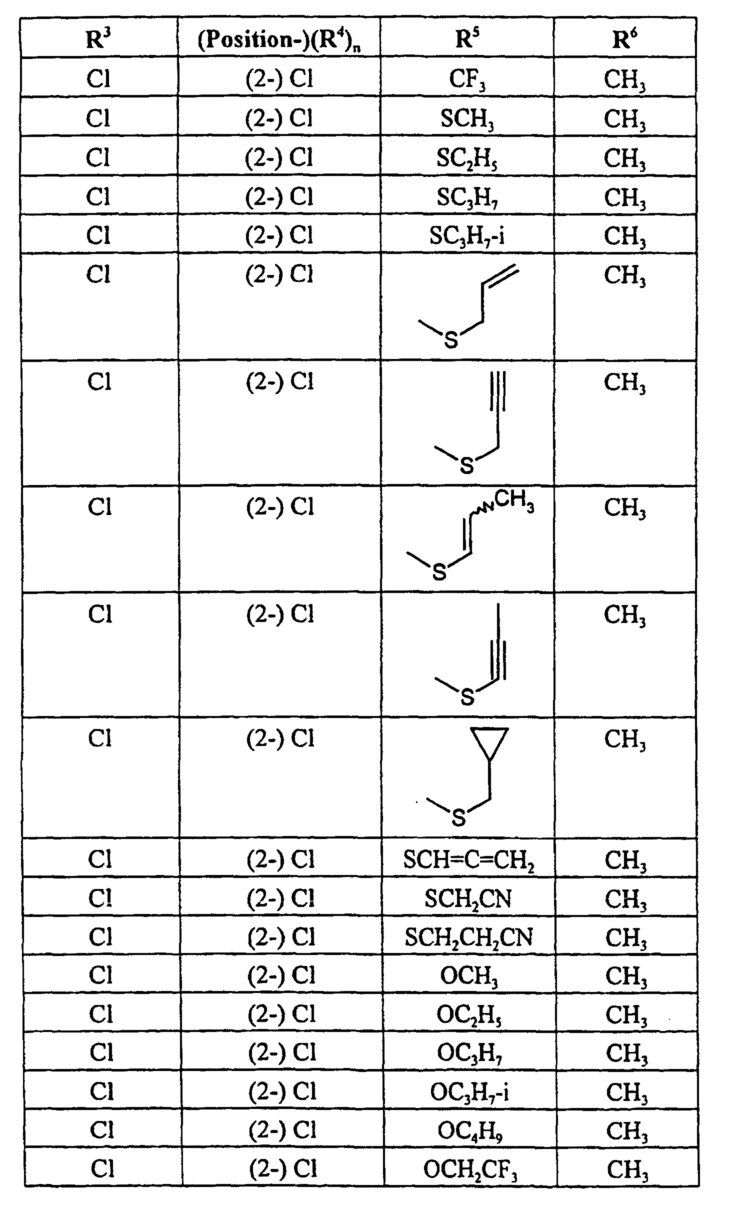

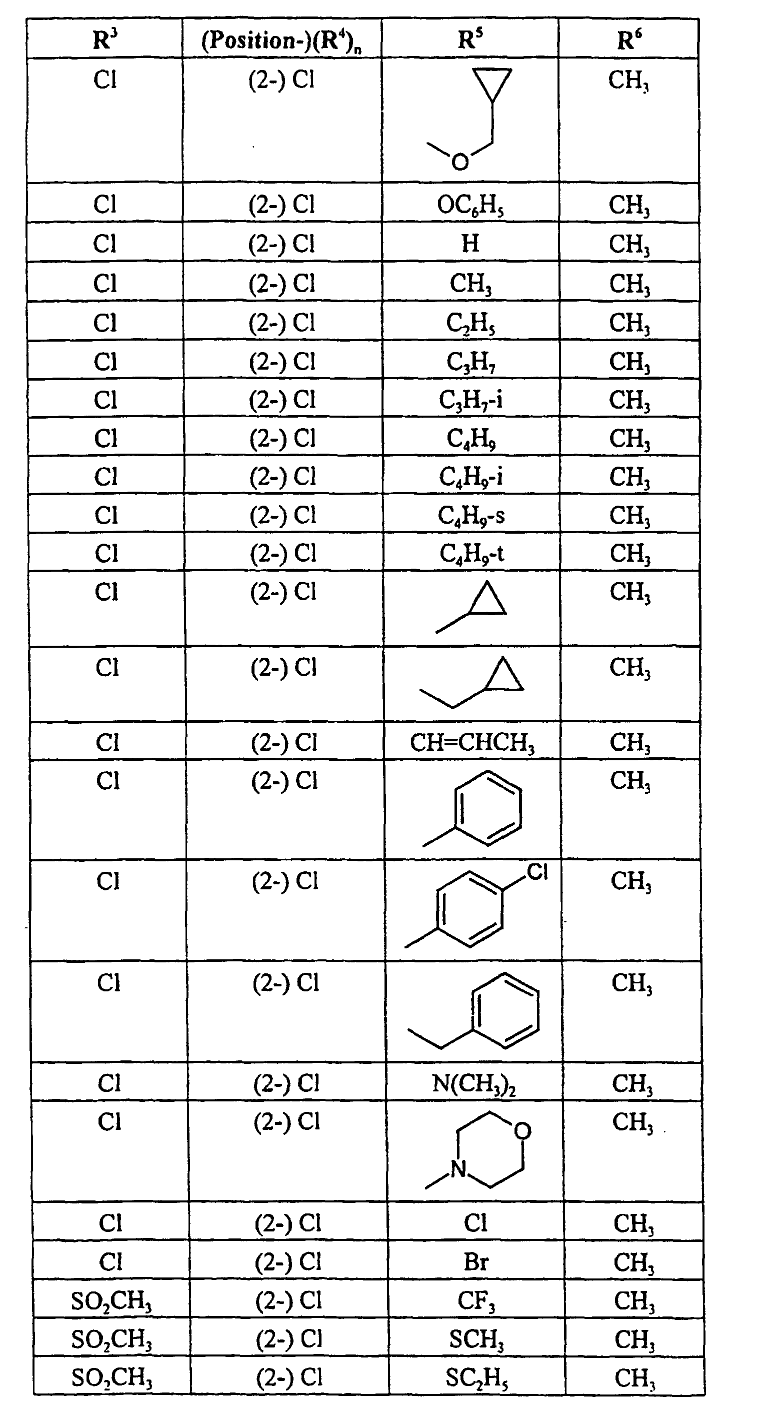

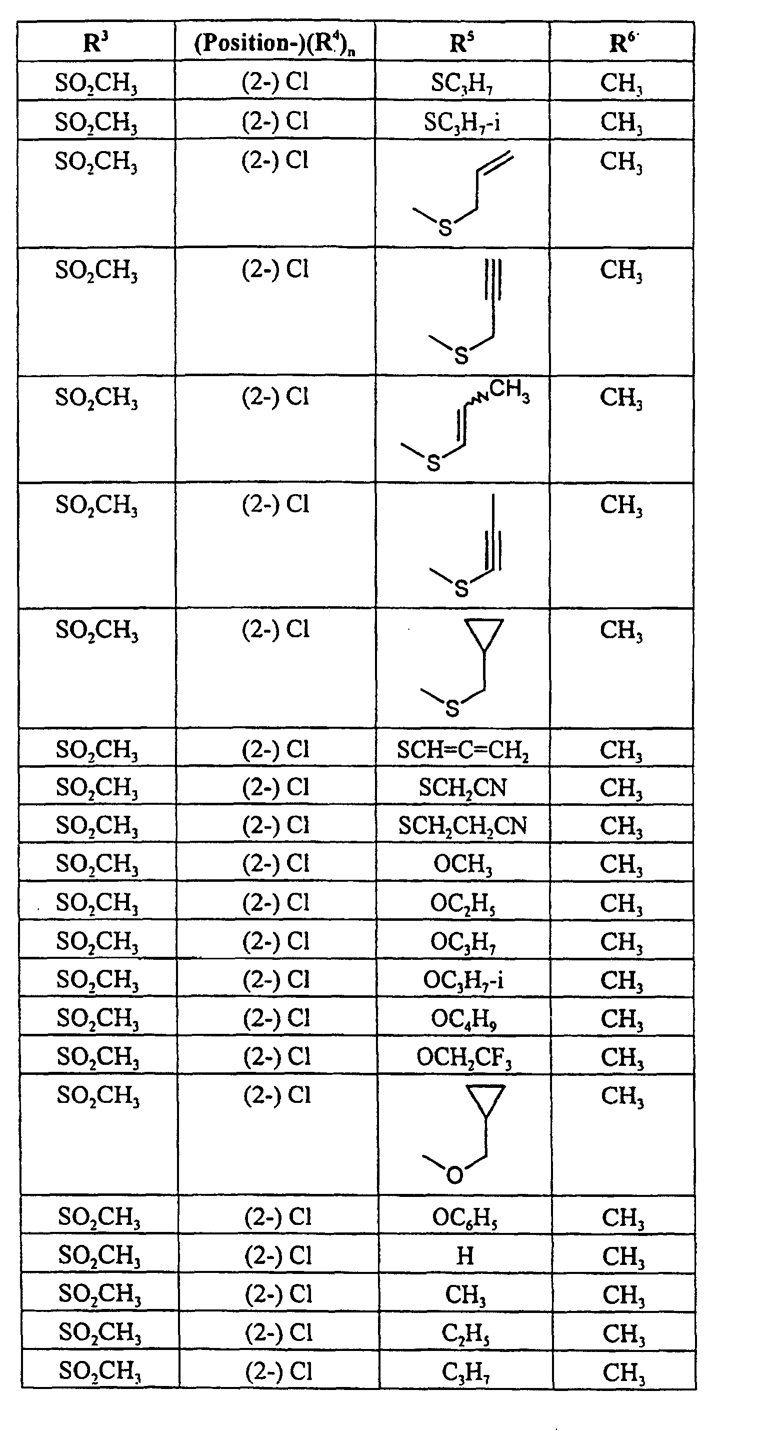

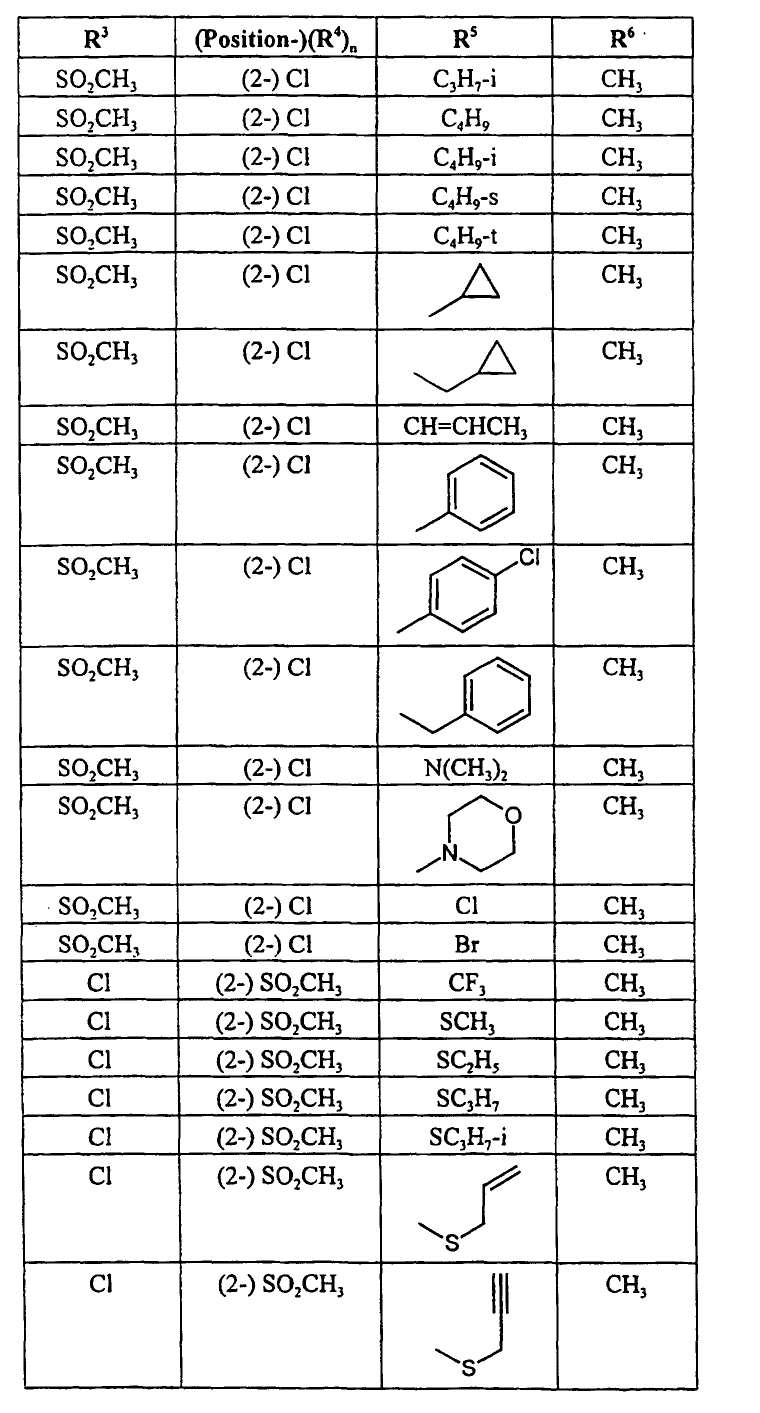

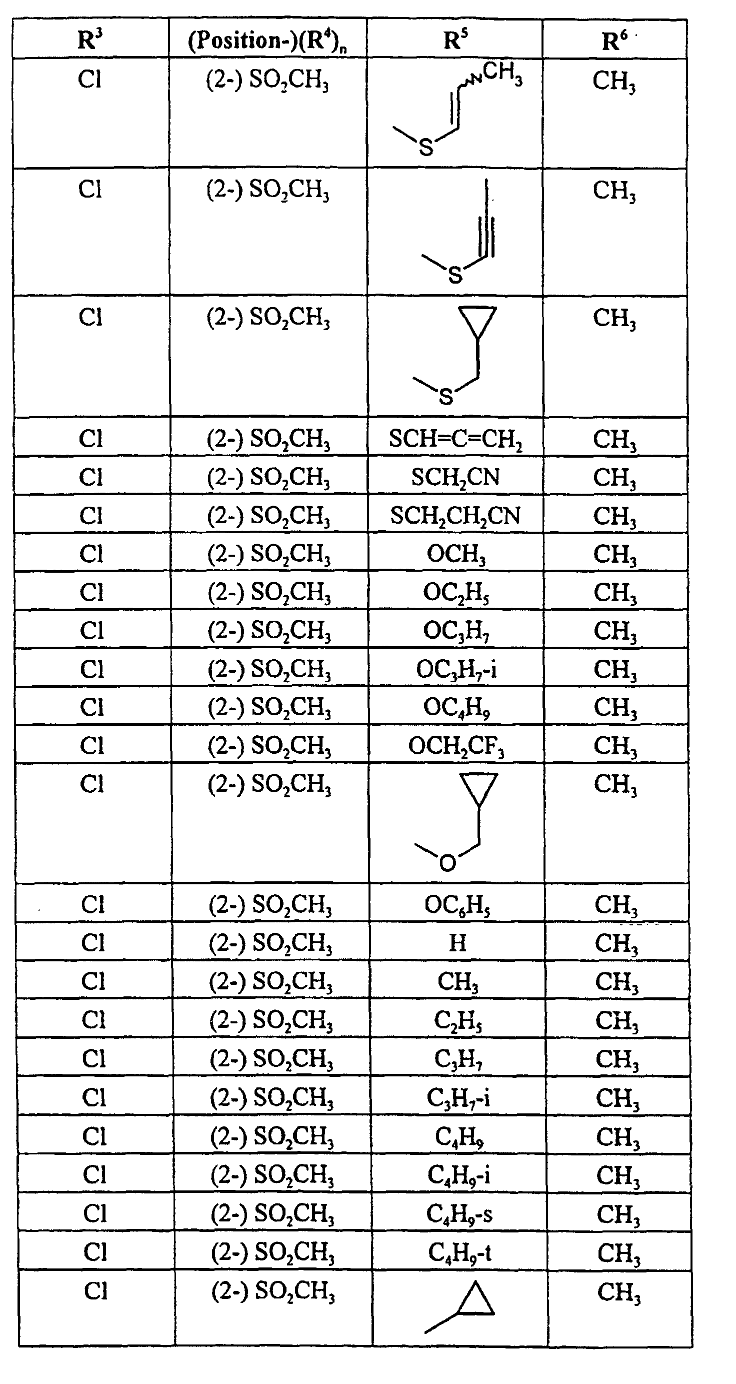

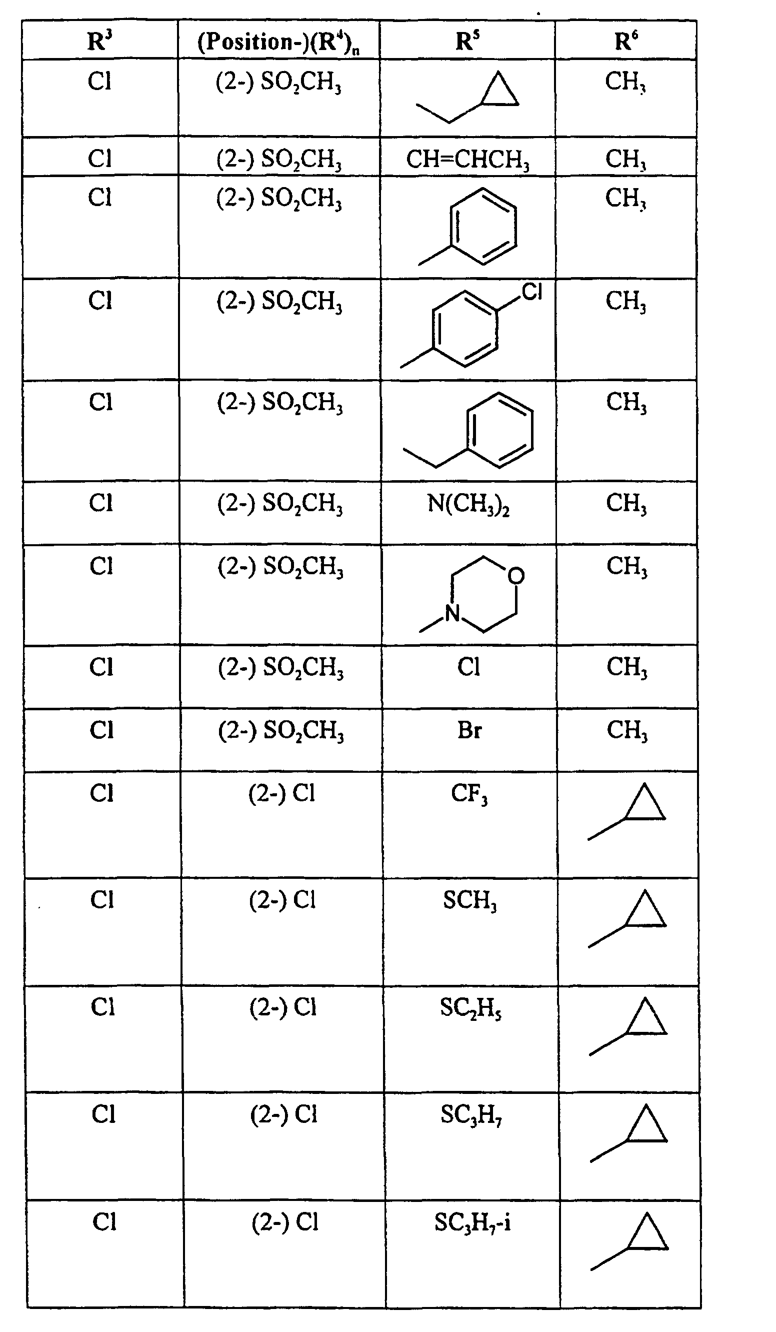

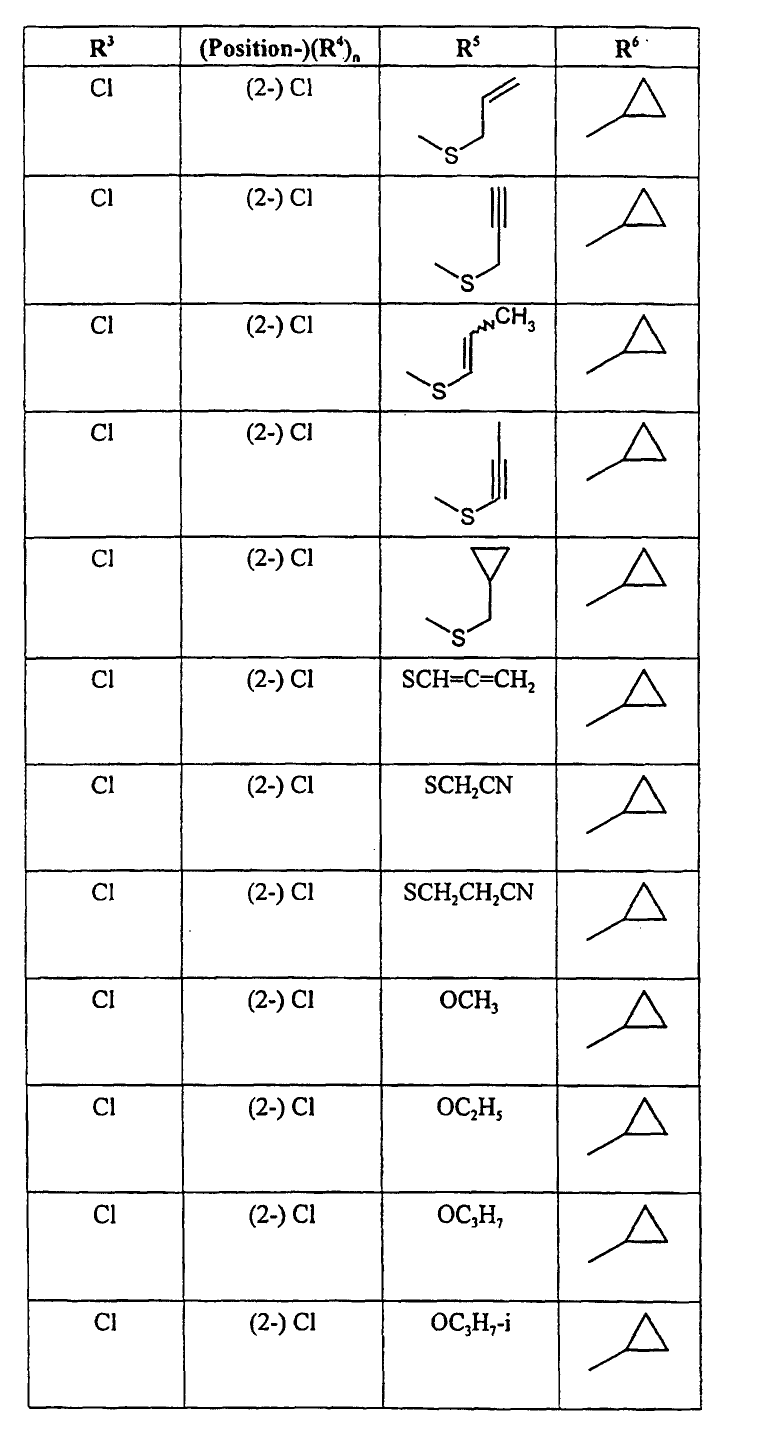

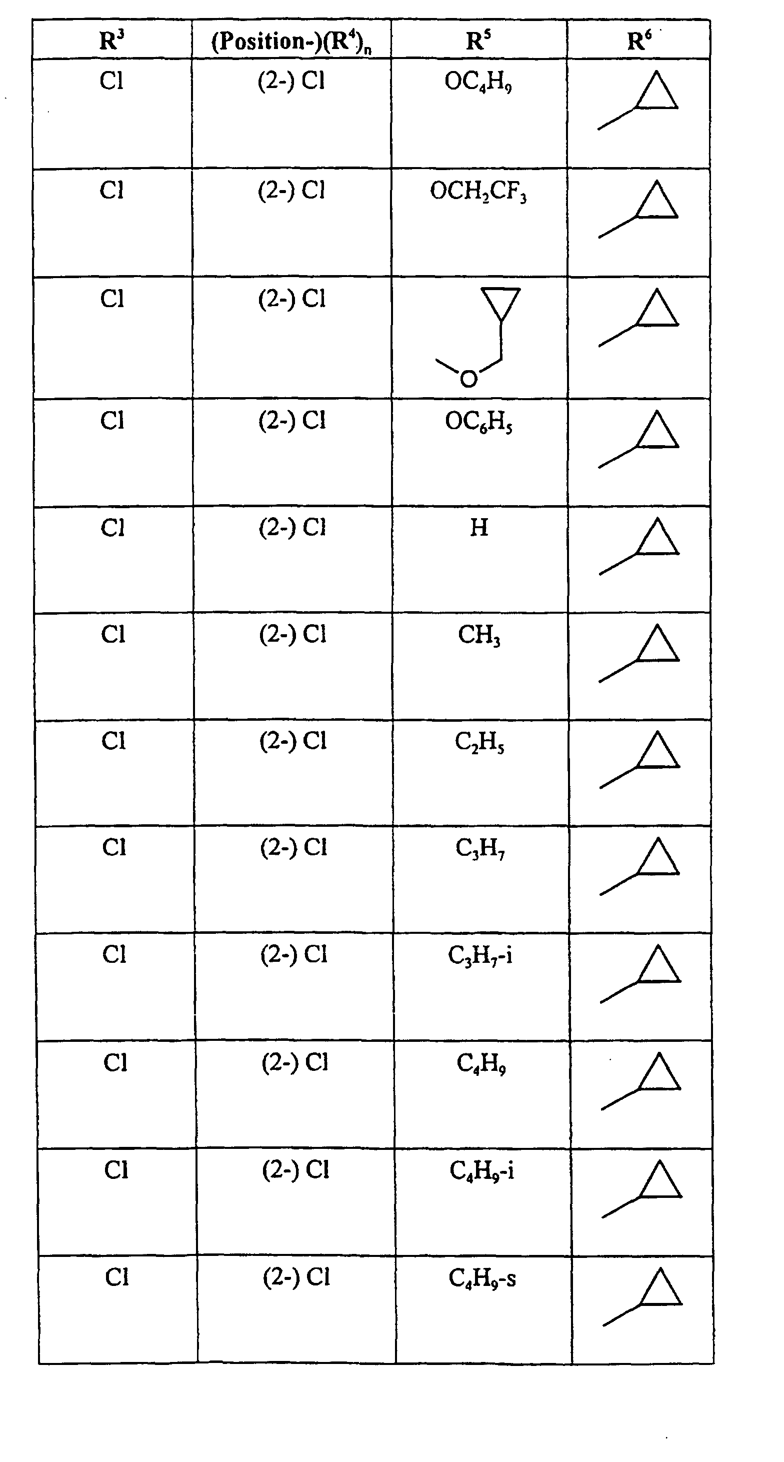

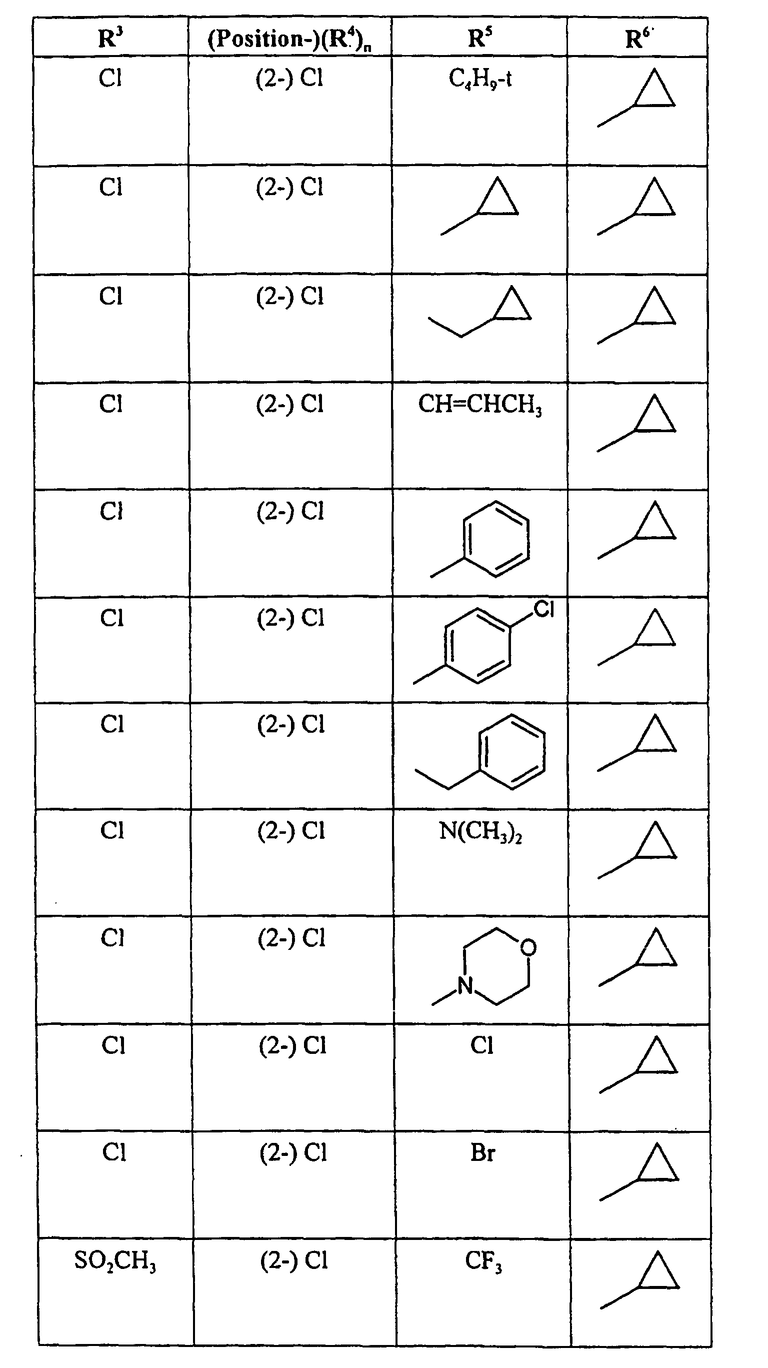

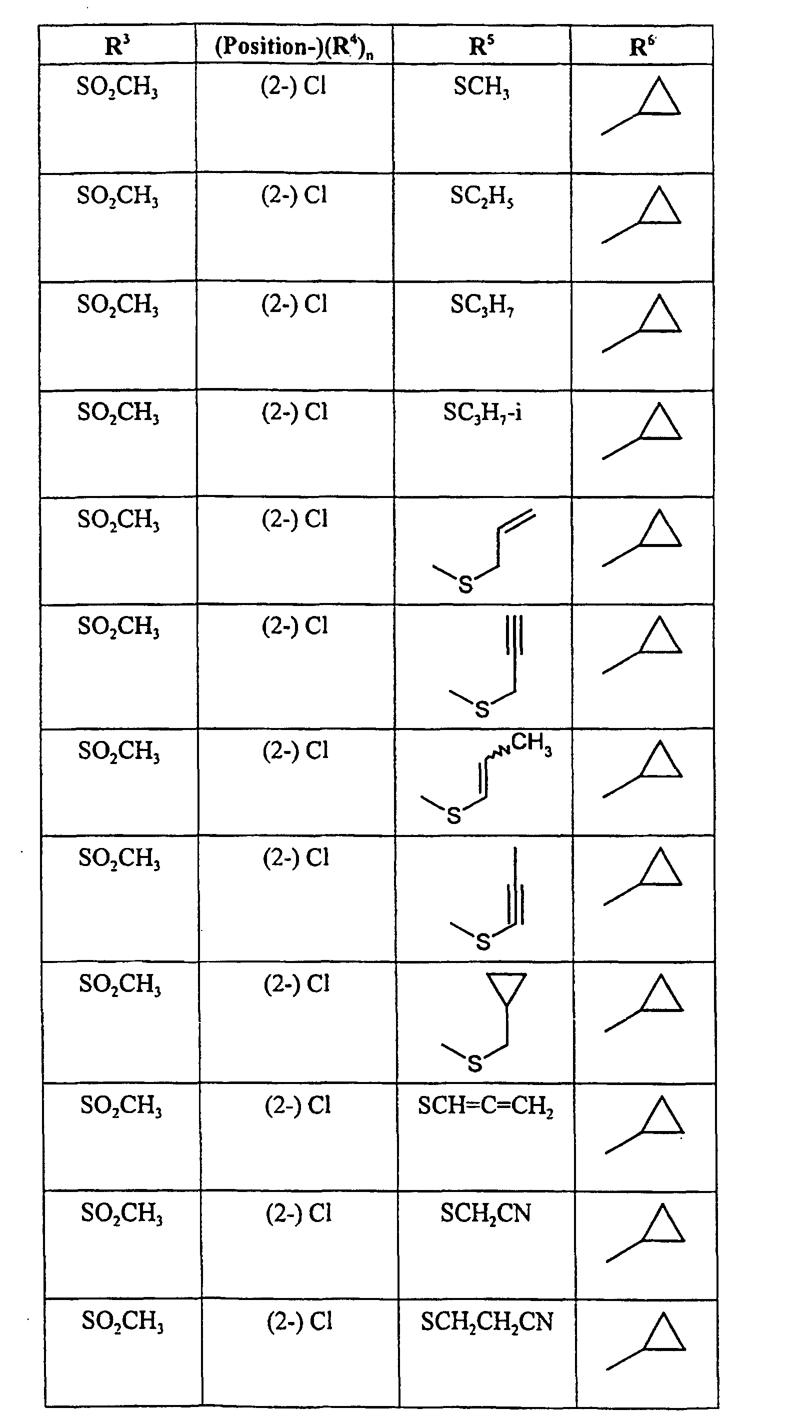

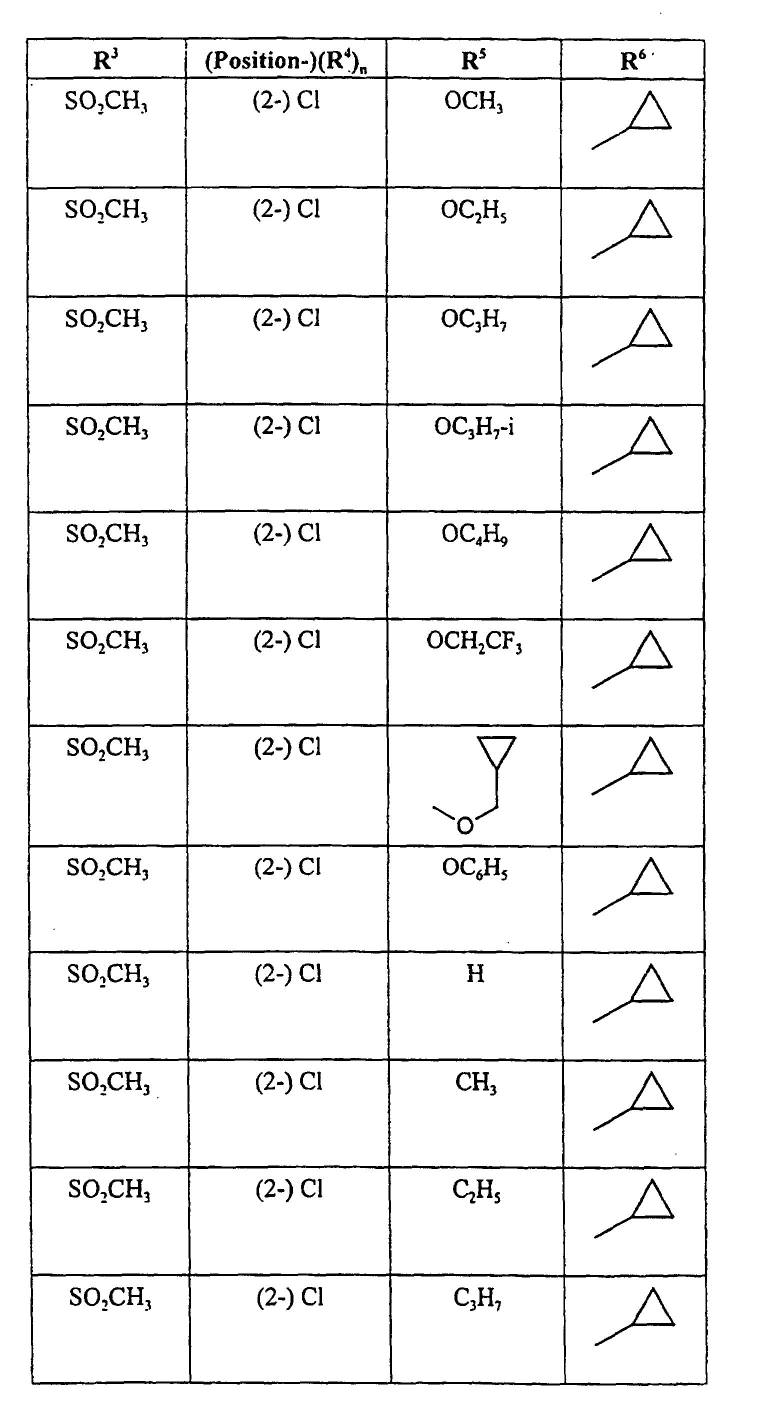

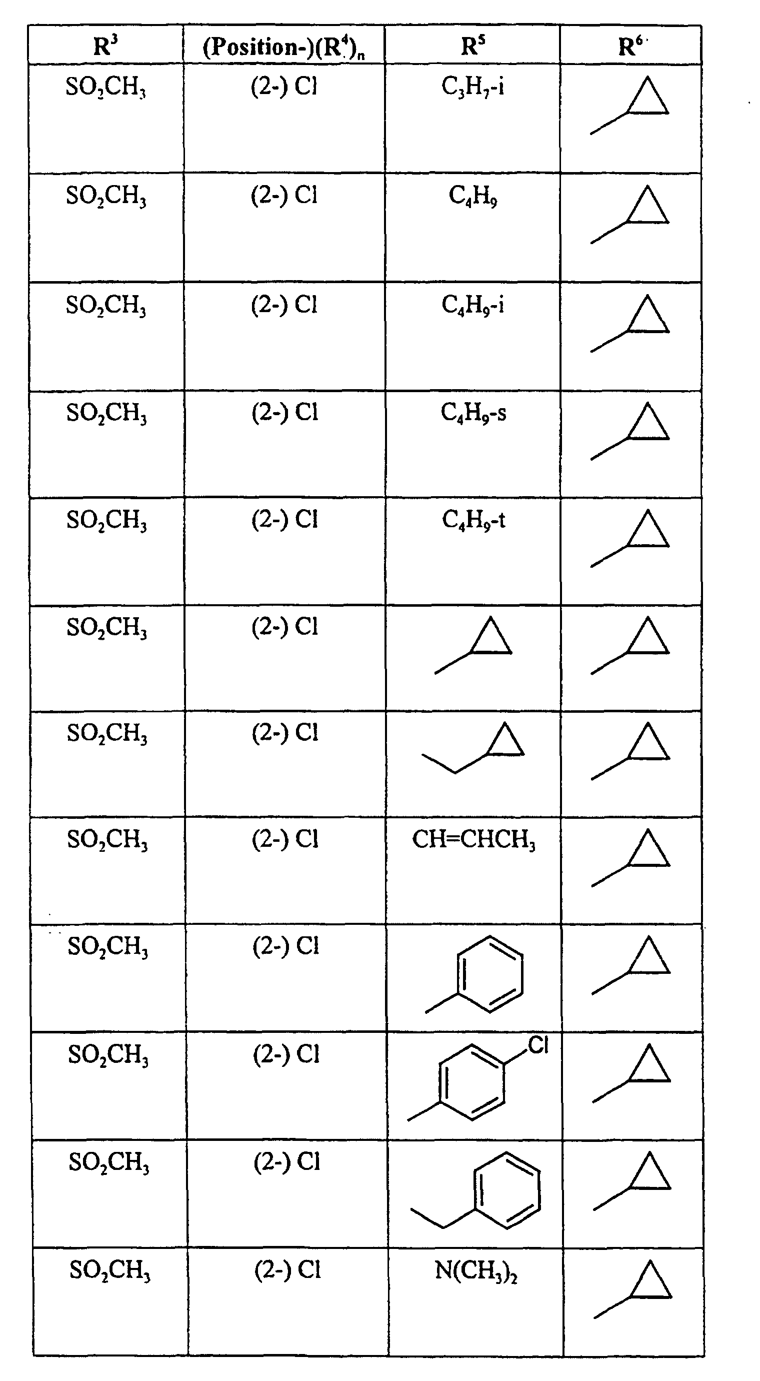

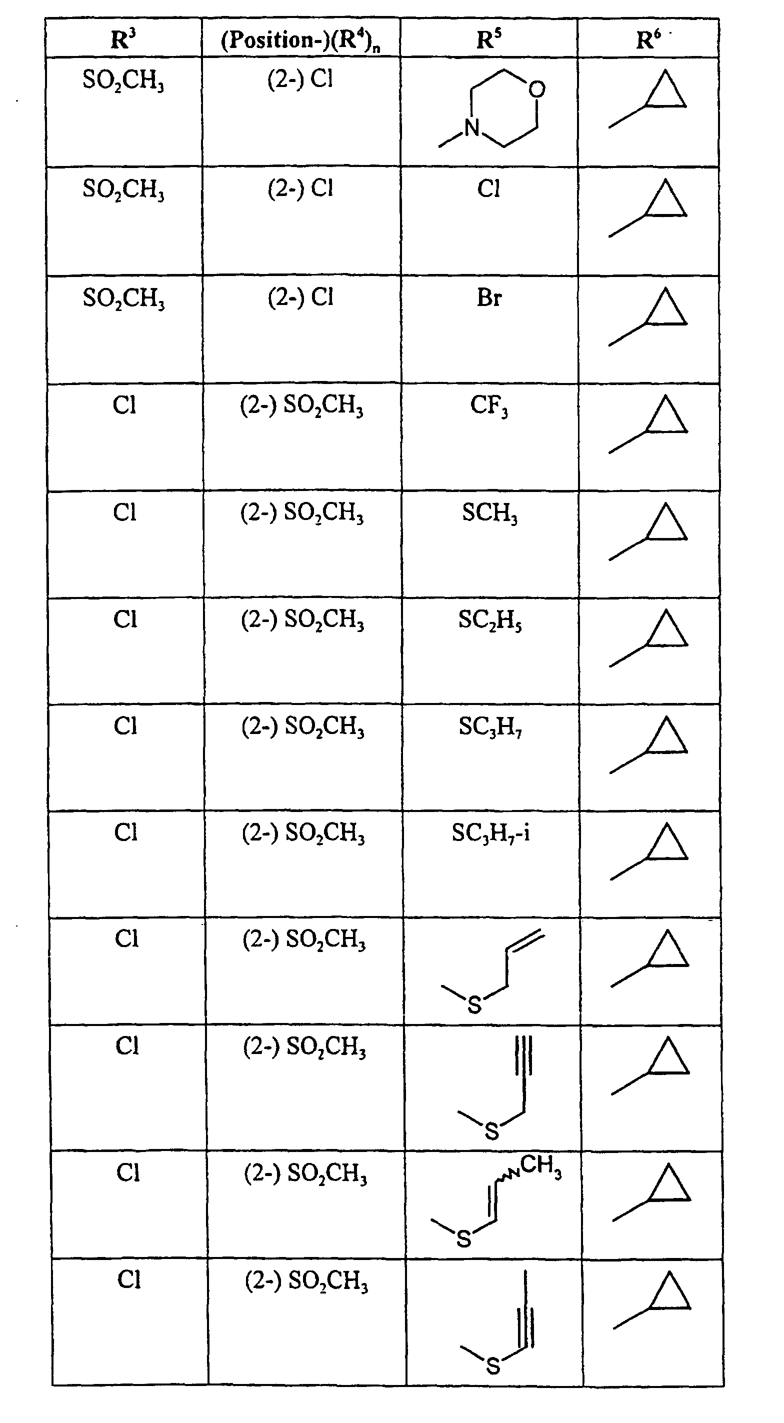

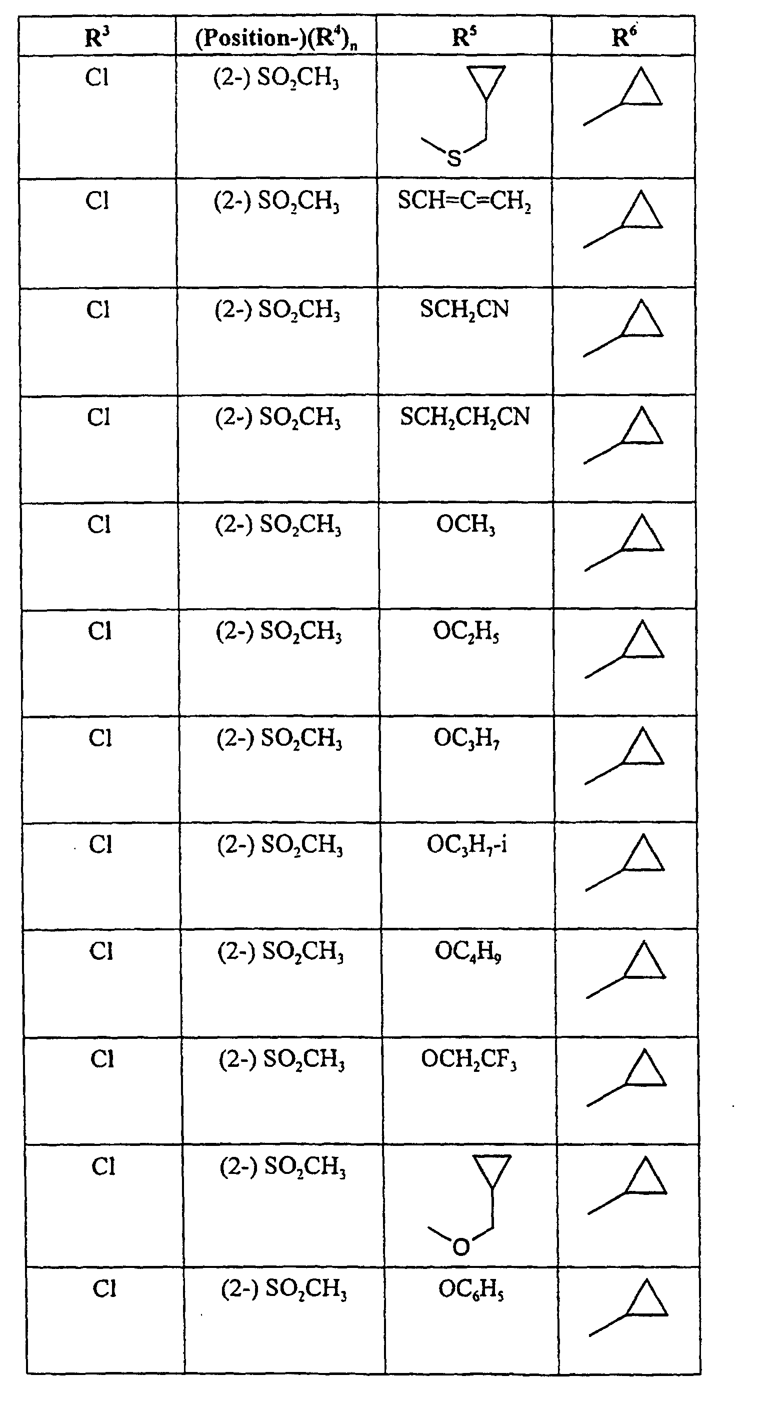

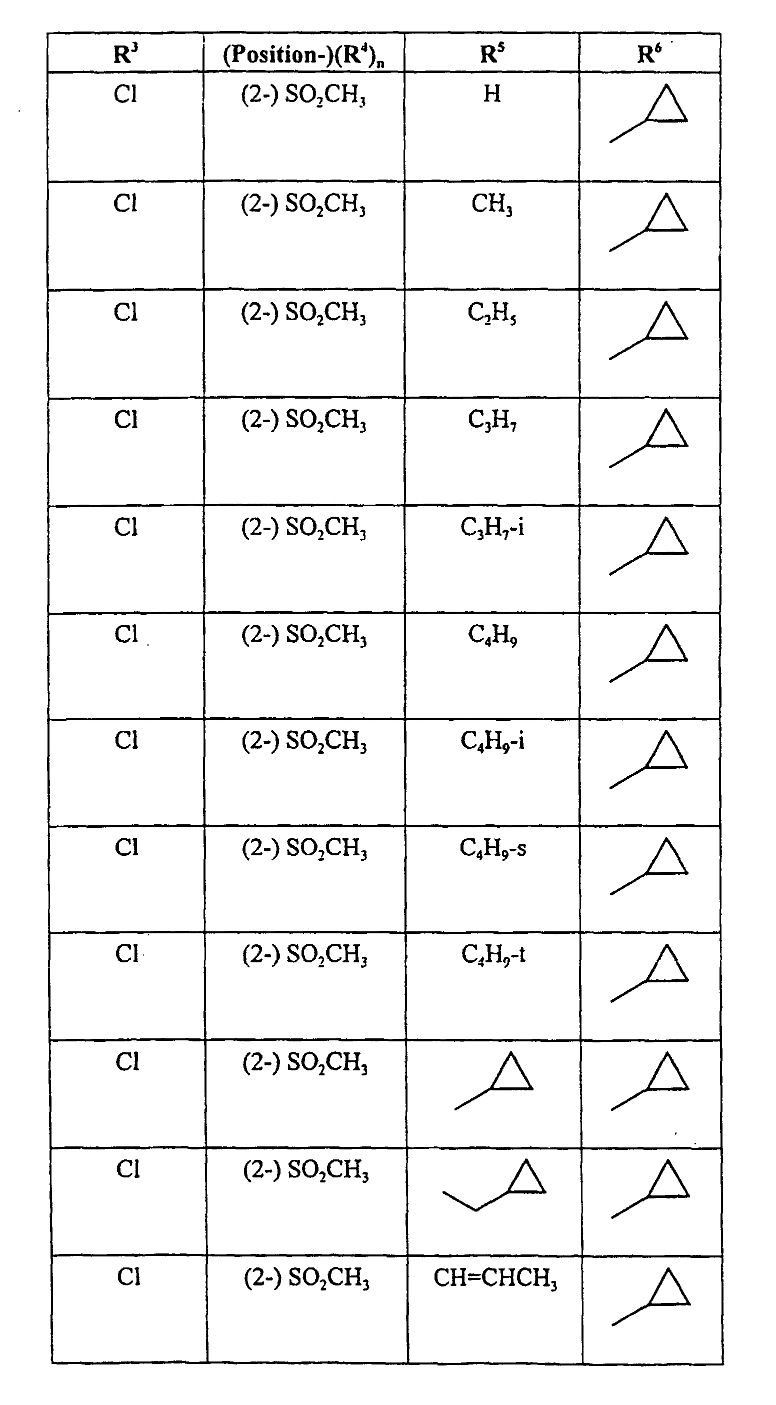

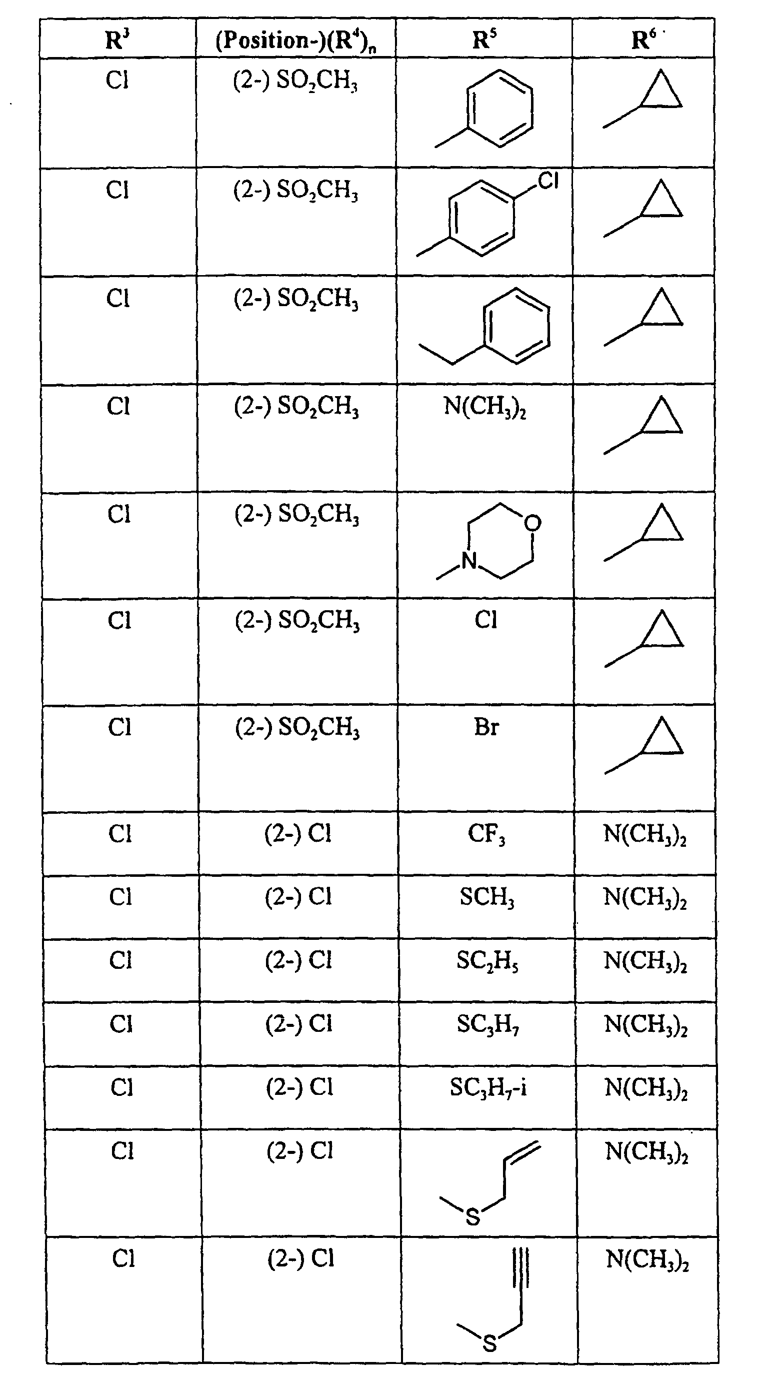

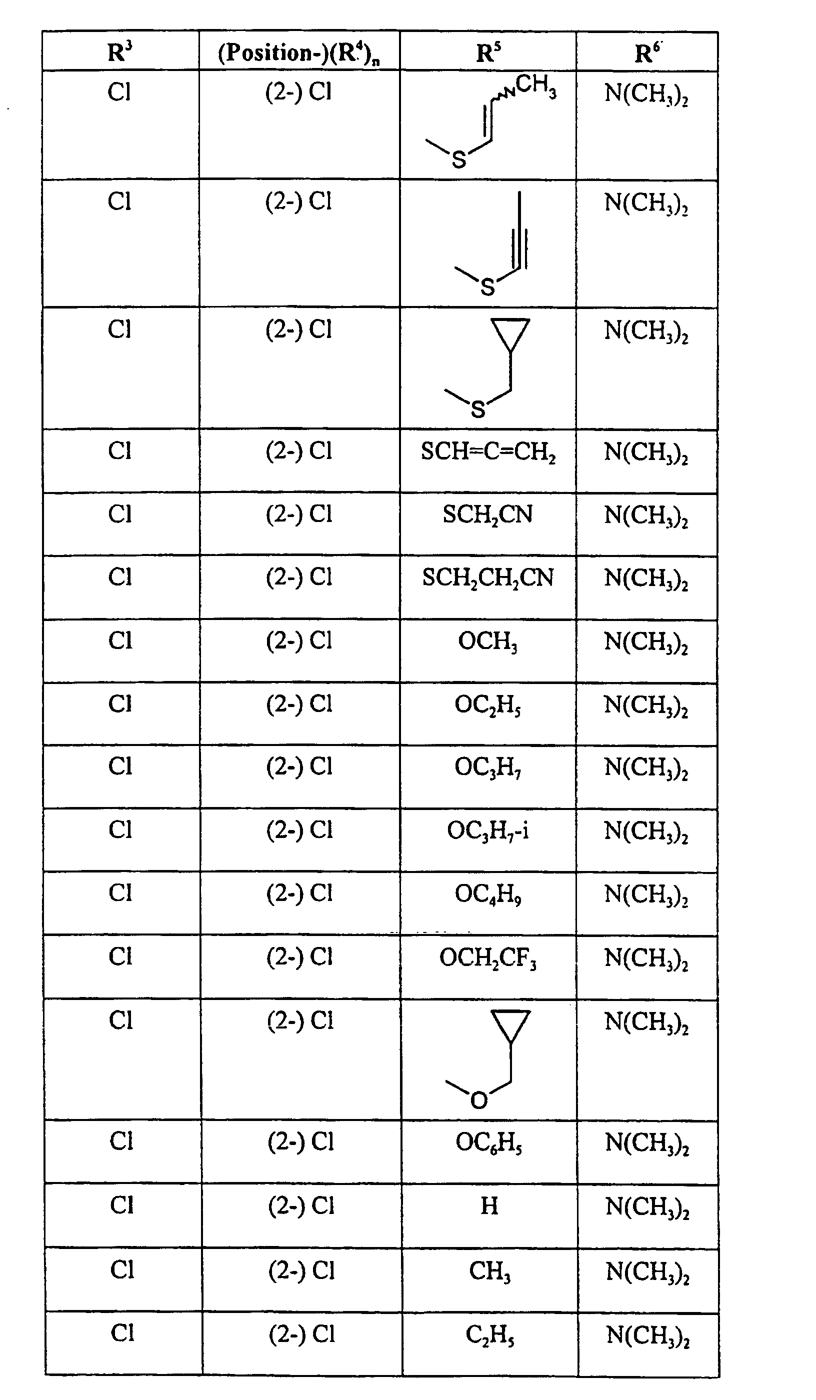

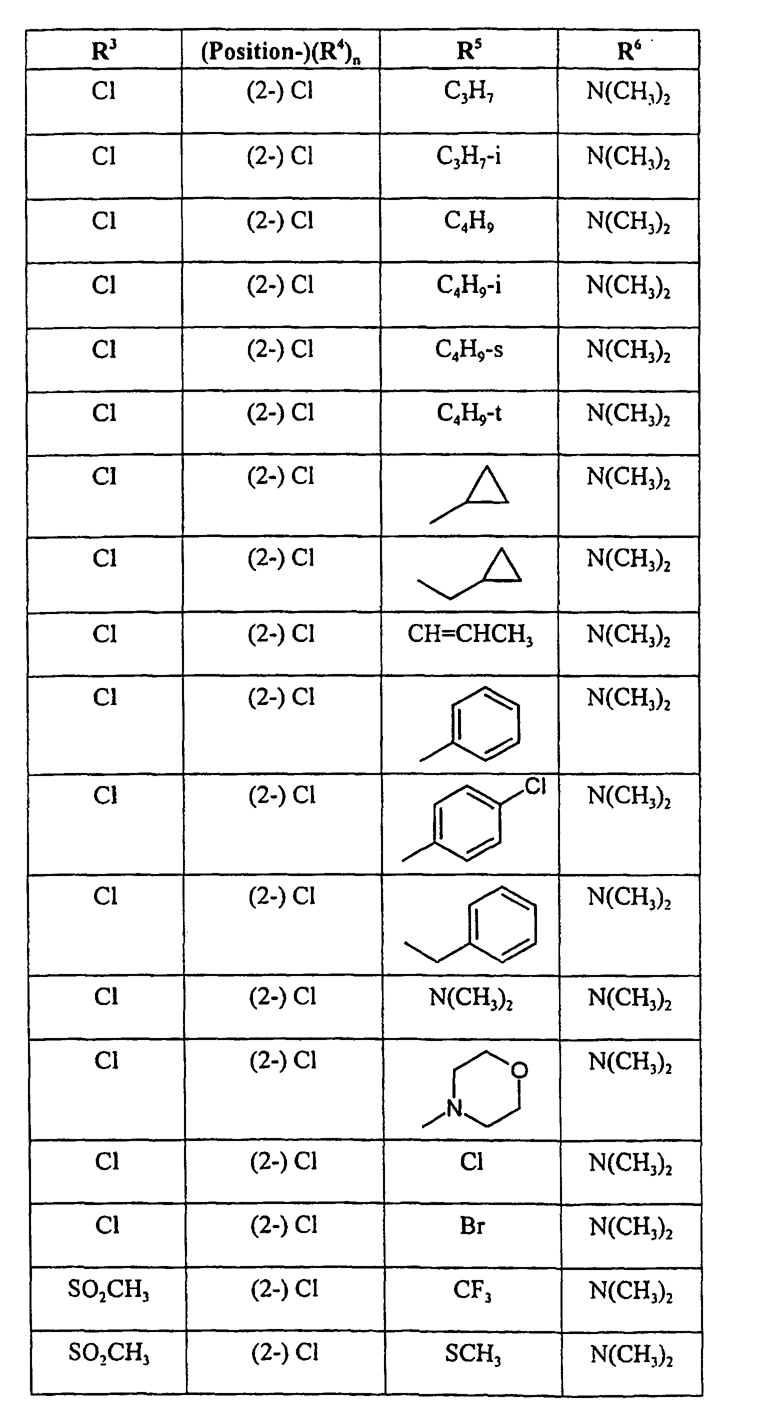

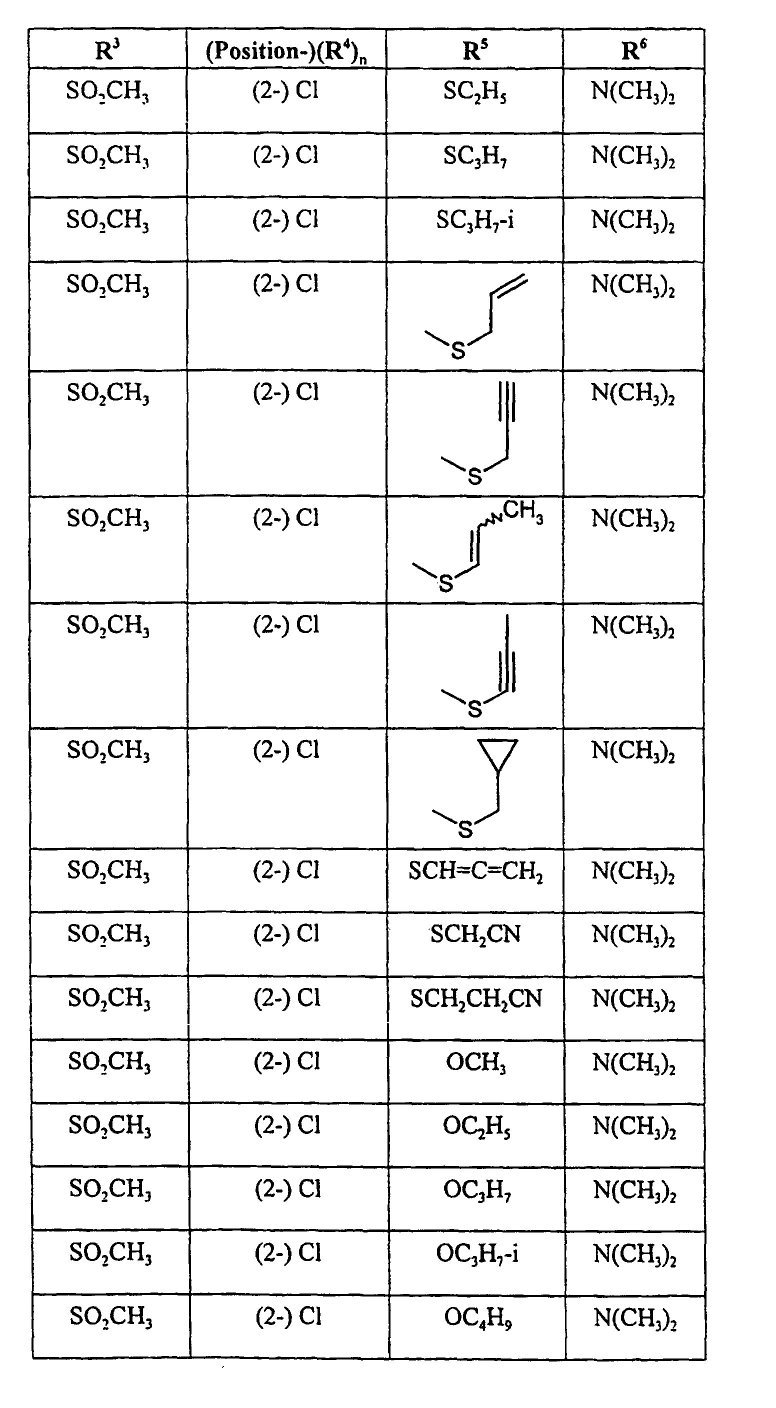

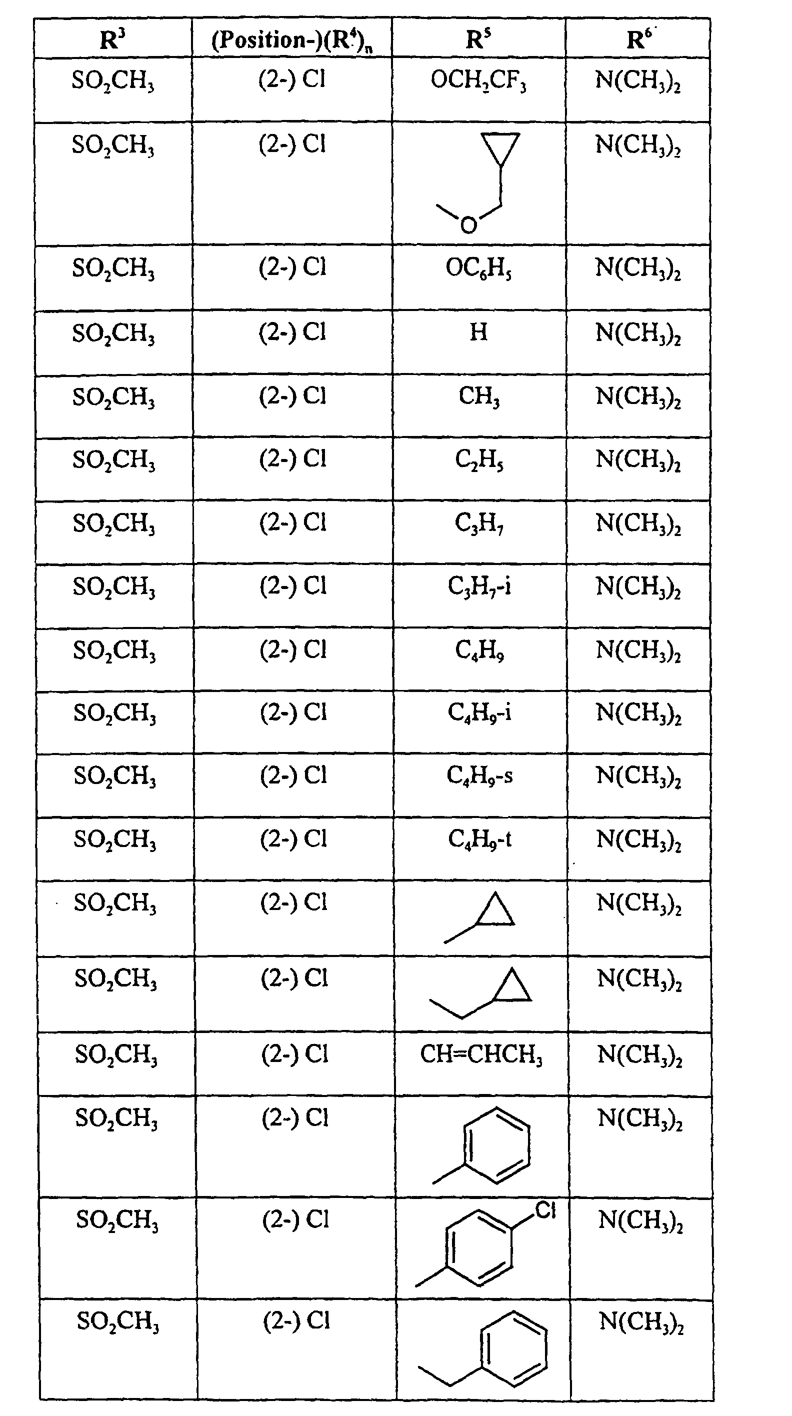

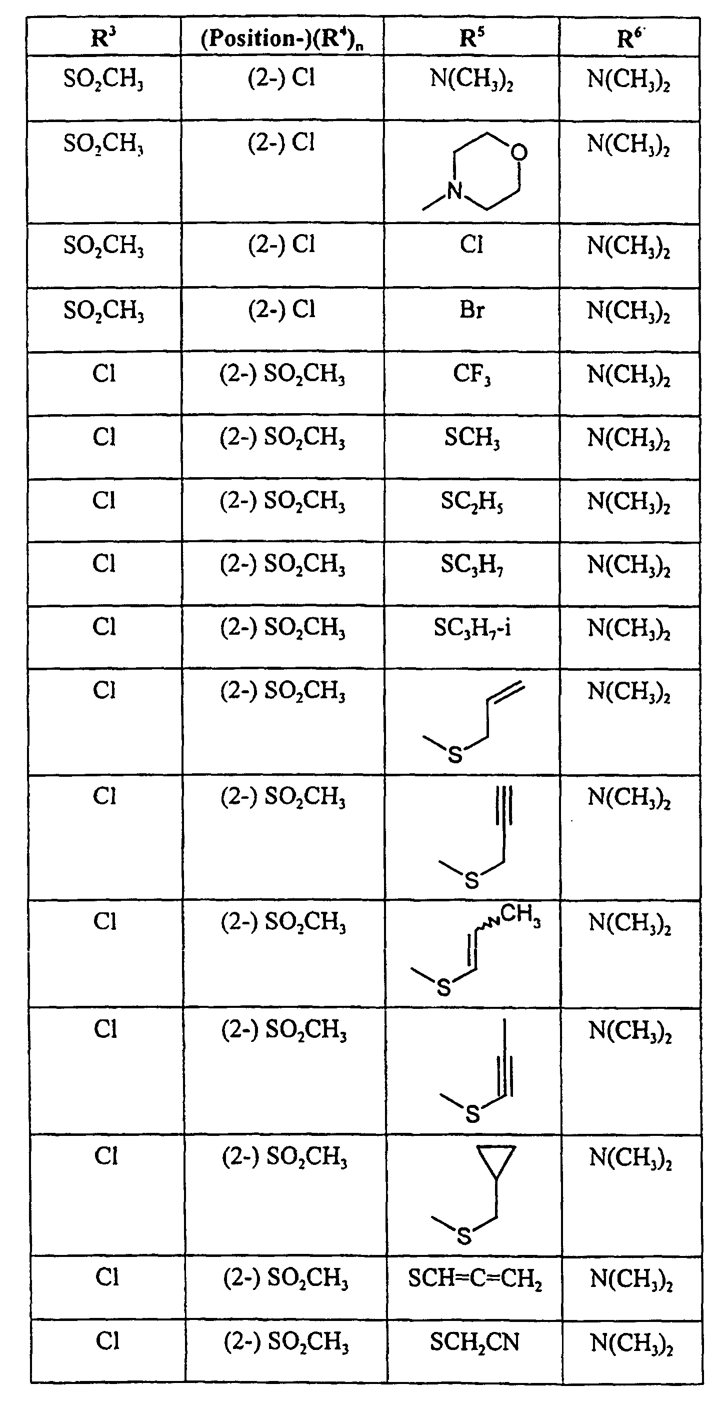

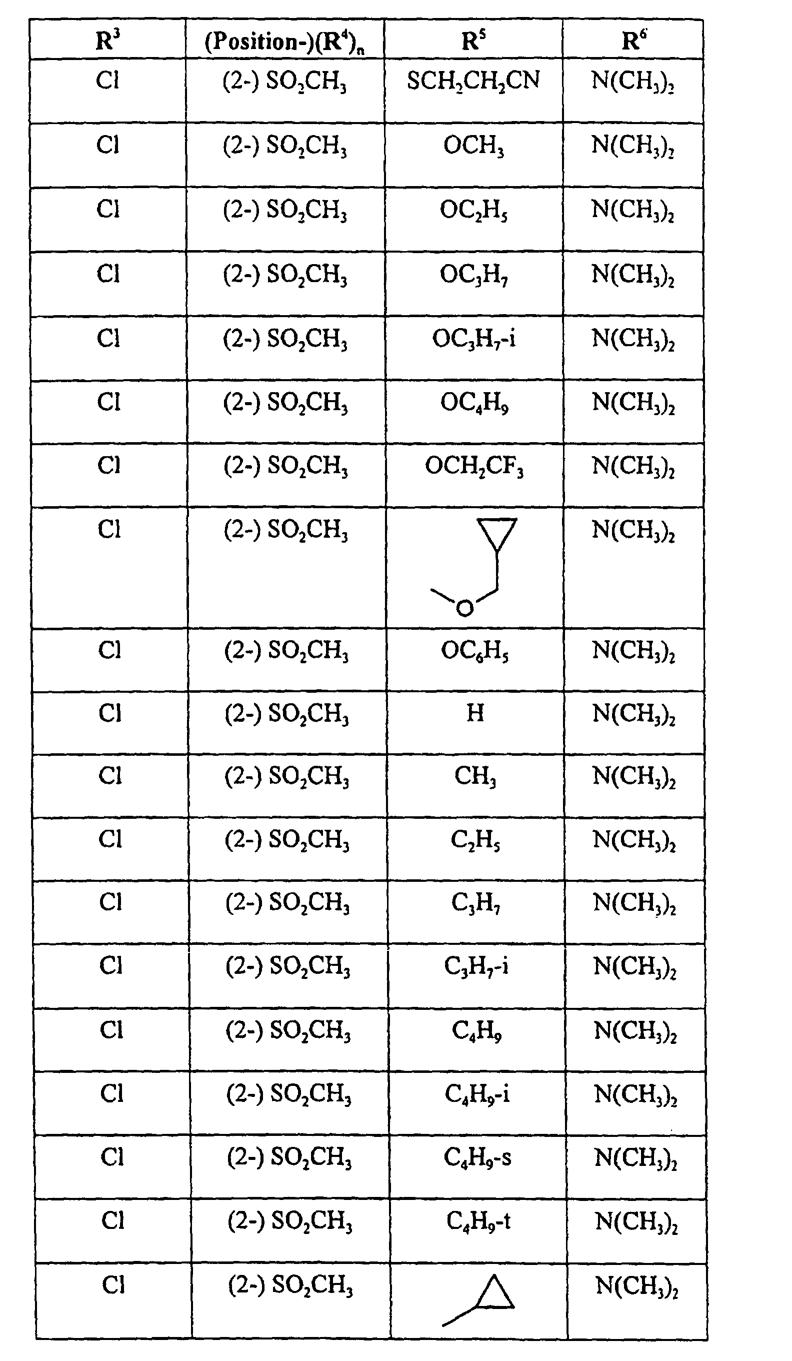

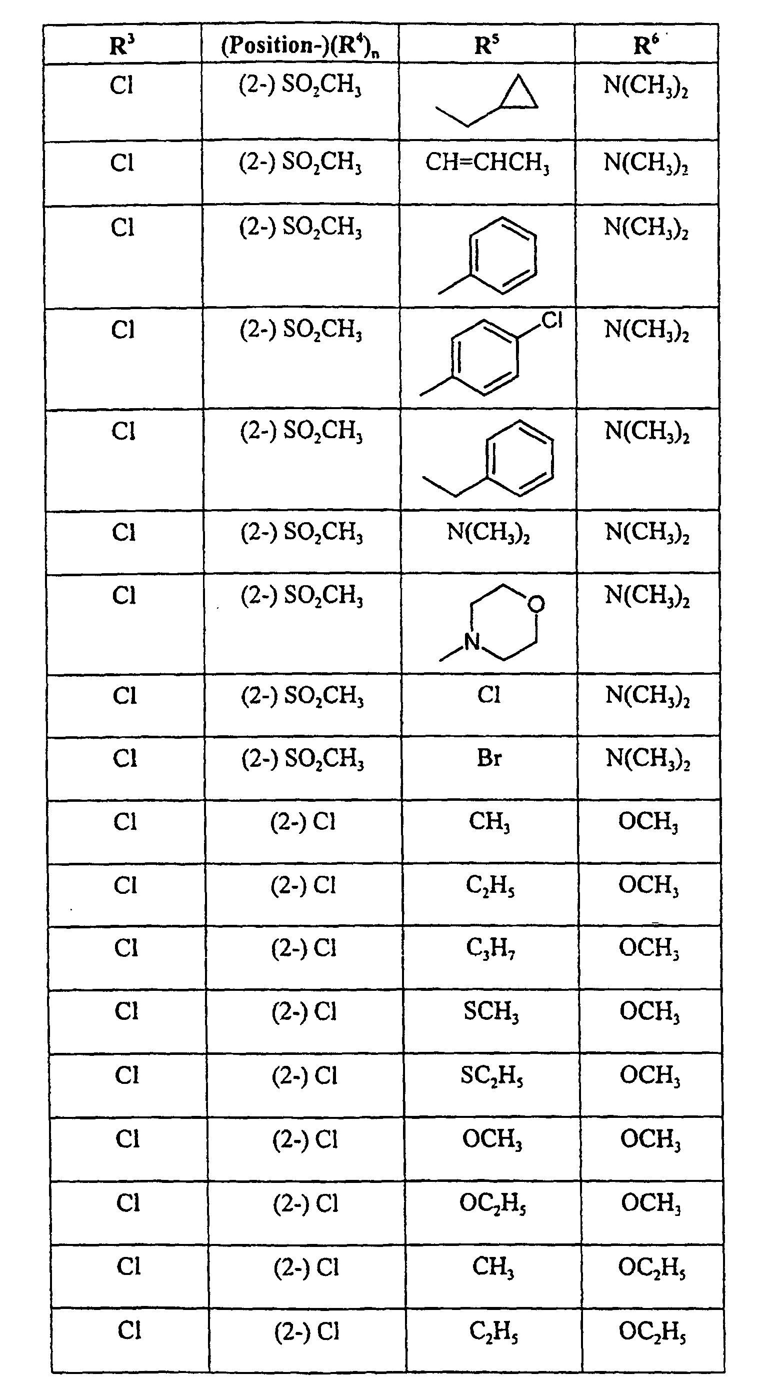

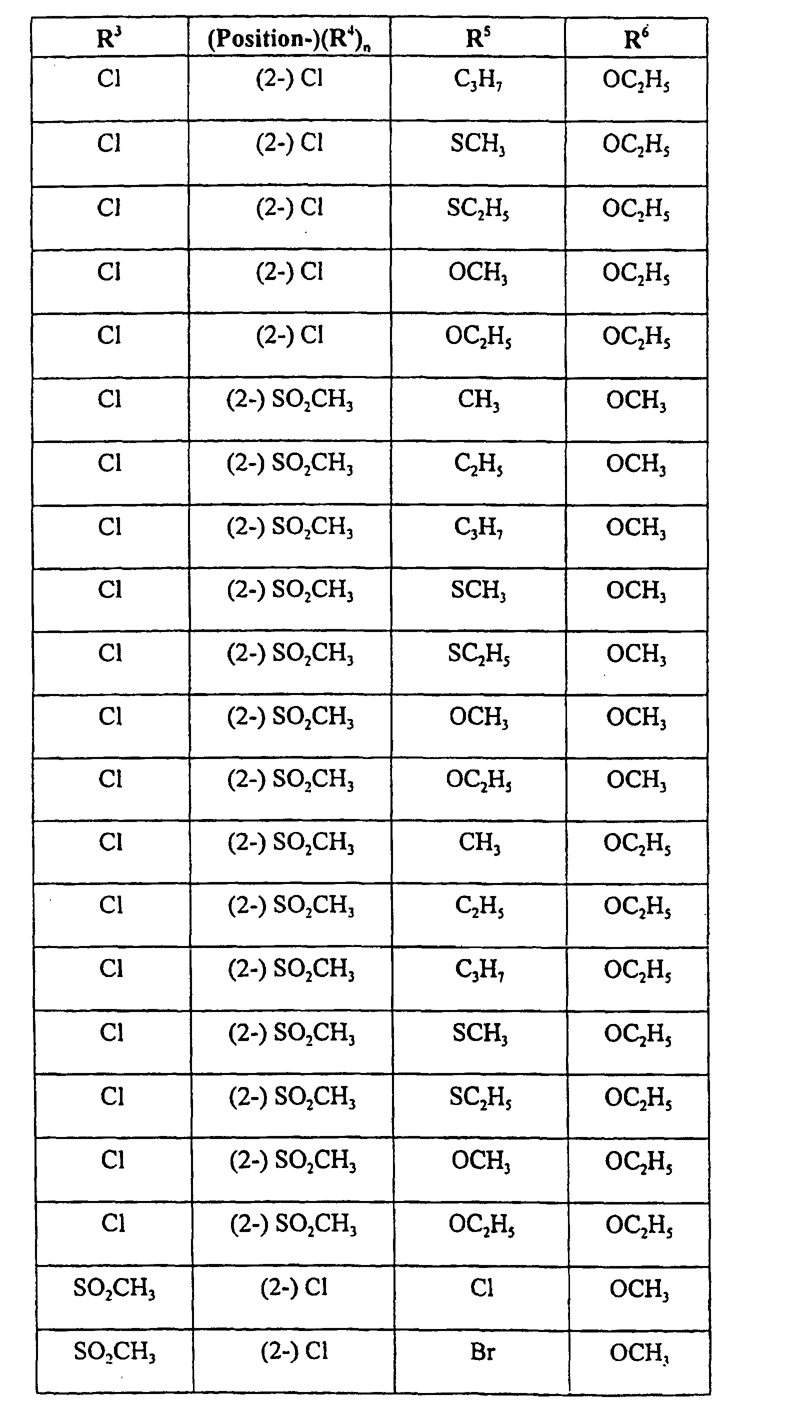

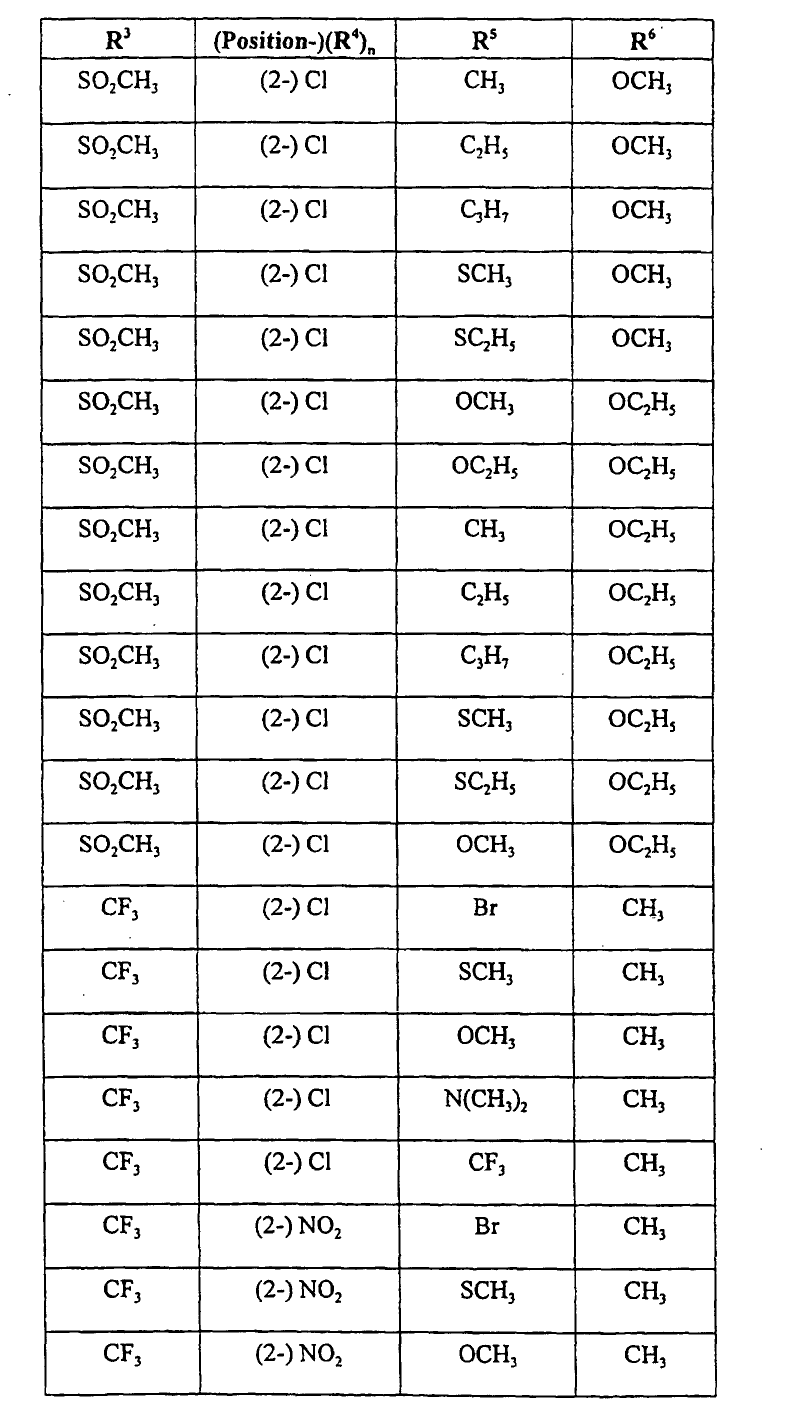

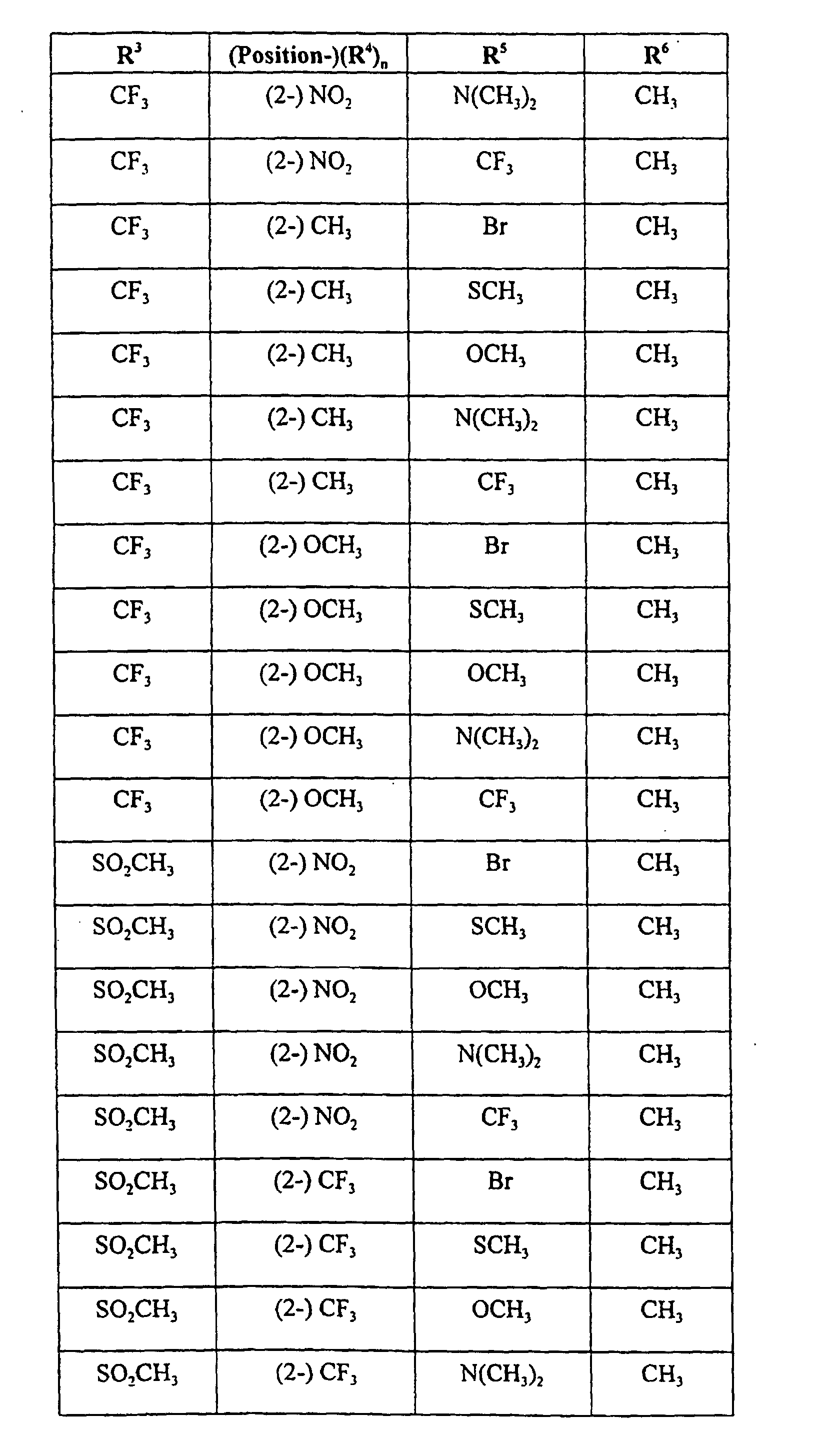

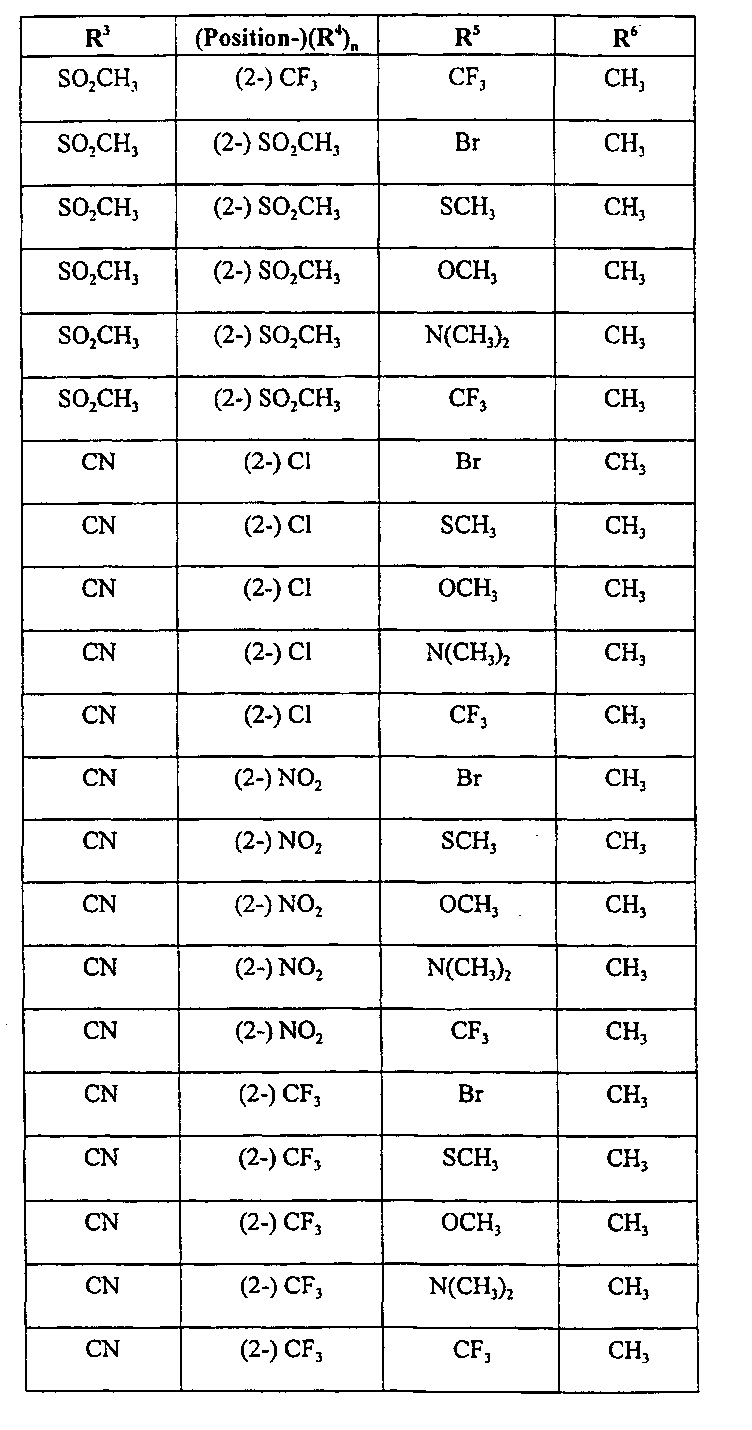

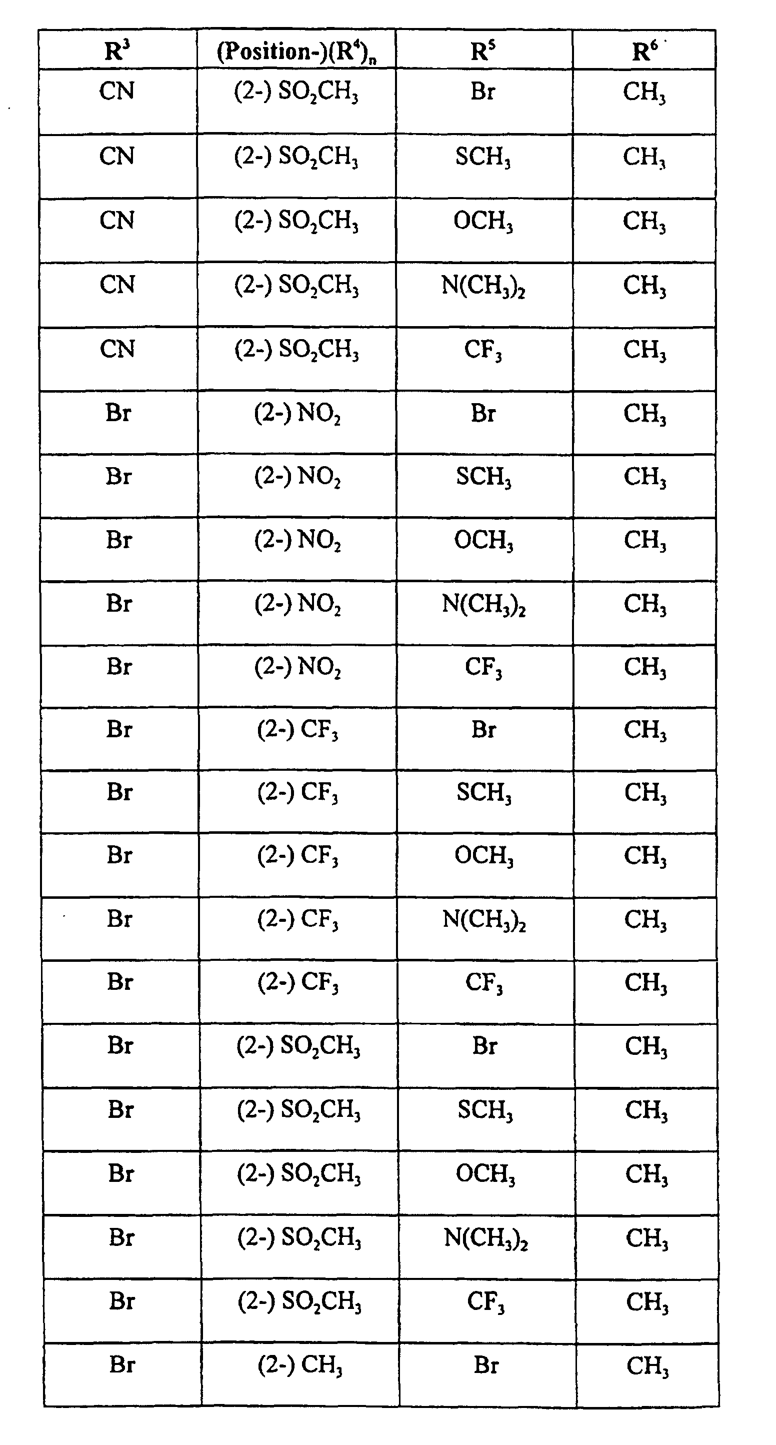

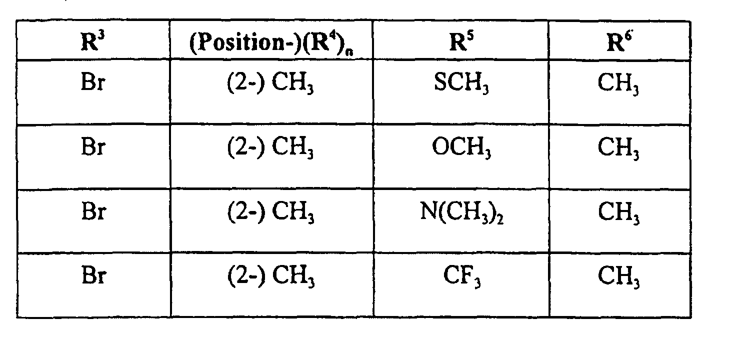

### Gruppe 3

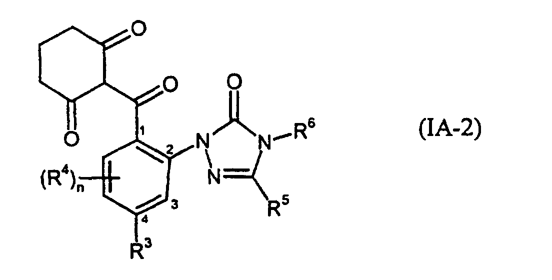

R³, (R⁴)ₙ, R⁵ und R⁶ haben hierbei beispielhaft die oben in Gruppe 1 angegebenen Bedeutungen.

### Gruppe 4

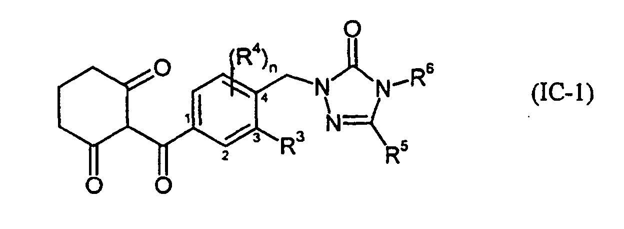

R³, (R⁴)ₙ, R⁵ und R⁶ haben hierbei beispielhaft die oben in Gruppe 2 angegebenen Bedeutungen.

Die neuen substituierten Benzoylcyclohexandione der allgemeinen Formel (I) zeichnen sich durch starke und selektive herbizide Wirksamkeit aus.

Man erhält die neuen substituierten Benzoylcyclohexandione der allgemeinen Formel (I), wenn man 1,3-Cyclohexandion oder dessen Derivate der allgemeinen Formel (II), 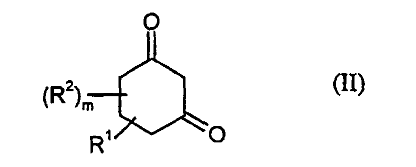 in welcher
m, R¹ und R² die oben angegebene Bedeutung haben,
mit substituierten Benzoesäuren der allgemeinen Formel (III), 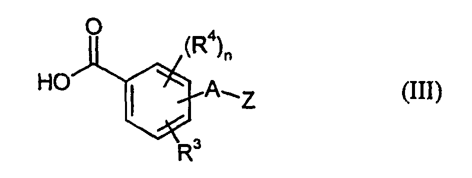 in welcher
n, A, R³, R⁴ und Z die oben angegebene Bedeutung haben,
in Gegenwart eines Dehydratisierungsmittels, gegebenenfalls in Gegenwart eines oder mehrerer Reaktionshilfsmittel und gegebenenfalls in Gegenwart eines Verdünnungsmittels, umsetzt,
und gegebenenfalls im Anschluß daran an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

Die Verbindungen der Formel (I) können nach üblichen Methoden in andere Verbindungen der Formel (I) gemäß obiger Definition umgewandelt werden, beispielsweise durch nucleophile Substitution (z.B. R⁵: Cl → OC₂H₅, SCH₃) oder durch Oxidation (z.B. R⁵: CH₂SCH₃ → CH₂S(O)CH₃).

Die Verbindungen der allgemeinen Formel (I) können prinzipiell auch wie im Folgenden schematisch dargestellt synthetisiert werden:

Umsetzung von 1,3-Cyclohexandion oder dessen Derivaten der allgemeinen Formel (II) - oben - mit reaktiven Derivaten der substituierten Benzoesäuren der allgemeinen Formel (III) - oben - insbesondere mit entsprechenden Carbonsäurechloriden, Carbonsäureanhydriden, Carbonsäure-cyaniden, Carbonsäure-methylestern oder -ethylestern - gegebenenfalls in Gegenwart von Reaktionshilfsmitteln, wie z.B. Triethylamin (und gegebenenfalls zusätzlich Zinkchlorid), und gegebenenfalls in Gegenwart eines Verdünnungsmittels, wie z.B. Methylenchlorid: 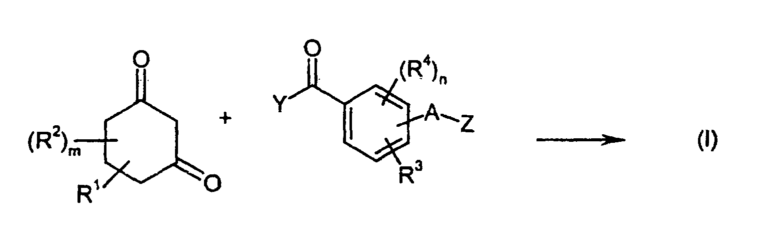 (Y z.B. CN, Cl)

Bei den oben skizzierten Umsetzungen zur Herstellung der Verbindungen der allgemeinen Formel (I) kommt es im allgemeinen neben der erwünschten C-Benzoylierung am Cyclohexandion auch zu einer O-Benzoylierung - vgl. nachstehendes Formelschema (vgl. Synthesis 1978, 925-927; Tetrahedron Lett. 37 (1996), 1007-1009, WO-A-91/05469). Die hierbei gebildeten O-Benzoyl-Verbindungen werden jedoch unter den Reaktionsbedingungen des erfindungsgemäßen Verfahrens zu den entsprechenden C-Benzoyl-Verbindungen der Formel (I) isomerisiert.

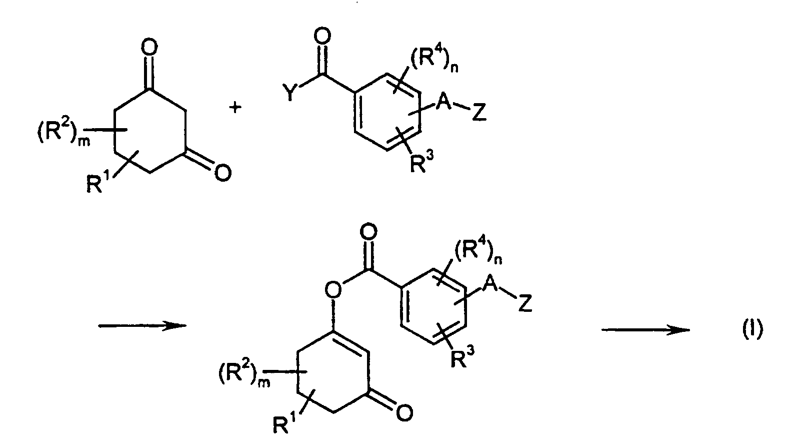

Verwendet man beispielsweise 1,3-Cyclohexandion und 2-(3-Carboxy-5-fluor-benzyl)-5-ethyl-4-methoxy-2,4-dihydro-3H-1,2,4-triazol-3-on als Ausgangsstoffe, so kann der Reaktionsablauf beim erfindungsgemäßen Verfahren durch das folgende Formelschema skizziert werden:

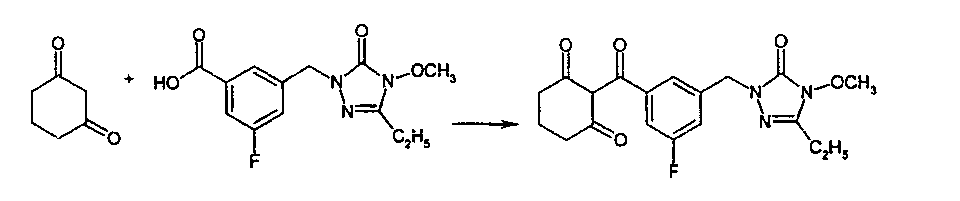

Die beim erfindungsgemäßen Verfahren zur Herstellung von Verbindungen der Formel (I) als Ausgangsstoffe zu verwendenden Cyclohexandione sind durch die Formel (II) allgemein definiert. In der Formel (II) haben m, R¹ und R² vorzugsweise diejenigen Bedeutungen, die bereits oben im Zusammenhang mit der Beschreibung der erfindungsgemäßen Verbindungen der Formel (I) als bevorzugt, besonders bevorzugt oder ganz besonders bevorzugt für m, R¹ und R² angegeben wurden.

Die Ausgangsstoffe der allgemeinen Formel (II) sind bekannt und/oder können nach an sich bekannten Verfahren hergestellt werden.

Die beim erfindungsgemäßen Verfahren zur Herstellung von Verbindungen der Formel (I) weiter als Ausgangsstoffe zu verwendenden substituierten Benzoesäuren sind durch die Formel (III) allgemein defmiert. In der Formel (III) haben n, A, R³, R⁴ und Z vorzugsweise diejenigen Bedeutungen, die bereits oben im Zusammenhang mit der Beschreibung der erfindungsgemäßen Verbindungen der Formel (I) als bevorzugt, besonders bevorzugt, ganz besonders bevorzugt oder am meisten bevorzugt für n, A, R³, R⁴ und Z angegeben wurden.

Die Ausgangsstoffe der allgemeinen Formel (III) sind mit Ausnahme von 2-(5-Carboxy-2,4-dichlor-phenyl)-4-difluormethyl-5-methyl-2,4-dihydro-3H-1,2,4-triazol-3-on - alias 2,4-Dichlor-5-(4-difluormethyl-4,5-dihydro-3-methyl-5-oxo-1H-1,2,4-triazol-1-yl)-benzoesäure (CAS-Reg.-Nr. 90208-77-8) und 2-(5-Carboxy-2,4-dichlor-phenyl)-4,5-dimethyl-2,4-dihydro-3H-1,2,4-triazol-3-on - alias 2,4-Dichlor-5-(4,5-dihydro-3,4-dimethyl-5-oxo-1H-1,2,4-triazol-1-yl)-benzoesäure (CAS-Reg.-Nr. 90208-76-7) - noch nicht aus der Literatur bekannt. Sie sind unter Ausnahme von 2-(5-Carboxy-2,4-dichlor-phenyl)-4-difluormethyl-5-methyl-2,4-dihydro-3H-1,2,4-triazol-3-on und 2-(5-Carboxy-2,4-dichlor-phenyl)-4,5-dimethyl-2,4-dihydro-3H-1,2,4-triazol-3-on (vgl. JP-A-58225070 - zitiert in Chem. Abstracts 100:209881, JP-A-02015069 - zitiert in Chem. Abstracts 113:23929) als neue Stoffe auch Gegenstand der vorliegenden Anmeldung.

Man erhält die neuen substituierten Benzoesäuren der allgemeinen Formel (III), wenn man Benzoesäurederivate der allgemeinen Formel (IV), 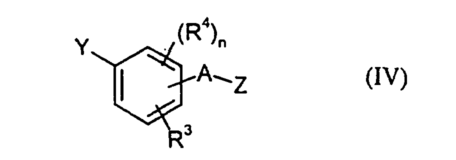 in welcher
n, A, R³ und R⁴ und Z die oben angegebene Bedeutung haben, und
- Y: für Cyano, Carbamoyl, Halogencarbonyl oder Alkoxycarbonyl steht,
mit Wasser, gegebenenfalls in Gegenwart eines Hydrolysehilfsmittels, wie z.B. Schwefelsäure, bei Temperaturen zwischen 50°C und 120°C umsetzt (vgl. die Herstellungsbeispiele).

Die als Vorprodukte benötigten Benzoesäurederivate der allgemeinen Formel (IV) sind bekannt und/oder können nach an sich bekannten Verfahren hergestellt werden (vgl. DE-A-3839480, DE-A-4239296, EP-A-597360, EP-A-609734, DE-A-4303676, EP-A-617026, DE-A-4405614, US-A-5378681).

Man erhält die neuen substituierten Benzoesäuren der allgemeinen Formel (III) auch, wenn man Halogen(alkyl)benzoesäuren der allgemeinen Formel (V), 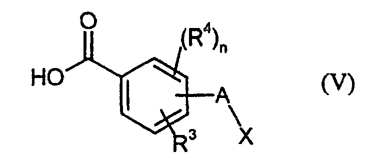 in welcher
n, A, R³ und R⁴ die oben angegebene Bedeutung haben und
- X: für Halogen (insbesondere Fluor, Chlor oder Brom) steht,
mit Verbindungen der allgemeinen Formel (VI), 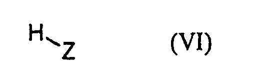 in welcher
- Z: die oben angegebene Bedeutung hat,
gegebenenfalls in Gegenwart eines Reaktionshilfsmittel, wie z.B. Triethylamin oder Kaliumcarbonat, und gegebenenfalls in Gegenwart eines Verdünnungsmittels, wie z.B. Aceton, Acetonitril, N,N-Dimethyl-formamid oder N,N-Dimethyl-acetamid, bei Temperaturen zwischen 50°C und 200°C umsetzt (vgl. die Herstellungsbeispiele).

An Stelle der Halogen(alkyl)benzoesäuren der allgemeinen Formel (V) können analog zur oben beschriebenen Methodik auch entsprechende Nitrile, Amide und Ester - insbesondere die Methylester oder die Ethylester - mit Verbindungen der allgemeinen Formel (VI) umgesetzt werden. Durch anschließende Hydrolyse nach üblichen Methoden, beispielsweise durch Umsetzung mit wässrig-ethanolischer Kalilauge, können dann die entsprechenden substituierten Benzoesäuren erhalten werden.

Die als Vorprodukte benötigten Halogen(alkyl)benzoesäuren der Formel (V) - bzw. entsprechende Nitrile oder Ester - sind bekannt und/oder können nach an sich bekannten Verfahren hergestellt werden (vgl. EP-A-90369, EP-A-93488, EP-A-399732, EP-A-480641, EP-A-609798, EP-A-763524, DE-A-2126720, WO-A-93/03722, WO-A-97/38977, US-A-3978127, US-A-4837333).

Die weiter als Vorprodukte benötigten Verbindungen der allgemeinen Formel (VI) sind bekannt und/oder können nach an sich bekannten Verfahren hergestellt werden.

Das erfindungsgemäße Verfahren zur Herstellung der neuen substituierten Benzoylcyclohexandione der allgemeinen Formel (I) wird unter Verwendung eines Dehydratisierungsmittels durchgeführt. Es kommen hierbei die üblichen zur Bindung von Wasser geeigneten Chemikalien in Betracht.

Als Beispiele hierfür seien Dicyclohexylcarbodiimid und Carbonyl-bis-imidazol genannt.

Als besonders gut geeignetes Dehydratisierungsmittel sei Dicyclohexylcarbodiimid genannt.

Das erfindungsgemäße Verfahren zur Herstellung der neuen substituierten Benzoylcyclohexandione der allgemeinen Formel (I) wird gegebenenfalls unter Verwendung eines Reaktionshilfsmittels durchgeführt.

Als Beispiele hierfür seien Natriumcyanid, Kaliumcyanid, Acetoncyanhydrin, 2-Cyano-2-(trimethylsilyloxy)-propan und Trimethylsilylcyanid genannt.

Als besonders gut geeignetes weiteres Reaktionshilfsmittel sei Trimethylsilylcyanid genannt.

Das erfindungsgemäße Verfahren zur Herstellung der neuen substituierten Benzoylcyclohexandione der allgemeinen Formel (I) wird gegebenenfalls unter Verwendung eines weiteren Reaktionshilfsmittels durchgeführt. Als weitere Reaktionshilfsmittel für das erfindungsgemäße Verfahren kommen im allgemeinen basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethylbenzylamin, Pyridin, 2-Methyl-, 3-Methyl-, 4-Methyl-, 2,4-Dimethyl-, 2,6-Dimethyl, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en . (DBN), oder 1,8-Diazabicyclo[5,4,0]-undec-7-en (DBU) in Betracht.

Als Verdünnungsmittel zur Durchführung des erfindungsgemäßen Verfahrens kommen vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan oder 1,2-Dichlor-ethan; Ether, wie Diethylether, Diisopropylether, Dioxan, Tetrahydrofuran, Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Dehydratisierungsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum. Hordeum, Avena. Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe fiir Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid. Ferrocyanblau und organische Farbstoffe, wie Alizarin-, Azo- und Metallphthalocyaninfarbstoffe und Spurennährstoffe wie Salze von Eisen, Mangan, Bor, Kupfer. Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise

Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzofenap, Benzoylprop(-ethyl), Bialaphos, Bifenox, Bispyribac(-sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlomitrofen, Chlorsulfuron, Chlortoluron, Cinidon(-ethyl), Cinmethylin, Cinosulfuron, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epoprodan, EPTC, Esprocarb, Ethalfluralin, Ethametsulfuron(-methyl), Ethofumesate, Ethoxyfen, Ethoxysulfuron, Etobenzanid, Fenoxaprop(-P-ethyl), Flamprop(-isopropyl), Flamprop(-isopropyl-L), Flamprop(-methyl), Flazasulfuron, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(-meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron, Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pentoxazone, Phenmedipham, Piperophos, Pretilachlor, Primisulfuron(-methyl), Procarbazone, Prometryn, Propachlor, Propanil, Propaquizafop, Propisochlor, Propyzamide, Prosulfocarb, Prosulfuron, Pyraflufen(ethyl), Pyrazolate, Pyrazosulfuron(-ethyl), Pyrazoxyfen, Pyribenzoxim, Pyributicarb, Pyridate, Pyriminobac(-methyl), Pyrithiobac(-sodium), Quinchlorac, Quinmerac, Quinoclamine, Quizalofop(-P-ethyl), Quizalofop(-P-tefuryl), Rimsulfuron, Sethoxydim, Simazine, Simetryn, Sulcotrione, Sulfentrazone, Sulfometuron-(methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

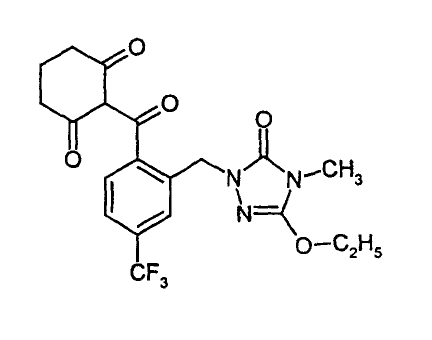

1,2 g (3,48 mMol) 5-Ethoxy-4-methyl-2-(2-carboxy-5-trifluormethyl-benzyl)-2,4-dihydro-3H-1,2,4-triazol-3-on werden in 30 ml Acetonitril suspendiert und mit 0,39 g (3,48 mMol) 1,3-Cyclohexandion und 0,76 g (3,7 mMol) Dicyclohexylcarbodiimid (DCC) bei Raumtemperatur (ca. 20°C) versetzt. Das Reaktionsgemisch wird über Nacht (ca. 15 Stunden) bei Raumtemperatur gerührt und dann mit 1,0 ml (7,0 mMol) Triethylamin und 0,10 ml (1,39 mmol) Trimethylsilylcyanid versetzt. Nach 3 Stunden bei Raumtemperatur wird mit 100 ml 5%iger wäßriger Natriumcarbonatlösung verrührt, der sich abscheidende Dicyclohexylharnstoff abgesaugt und die alkalische wäßrige Phase mehrfach mit Ethylacetat extrahiert. Dann wird die wäßrige Phase mit 35%iger Salzsäure auf pH 2 eingestellt und mehrfach mit Methylenchlorid extrahiert. Die Methylenchloridphasen werden über Natriumsulfat getrocknet und eingeengt.

Man erhält 0.8 g (52 % der Theorie) 5-Ethoxy-4-methyl-2-[2-(2,6-dioxo-cyclohexylcarbonyl)-5-trifluormethyl-benzyl)-2,4-dihydro-3H-1,2,4-triazol-3-on als amorphen Rückstand.
logP (bei pH=2 bestimmt): 2,70.

### Beispiel 2

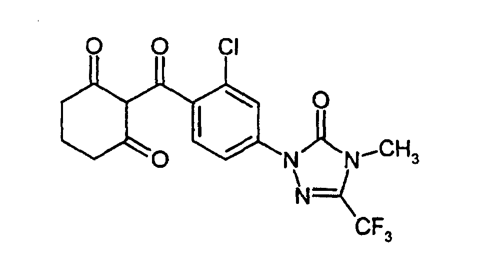

Zu einer Suspension aus 2,15 g (6,5 mMol) 2-(4-Carboxy-3-chlor-phenyl)-4-methyl-5-trifluormethyl-2,4-dihydro-3H-1,2,4-triazol-3-on, 0,83 g (7,2 mMol) 1,3-Cyclohexandion und 40 ml Acetonitril wird eine Lösung von 1,5 g (7,2 mMol) Dicyclohexylcarbodiimid in 40 ml Acetonitril gegeben und die Reaktionsmischung wird 16 Stunden bei 20°C gerührt. Dann werden 1,3 g (13 mMol) Triethylamin und 0,26 g (2,6 mMol) Trimethylsilylcyanid dazu gegeben und das Reaktionsgemisch wird weitere 4 Stunden bei 20°C gerührt. Dann wird mit 180 ml 2%iger wässriger Sodalösung verrührt und abgesaugt. Die Mutterlauge wird mit Essigsäureethylester extrahiert. Dann wird die wässrige Phase mit 2N-Salzsäure angesäuert und mit Methylenchlorid extrahiert. Die organische Phase wird getrocknet, im Wasserstrahlvakuum eingeengt und mit Diethylether/Petrolether digeriert. Das kristallin angefallene Produkt wird durch Absaugen isoliert.

Man erhält 1,6 g (59% der Theorie) 2-[4-(2,6-Dioxocyclohexylcarbonyl)-3-chlorphenyl]-4-methyl-5-trifluormethyl-2,4-dihydro-3H-1,2,4-triazol-3-on vom Schmelzpunkt 182°C.
logP (bei pH=2 bestimmt): 3,13.

Analog zu den Herstellungsbeispielen 1 und 2 sowie entsprechend der allgemeinen Beschreibung der erfindungsgemäßen Herstellungsverfahren können beispielsweise auch die in den nachstehenden Tabellen 1 und 2 aufgeführten Verbindungen der Formel (I) - bzw. der Formeln (IA-3), (IB-2), (IC-2) oder (ID) - hergestellt werden.

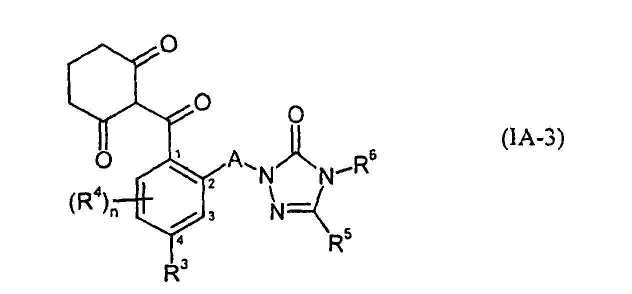

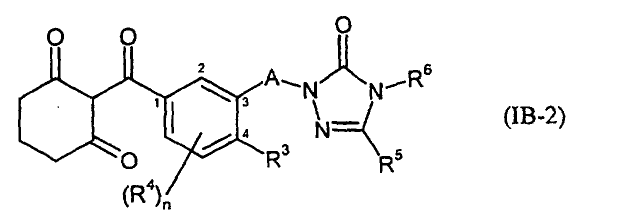

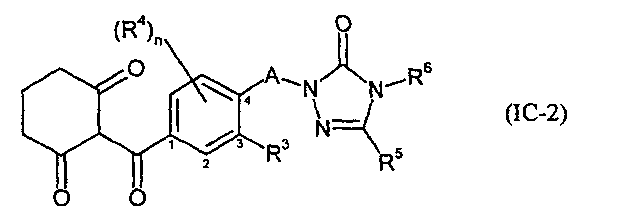

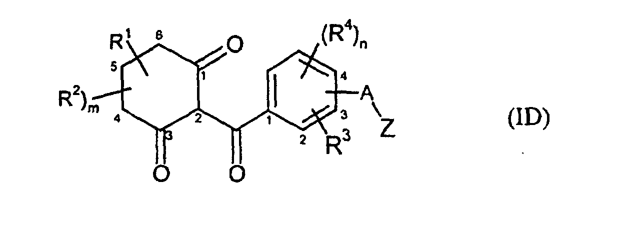

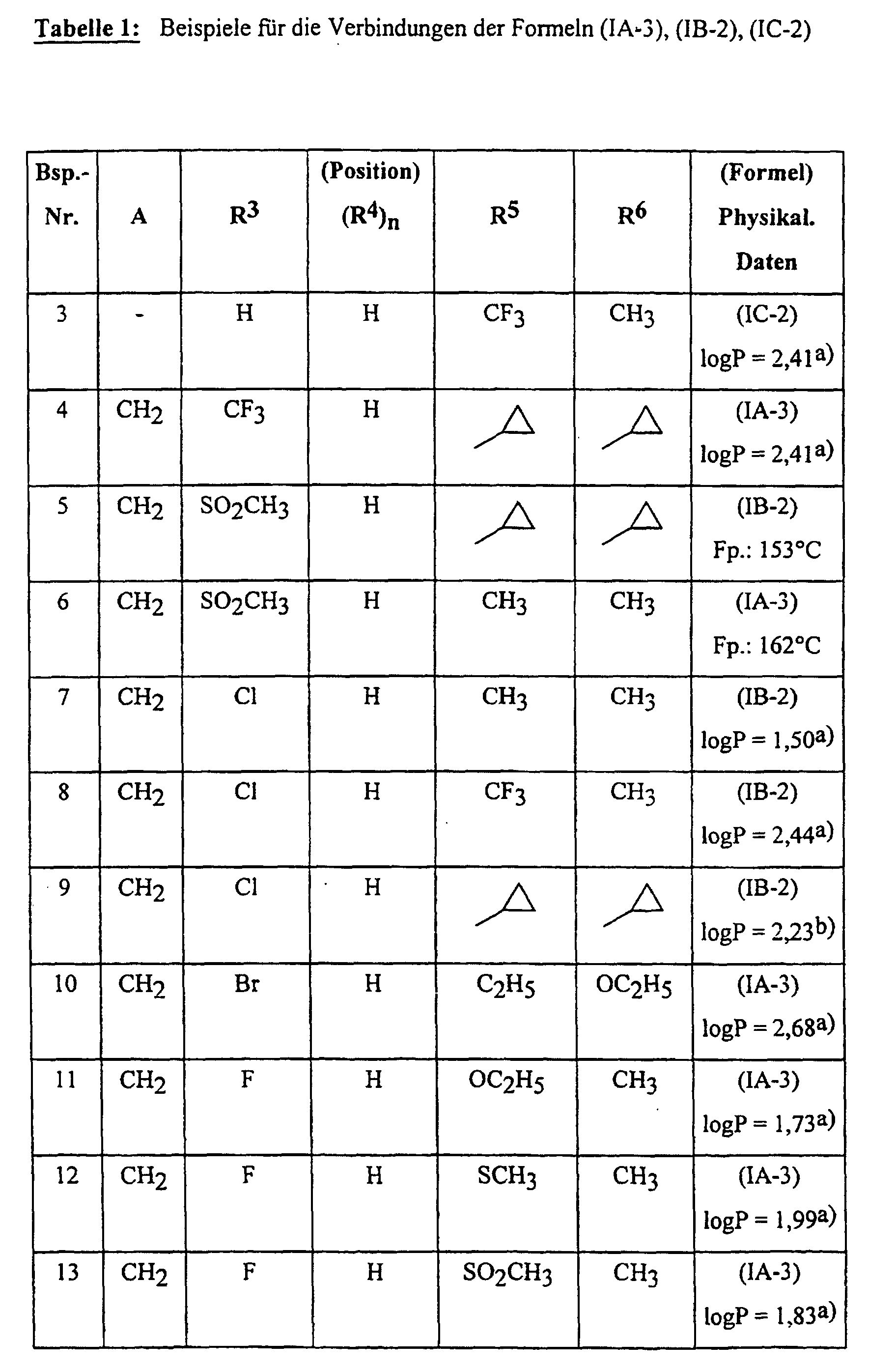

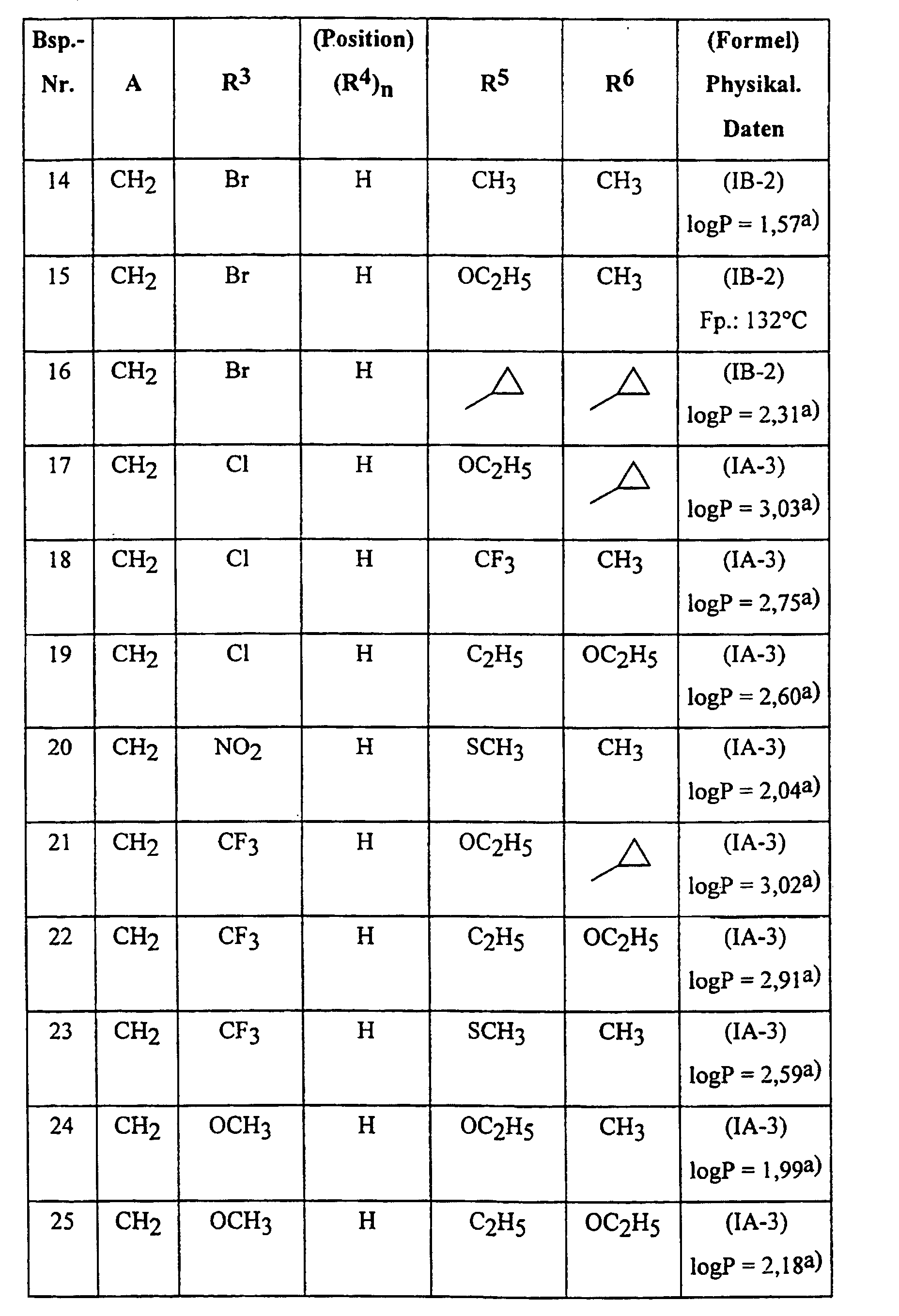

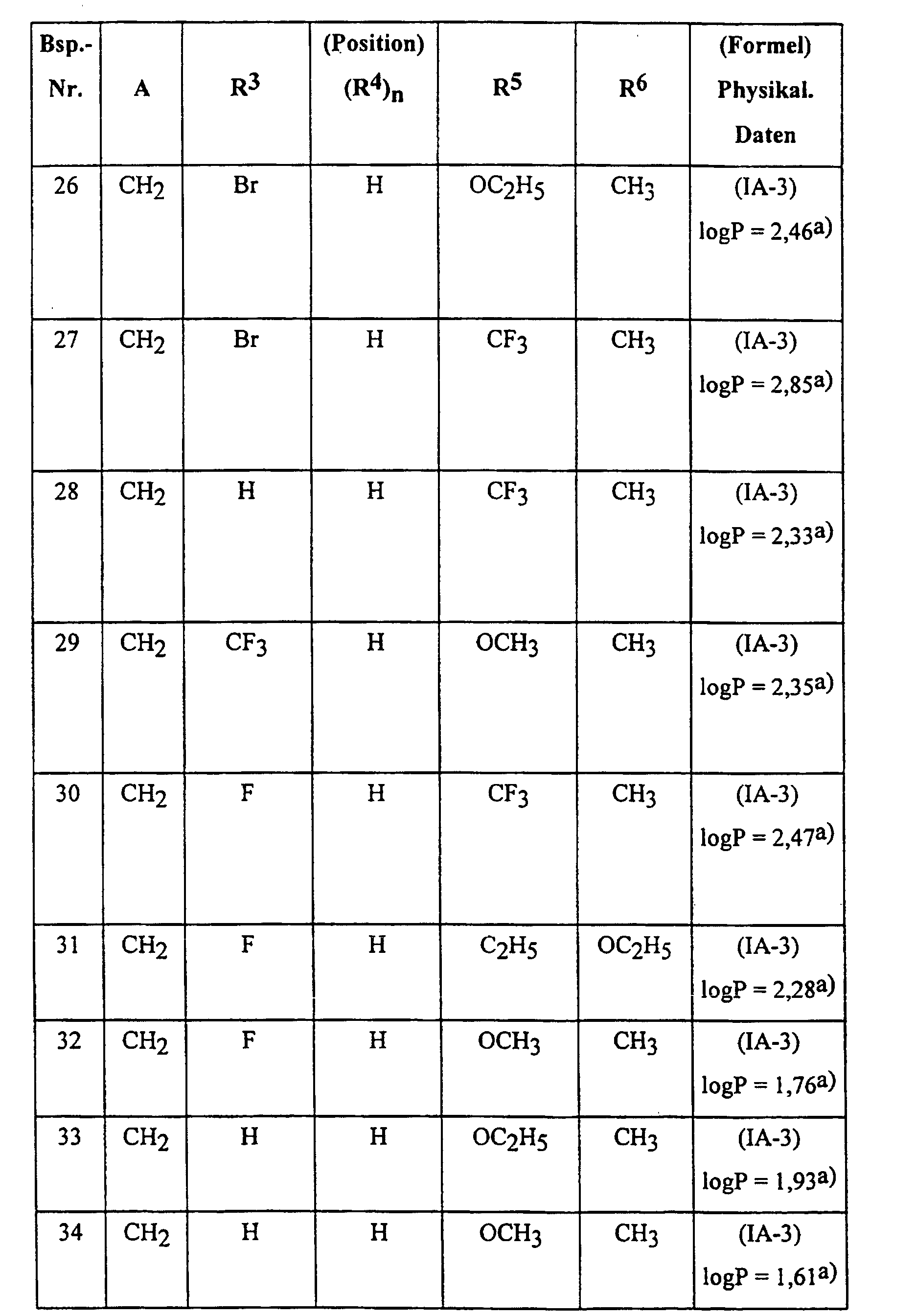

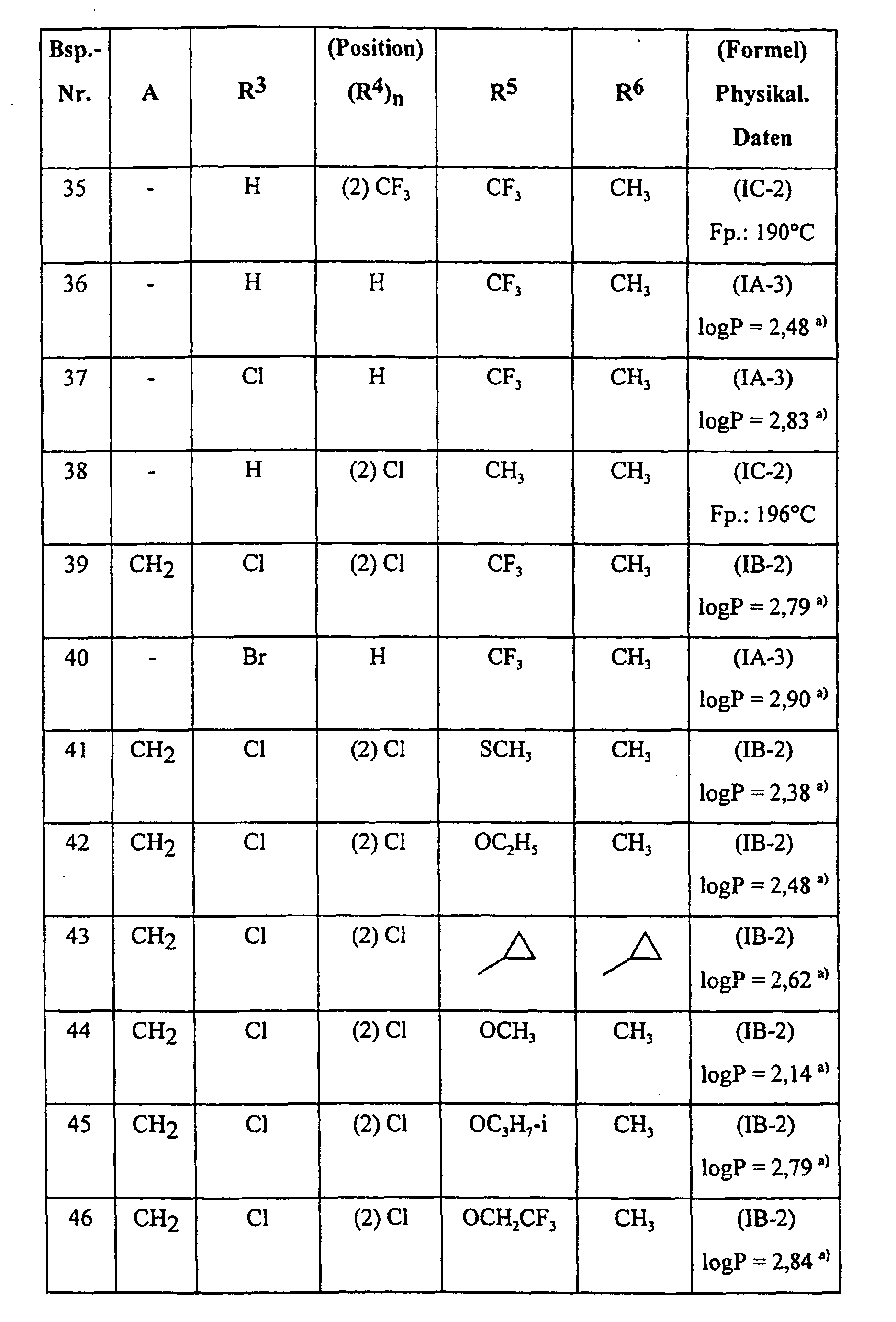

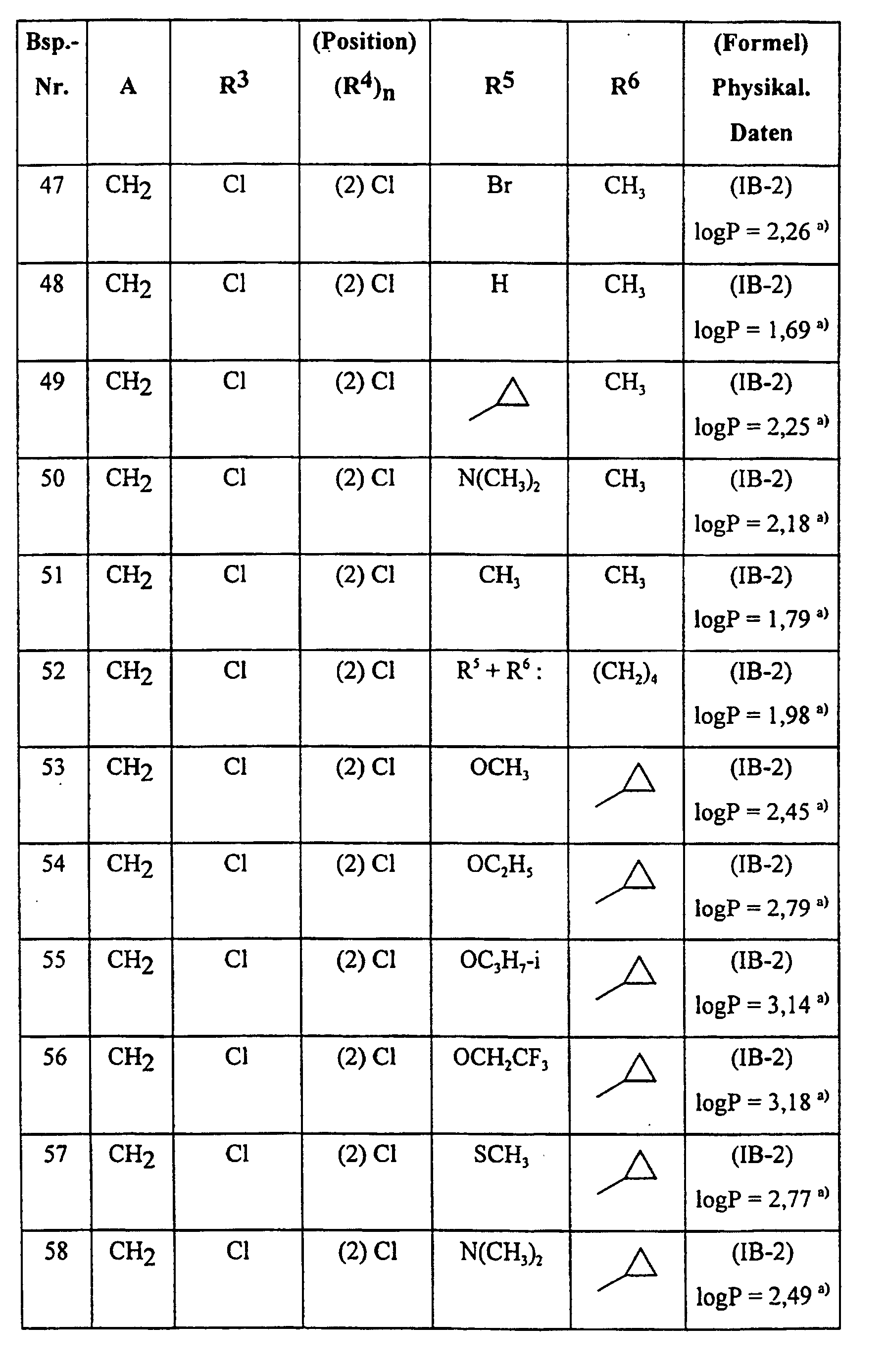

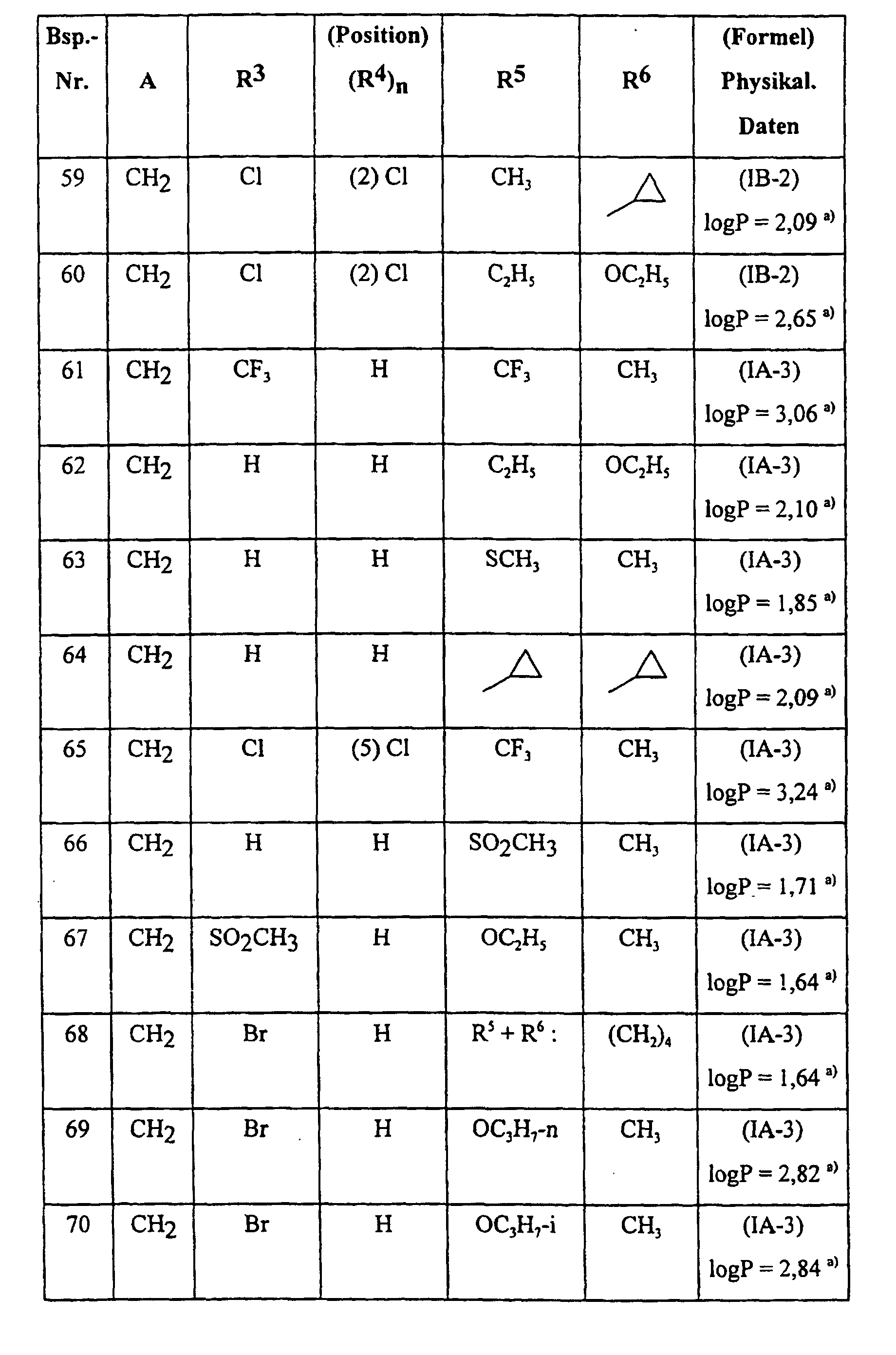

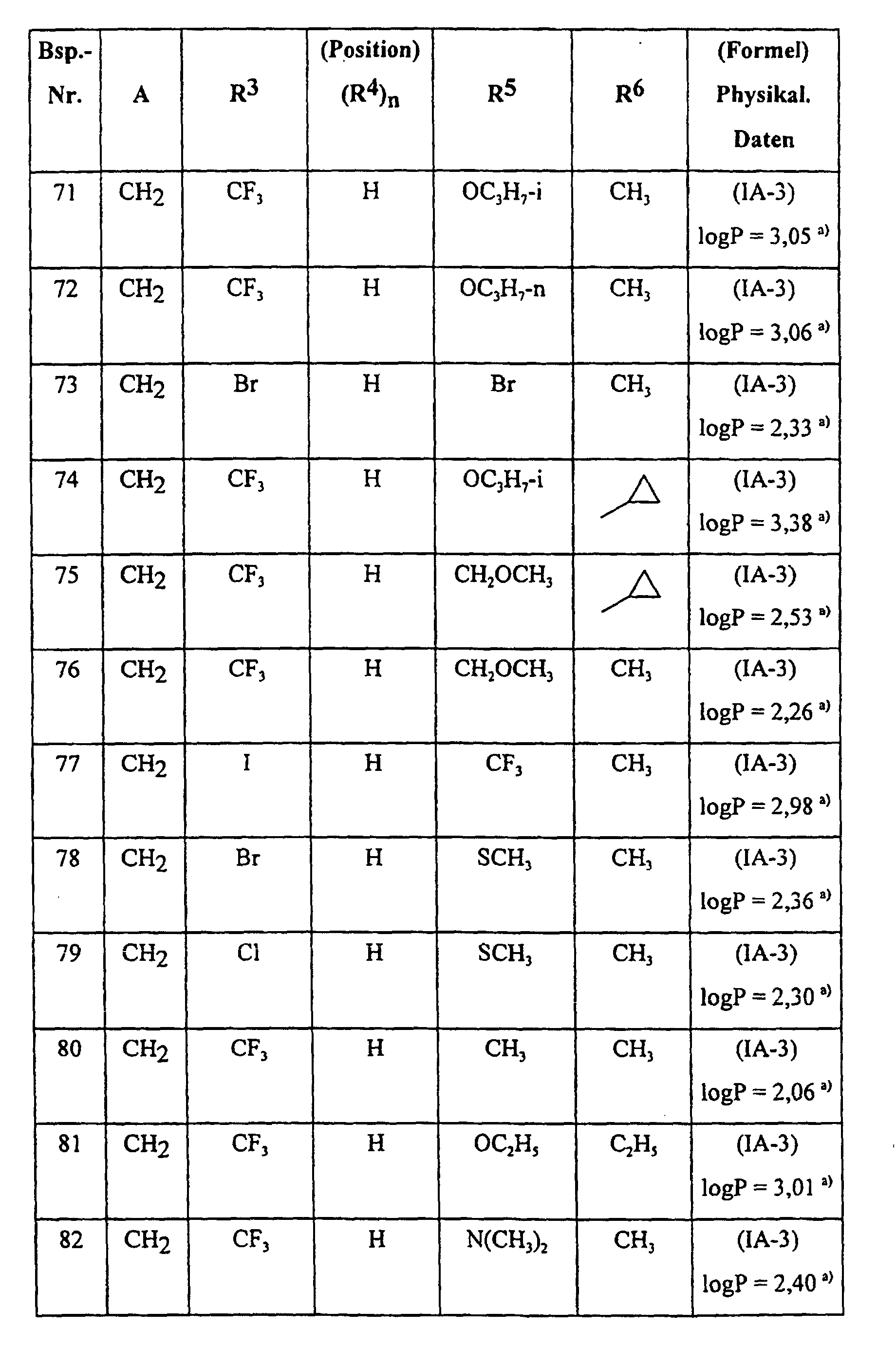

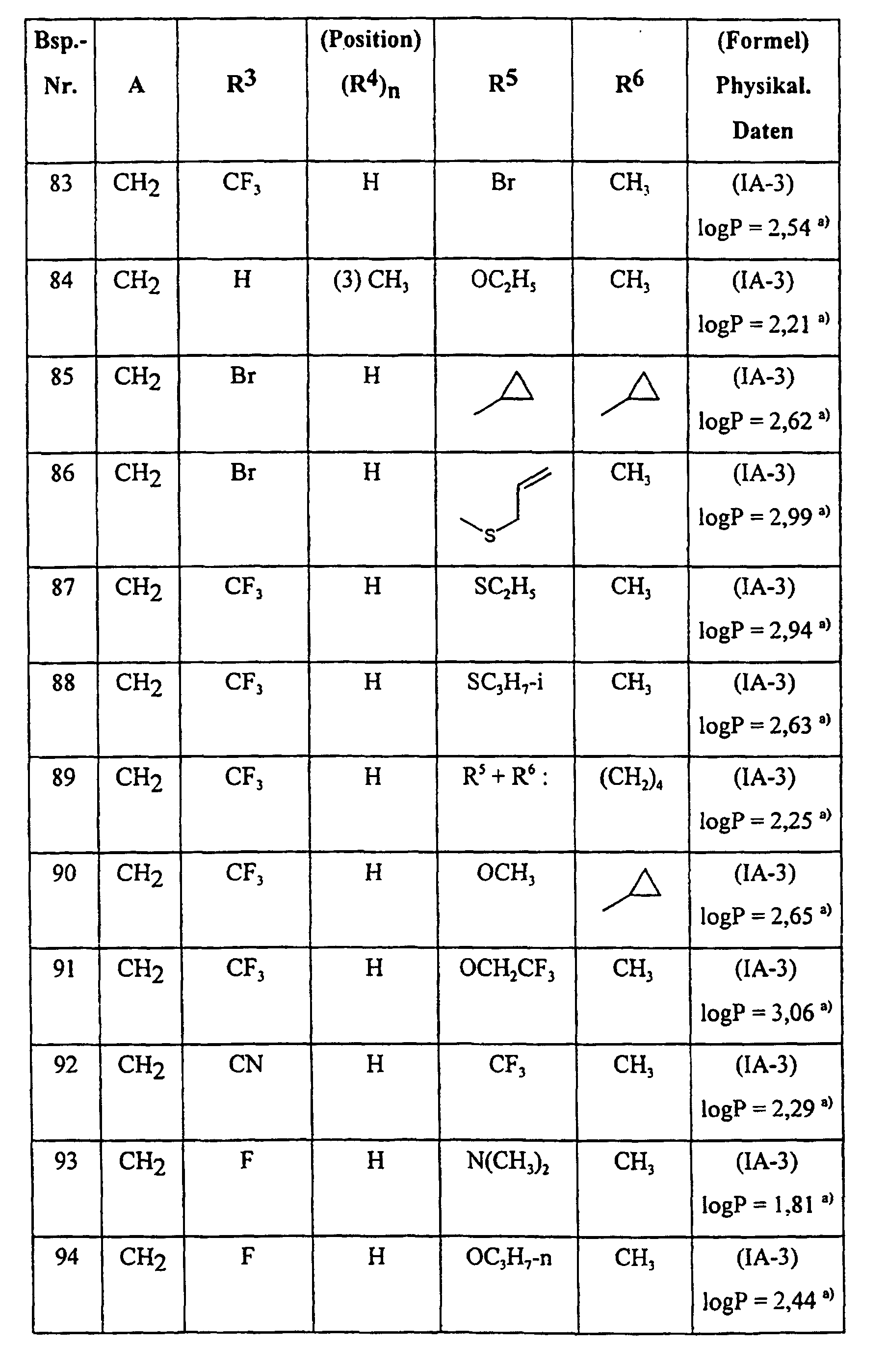

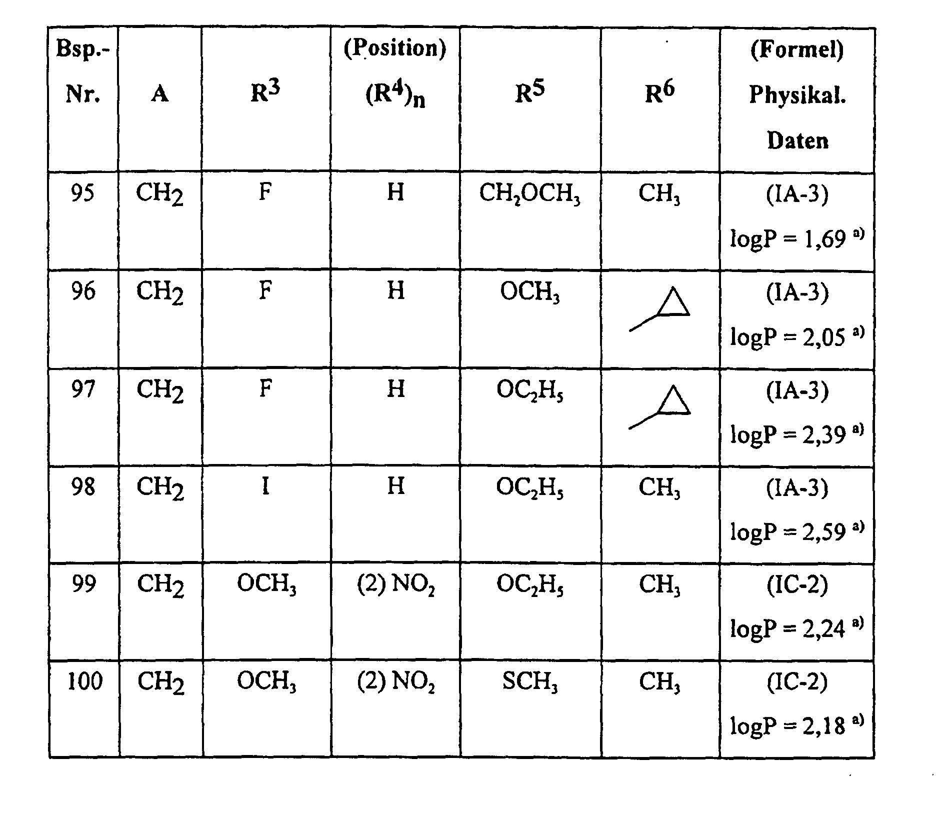

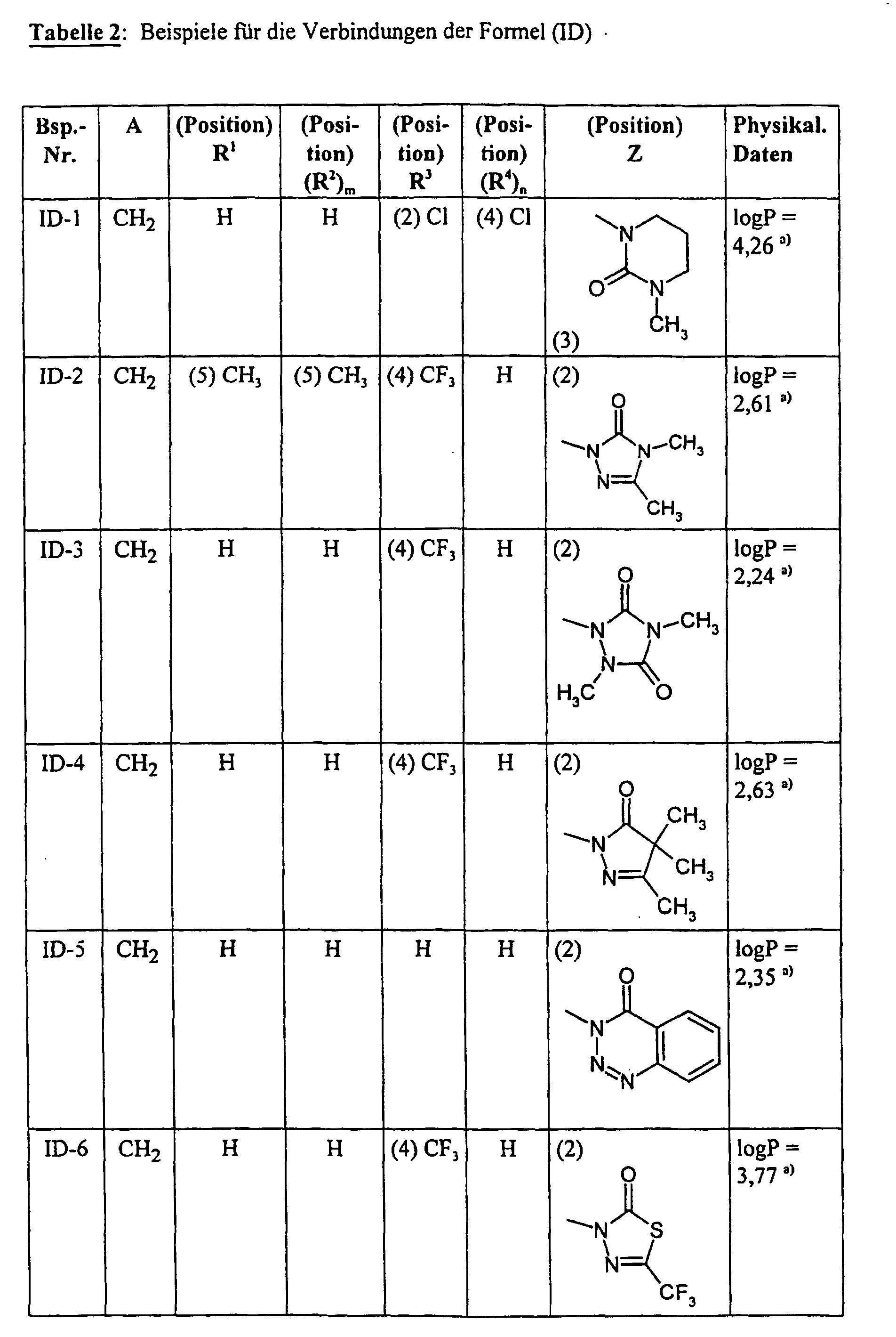

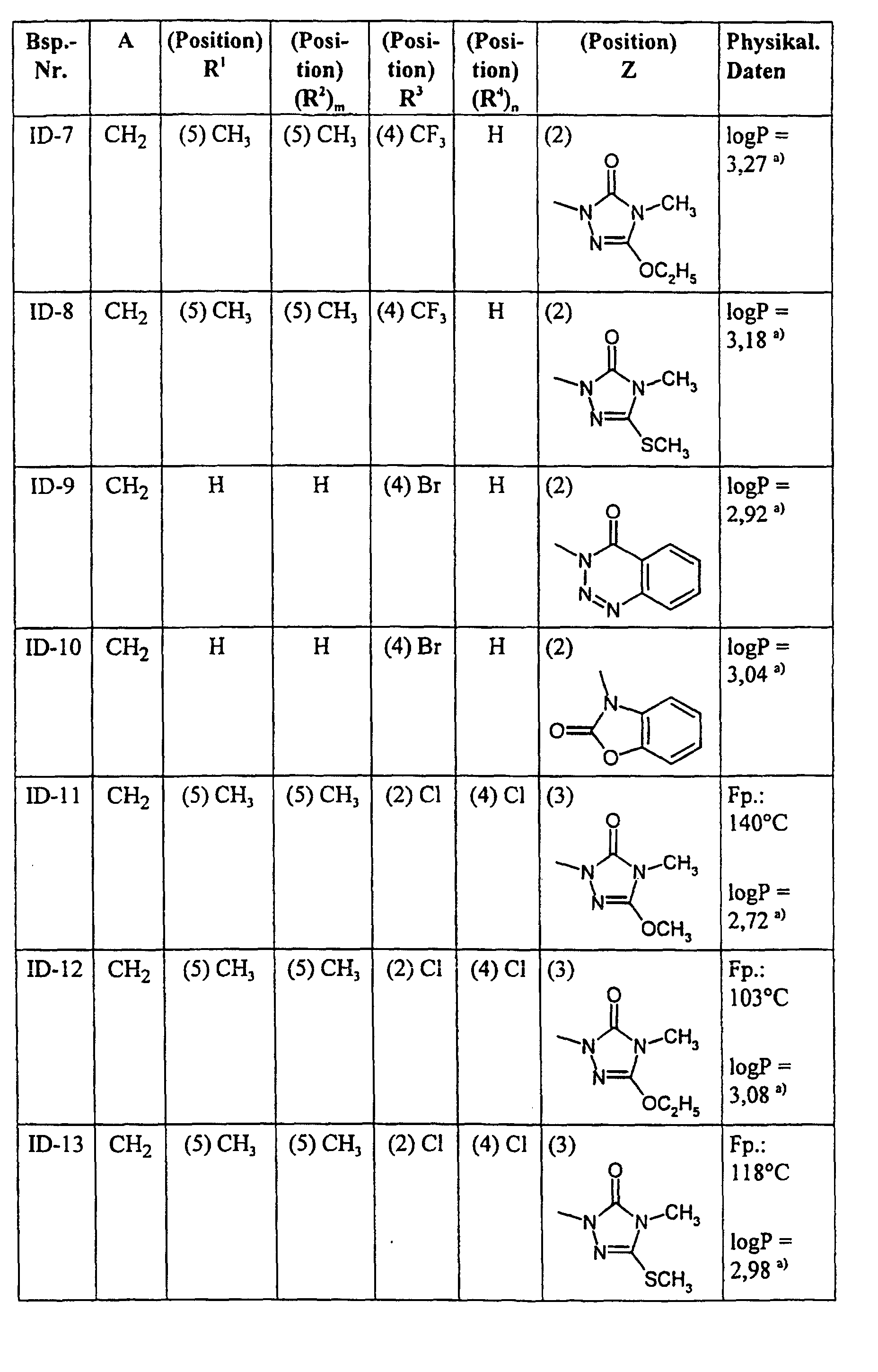

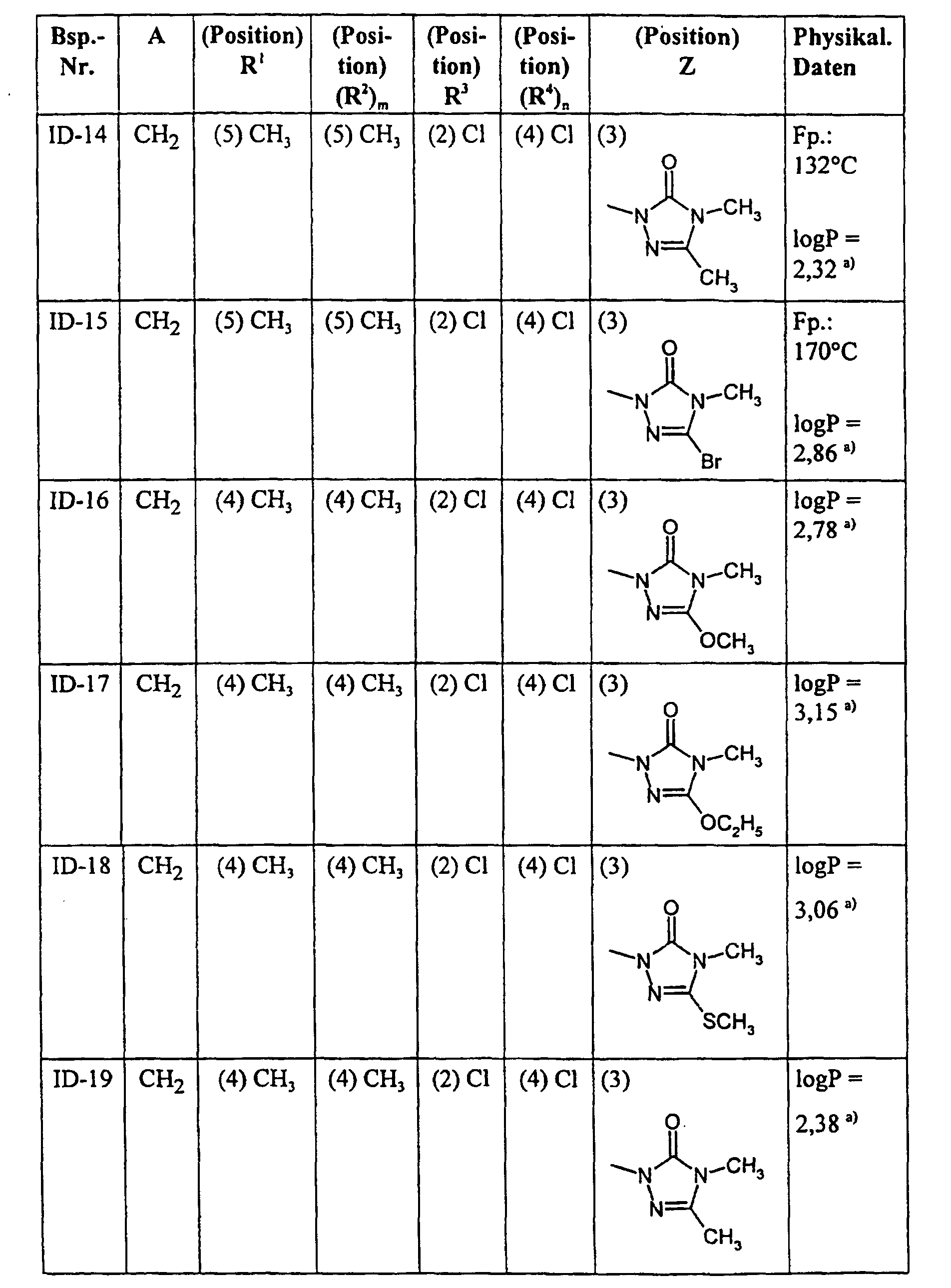

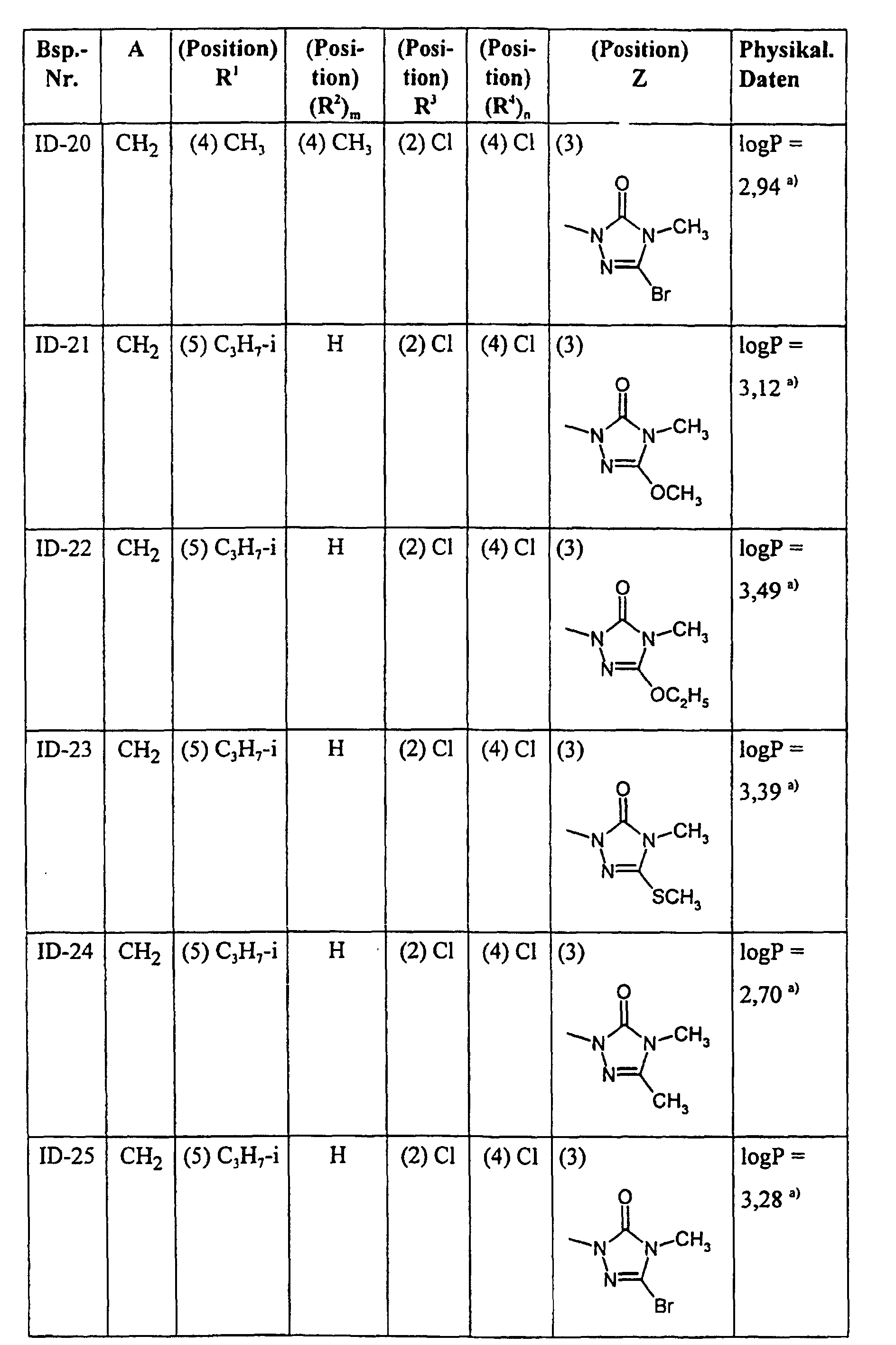

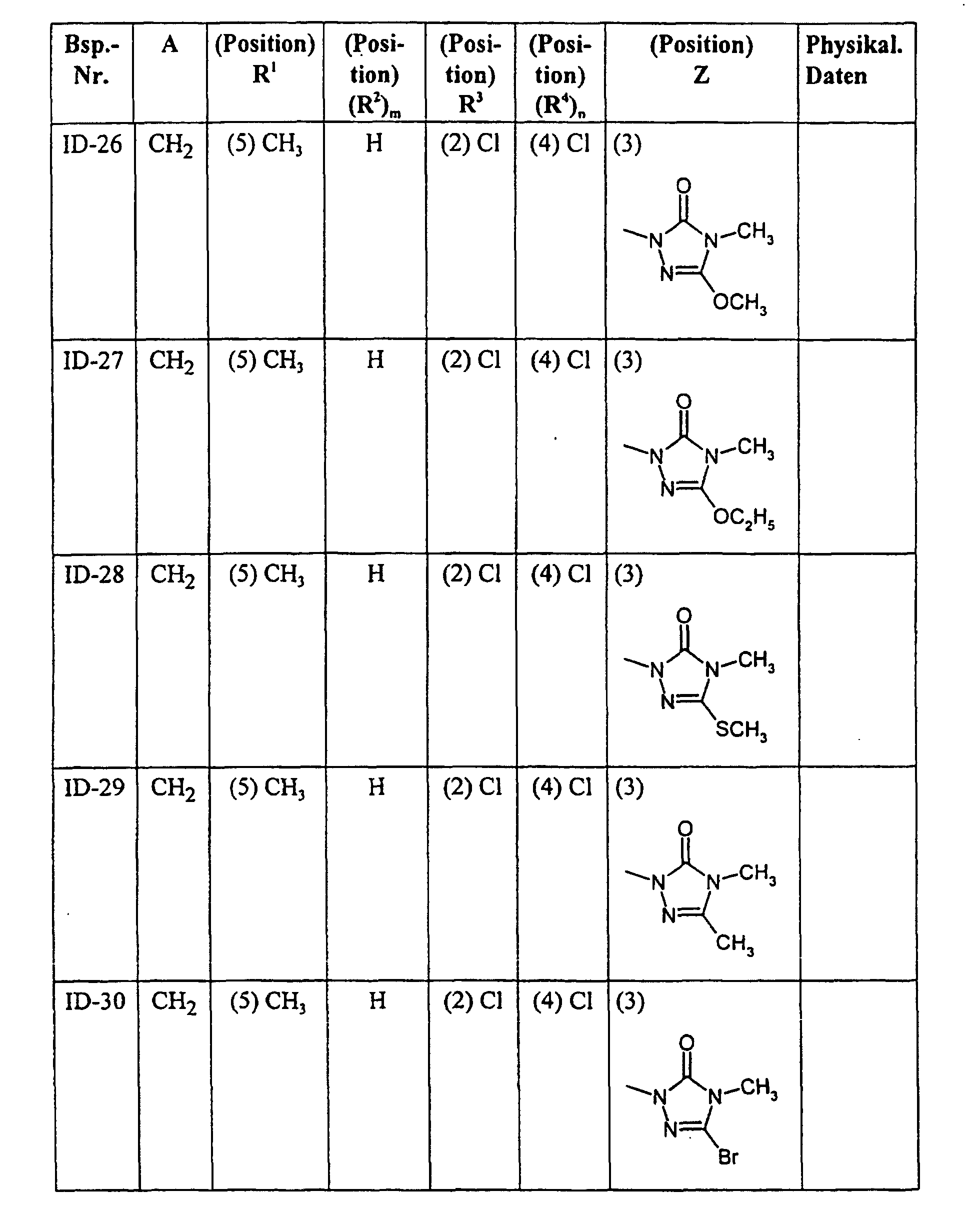

### Ausgangsstoffe der Formel (III):

### Beispiel (III-1)

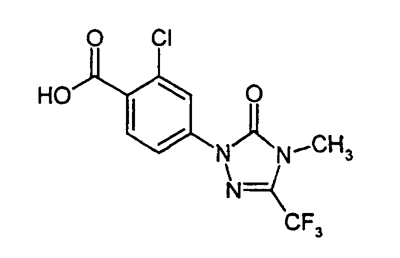

4,5 g (15 mMol) 2-(3-Chlor-4-cyano-phenyl)-4-methyl-5-trifluormethyl-2,4-dihydro-3H-1,2,4-triazol-3-on werden in 80 ml 60%iger Schwefelsäure aufgenommen und die Mischung wird 6 Stunden unter Rückfluß erhitzt. Nach Abkühlen auf Raumtemperatur wird das kristallin angefallene Produkt durch Absaugen isoliert.

Man erhält 4,5 g (91% der Theorie) 2-(3-Carboxy-4-chlor-phenyl)-4-methyl-5-trifluormethyl-2,4-dihydro-3H-1,2,4-triazol-3-on vom Schmelzpunkt 223°C.

### Beispiel (III-2)

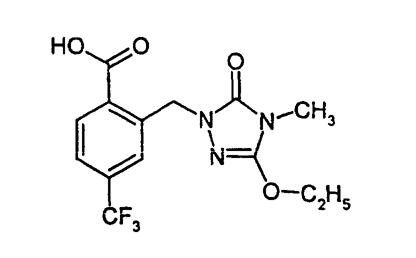

2 g (4,9 mMol) 5-Brom-4-methyl-2-(2-ethoxycarbonyl-5-trifluormethyl-benzyl)-2,4-dihydro-3H-1,2,4-triazol-3-on (vgl. Beispiel IV-1) werden in 30 ml 10%iger ethanolischer Kalilauge gelöst und 2 Stunden unter Rückfluß erhitzt. Das Reaktionsgemisch wird im Wasserstrahlvakuum eingeengt, in 20 ml Wasser aufgenommen und mit verdünnter Salzsäure angesäuert. Der ausfallende Feststoff wird filtriert und getrocknet.

Man erhält 1,2 g (71% der Theorie) 5-Ethoxy-4-methyl-2-(2-carboxy-5-trifluormethyl-benzyl)-2,4-dihydro-3H-1,2,4-triazol-3-on als festes Produkt.
logP: 2,18^{a)}

### Beispiel (III-3)

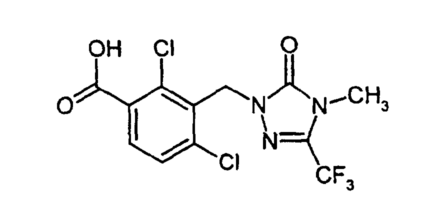

13,4 g (35 mMol) 4-Methyl-5-trifluormethyl-2-(2,6-dichlor-3-methoxycarbonylbenzyl)-2,4-dihydro-3H-1,2,4-triazol-3-on werden in 60 ml 1,4-Dioxan vorgelegt und eine Lösung von 1,54 g (38,5 mMol) Natriumhydroxid in 20 ml Wasser wird bei Raumtemperatur langsam eindosiert. Die Reaktionsmischung wird 150 Minuten bei 60°C gerührt und anschließend im Wasserstrahlvakuum eingeengt. Der Rückstand wird in 100 ml Wasser gelöst und durch Zugabe von konz. Salzsäure wird der pH-Wert der Lösung auf 1 eingestellt. Das hierbei kristallin angefallene Produkt wird durch Absaugen isoliert.

Man erhält 11,7 g (90% der Theorie) 4-Methyl-5-trifluormethyl-2-{2,6-dichlor-3-carboxy-benzyl)-2,4-dihydro-3H-1,2,4-triazol-3-on vom Schmelzpunkt 207°C.

Analog zu den Beispielen (III-1) bis (III-3) können beispielsweise auch die in der nachstehenden Tabelle 2 aufgeführten Verbindungen der allgemeinen Formel (III) hergestellt werden.

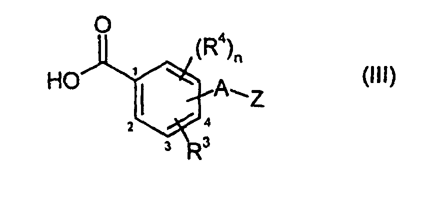

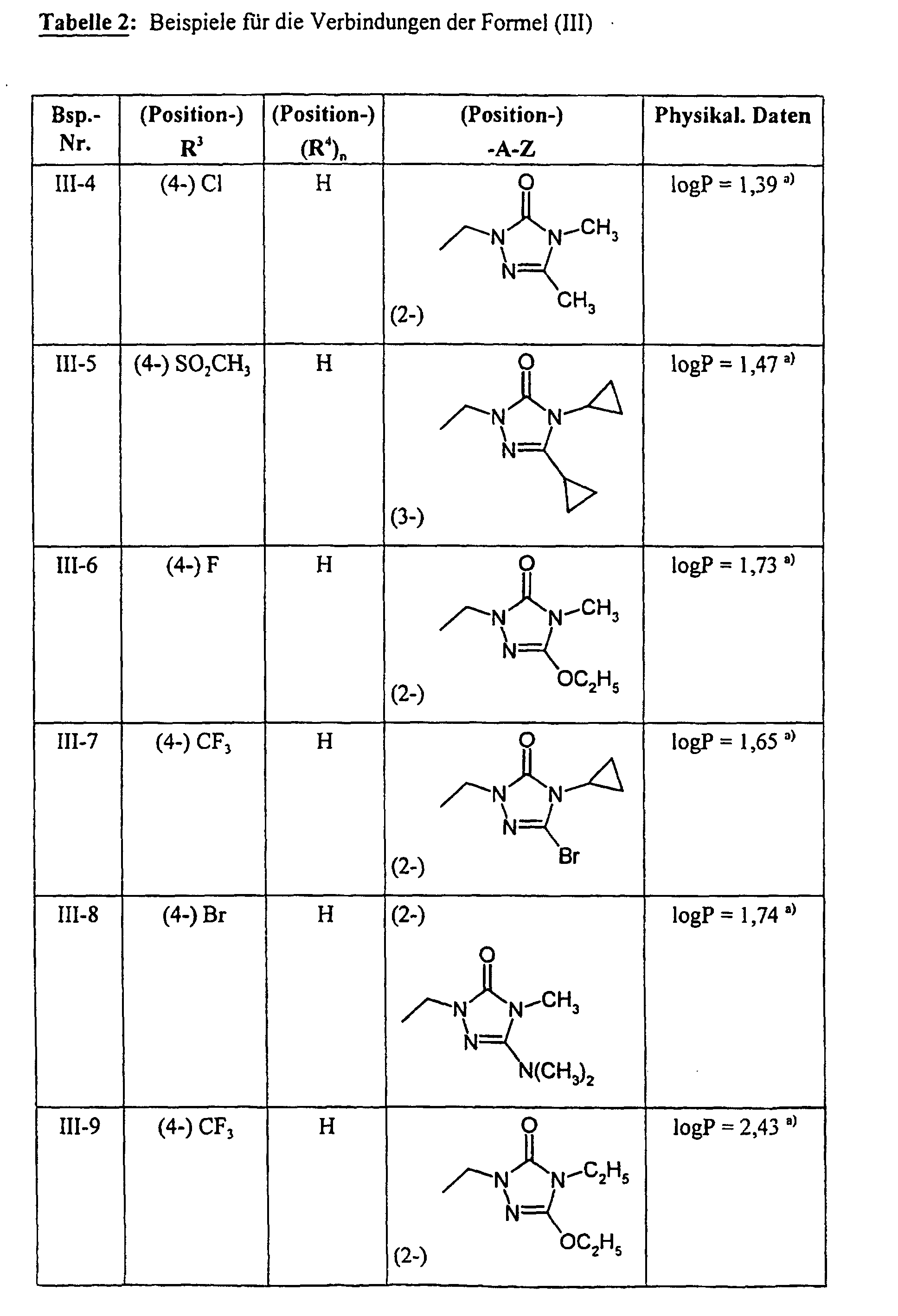

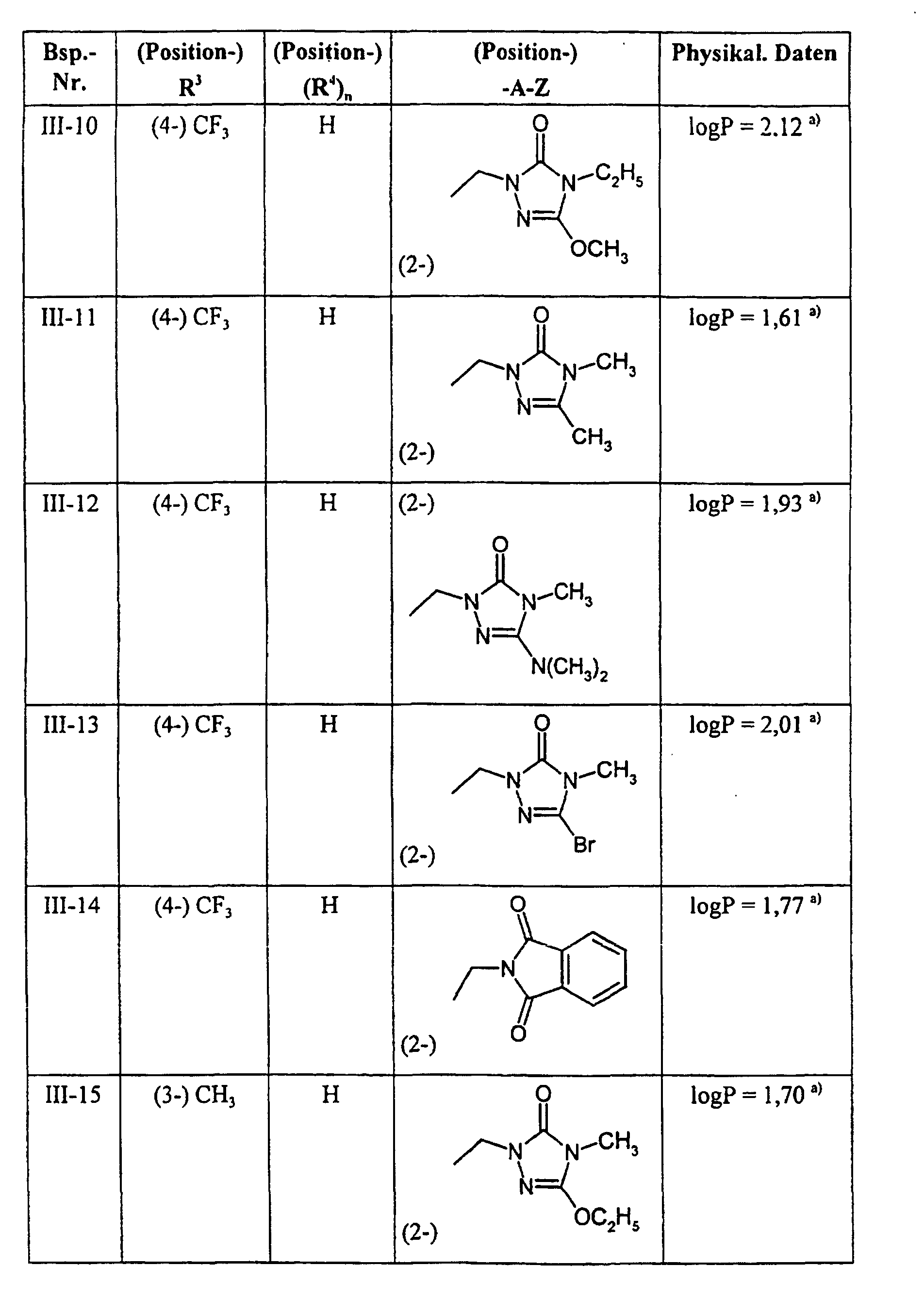

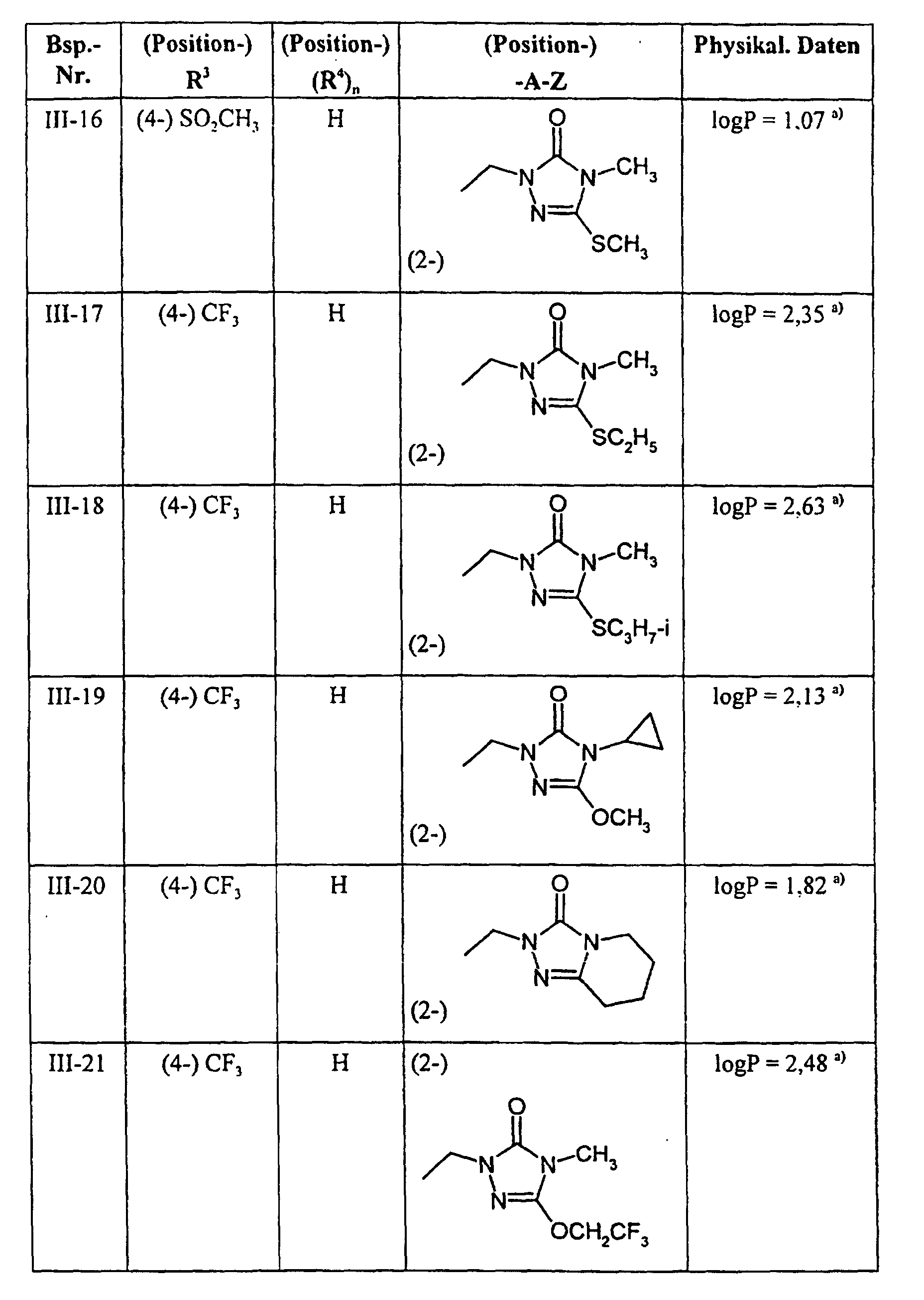

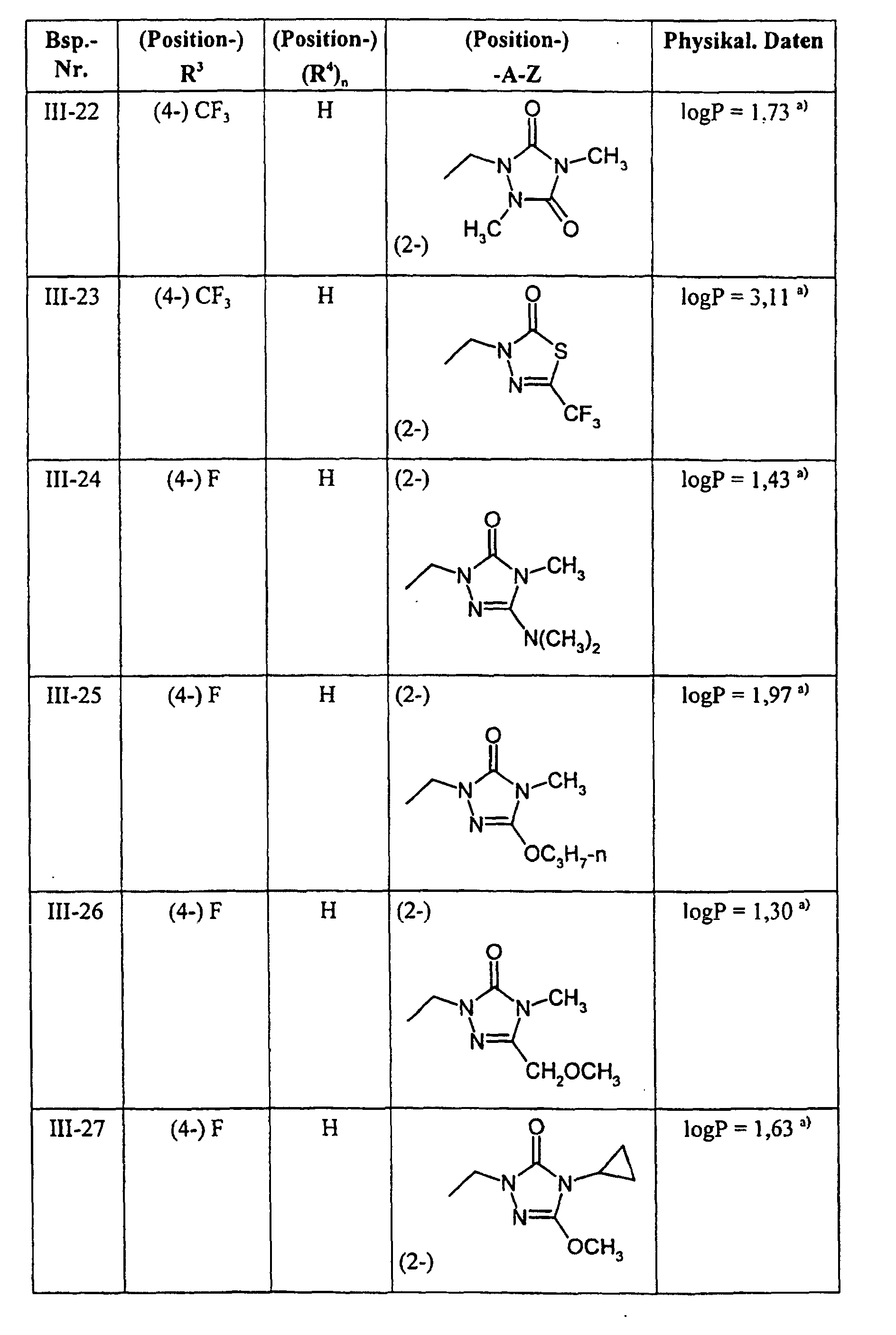

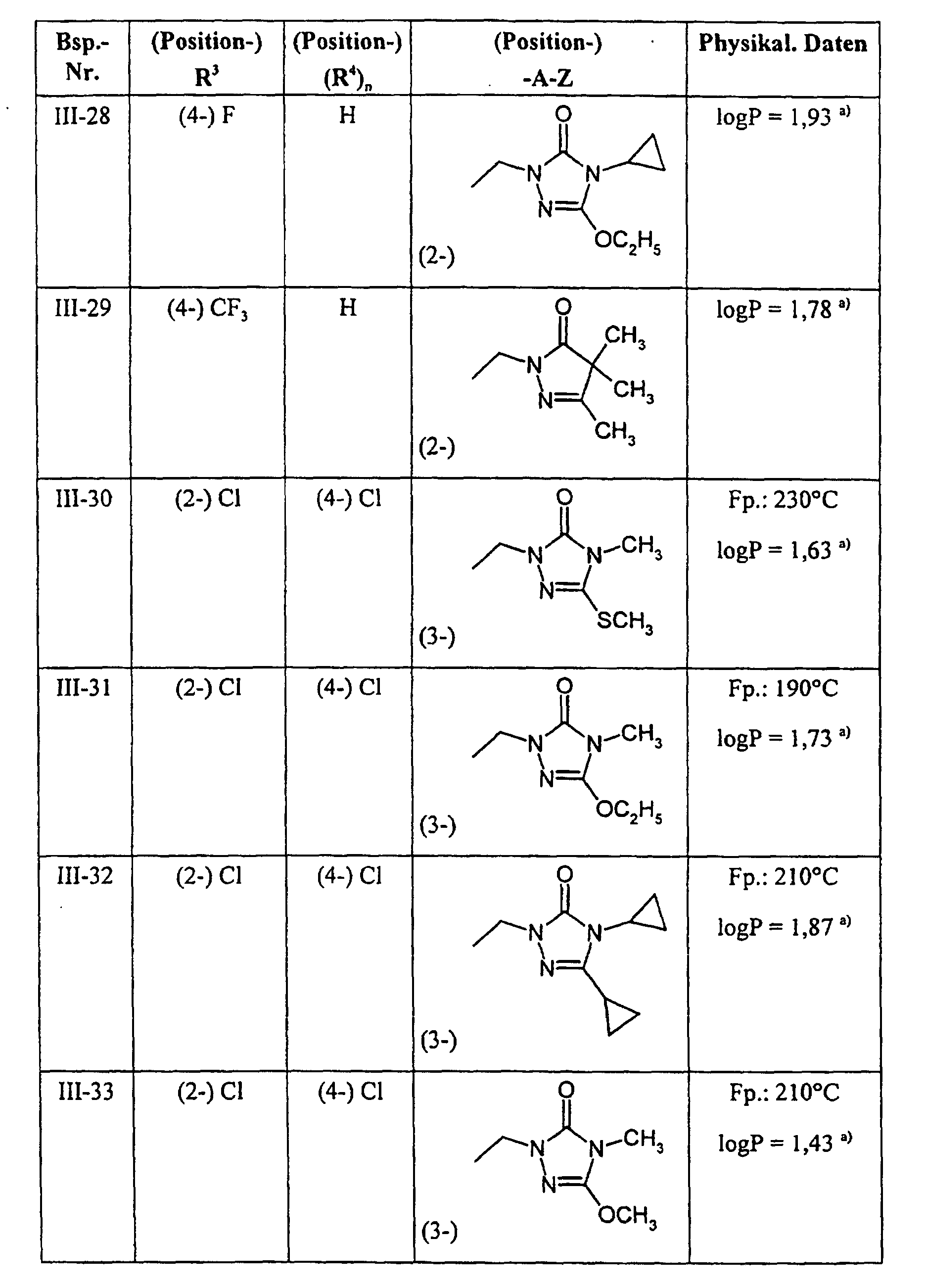

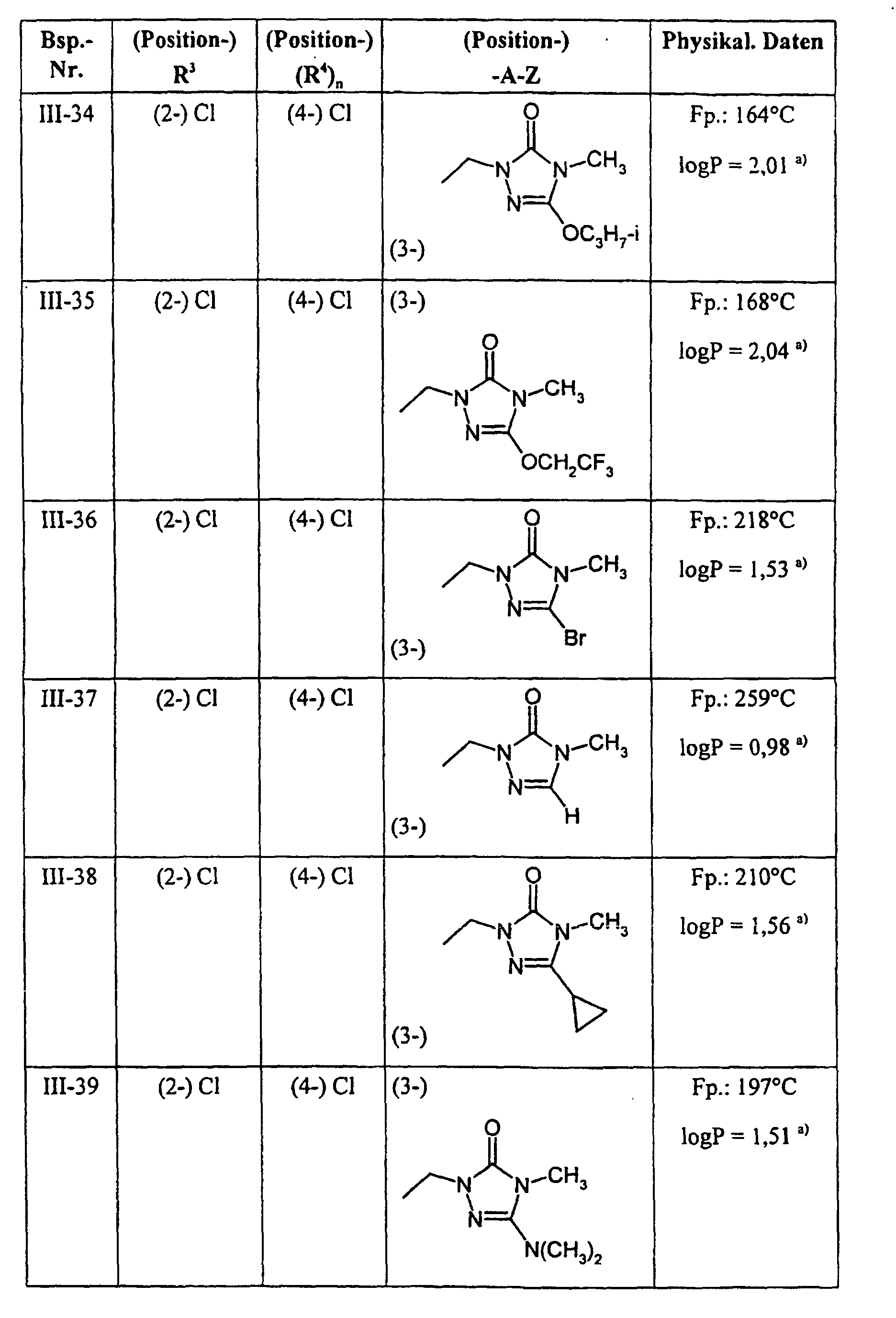

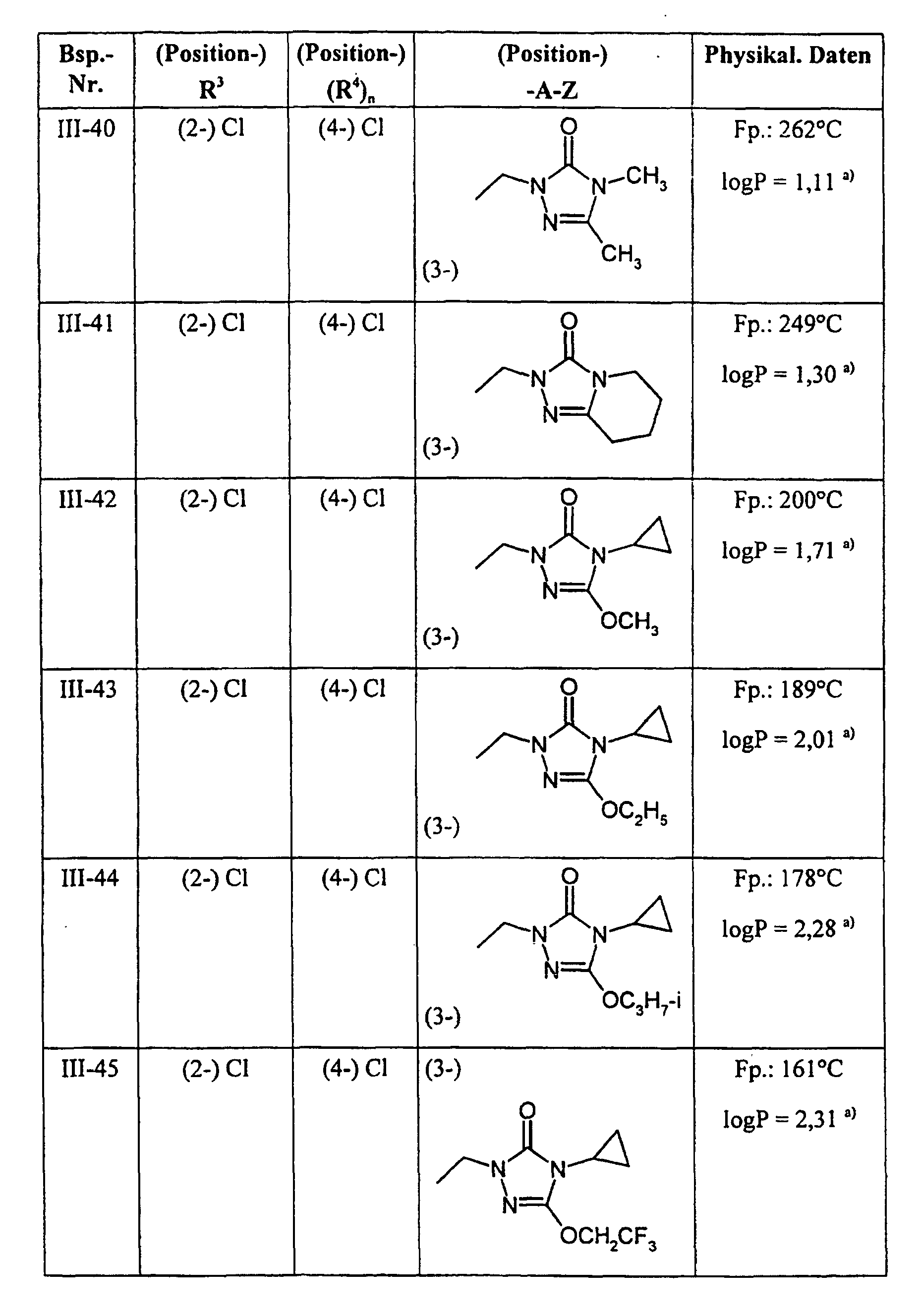

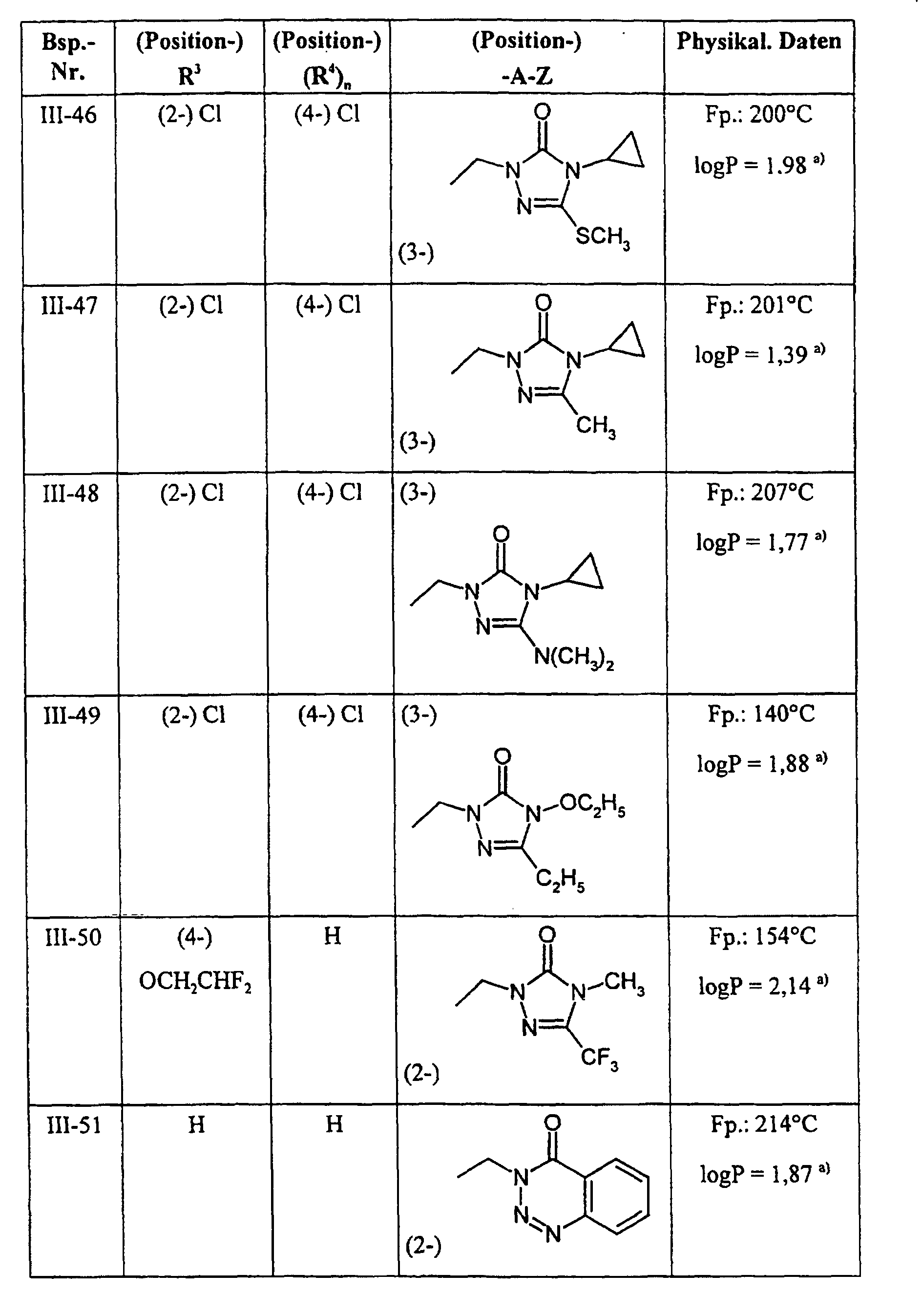

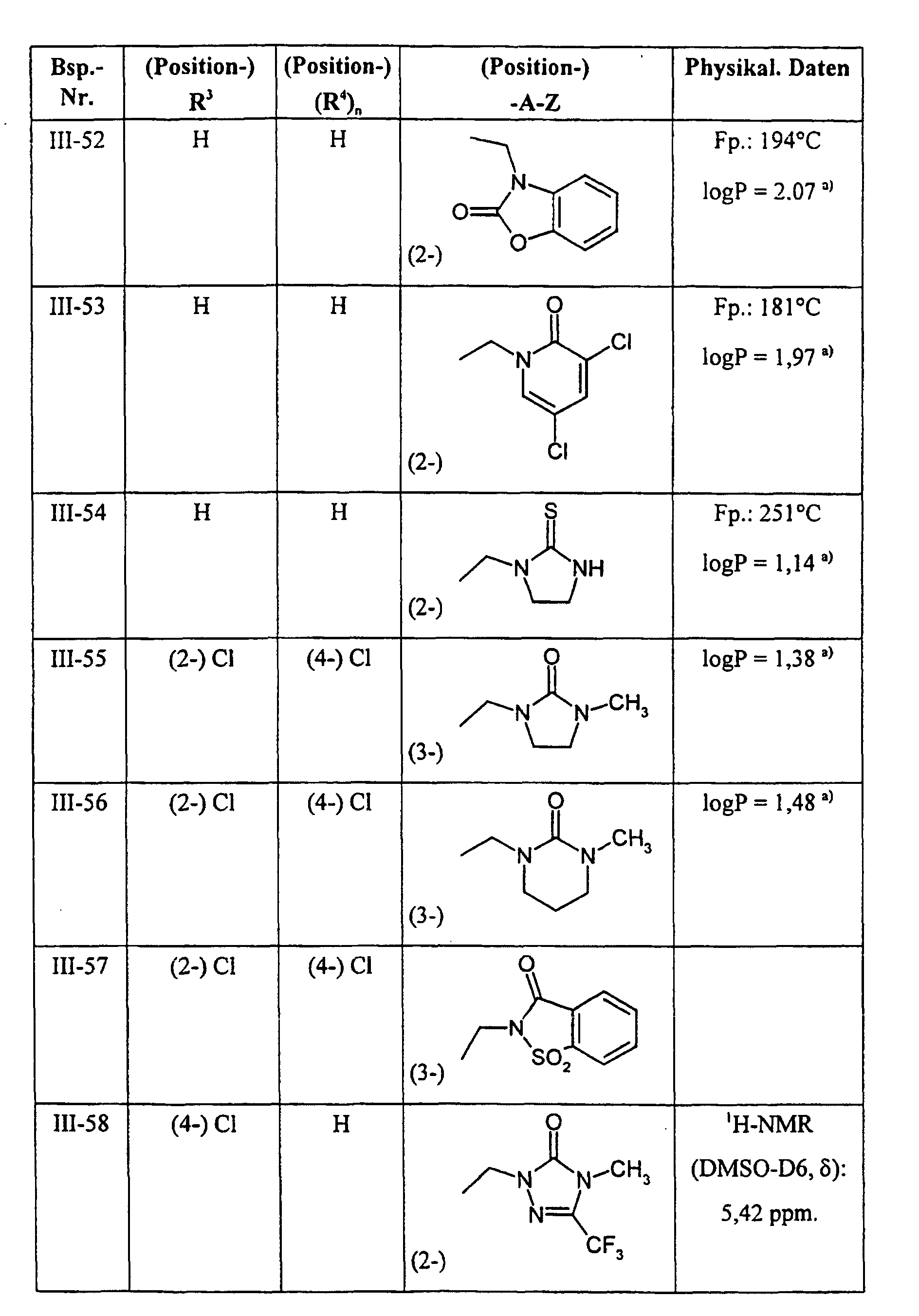

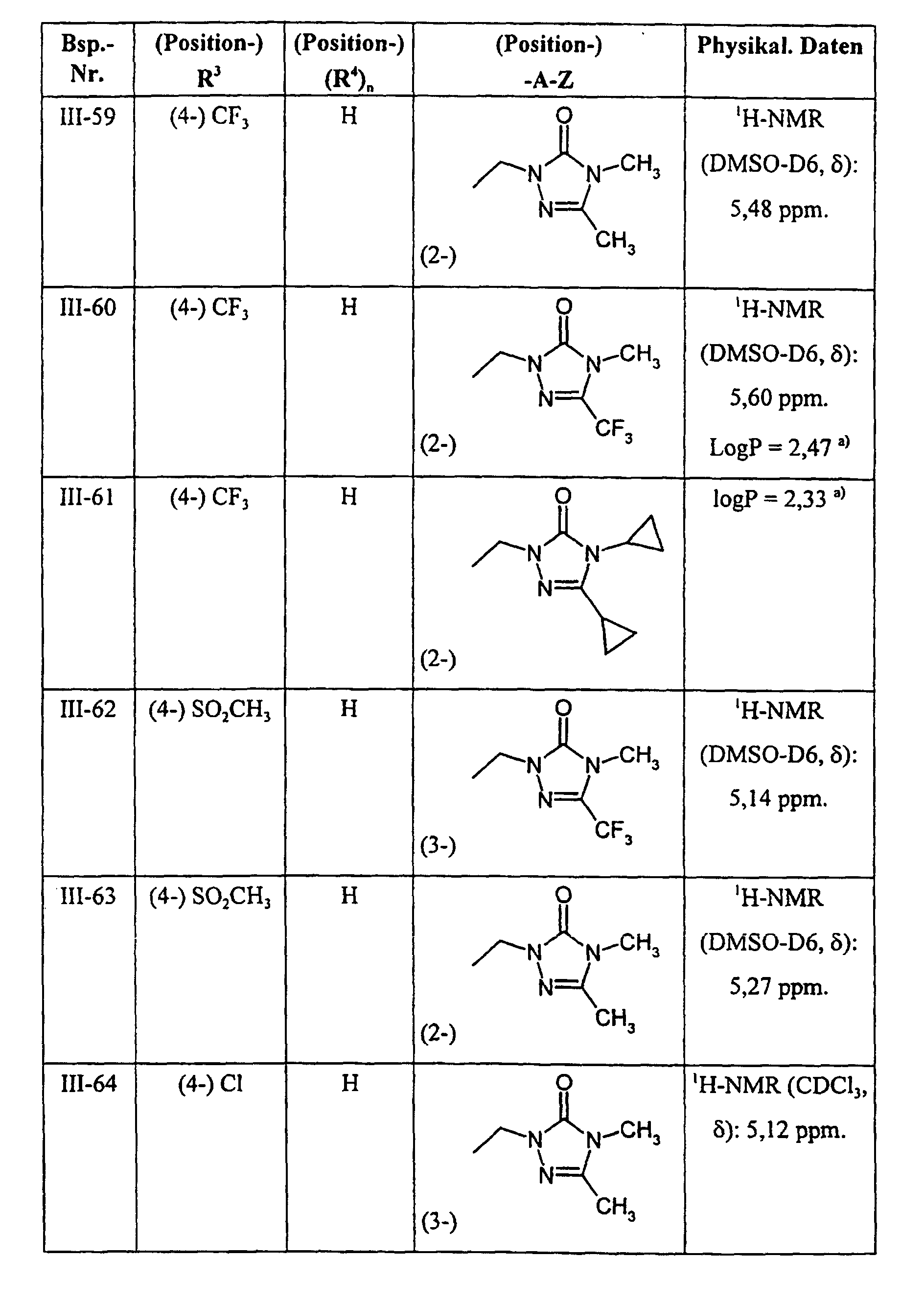

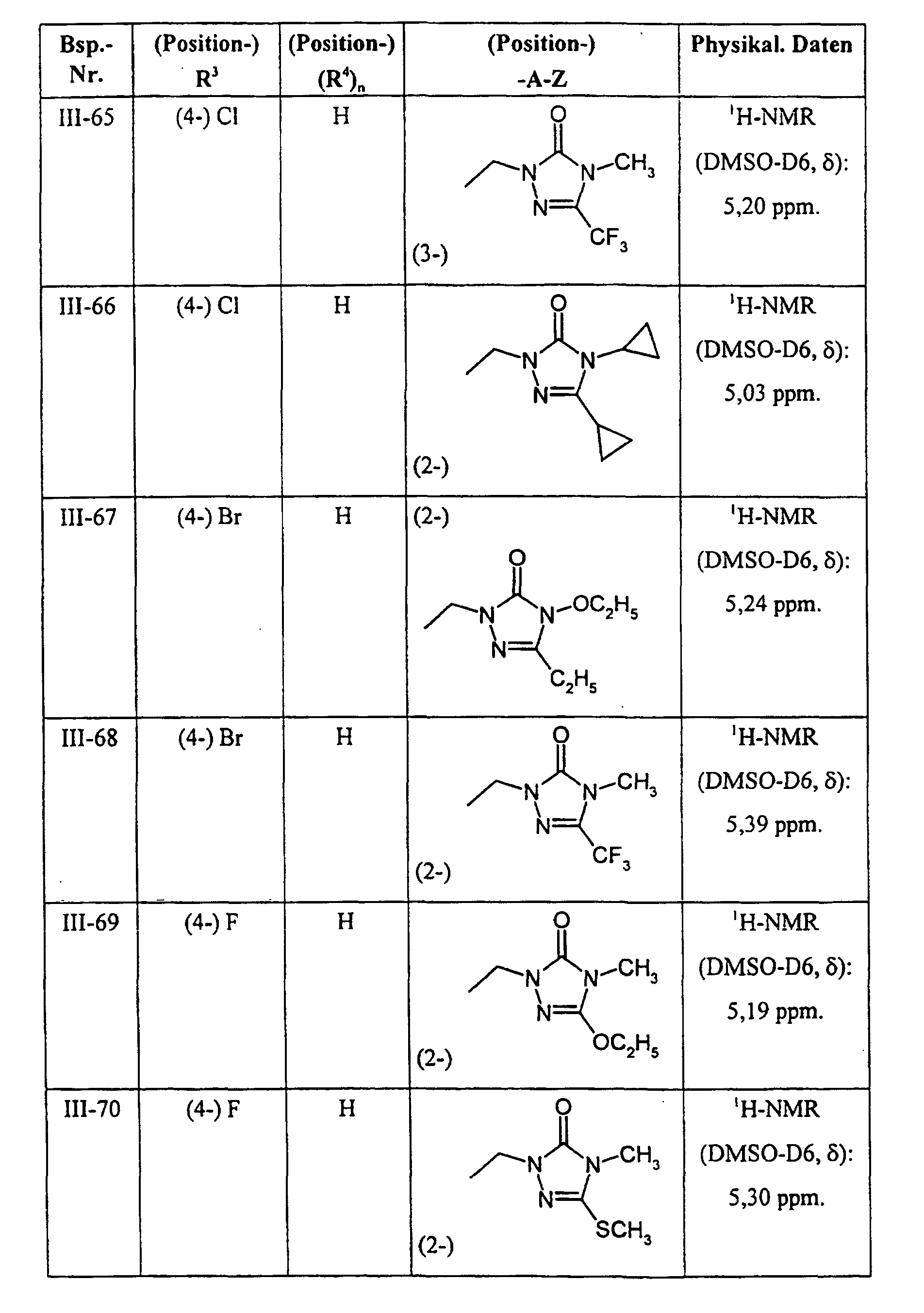

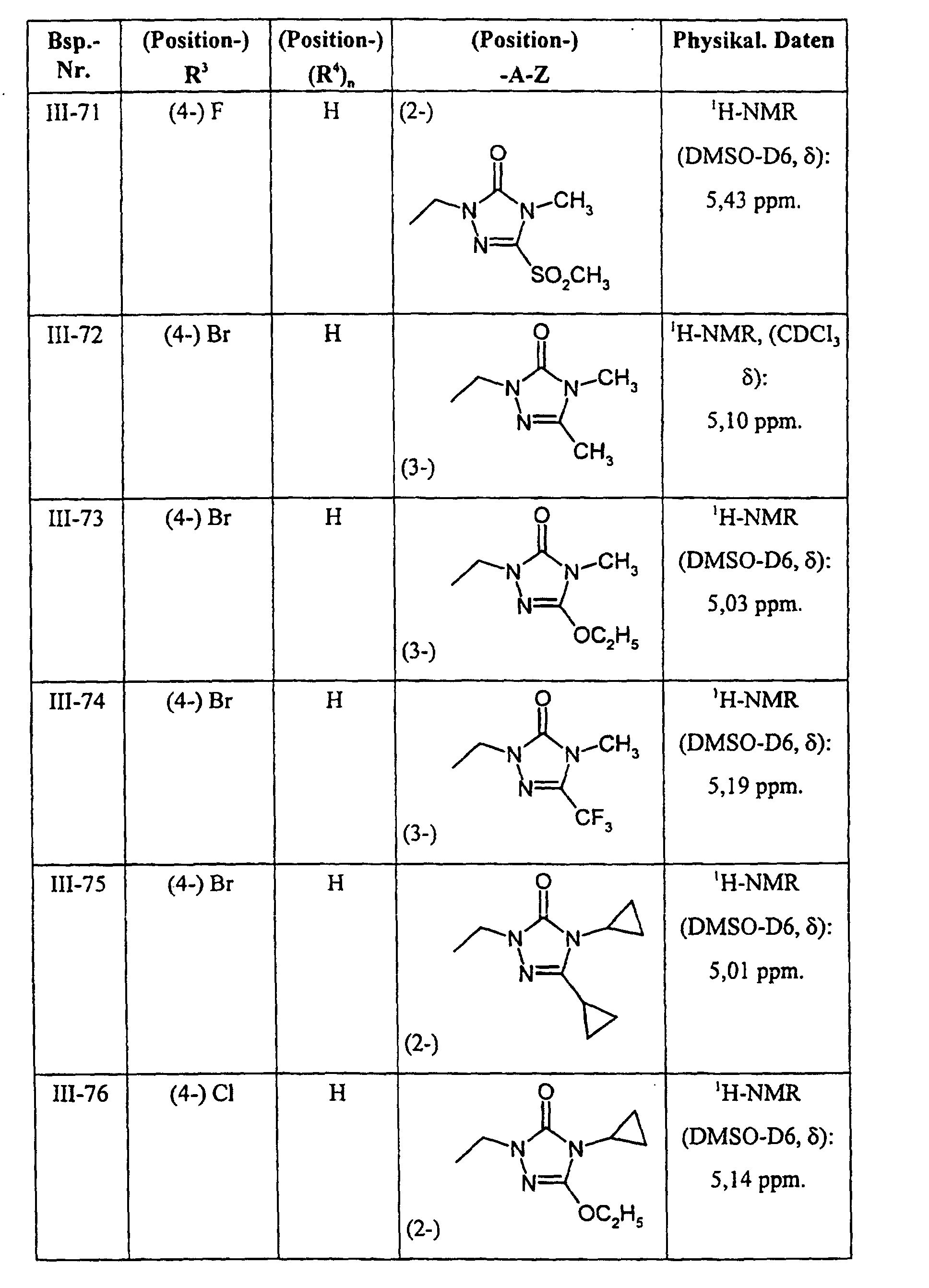

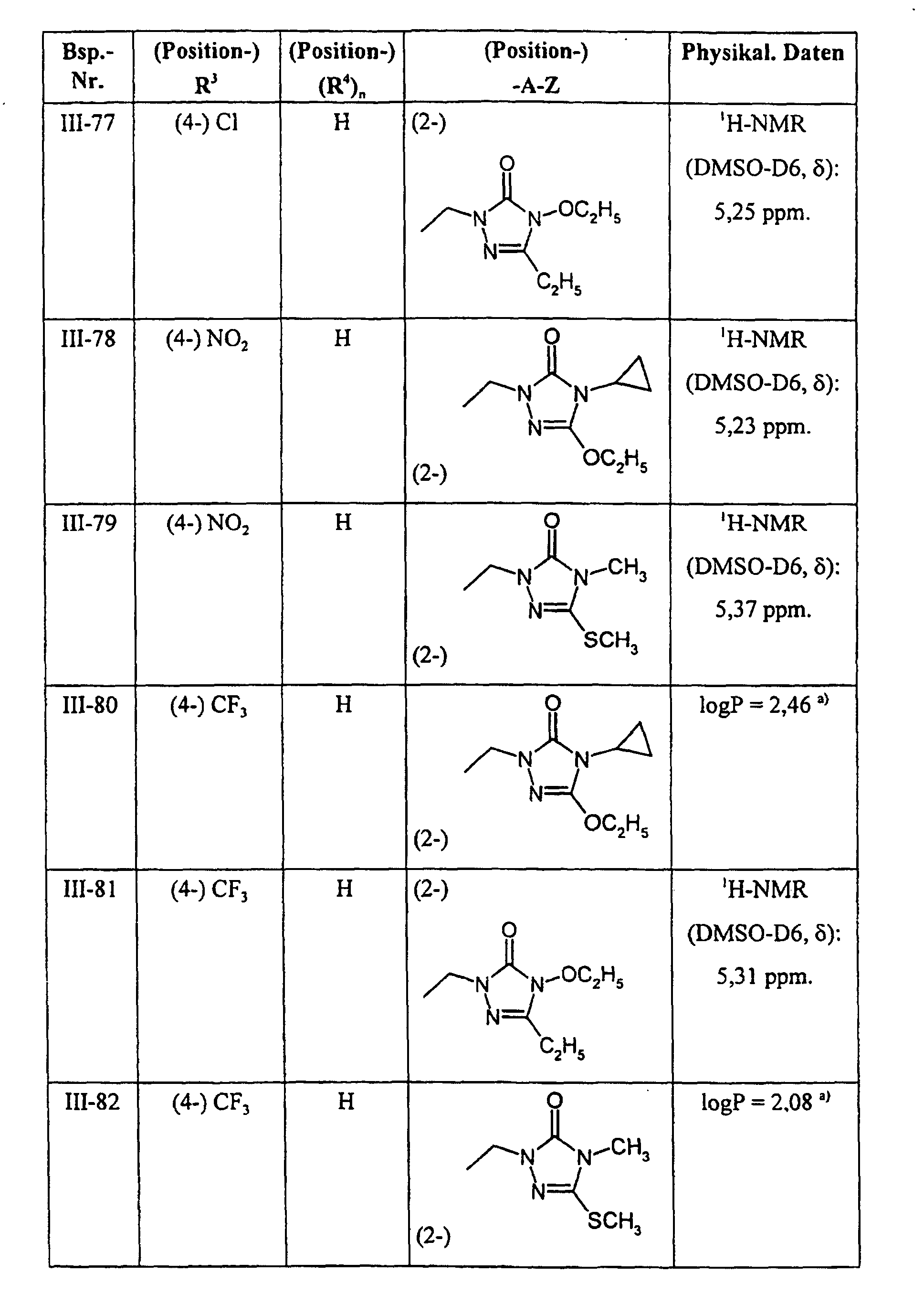

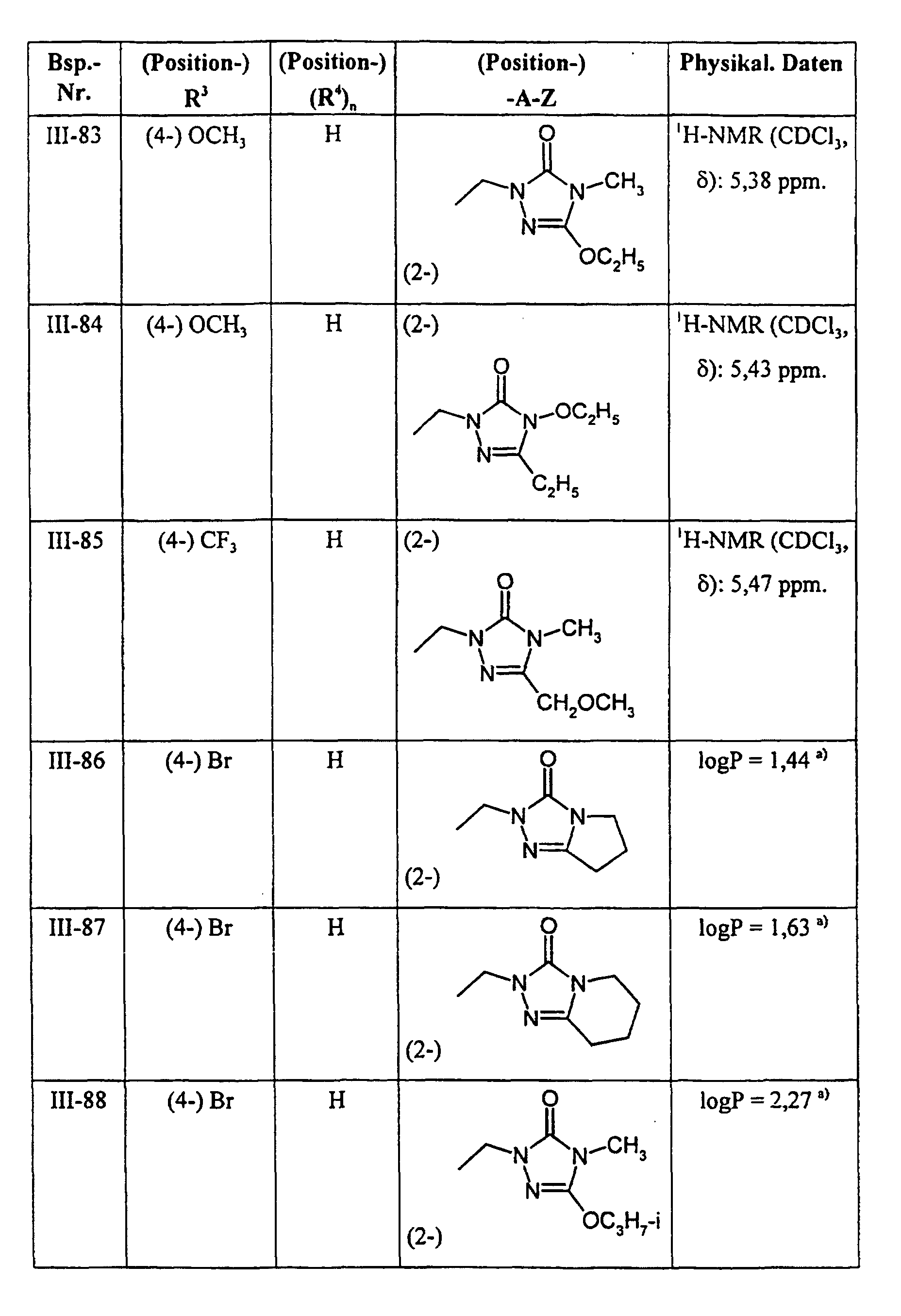

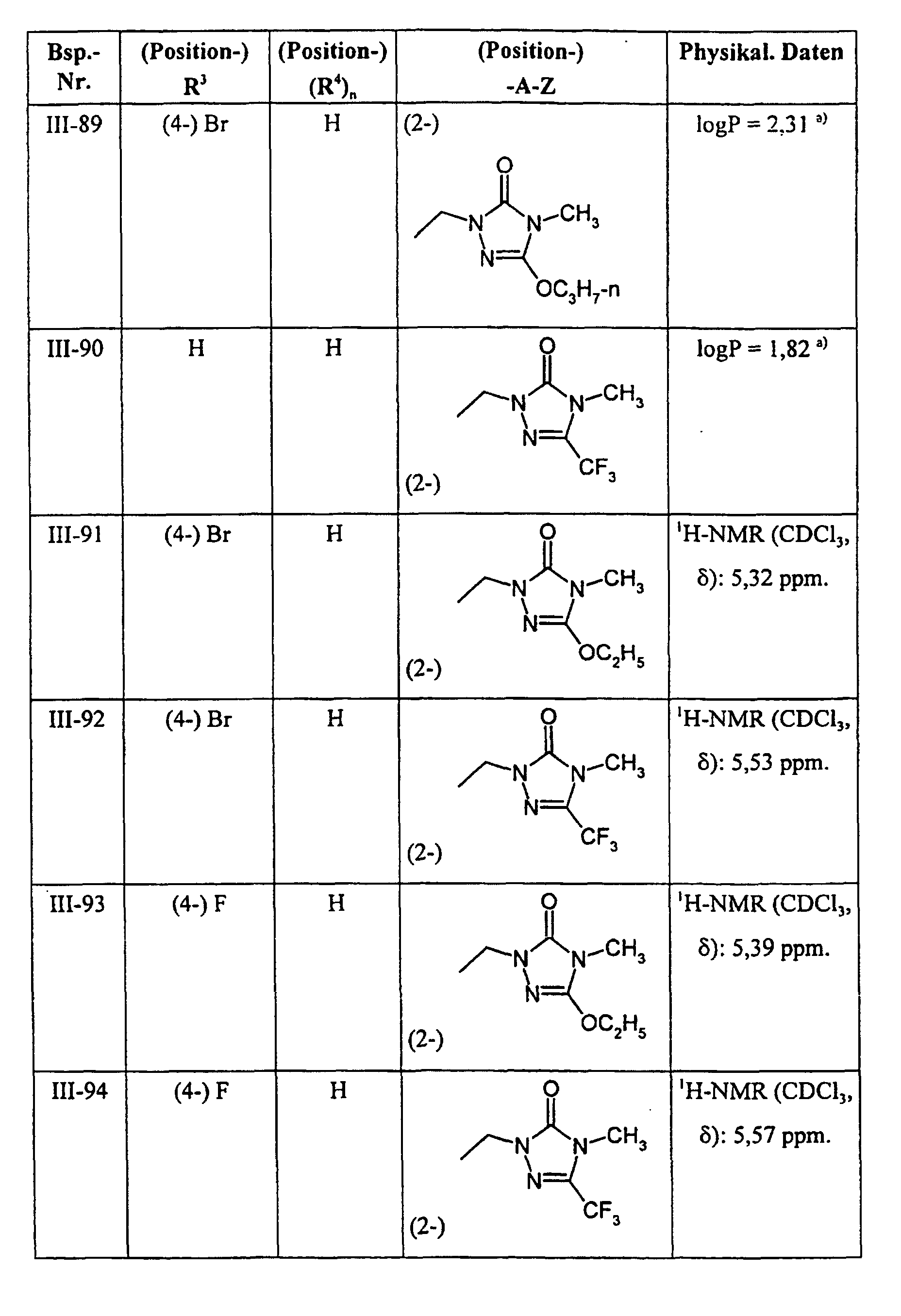

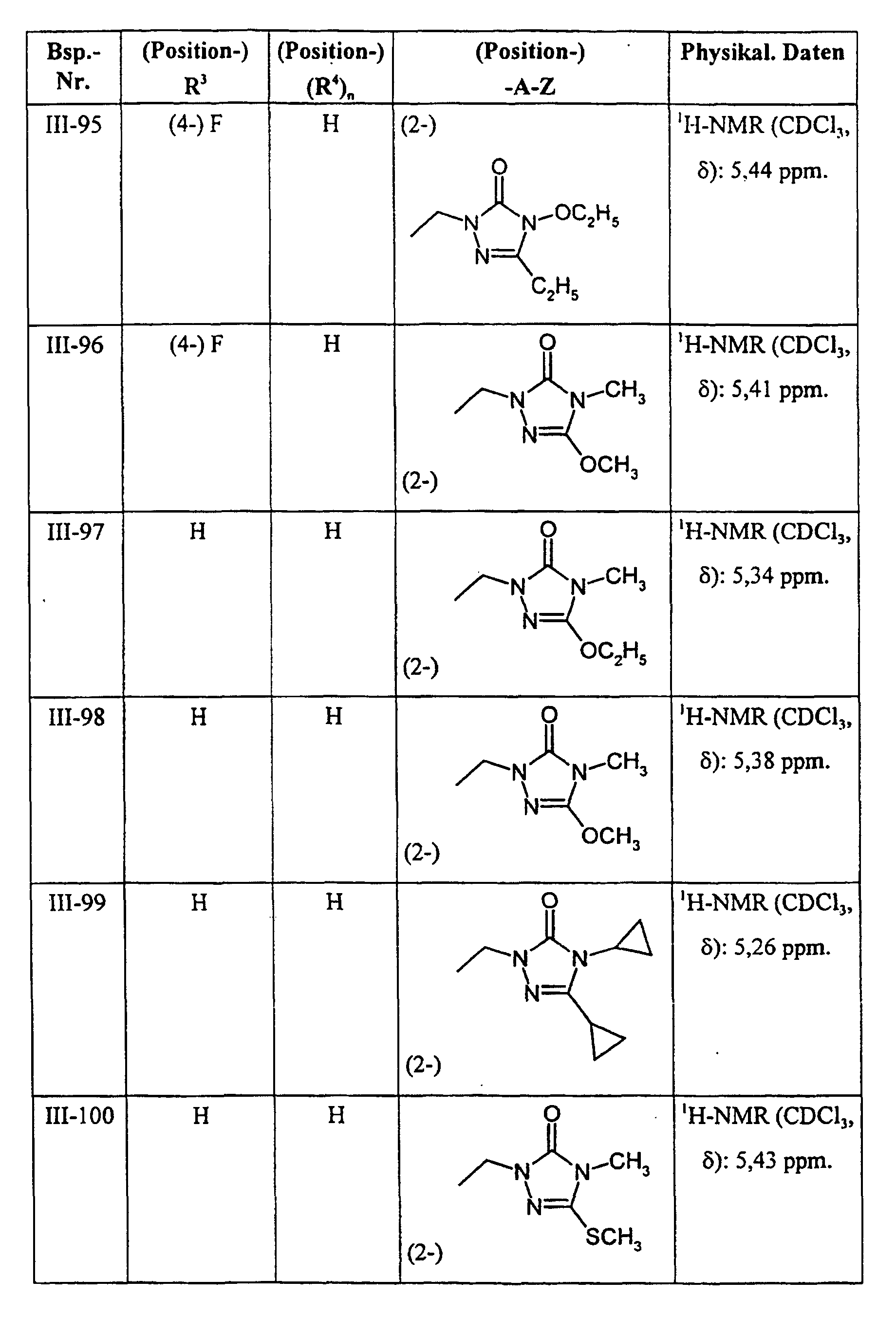

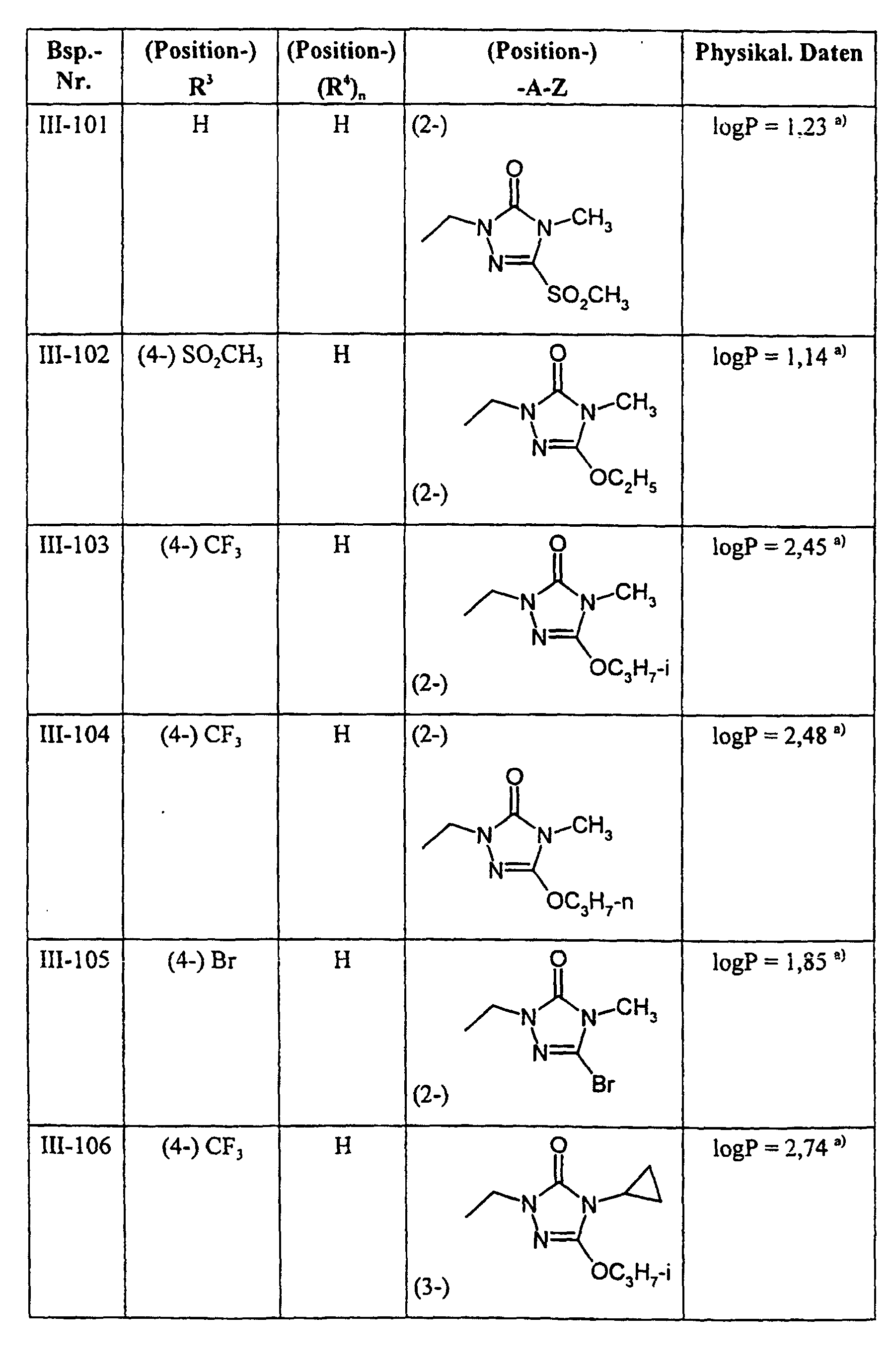

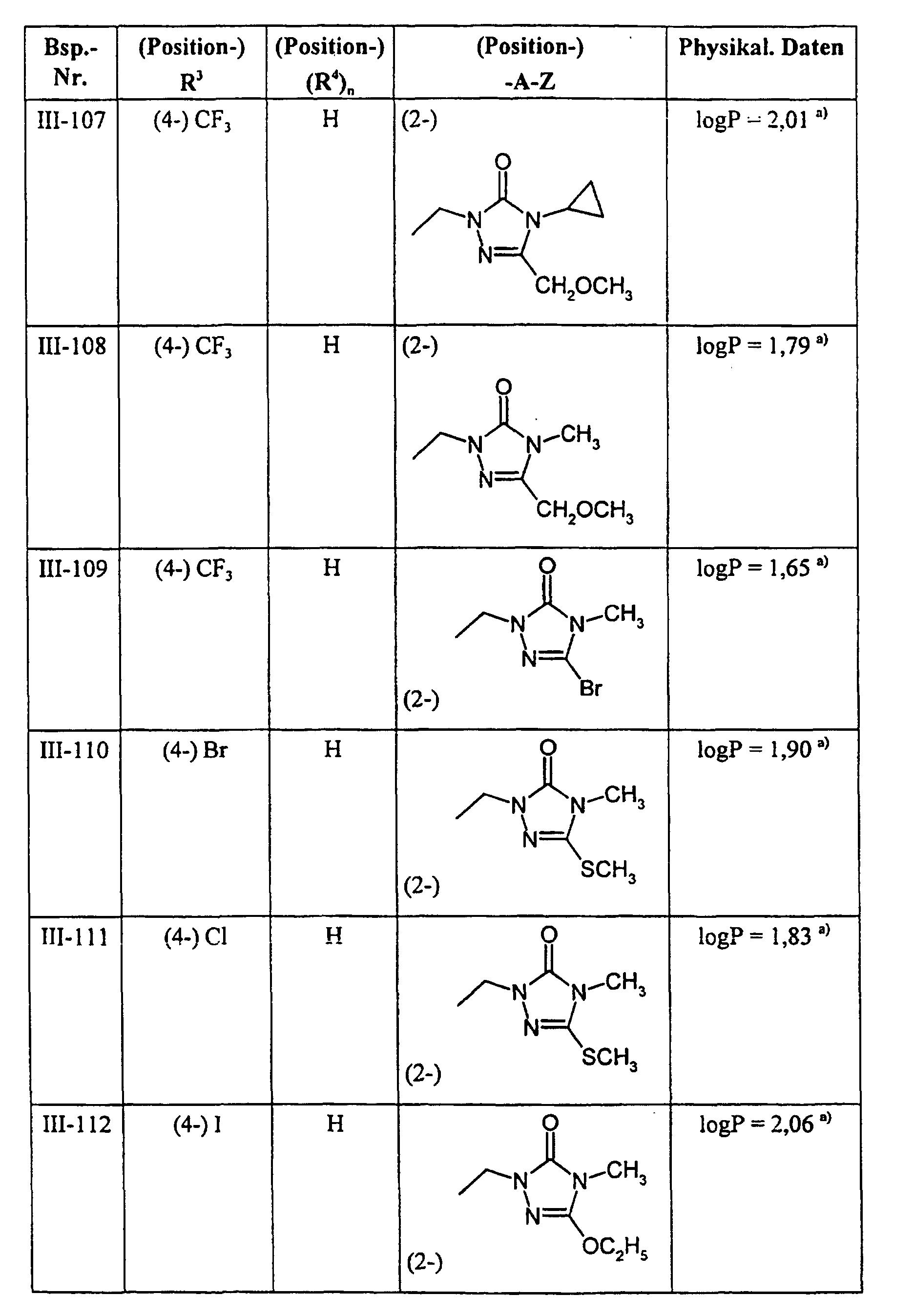

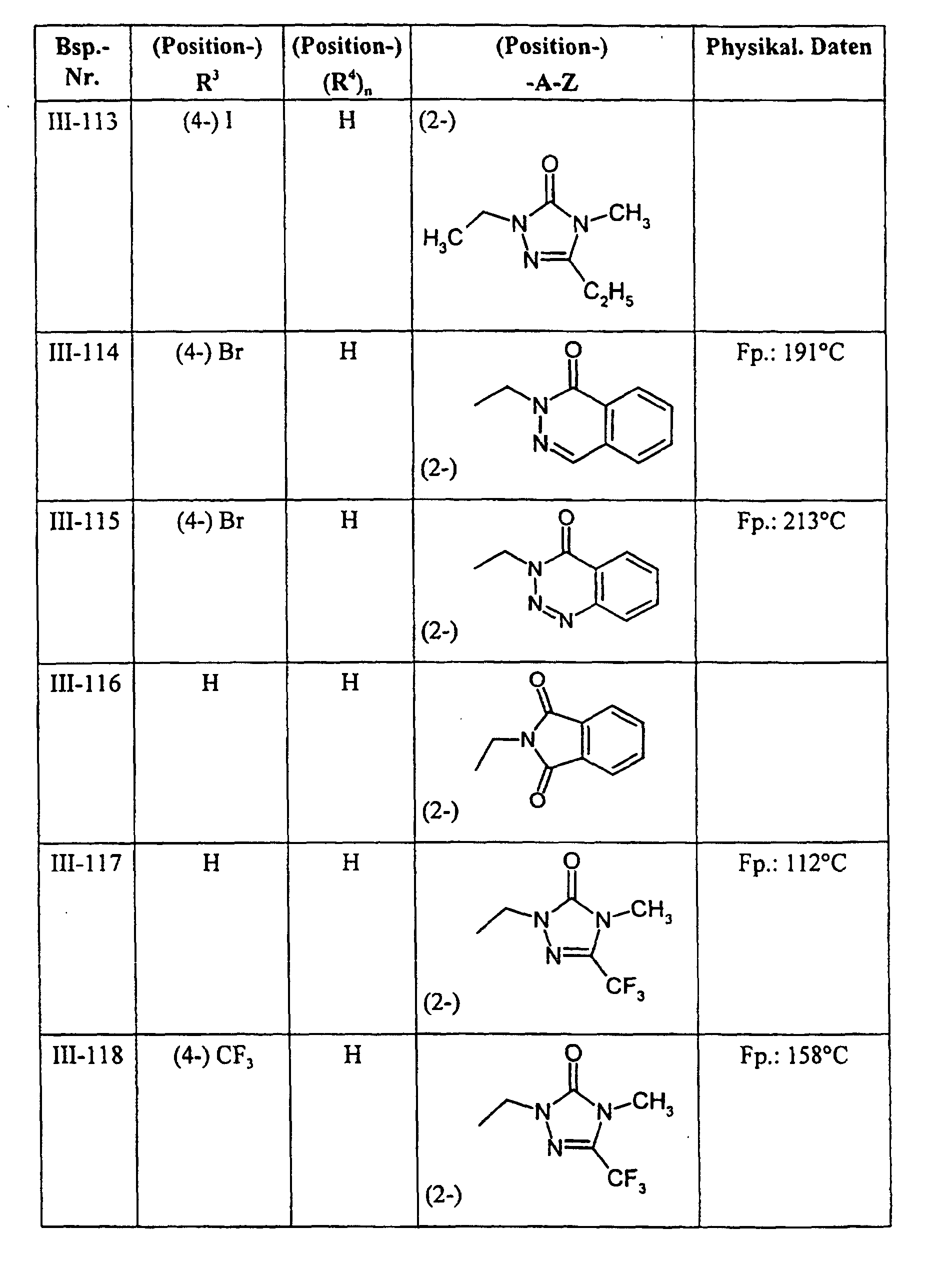

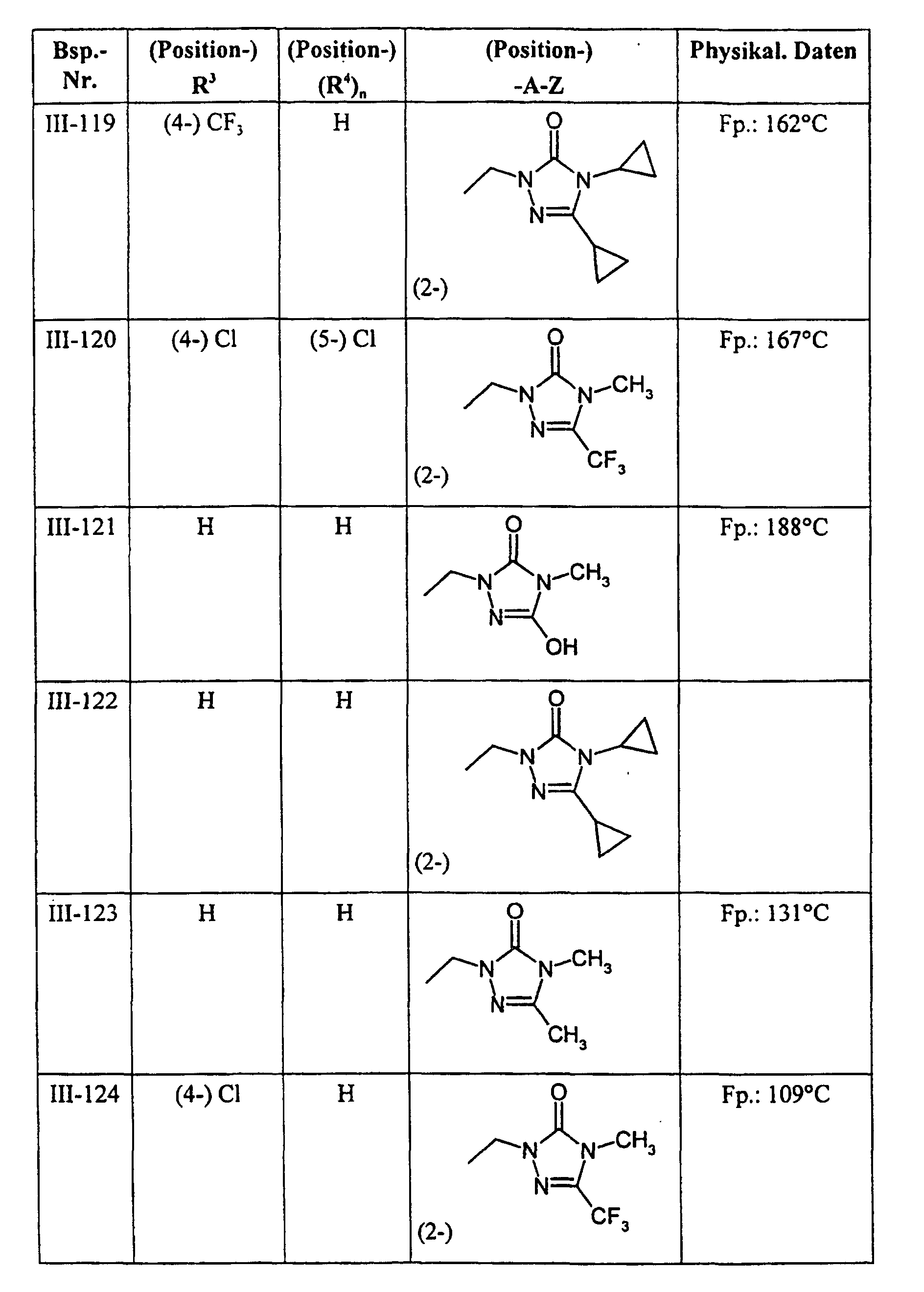

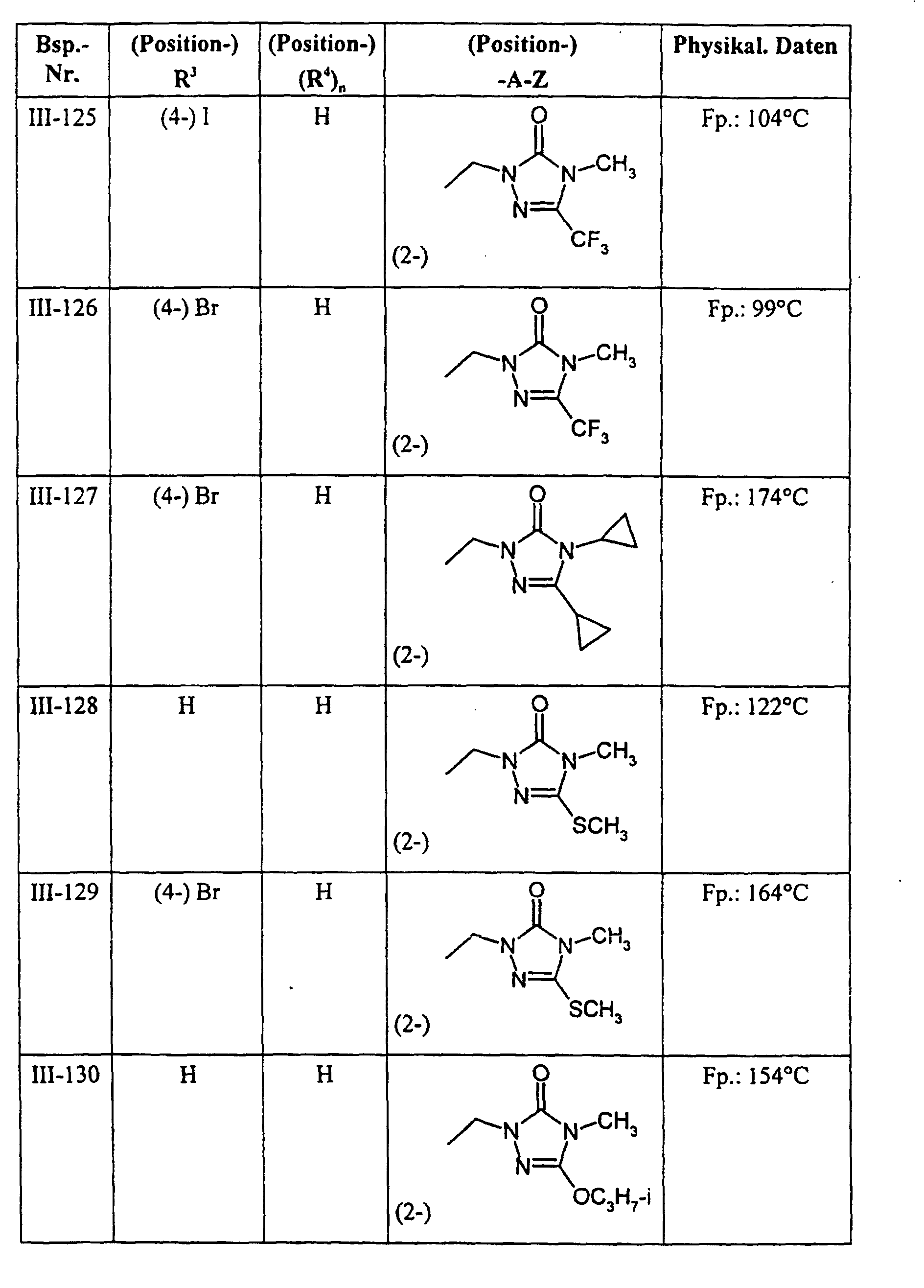

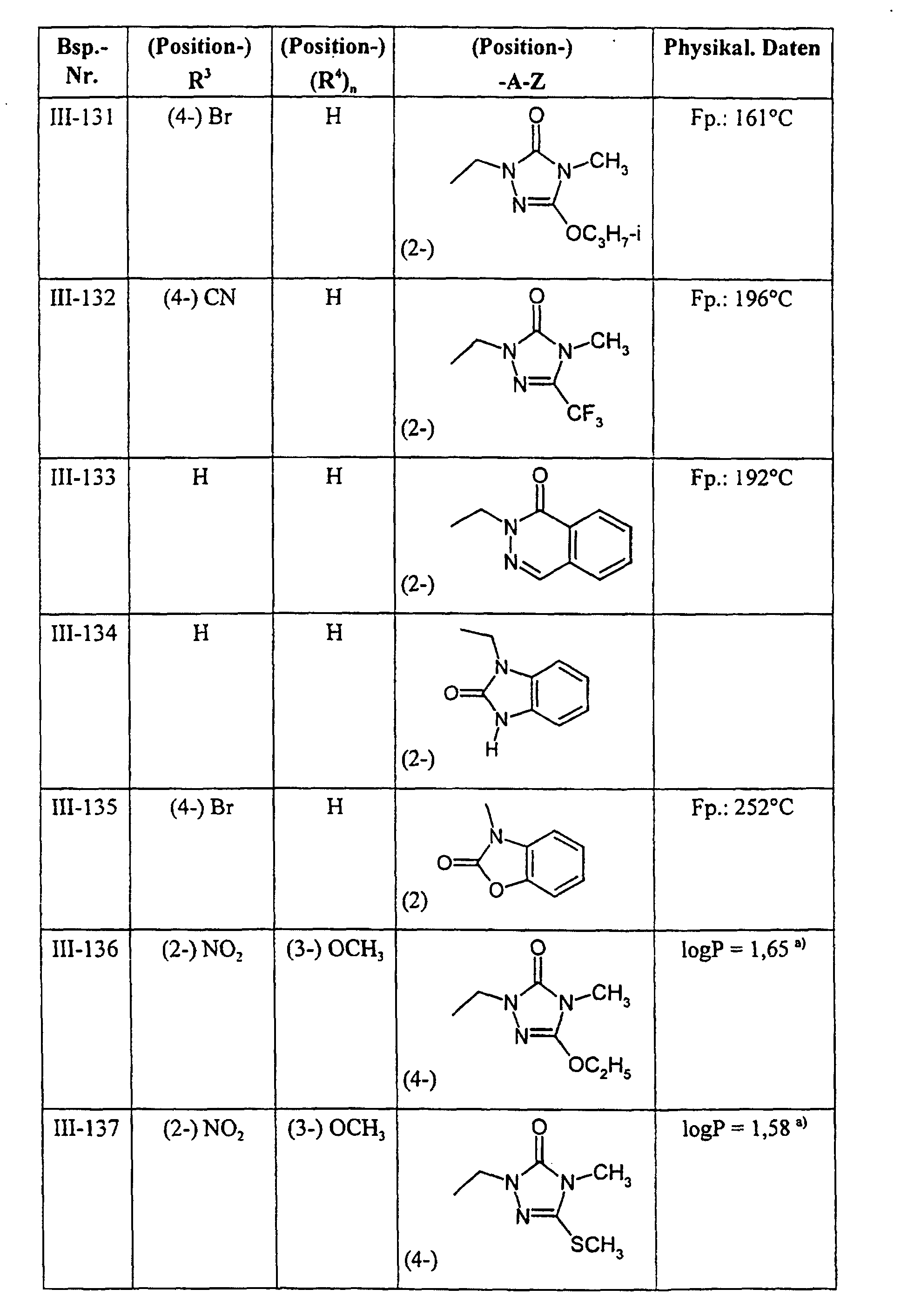

### Ausgangsstoffe der Formel (IV):

### Beispiel (IV-1)

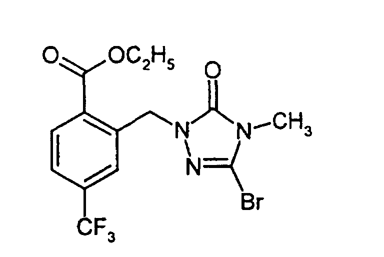

### Stufe 1

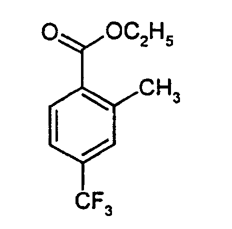

10 g (49 mMol) 2-Methyl-4-trifluormethyl-benzoesäure werden in 150 ml Ethanol gelöst und mit 1 ml konz. Schwefelsäure versetzt. Nach 24 Stunden Erhitzen unter Rückfluß wird die Lösung eingeengt, in Methylenchlorid aufgenommen und mit gesättigter wäßriger Natriumhydrogencarbonat-Lösung extrahiert. Die Methylenchlorid-Phase wird über Natriumsulfat getrocknet und im Wasserstrahlvakuum eingeengt.

Man erhält 9 g (80% der Theorie) 2-Methyl-4-trifluormethyl-benzoesäure-ethylester als amorphen Rückstand.

### Stufe 2

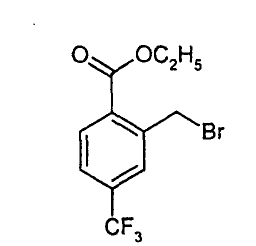

9 g (39 mMol) 2-Methyl-4-trifluormethyl-benzoesäure-ethylester werden in 200 ml Tetrachlormethan gelöst und mit 7 g (39 mMol) *N*-Brom-succinimid und 0,1 g Dibenzoylperoxid versetzt. Nach 6 Stunden Erhitzen unter Rückfluß wird das abgeschiedene Succinimid abfiltriert und das Filtrat im Wasserstrahlvakuum eingeengt.

Man erhält 12 g eines amorphen Rückstandes, der neben 2-Brommethyl-4-trifluormethyl-benzoesäure-ethylester noch 17% 2,2-Dibrommethyl-4-trifluormethyl-benzoesäure-ethylester und 12% 2-Methyl-4-trifluormethyl-benzoesäure-ethylester enthält.

### Stufe 3

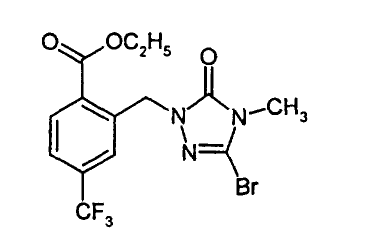

4 g 2-Brommethyl-4-trifluormethyl-benzoesäure-ethylester (ca. 70%ig) und 2.28 g (12,8 mMol) 5-Brom-4-methyl-2,4-dihydro-3H-1,2,4-triazol-3-on werden in 150 ml Acetonitril gelöst, mit 5,3 g (38,4 mMol) Kaliumcarbonat versetzt und unter kräftigem Rühren 2 Stunden zum Rückfluß erhitzt. Das Reaktionsgemisch wird in Wasser aufgenommen und mit Methylenchlorid mehrfach extrahiert. Die gesammelten Methylenchlorid-Phasen werden über Natriumsulfat getrocknet, im Wasserstrahlvakuum eingeengt und chromatographiert.

Man erhält 2 g (38 % der Theorie) 5-Brom-4-methyl-2-(2-ethoxycarbonyl-5-trifluormethyl-benzyl)-2,4-dihydro-3H-1,2,4-triazol-3-on als amorphes Produkt.
¹H-NMR (CDCl₃, δ): 5,46 ppm.

### Beispiel (IV-2)

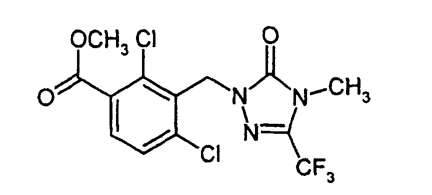

6,7 g (40 mMol) 4-Methyl-5-trifluormethyl-2,4-dihydro-3H-1,2,4-triazol-3-on werden in 150 ml Acetonitril vorgelegt und mit 11 g (80 mMol) Kaliumcarbonat verrührt. Nach Erwärmen der Mischung auf 50°C wird dann eine Lösung von 13,1 g (44 mMol) 3-Brommethyl-2,4-dichlor-benzoesäure-methylester in 20 ml Acetonitril unter Rühren tropfenweise dazu gegeben und die Reaktionsmischung wird noch 15 Stunden unter Rühren zum Rückfluß erhitzt. Anschließend wird im Wasserstrahlvakuum eingeengt, der Rückstand in Methylenchlorid aufgenommen, mit 1N-Salzsäure gewaschen, mit Natriumsulfat getrocknet und filtriert. Das Filtrat wird unter vermindertem Druck eingeengt, der Rückstand mit Petrolether digeriert und das kristallin angefallene Produkt durch Absaugen isoliert.

Man erhält 14,9 g (97% der Theorie) 4-Methyl-5-trifluormethyl-2-(2,6-dichlor-3-methoxycarbonyl-benzyl)-2,4-dihydro-3H-1,2,4-triazol-3-on vom Schmelzpunkt 109°C.

Analog zu den Beispielen (IV-1) und (IV-2) können beispielsweise auch die in der nachstehenden Tabelle 3 aufgeführten Verbindungen der allgemeinen Formel (IVa) hergestellt werden.

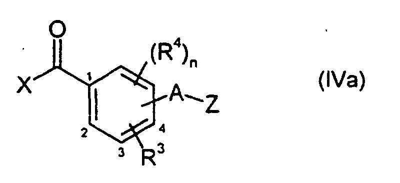

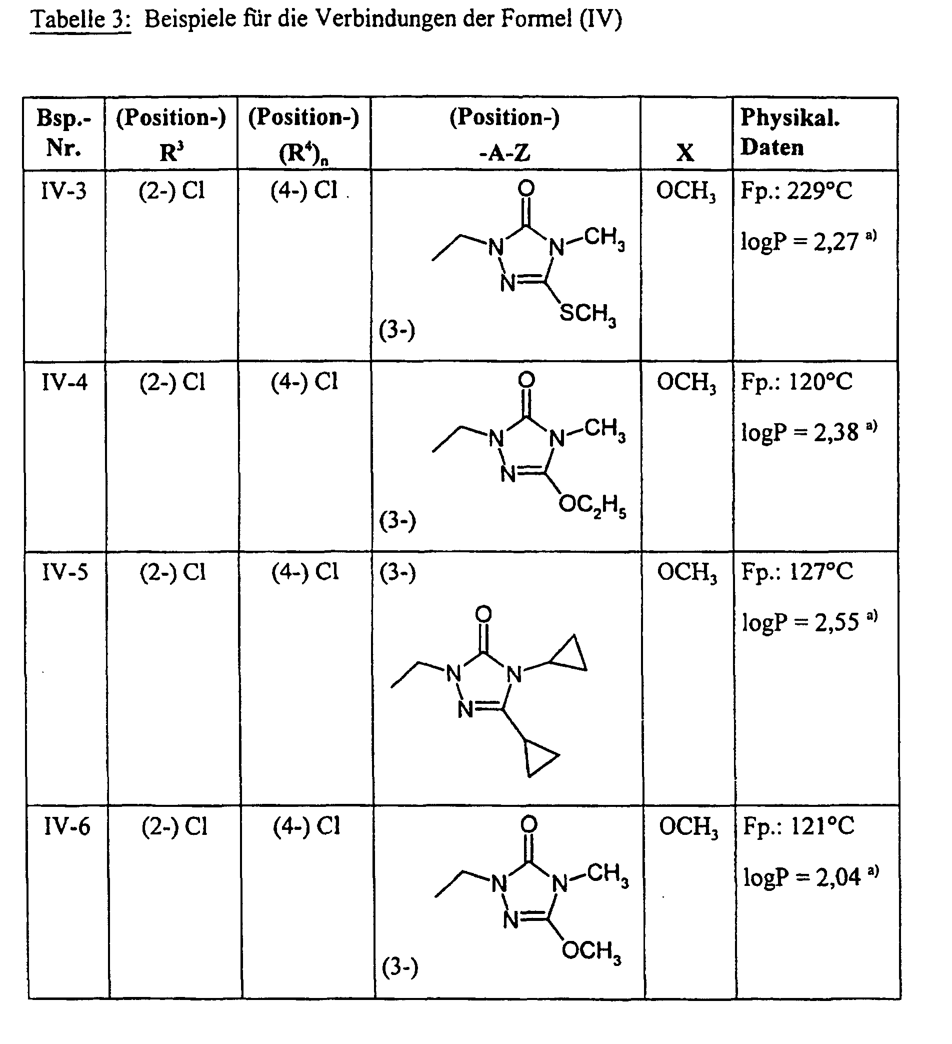

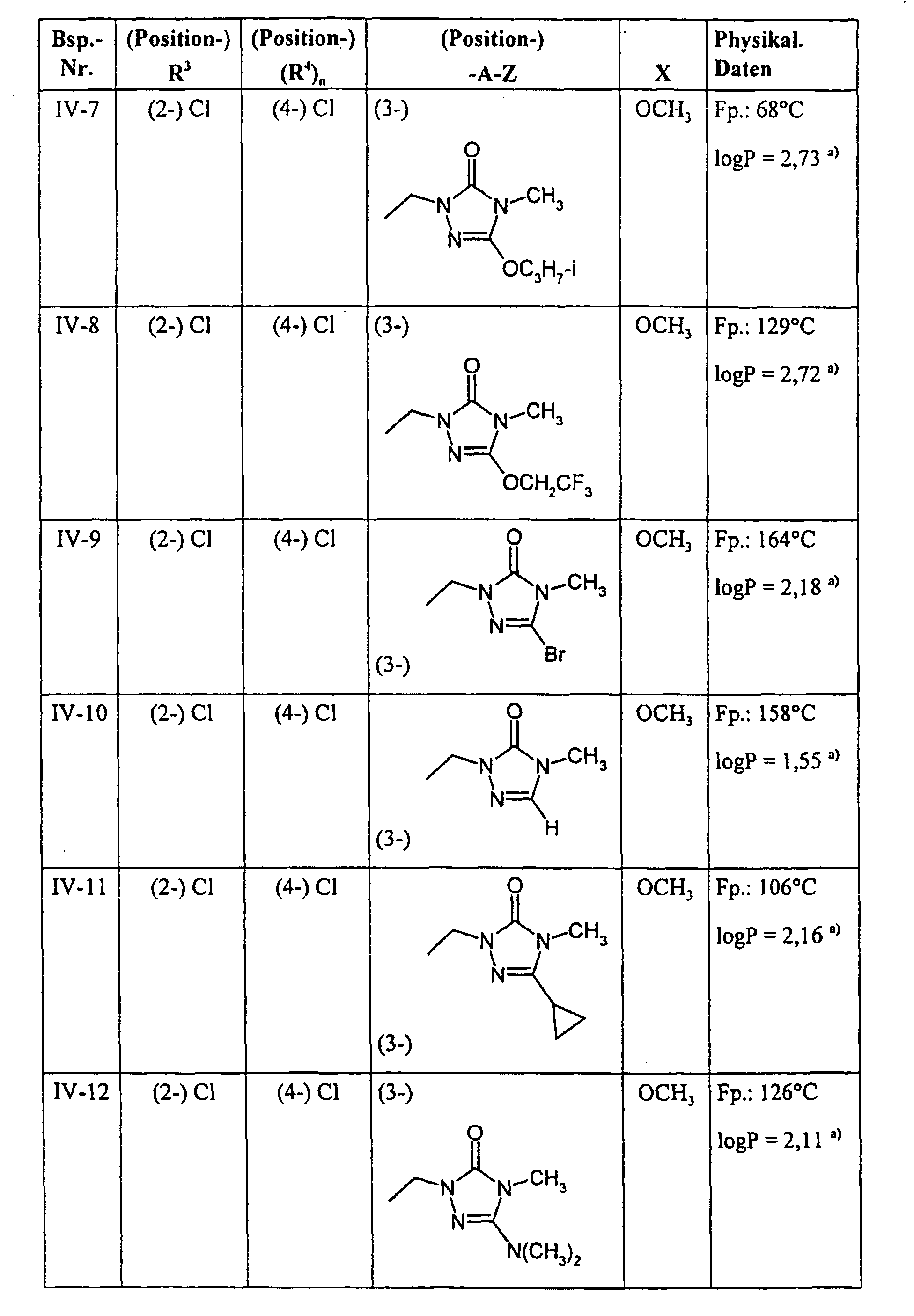

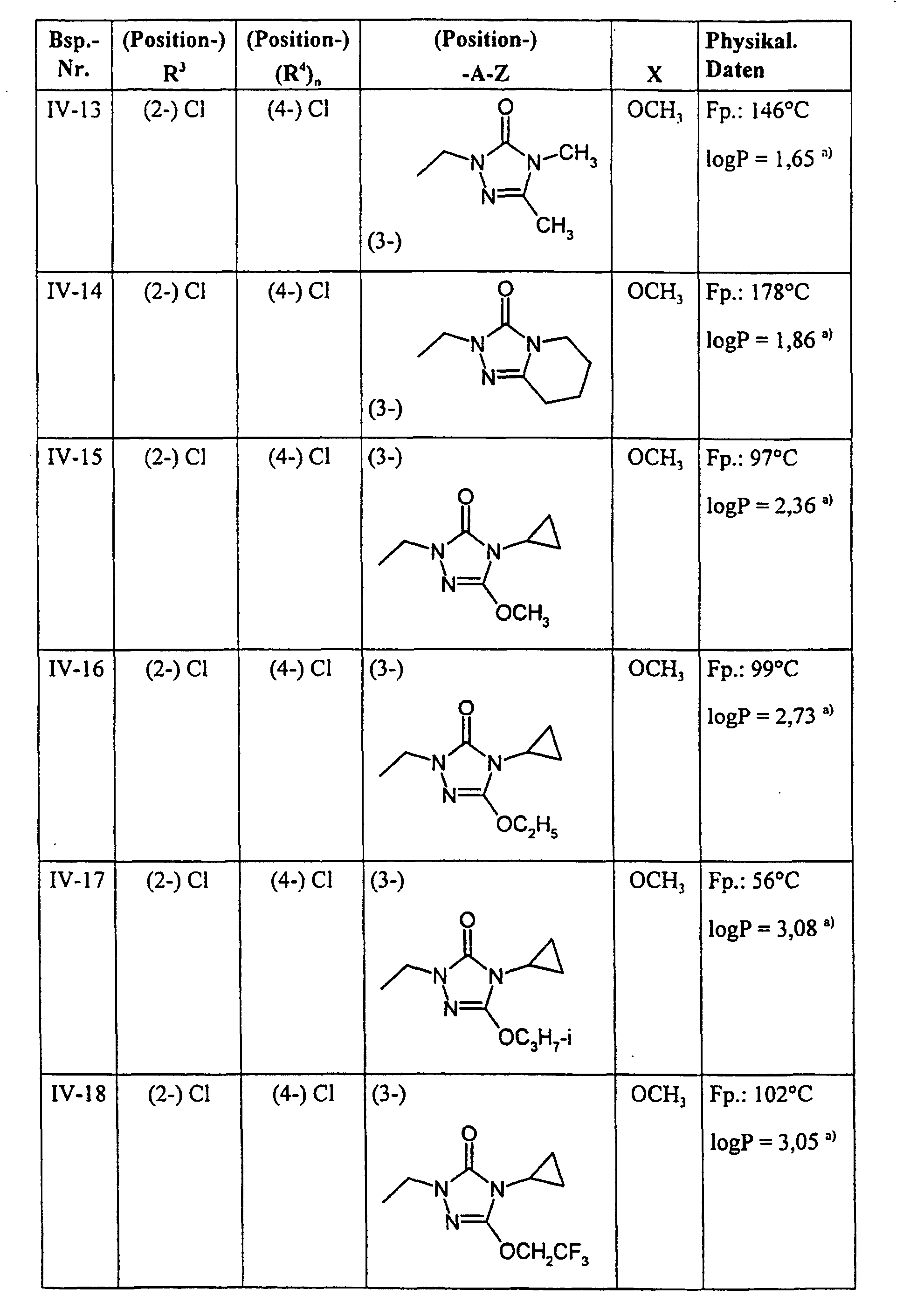

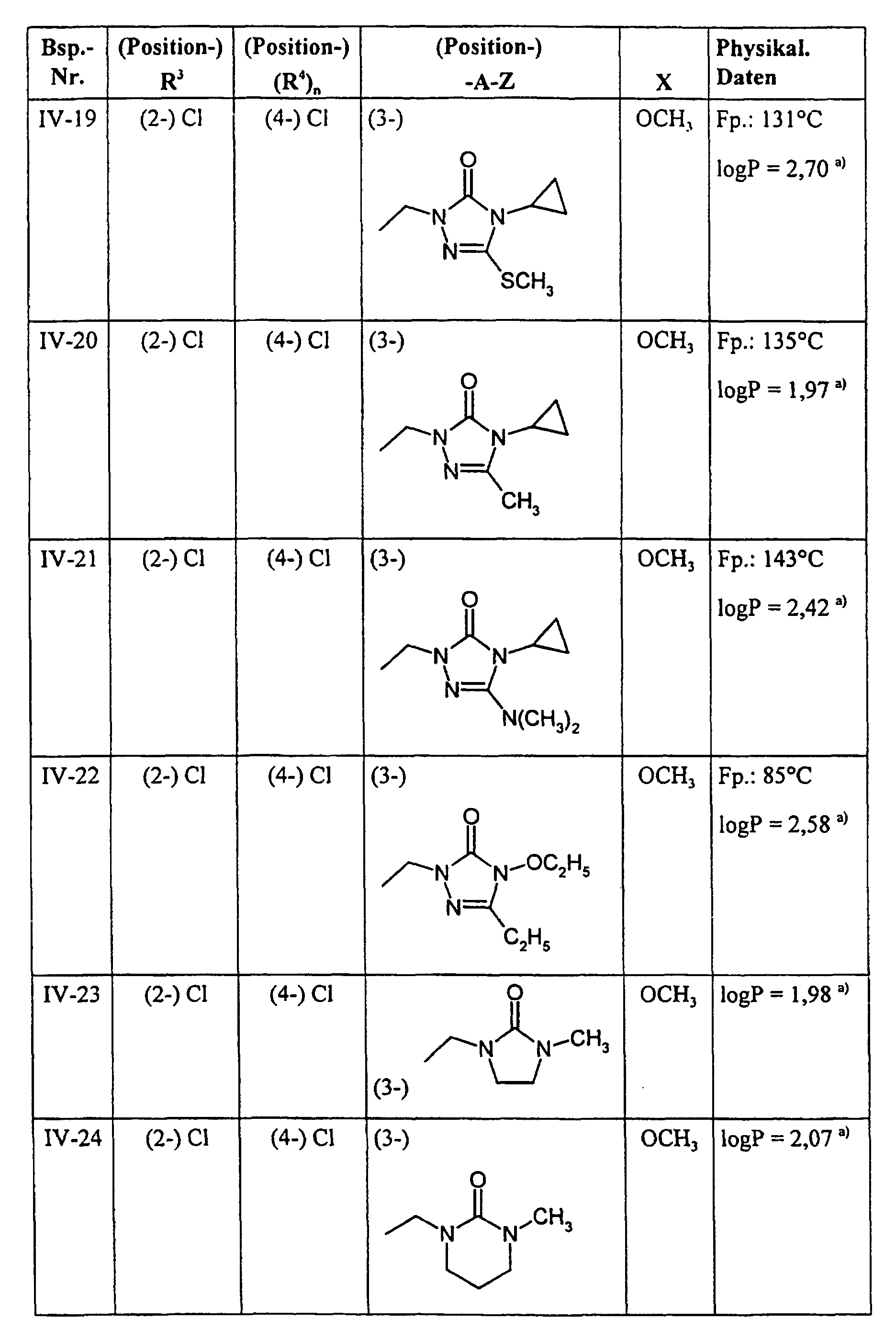

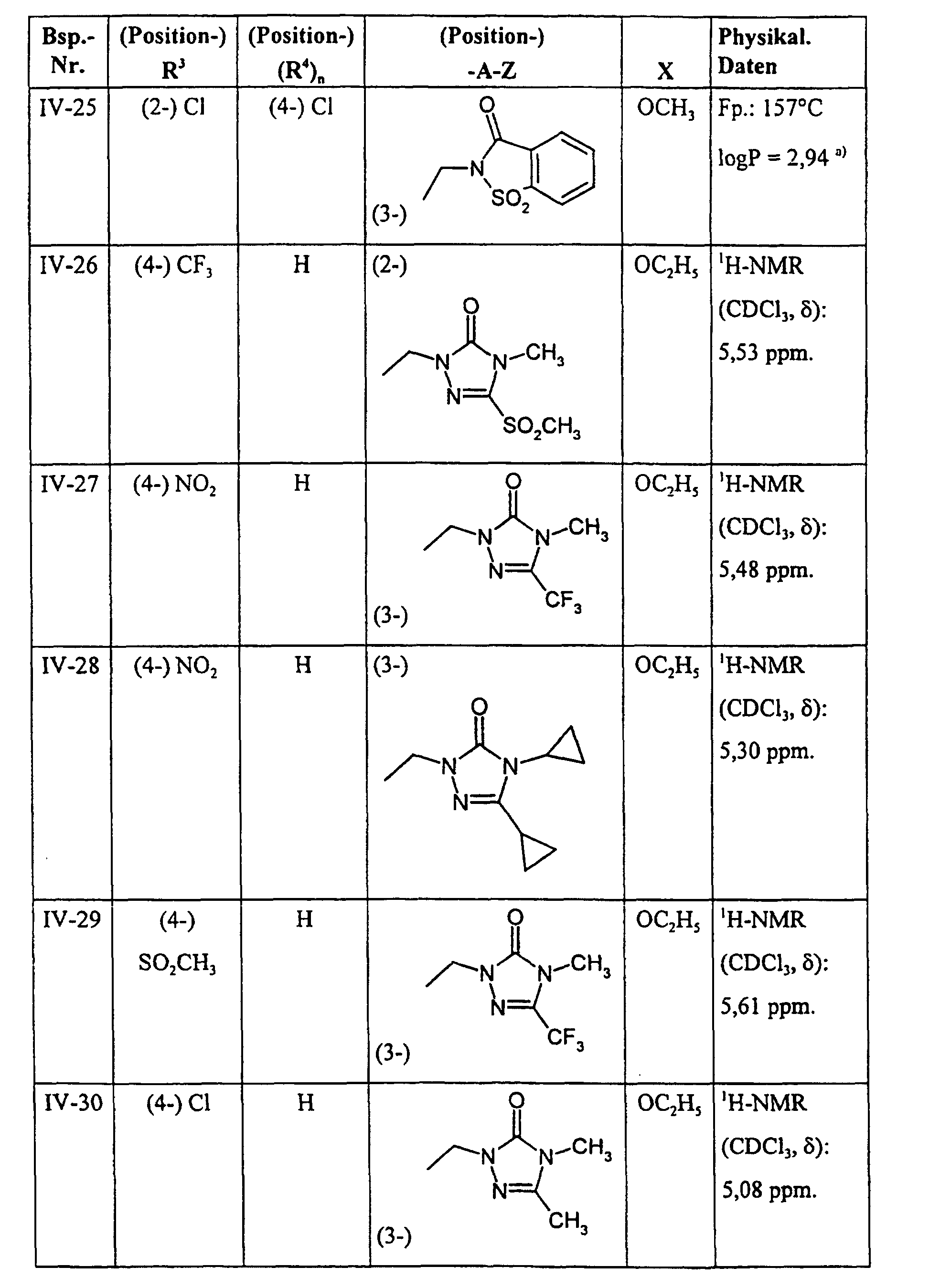

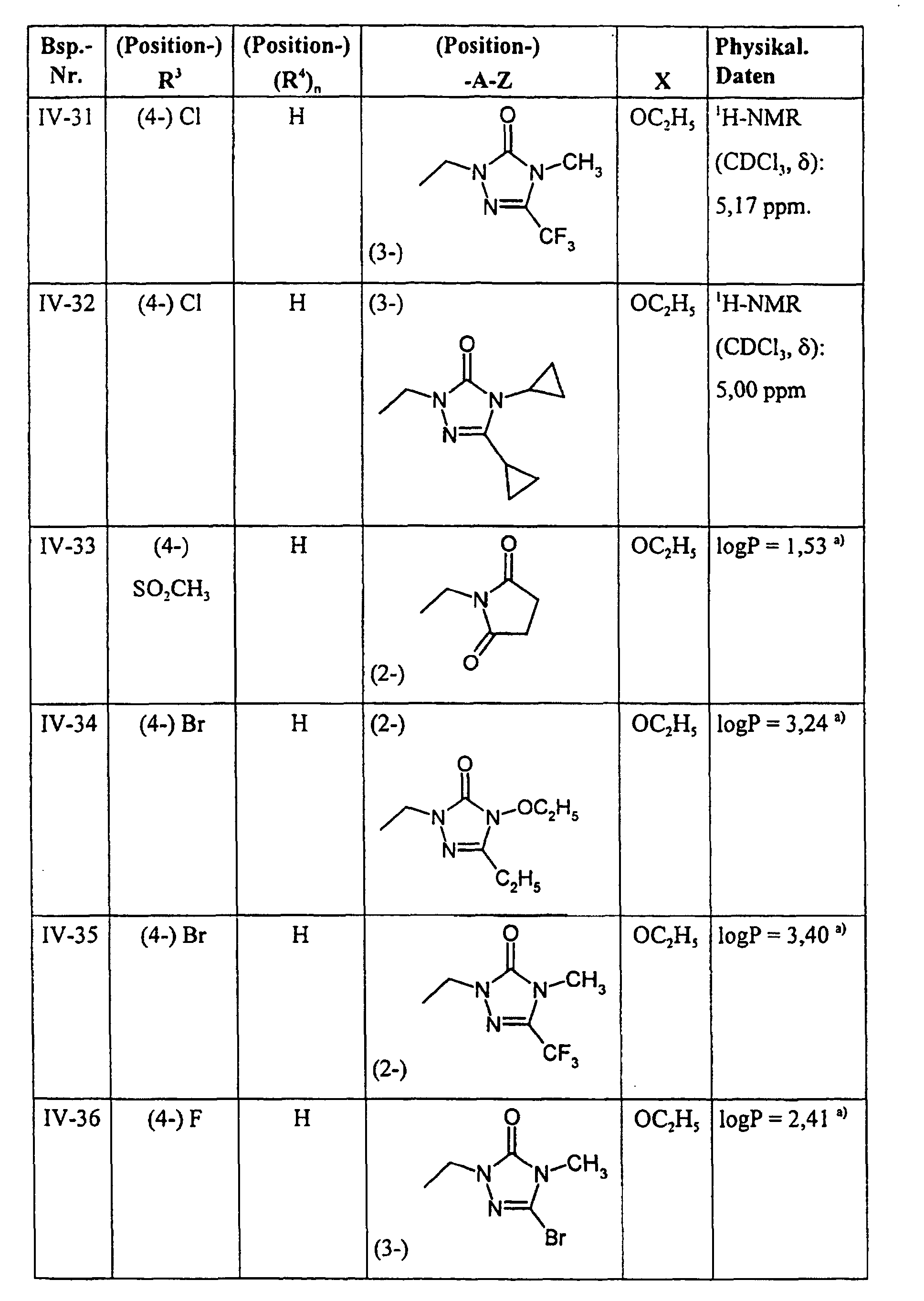

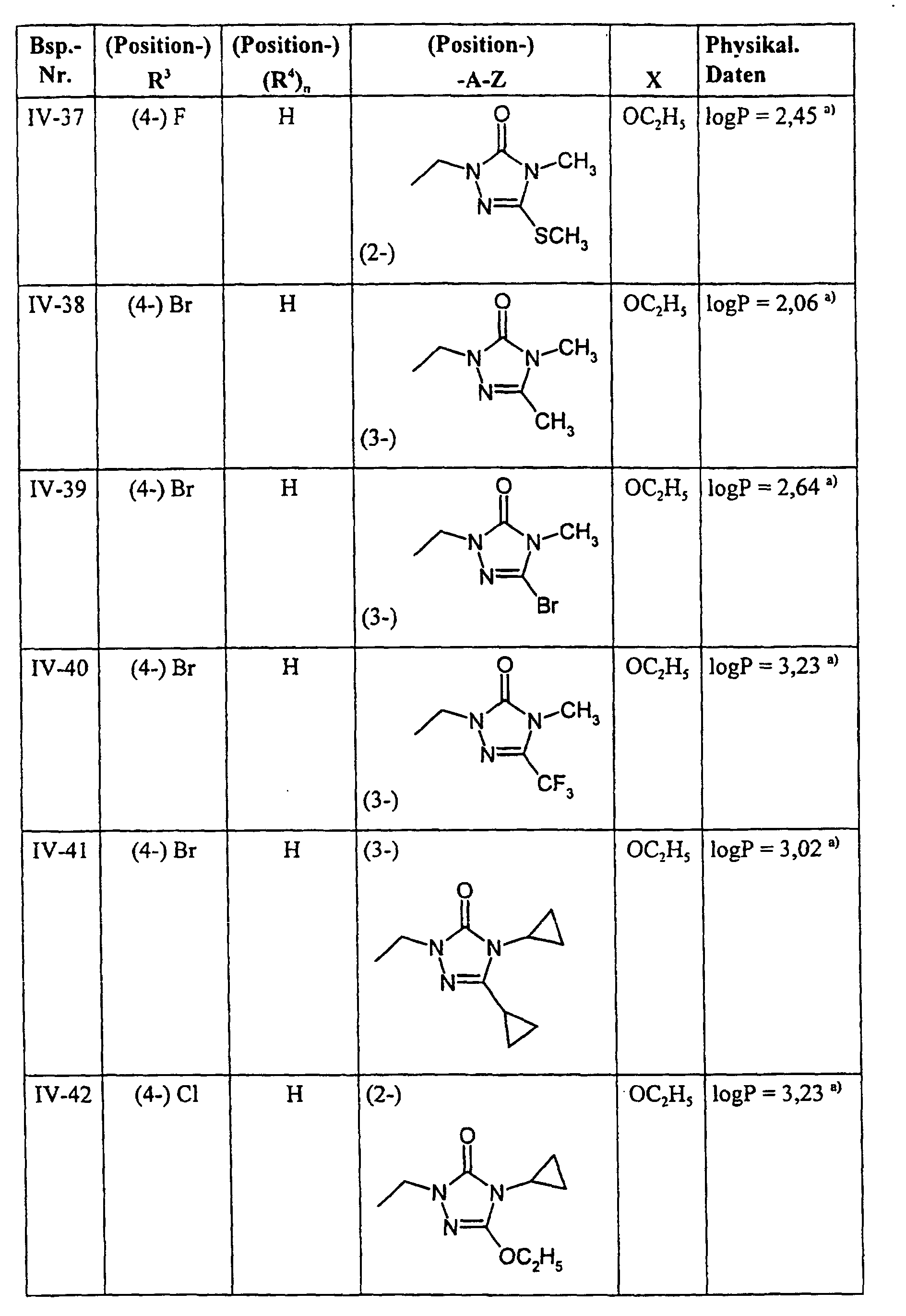

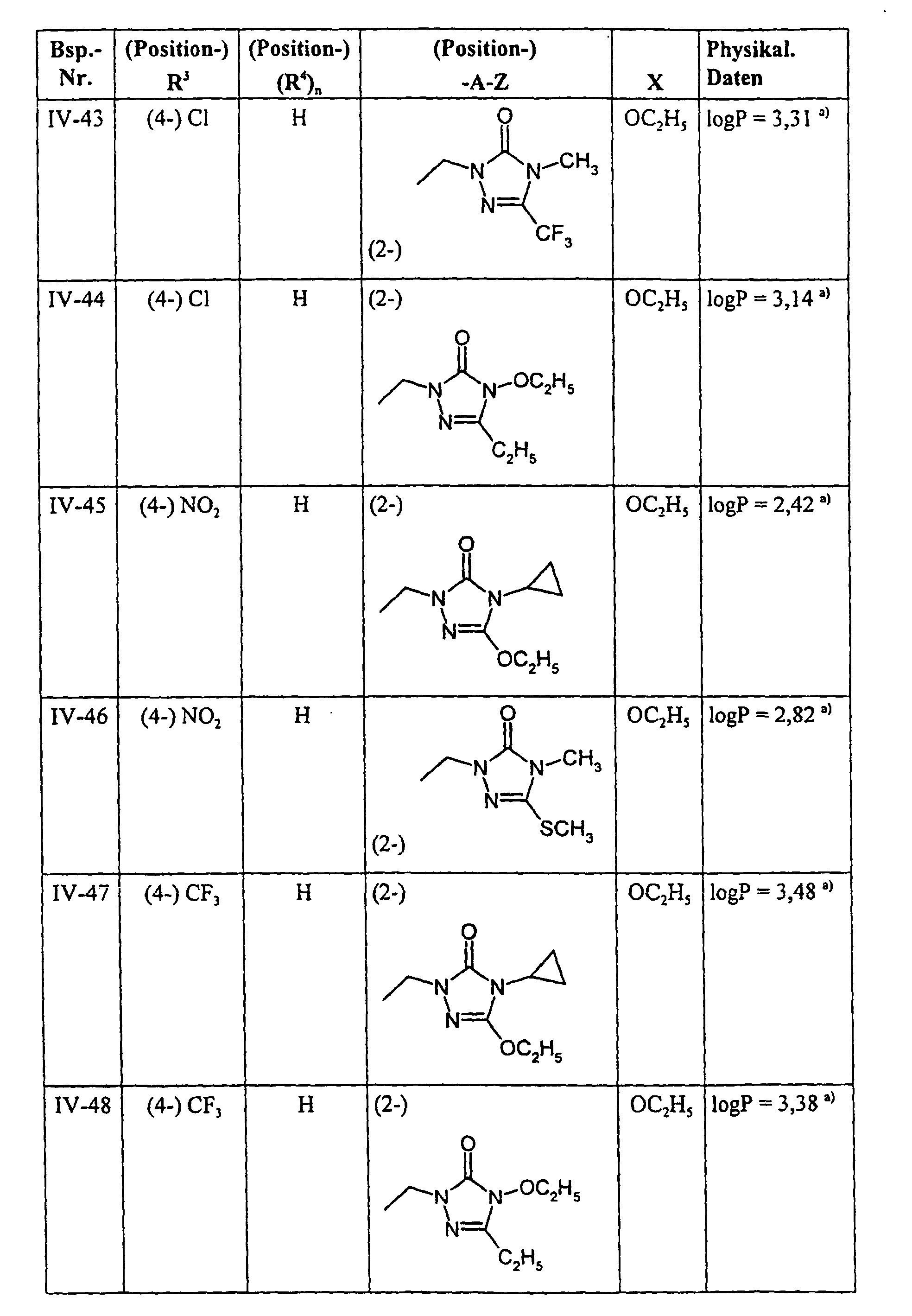

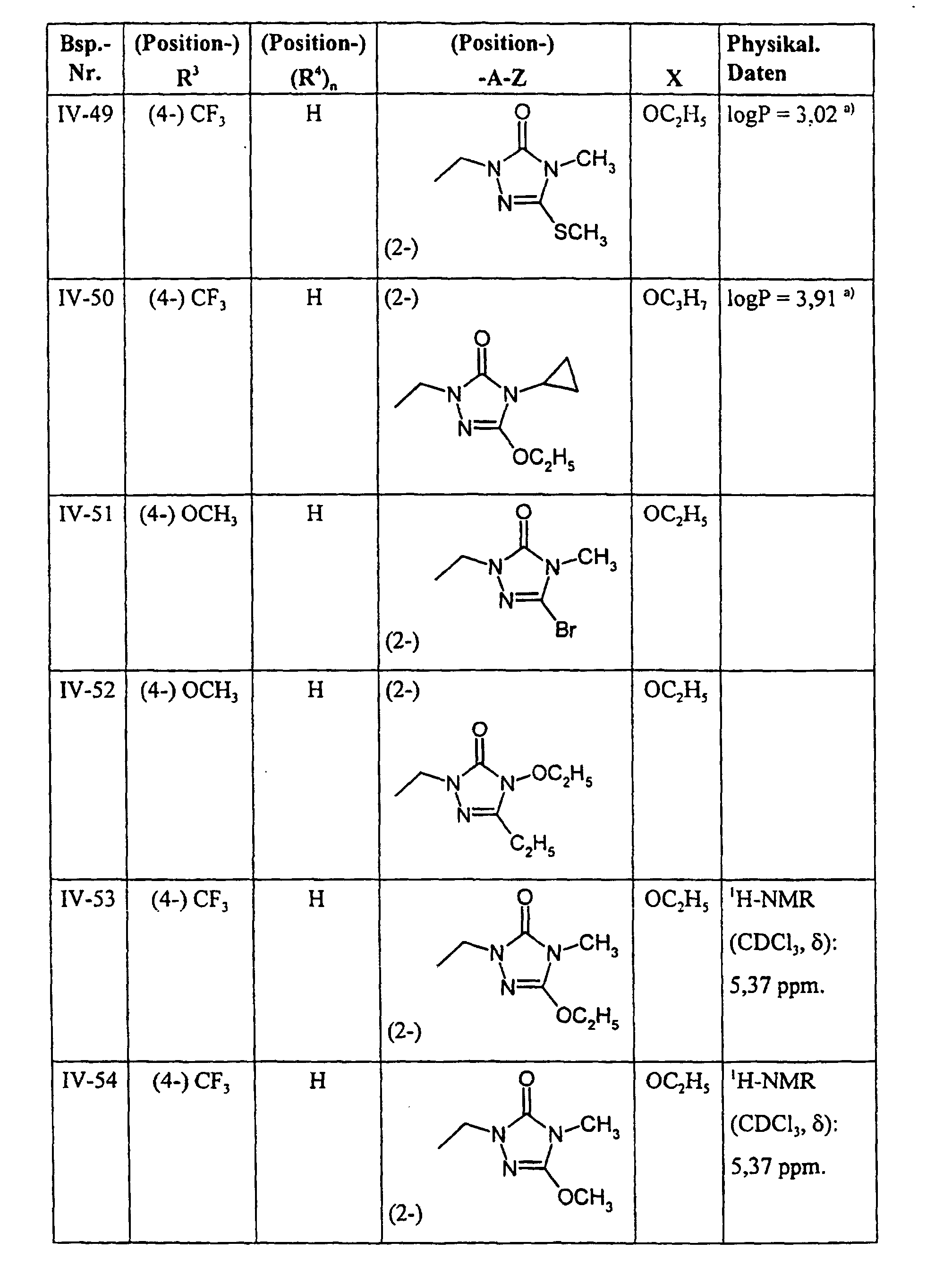

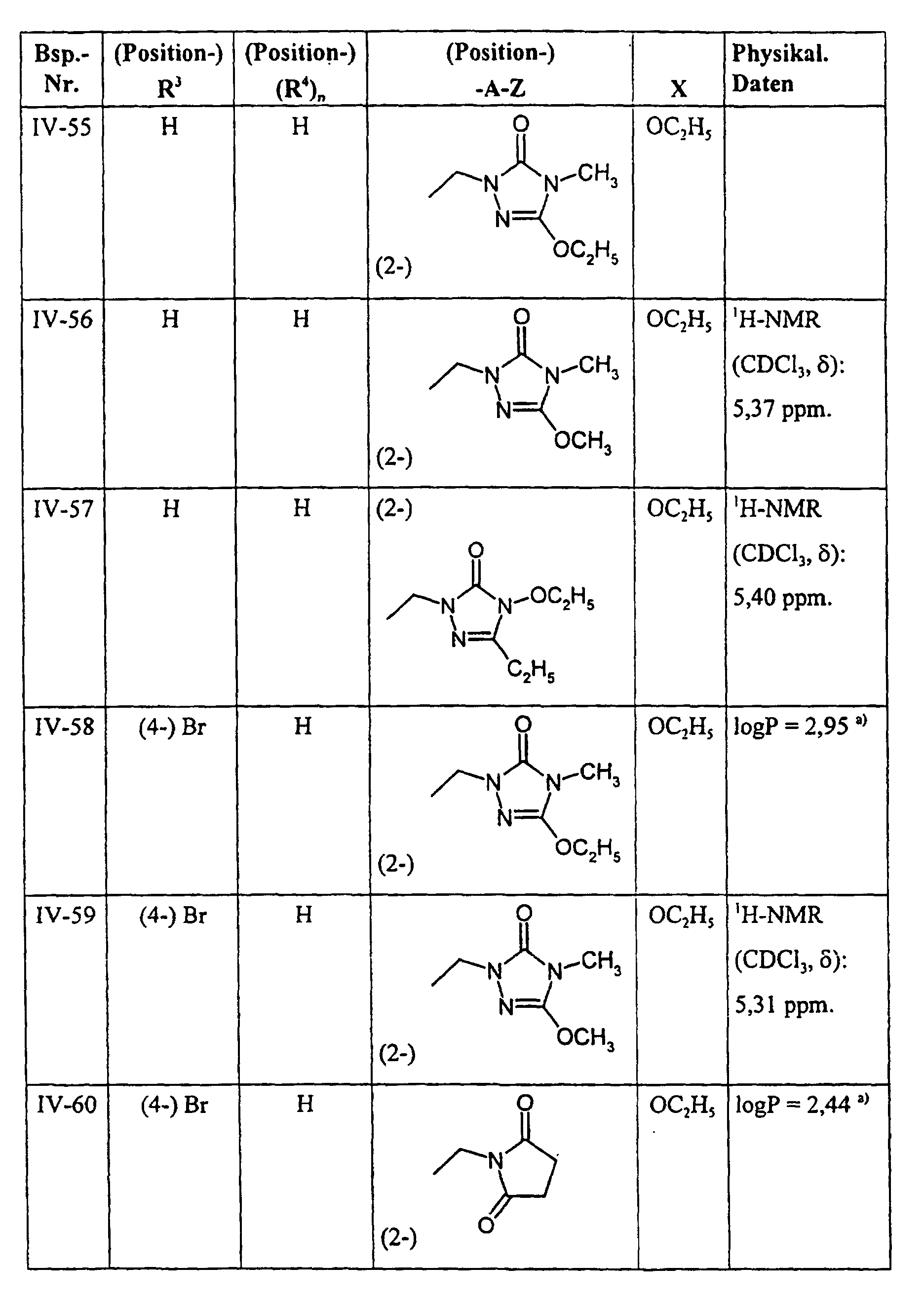

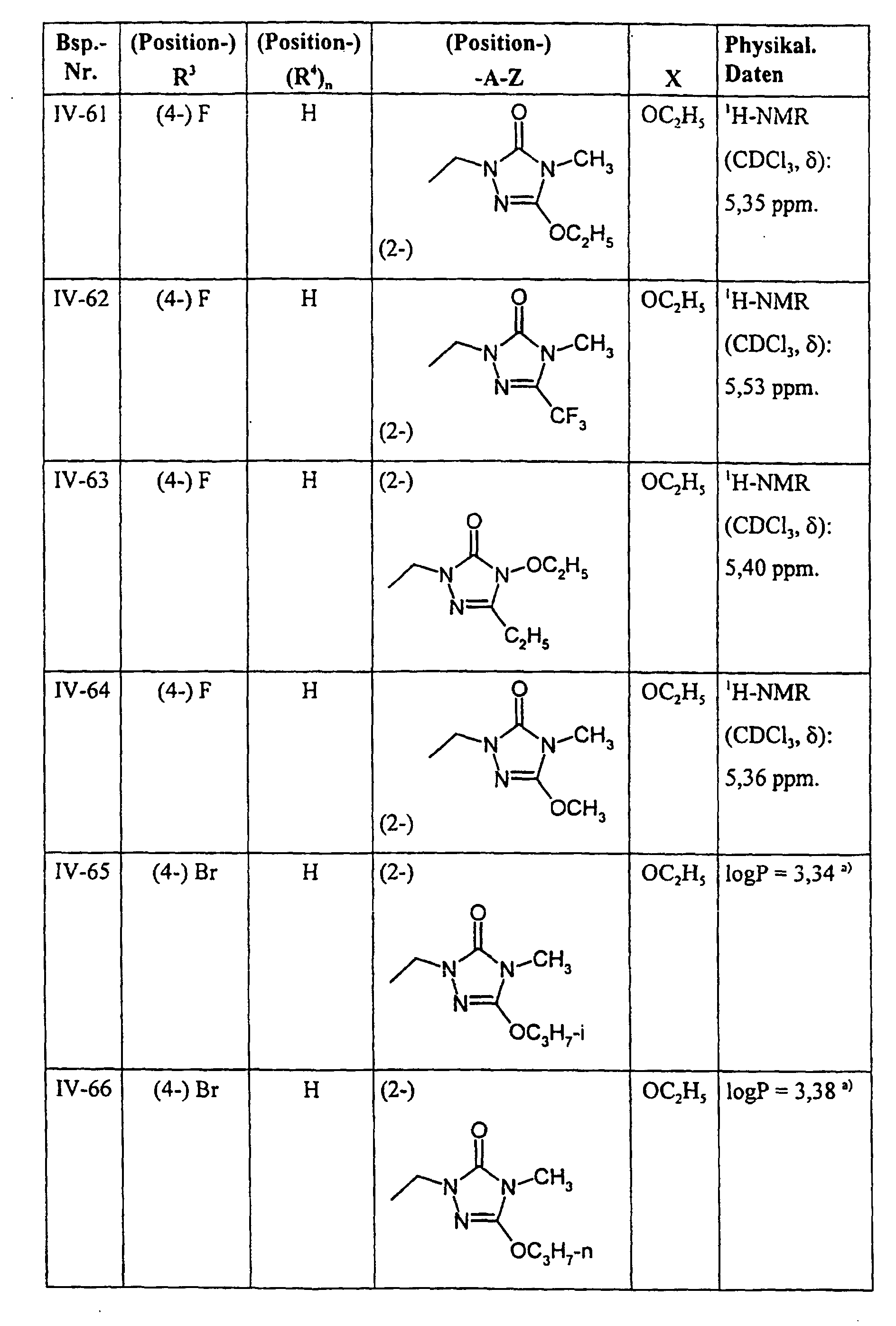

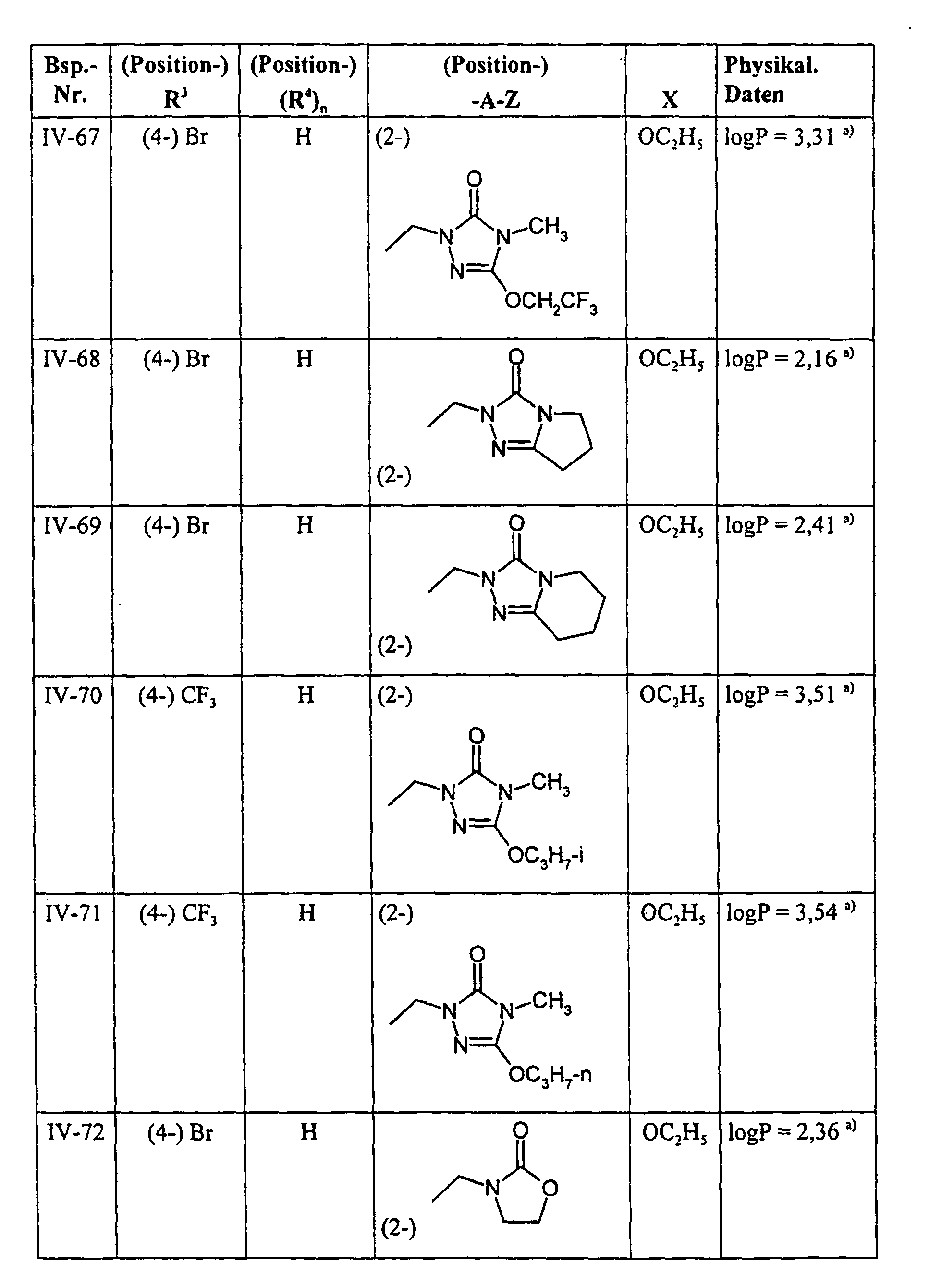

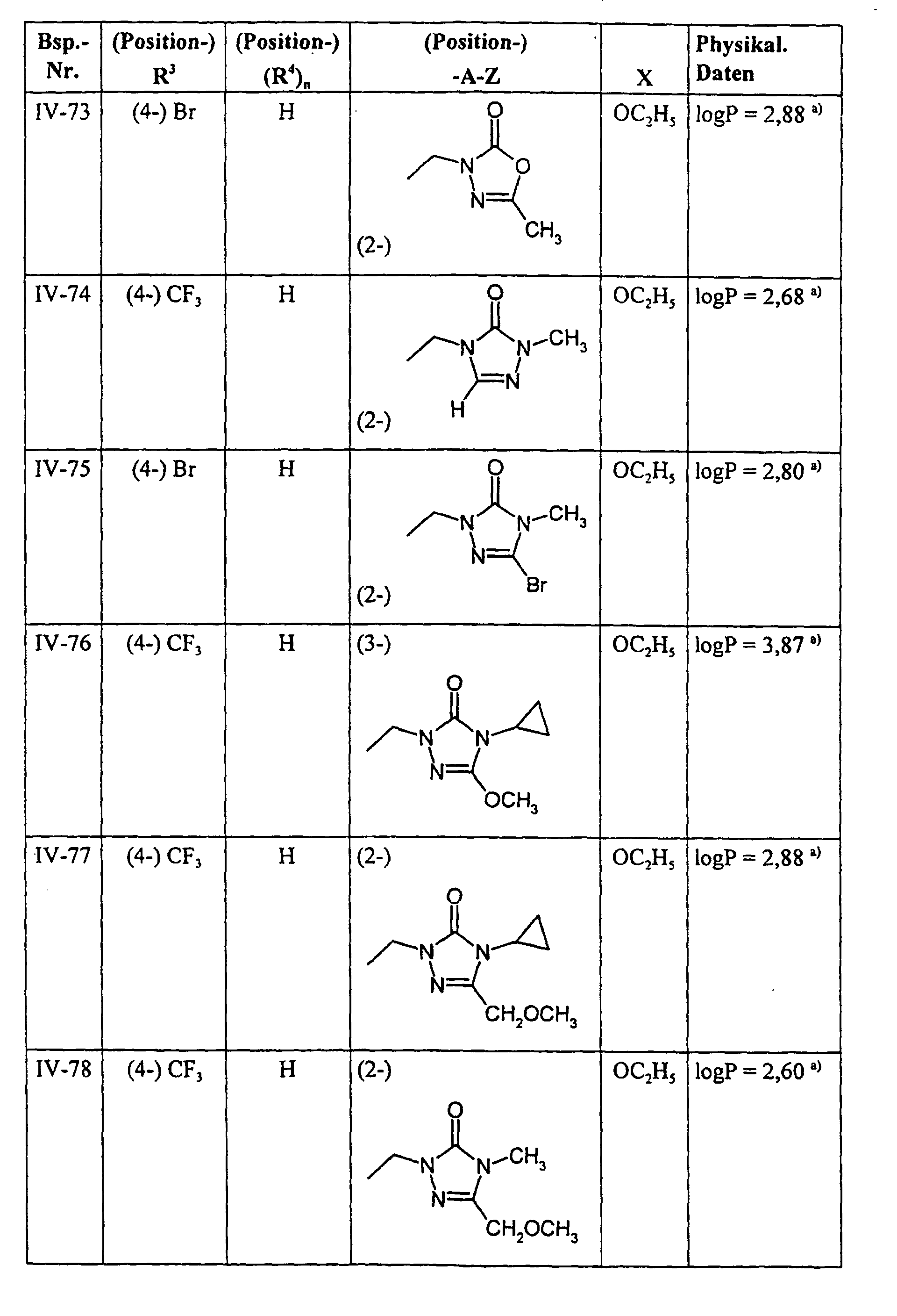

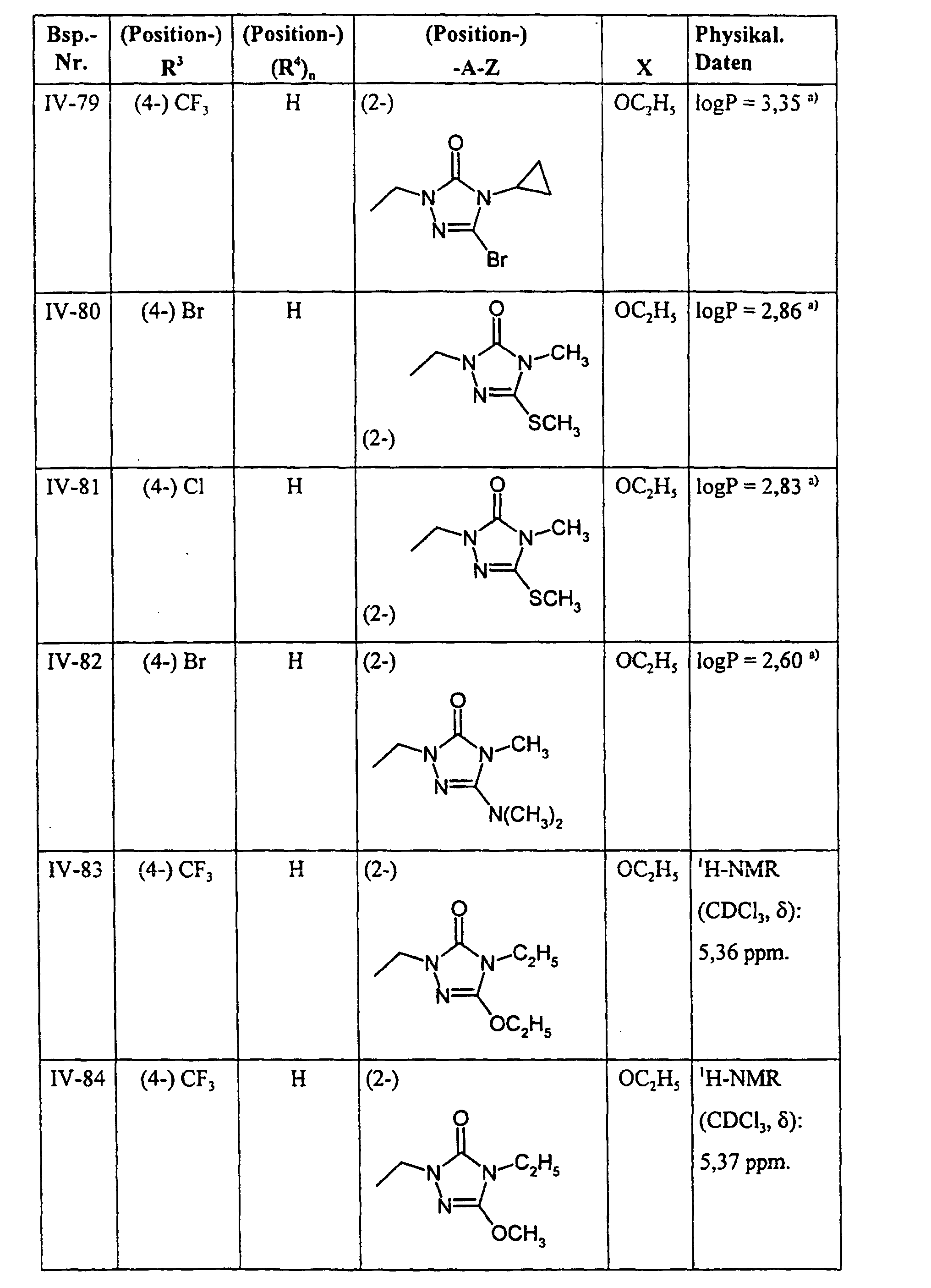

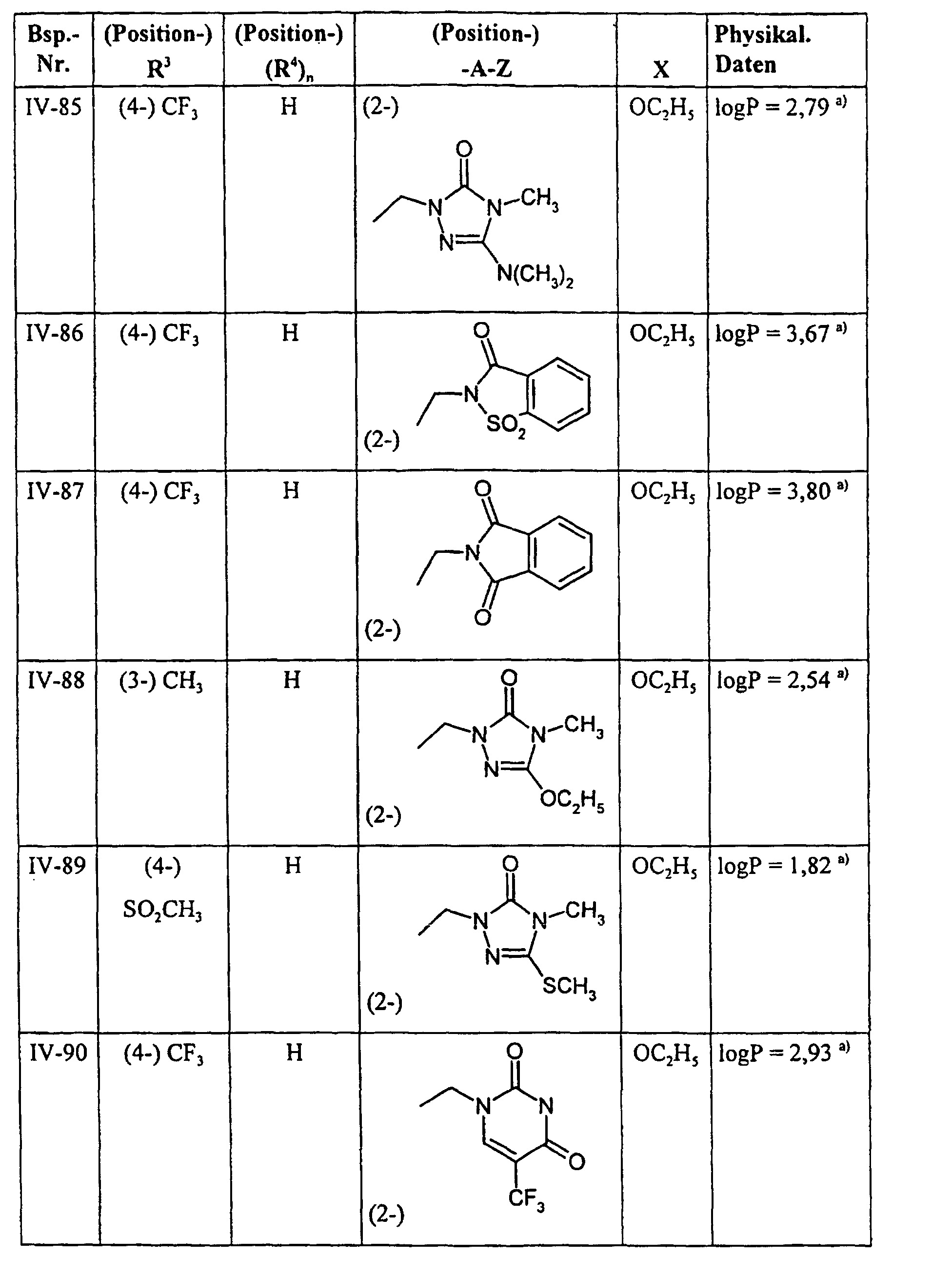

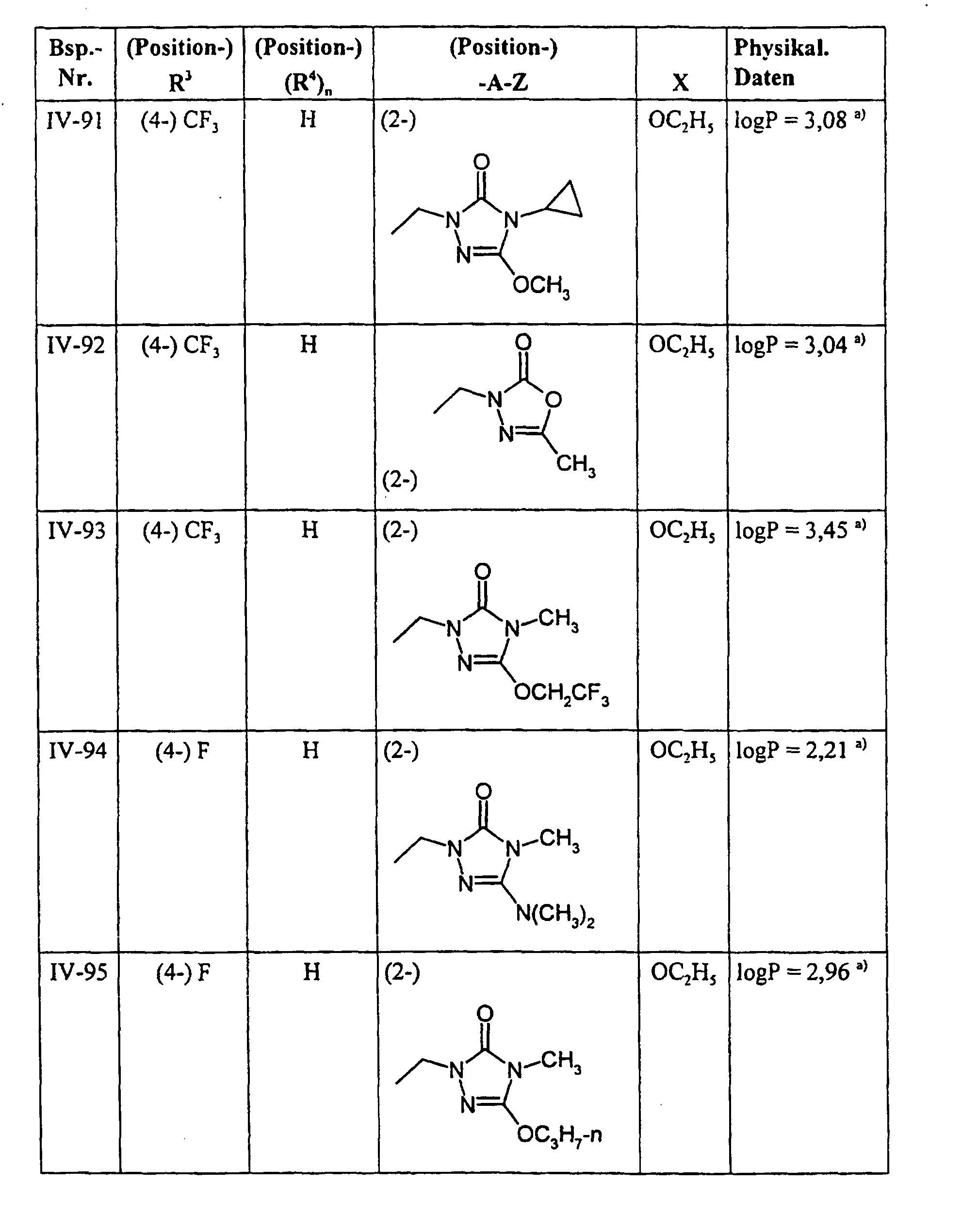

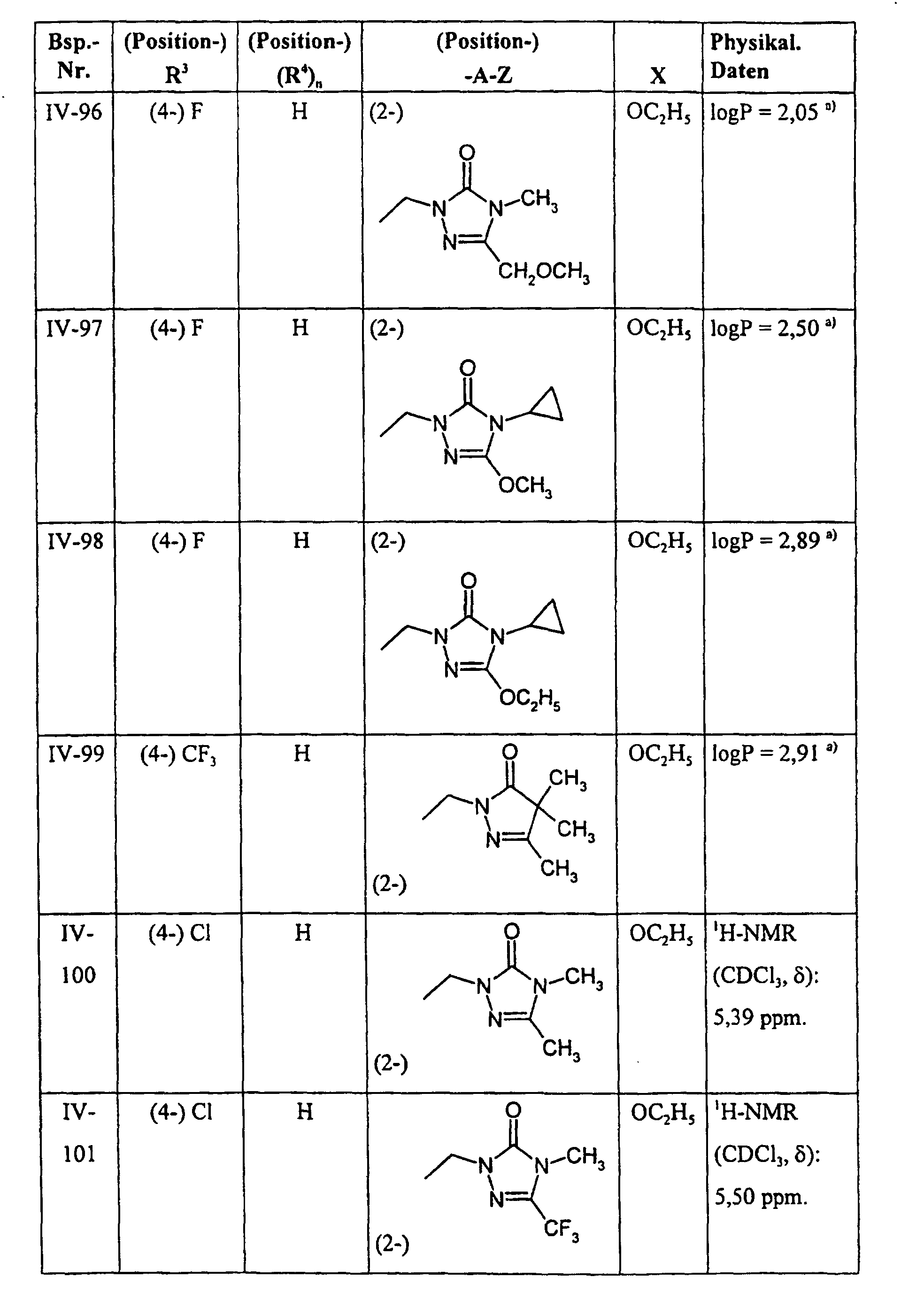

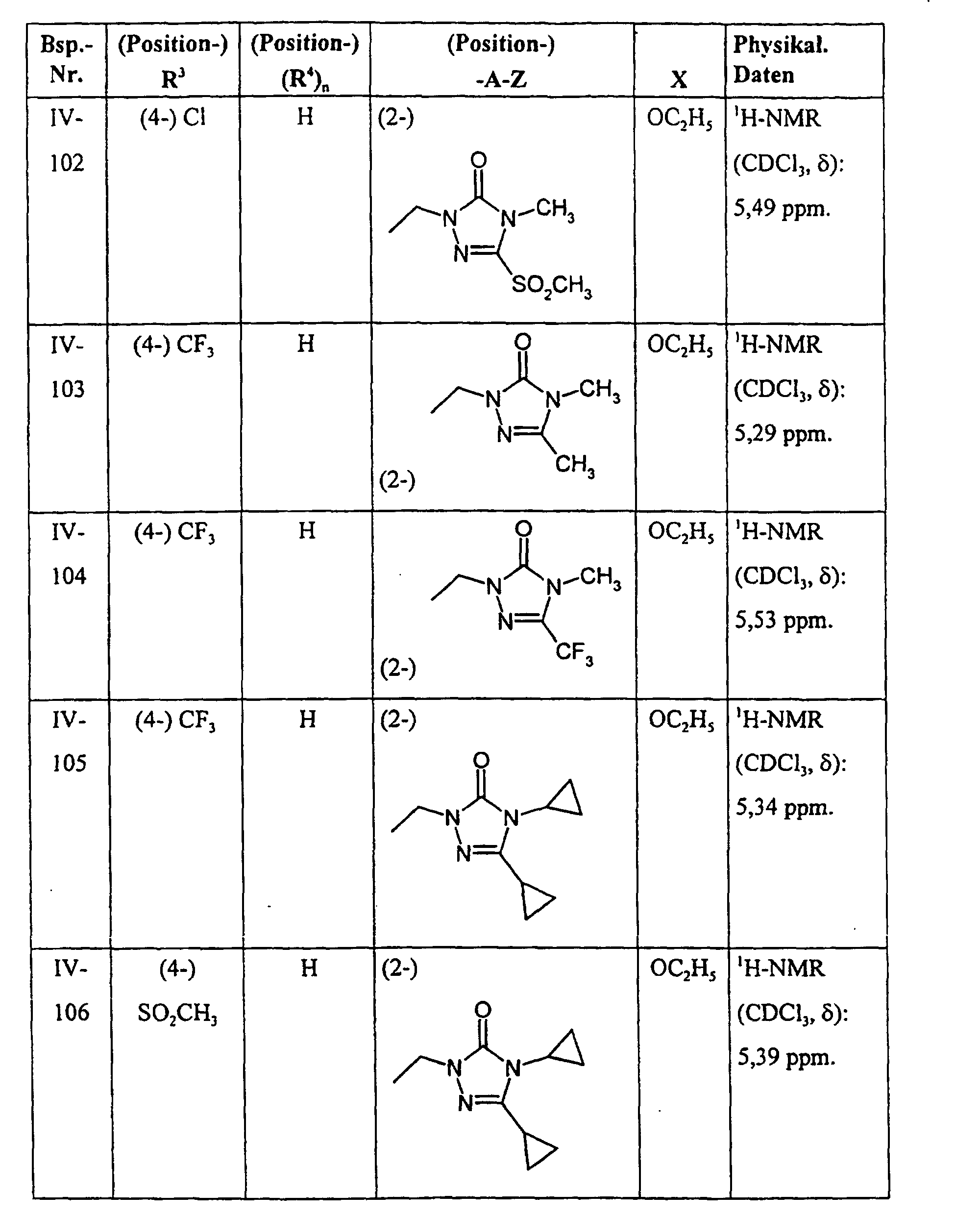

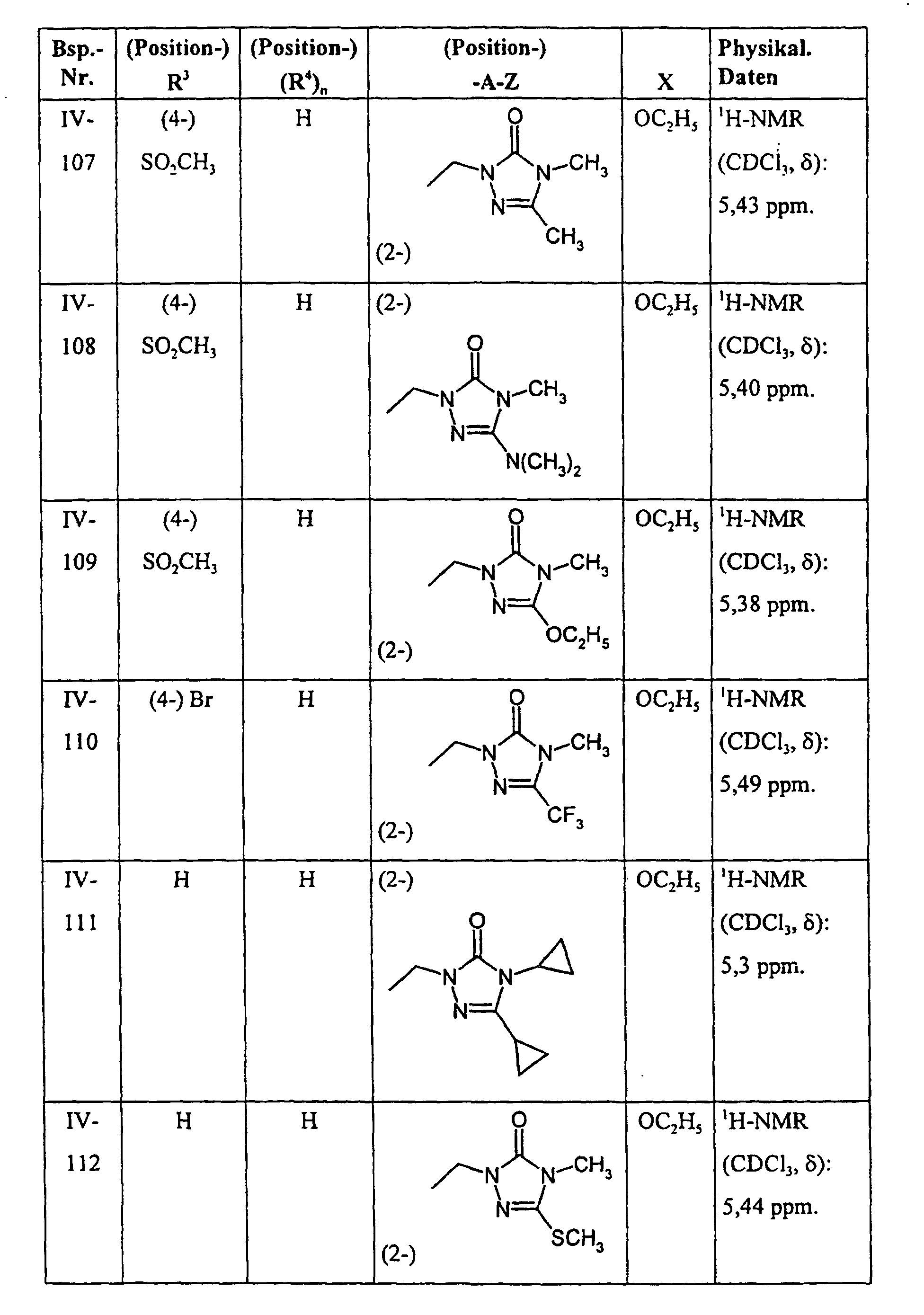

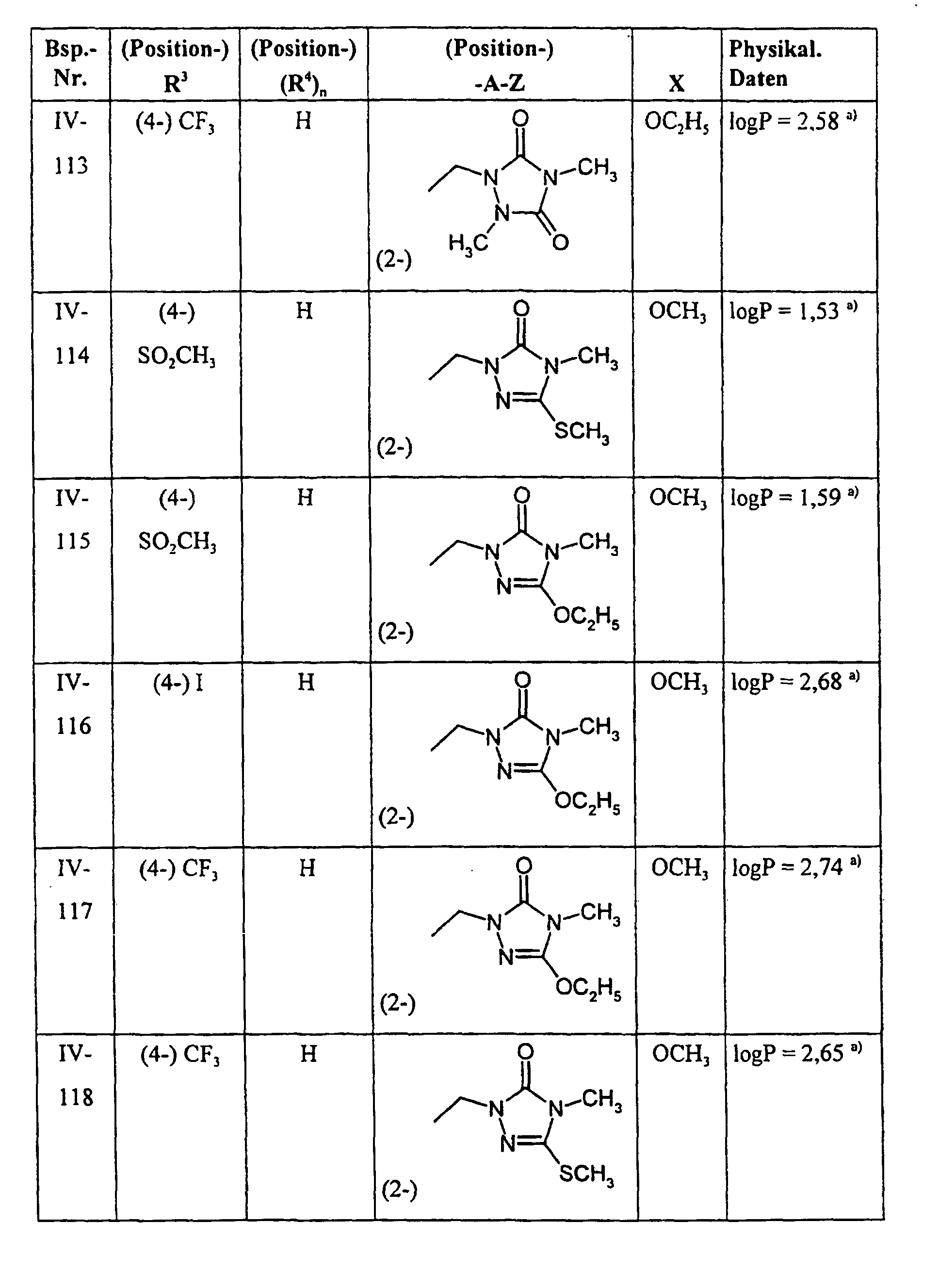

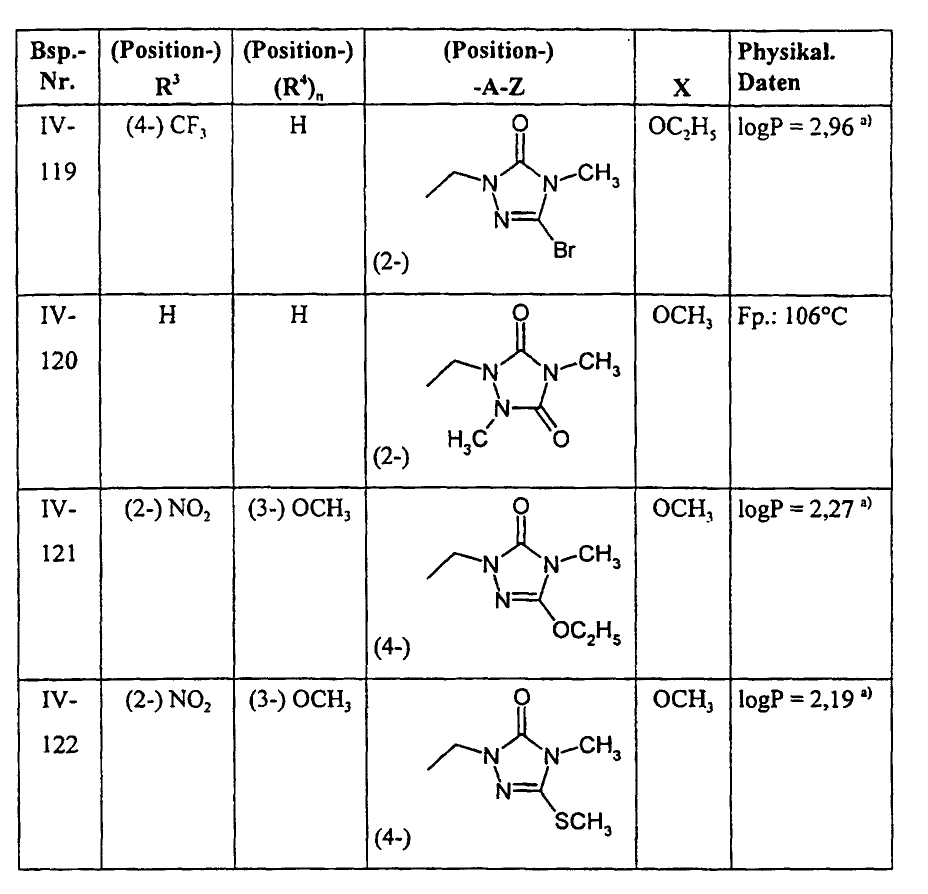

Die Bestimmung der in den Tabellen angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1 % wässrige Phosphorsäure, Acetonitril; linearer Gradient von 10 % Acetonitril bis 90 % Acetonitril - entsprechende Messergebnisse sind in den Tabellen mit ^{a)} markiert.
(b) Eluenten für die Bestimmung im neutralen Bereich: 0.01-molare wässrige Phosphatpuffer-Lösung, Acetonitril; linearer Gradient von 10 % Acetonitril bis 90 % Acetonitril - entsprechende Messergebnisse sind in den Tabellen mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, daß die jeweils gewünschte Wirkstoffmenge pro Flächeneinheit ausgebracht wird. Die Konzentration der Spritzbrühe wird so gewählt, daß in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
- 0 % =: keine Wirkung (wie unbehandelte Kontrolle)
- 100 % =: totale Vernichtung

In diesem Test zeigen beispielsweise die Verbindungen gemäß Herstellungsbeispiel 1 und 10 bei teilweise guter Verträglichkeit gegenüber Kulturpflanzen, wie z.B. Mais, starke Wirkung gegen Unkräuter.

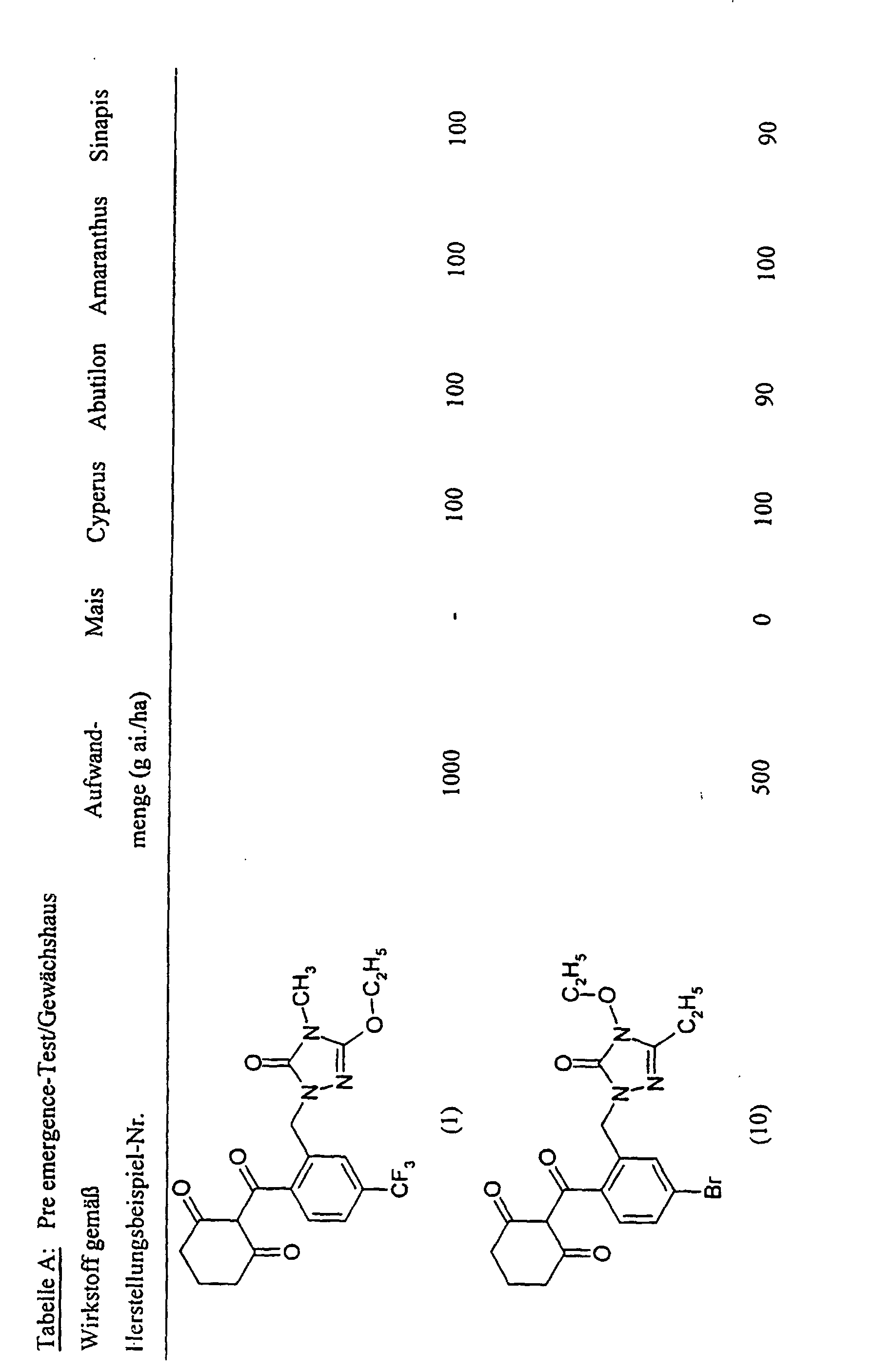

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden.
Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 10 und 15 bei teilweise guter Verträglichkeit gegenüber Kulturpflanzen, wie z.B. Mais, starke Wirkung gegen Unkräuter.

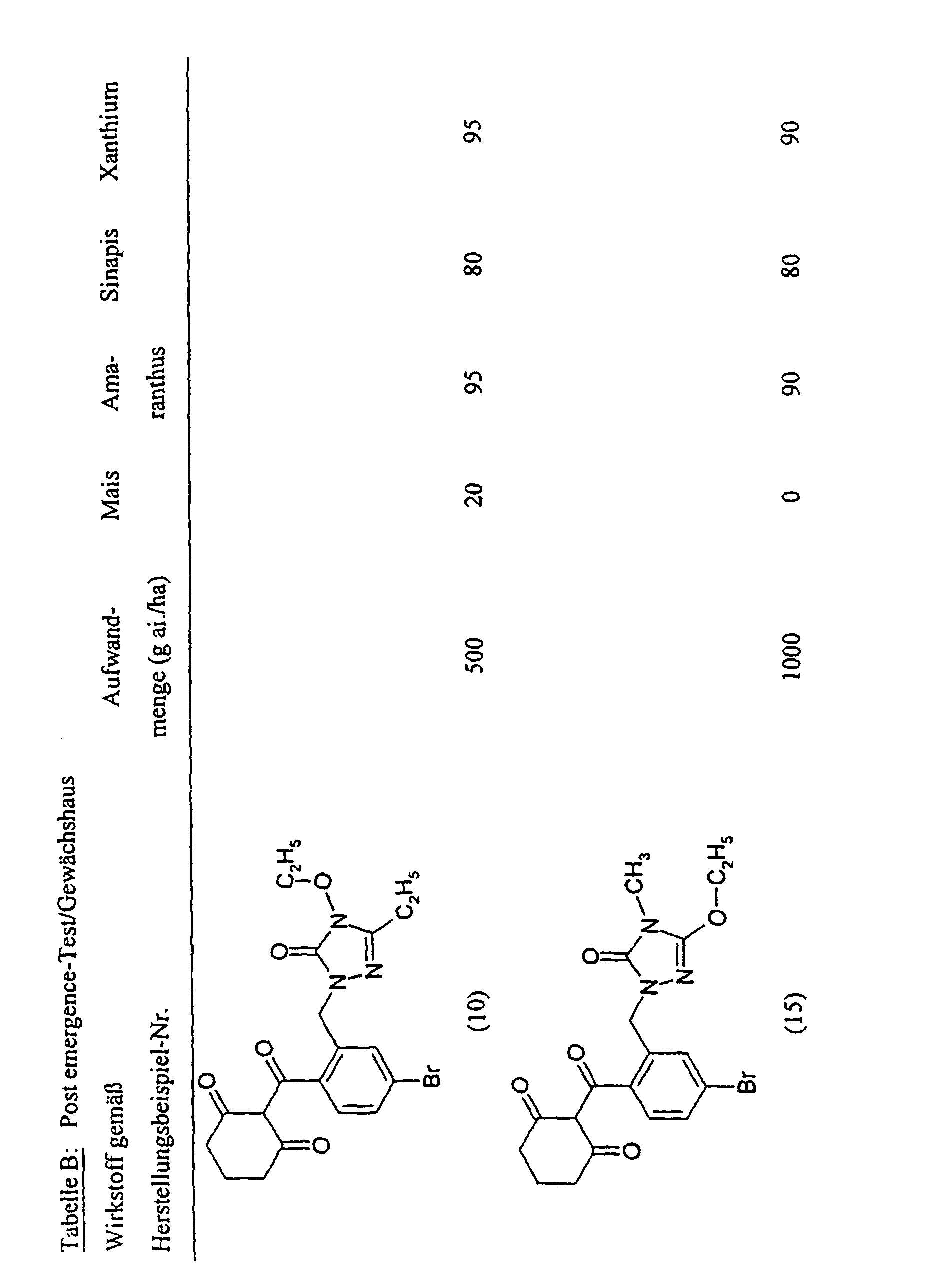

## Patentansprüche

1. Verbindungen der allgemeinen Formel (I), 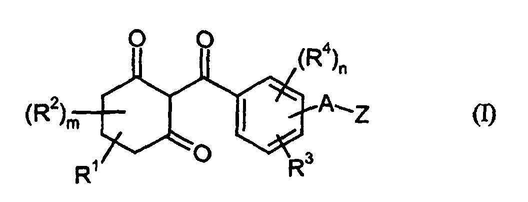 in welcher
m für die Zahlen 0, 1 oder 2 steht,
n für die Zahlen 0, 1 oder 2 steht,
A für Alkandiyl (Alkylen) mit 1 bis 4 Kohlenstoffatomen steht,
R¹ für Wasserstoff, für gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl mit 1 bis 6 Kohlenstoffatomen oder für Alkoxycarbonyl mit bis zu 6 Kohlenstoffatomen steht,
R² für gegebenenfalls durch Halogen substituiertes Alkyl mit 1 bis 6 Kohlenstoffatomen steht, oder zusammen mit R¹ für Alkandiyl (Alkylen) mit 2 bis 5 Kohlenstoffatomen steht, wobei in diesem Fall m für 1 steht und R¹ und R² am gleichen Kohlenstoffatom ("geminal") oder an zwei benachbarten Kohlenstoffatomen ("vicinal") stehen,
R³ für Wasserstoff, Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht,
R⁴ für Nitro, Cyano, Carboxy, Carbamoyl, Thiocarbamoyl, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkoxy, Alkylthio, Alkylsulfinyl oder Alkylsulfonyl mit jeweils bis zu 4 Kohlenstoffatomen in den Alkylgruppen, oder für Alkylamino, Dialkylamino oder Dialkylaminosulfonyl mit jeweils bis zu 4 Kohlenstoffatomen in den Alkylgruppen steht, und
Z für eine der nachstehenden heterocyclischen Gruppierungen steht 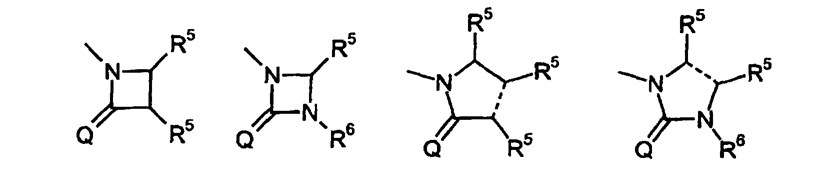 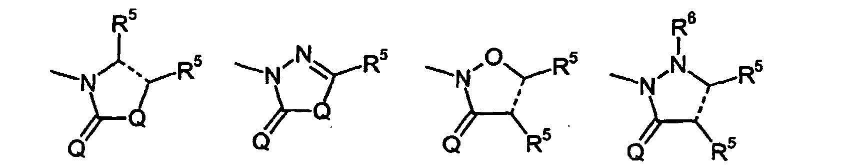 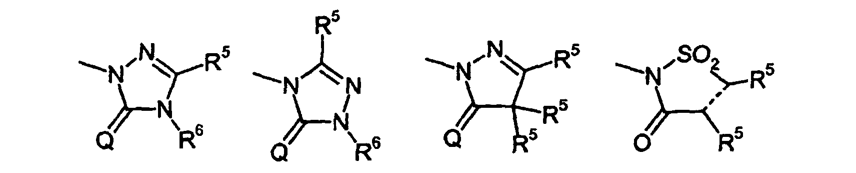 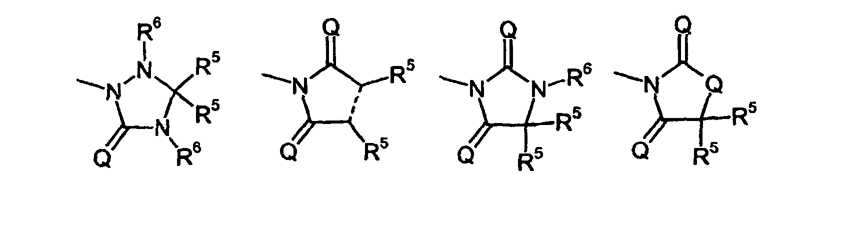 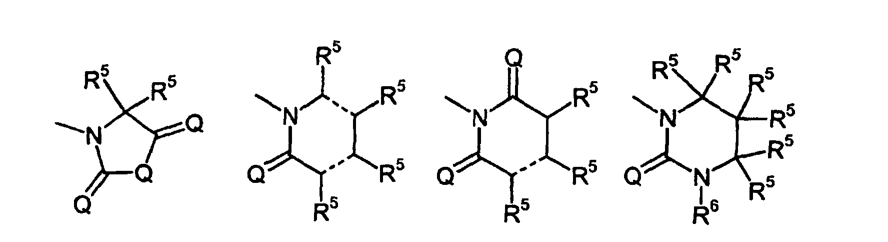 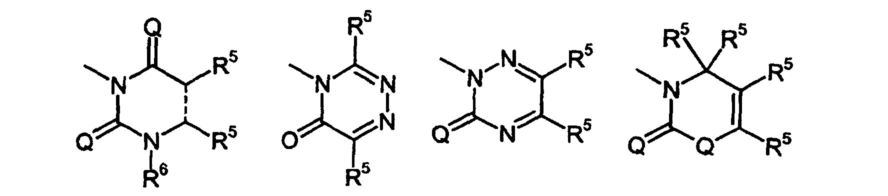 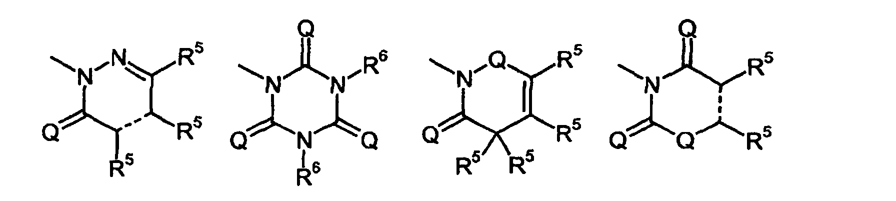 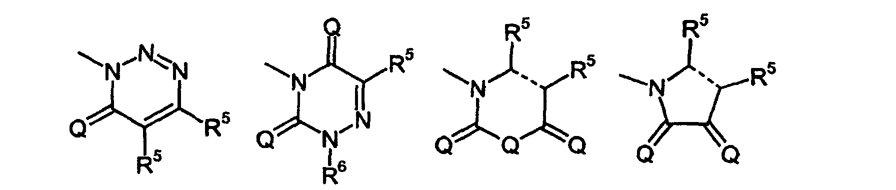 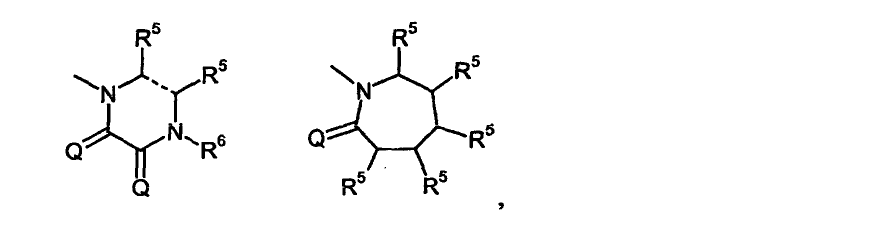
worin jeweils die gestrichelt gezeichnete Bindung eine Einfachbindung oder eine Doppelbindung ist,
Q für Sauerstoff oder Schwefel steht,
R⁵ für Wasserstoff, Hydroxy, Mercapto, Cyano, Halogen, für jeweils gegebenenfalls durch Halogen, C₁-C₄-Alkoxy, C₁-C₄-Alkylthio, C₁-C₄-Alkylsulfinyl oder C₁-C₄-Alkylsulfonyl substituiertes Alkyl, Alkylcarbonyl, Alkoxy, Alkoxycarbonyl, Alkylthio, Alkylsulfinyl oder Alkylsulfonyl mit jeweils bis zu 6 Kohlenstoffatomen in den Alkylgruppen, für jeweils gegebenenfalls durch Halogen substituiertes Alkylamino oder Dialkylamino mit jeweils bis zu 6 Kohlenstoffatomen in den Alkylgruppen, für jeweils gegebenenfalls durch Halogen substituiertes Alkenyl, Alkinyl, Alkenyloxy, Alkenylthio oder Alkenylamino mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl, Cycloalkyloxy, Cycloalkylthio oder Cycloalkylamino mit jeweils 3 bis 6 Kohlenstoffatomen in den Cycloalkylgruppen und gegebenenfalls bis zu 4 Kohlenstoffatomen im Alkylteil, oder für jeweils gegebenenfalls durch Halogen, C₁-C₄-Alkyl oder C₁-C₄-Alkoxy substituiertes Phenyl, Phenyloxy, Phenylthio, Phenylamino, Benzyl, Benzyloxy, Benzylthio oder Benzylamino steht, und
R⁶ für Wasserstoff, Hydroxy, Amino, Alkylidenamino mit bis zu 4 Kohlenstoffatomen, für jeweils gegebenenfalls durch Halogen oder C₁-C₄-Alkoxy substituiertes Alkyl, Alkoxy, Alkylamino, Dialkylamino oder Alkanoylamino mit jeweils bis zu 6 Kohlenstoffatomen in den Alkylgruppen, für jeweils gegebenenfalls durch Halogen substituiertes Alkenyl, Alkinyl oder Alkenyloxy mit jeweils bis zu 6 Kohlenstoffatomen in den Alkenyl- bzw. Alkinylgruppen, für jeweils gegebenenfalls durch Halogen substituiertes Cycloalkyl, Cycloalkylalkyl oder Cycloalkylamino mit jeweils 3 bis 6 Kohlenstoffatomen in den Cycloalkylgruppen und gegebenenfalls bis zu 3 Kohlenstoffatomen im Alkylteil, oder für jeweils gegebenenfalls durch Halogen, C₁-C₄-Alkyl oder C₁-C₄-Alkoxy substituiertes Phenyl oder Benzyl steht, oder zusammen mit einem benachbarten Rest R⁵ oder R⁶ für gegebenenfalls durch Halogen oder C₁-C₄-Alkyl substituiertes Alkandiyl mit 3 bis 5 Kohlenstoffatomen steht, oder - für den Fall, daß zwei benachbarte Reste R⁵ und R⁵ sich an einer Doppelbindung befinden - zusammen mit dem benachbarten Rest R⁵ auch für eine Benzogruppierung steht.

2. Verbindungen der Formel (I) gemäß Anspruch 1, **dadurch gekennzeichnet, daß**
m für die Zahlen 0, 1 oder 2 steht,
n für die Zahlen 0, 1 oder 2 steht,
A für Methylen, Ethyliden (Ethan-1,1-diyl) oder Dimethylen (Ethan-1,2-diyl) steht,
R¹ für Wasserstoff, für jeweils gegebenenfalls durch Fluor, Chlor, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i- oder s-Butyl, oder für Methoxycarbonyl, Ethoxycarbonyl, n- oder i-Propoxycarbonyl steht,
R² für Methyl, Ethyl, n- oder i-Propyl, oder zusammen mit R¹ für Methylen, Ethan-1,1-diyl (Ethyliden, -CH(CH₃)-), Ethan-1,2-diyl (Dimethylen, -CH₂CH₂-), Propan-1,3-diyl (Trimethylen, -CH₂CH₂CH₂-), Butan-1,4-diyl (Tetramethylen, -CH₂CH₂CH₂CH₂-) oder Pentan-1,5-diyl (Pentamethylen, -CH₂CH₂CH₂CH₂CH₂-) steht, wobei in diesem Fall m für 1 steht und R¹ und R² am gleichen Kohlenstoffatom ("geminal") oder an zwei benachbarten Kohlenstoffatomen ("vicinal") stehen,
R³ für Wasserstoff, Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht,
R⁴ für Nitro, Cyano, Carboxy, Carbamoyl, Thiocarbamoyl, Fluor, Chlor, Brom, für jeweils gegebenenfalls durch Fluor und/oder Chlor, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl substituiertes Methyl, Ethyl, n- oder i-Propyl, n-, i-, s- oder t-Butyl, für jeweils gegebenenfalls durch Fluor und/oder Chlor, Methoxy, Ethoxy, n- oder i-Propoxy substituiertes Methoxy, Ethoxy, n- oder i-Propoxy, für jeweils gegebenenfalls durch Fluor und/oder Chlor substituiertes Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Methylamino, Ethylamino, n- oder i-Propylamino, Dimethylamino, Diethylamino, Dimethylaminosulfonyl oder Diethylaminosulfonyl steht, und
Z für eine der nachstehenden heterocyclischen Gruppierungen steht 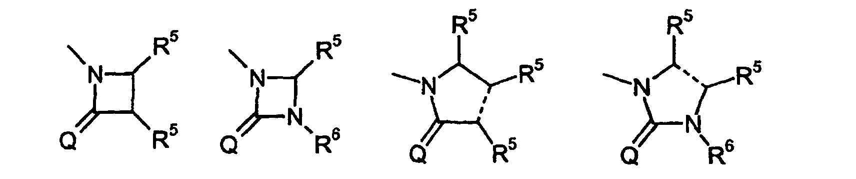 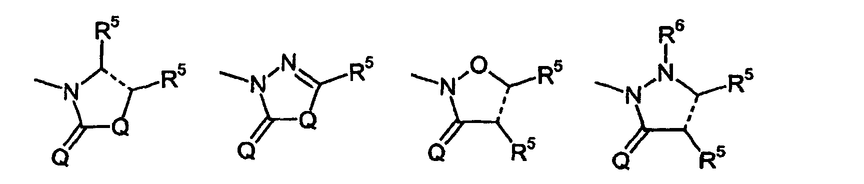 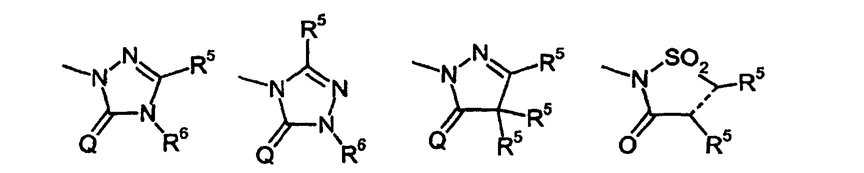 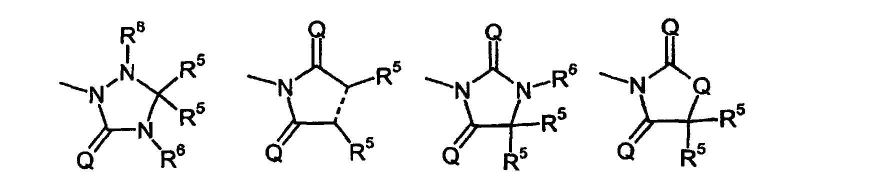 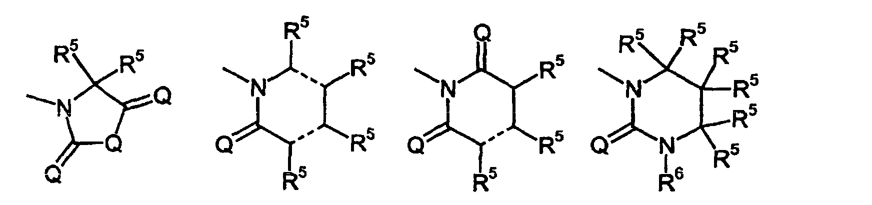 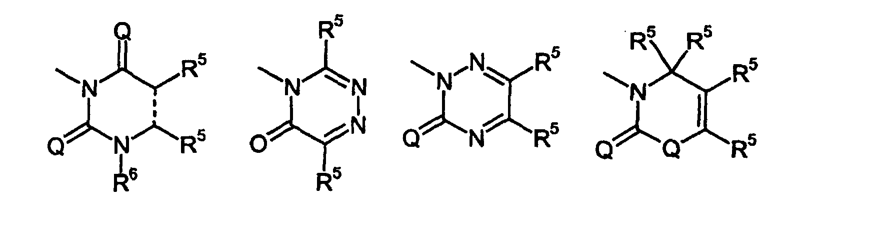 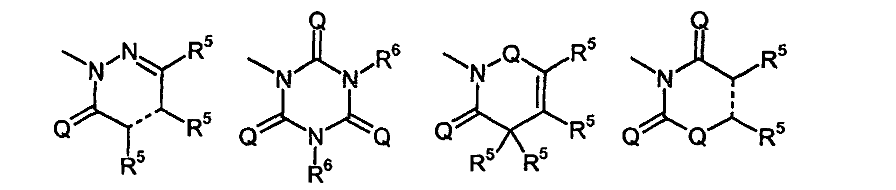 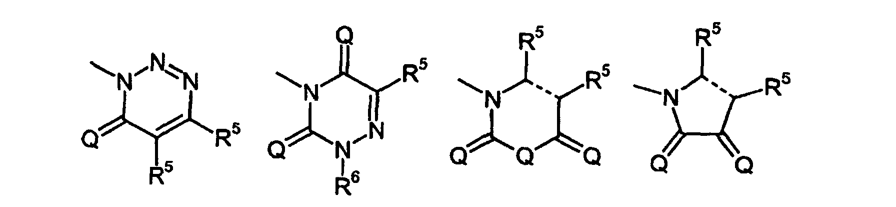 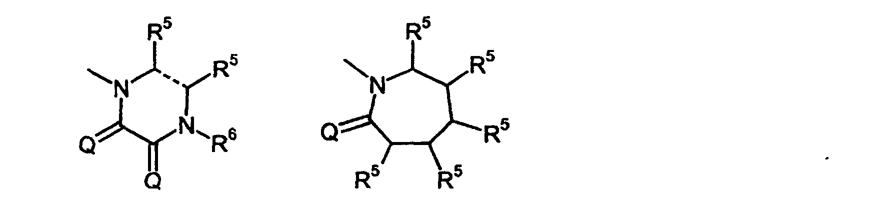
worin jeweils die gestrichelt gezeichnete Bindung eine Einfachbindung oder eine Doppelbindung ist,
Q für Sauerstoff oder Schwefel steht,
R⁵ für Wasserstoff, Hydroxy, Mercapto, Cyano, Fluor, Chlor, Brom, Iod, für jeweils gegebenenfalls durch Fluor, Chlor, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl substituiertes Methyl, Ethyl, n- oder i-Propyl, n-, i-, s - oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, für Methylamino, Ethylamino, n- oder i-Propylamino, n-, i-, s- oder t-Butylamino, Dimethylamino, Diethylamino, Di-n-propylamino oder Di-i-propylamino, für jeweils gegebenenfalls durch Fluor und/oder Chlor substituiertes Ethenyl, Propenyl, Butenenyl, Ethinyl, Propinyl, Butinyl, Propenyloxy, Butenyloxy, Propenylthio, Butenylthio, Propenylamino oder Butenylamino, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl, Phenyloxy, Phenylthio, Phenylamino, Benzyl, Benzyloxy, Benzylthio oder Benzylamino steht, und
R⁶ für Wasserstoff, Hydroxy, Amino, für jeweils gegebenenfalls durch Fluor und/oder Chlor, Methoxy oder Ethoxy substituiertes Methyl, Ethyl, n- oder i-Propyl, n-, i- oder s-Butyl, Methoxy, Ethoxy, n- oder i-Propoxy, Methylamino, Ethylamino oder Dimethylamino, für jeweils gegebenenfalls durch Fluor und/oder Chlor substituiertes Ethenyl, Propenyl, Ethinyl, Propinyl oder Propenyloxy, für jeweils gegebenenfalls durch Fluor und/oder Chlor substituiertes Cyclopropyl, Cyclobutyl, Cyclopentyl, Cyclohexyl, Cyclopropylmethyl, Cyclobutylmethyl, Cyclopentylmethyl, Cyclohexylmethyl, oder für jeweils gegebenenfalls durch Fluor, Chlor, Methyl, Ethyl, n- oder i-Propyl, n-, i-, s- oder t-Butyl, Methoxy, Ethoxy, n- oder i-Propoxy substituiertes Phenyl oder Benzyl steht, oder zusammen mit einem benachbarten Rest R⁵ oder R⁶ für jeweils gegebenenfalls durch Methyl und/oder Ethyl substituiertes Propan-1,3-diyl (Trimethylen) oder Butan-1,4-diyl (Tetramethylen) steht, oder - für den Fall, daß zwei benachbarte Reste R⁵ und R⁵ sich an einer Doppelbindung befinden - zusammen mit dem benachbarten Rest R⁵ auch für eine Benzogruppierung steht.

3. Verbindungen der allgemeinen Formel (IA), gemäß Anspruch 1 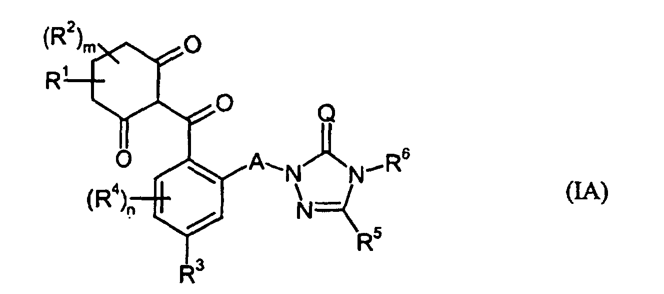 in welcher
m für die Zahlen 0, 1 oder 2 steht,
n für die Zahlen 0, 1 oder 2 steht,
A für Methylen steht,
Q für Sauerstoff oder Schwefel steht,
R¹ für Wasserstoff, Methyl, Ethyl, n- oder i-Propyl steht,
R² für Methyl steht,
R³ für Wasserstoff, Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁴ für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁵ für Methyl, Ethyl, n- oder i-Propyl, Trifluormethyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Cyclopropyl steht, und
R⁶ für Methyl, Ethyl, Methoxy, Ethoxy oder Cycloproypyl steht.

4. Verbindungen der allgemeinen Formel (IB), gemäß Anspruch 1 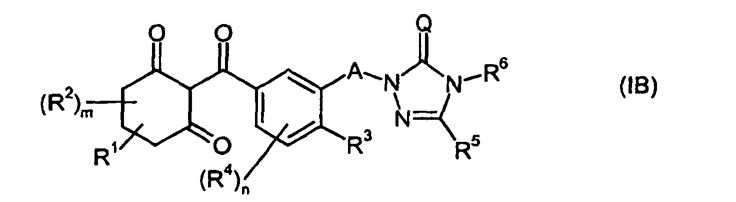 in welcher
m für die Zahlen 0, 1 oder 2 steht,
n für die Zahlen 0, 1 oder 2 steht,
A für Methylen steht,
Q für Sauerstoff oder Schwefel steht,
R¹ für Wasserstoff, Methyl, Ethyl, n- oder i-Propyl steht,
R² für Methyl steht,
R³ für Wasserstoff, Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁴ für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁵ für Methyl, Ethyl, n- oder i-Propyl, Trifluormethyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Cyclopropyl steht, und
R⁶ für Methyl, Ethyl, Methoxy, Ethoxy oder Cycloproypyl steht.

5. Verbindungen der allgemeinen Formel (IC), gemäß Anspruch 1 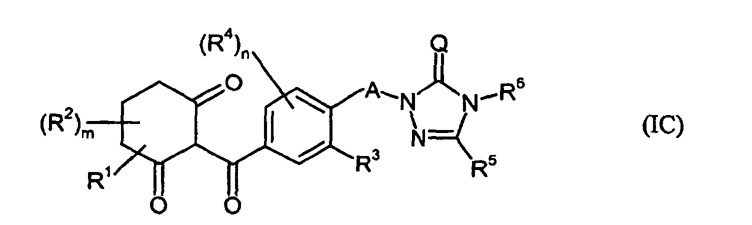 in welcher
m für die Zahlen 0, 1 oder 2 steht,
n für die Zahlen 0, 1 oder 2 steht,
A für Methylen steht,
Q für Sauerstoff oder Schwefel steht,
R¹ für Wasserstoff, Methyl, Ethyl, n- oder i-Propyl steht,
R² für Methyl steht,
R³ für Wasserstoff, Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluonmethoxy, Trifluonnethoxy, Methylthio, Ethylthio, Methylsulfinyl, Ethylsulfinyl, Methylsulfonyl, Ethylsulfonyl oder Dimethylaminosulfonyl steht,
R⁴ für Nitro, Cyano, Fluor, Chlor, Brom, Methyl, Ethyl, Trifluormethyl, Methoxymethyl, Methylthiomethyl, Methylsulfinylmethyl, Methylsulfonylmethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl oder Dimethylaminosulfonyl steht,
R⁵ für Methyl, Ethyl, n- oder i-Propyl, Trifluormethyl, Methoxy, Ethoxy, n- oder i-Propoxy, Methylthio, Ethylthio, n- oder i-Propylthio, Methylsulfinyl, Ethylsulfinyl, n- oder i-Propylsulfinyl, Methylsulfonyl, Ethylsulfonyl, n- oder i-Propylsulfonyl, oder für Cyclopropyl steht, und
R⁶ für Methyl, Ethyl, Methoxy, Ethoxy oder Cycloproypyl steht.

6. Verbindungen der Formel (I), (IA), (IB), (IC) gemäß einem der Ansprüche 1 bis 5, **dadurch gekennzeichnet, daß** es sich bei den Salzen um die Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium, Tri-(C₁-C₄-alkyl)-sulfonium-, C₅- oder C₆-Cycloalkyl-ammonium- und Di-(C₁-C₂-alkyl)-benzyl-ammonium-Salze handelt.

7. Verfahren zum Herstellen von Verbindungen der Formel (I), (IA), (IB), (IC) gemäß einem der Ansprüche 1 bis 5, **dadurch gekennzeichnet, daß** man 1,3-Cyclohexandion oder dessen Derivate der allgemeinen Formel (II), 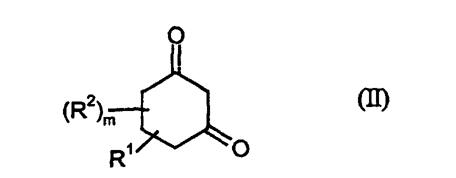 in welcher
m, R¹ und R² die in einem der Ansprüche 1 bis 5 angegebene Bedeutung haben,
mit substituierten Benzoesäuren der allgemeinen Formel (III), 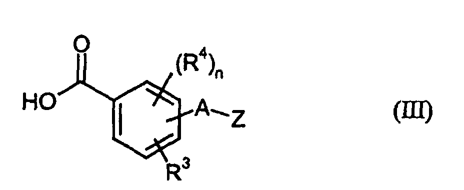 in welcher
n, A, R³, R⁴ und Z die in einem der Ansprüche 1 bis 5 angegebene Bedeutung haben,
in Gegenwart eines Dehydratisierungsmittels, gegebenenfalls in Gegenwart eines oder mehrerer Reaktionshilfsmittel und gegebenenfalls in Gegenwart eines Verdünnungsmittels, umsetzt,
und gegebenenfalls im Anschluß daran an den so erhaltenen Verbindungen der Formel (I) im Rahmen der Substituentendefinition auf übliche Weise elektrophile oder nucleophile bzw. Oxidations- oder Reduktionsreaktionen durchführt oder die Verbindungen der Formel (I) auf übliche Weise in Salze überführt.

8. Verbindungen der allgemeinen Formel (III), 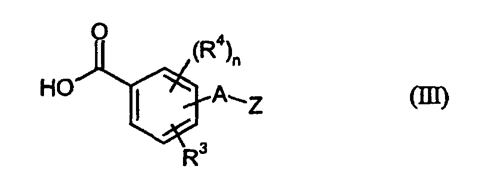 in welcher
n, A, R³, R⁴ und Z die in einem der Ansprüche 1 bis 5 angegebene Bedeutung haben.

9. Verwendung von mindestens einer Verbindung der Formel (I), (IA), (IB), (IC) gemäß einem der Ansprüche 1 bis 5 zur Bekämpfung von unerwünschten Pflanzen.

10. Herbizide Mittel, **gekennzeichnet durch** den Gehalt von mindestens einer Verbindung der Formel (I), (IA), (IB), (IC) gemäß einem der Ansprüche 1 bis 5 und üblichen Streckmitteln.

## Claims

1. Compounds of the general formula (I), 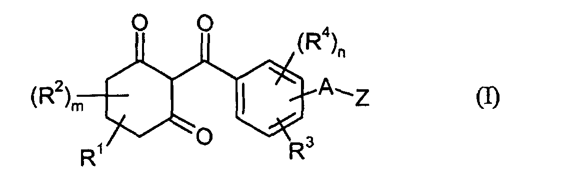 in which
m represents the numbers 0, 1 or 2,
n represents the numbers 0, 1 or 2,
A represents alkanediyl (alkylene) having 1 to 4 carbon atoms,
R¹ represents hydrogen, represents optionally halogen-, C₁-C₄-alkoxy-, C₁-C₄-alkylthio-, C₁-C₄-alkylsulphinyl- or C₁-C₄-alkylsulphonyl-substituted alkyl having 1 to 6 carbon atoms or represents alkoxycarbonyl having up to 6 carbon atoms,
R² represents optionally halogen-substituted alkyl having 1 to 6 carbon atoms, or together with R¹ represents alkanediyl (alkylene) having 2 to 5 carbon atoms, where in this case m represents 1 and R¹ and R² are located at the same carbon atom ("geminal") or at two adjacent carbon atoms ("vicinal"),
R³ represents hydrogen, nitro, cyano, carboxyl, carbamoyl, thiocarbamoyl, halogen, represents in each case optionally halogen-, C₁-C₄-alkoxy-, C₁-C₄-alkylthio-, C₁-C₄-alkylsulphinyl- or C₁-C₄-alkylsulphonyl-substituted alkyl, alkoxy, alkylthio, alkylsulphinyl or alkylsulphonyl having in each case up to 4 carbon atoms in the alkyl groups, or represents alkylamino, dialkylamino or dialkylaminosulphonyl having in each case up to 4 carbon atoms in the alkyl groups,
R⁴ represents nitro, cyano, carboxyl, carbamoyl, thiocarbamoyl, halogen, represents in each case optionally halogen-, C₁-C₄-alkoxy-, C₁-C₄-alkylthio-, C₁-C₄-alkylsulphinyl- or C₁-C₄-alkylsulphonyl-substituted alkyl, alkoxy, alkylthio, alkylsulphinyl or alkylsulphonyl having in each case up to 4 carbon atoms in the alkyl groups, or represents alkylamino, dialkylamino or dialkylaminosulphonyl having in each case up to 4 carbon atoms in the alkyl groups, and
Z represents one of the heterocyclic groupings below 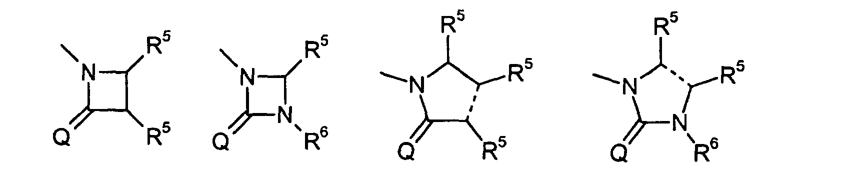 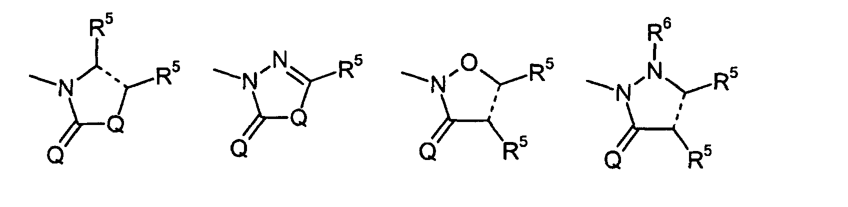 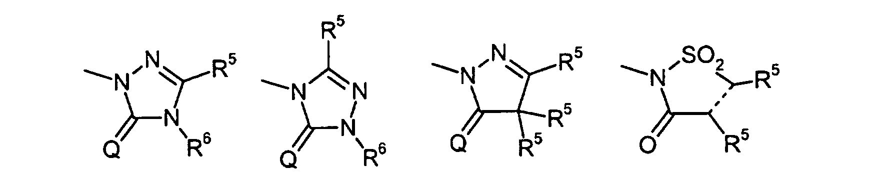 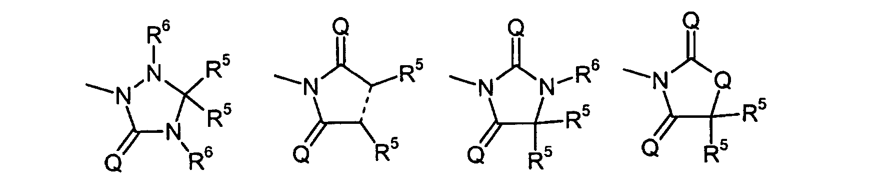 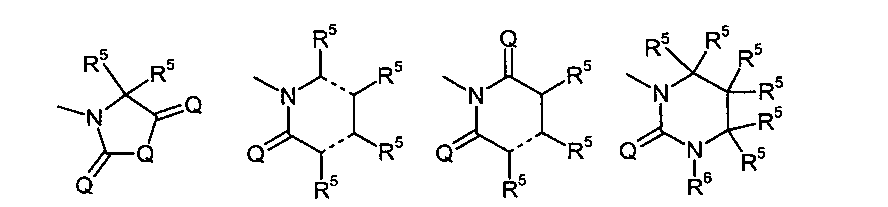 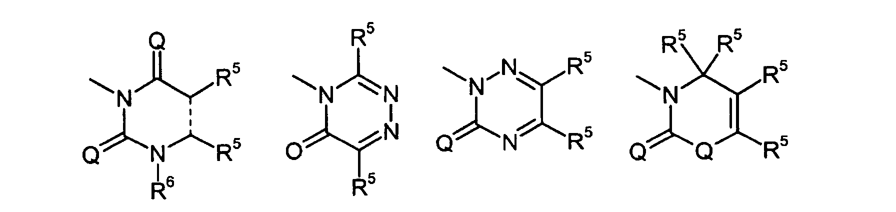 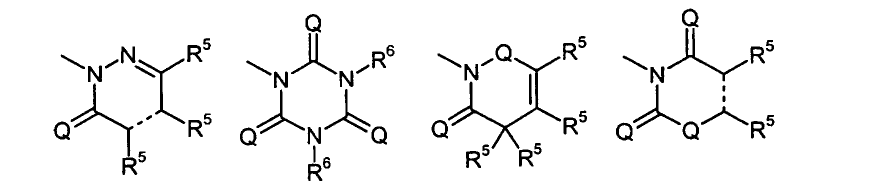 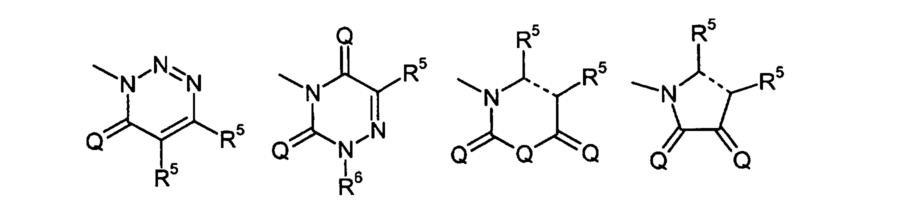 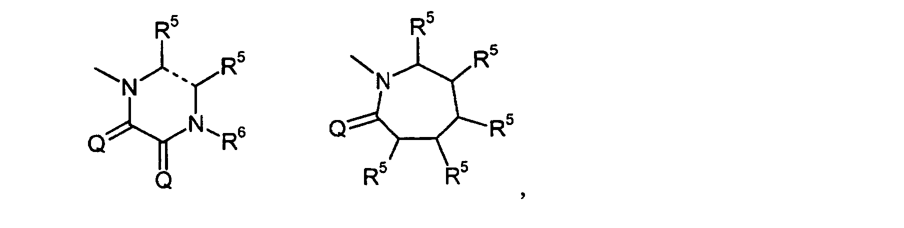
in which the bond drawn broken in each case denotes a single bond or a double bond,
Q represents oxygen or sulphur,
R⁵ represents hydrogen, hydroxyl, mercapto, cyano, halogen, represents in each case optionally halogen-, C₁-C₄-alkoxy-, C₁-C₄-alkylthio-, C₁-C₄-alkylsulphinyl- or C₁-C₄-alkylsulphonyl-substituted alkyl, alkylcarbonyl, alkoxy, alkoxycarbonyl, alkylthio, alkylsulphinyl or alkylsulphonyl having in each case up to 6 carbon atoms in the alkyl groups, represents in each case optionally halogen-substituted alkylamino or dialkylamino having in each case up to 6 carbon atoms in the alkyl groups, represents in each case optionally halogen-substituted alkenyl, alkinyl, alkenyloxy, alkenylthio or alkenylamino having in each case up to 6 carbon atoms in the alkenyl or alkinyl groups, represents in each case optionally halogen-substituted cycloalkyl, cycloalkylalkyl, cycloalkyloxy, cycloalkylthio or cycloalkylamino having in each case 3 to 6 carbon atoms in the cycloalkyl groups and optionally up to 4 carbon atoms in the alkyl moiety, or represents in each case optionally halogen-, C₁-C₄-alkyl- or C₁-C₄-alkoxy-substituted phenyl, phenyloxy, phenylthio, phenylamino, benzyl, benzyloxy, benzylthio or benzylamino, and
R⁶ represents hydrogen, hydroxyl, amino, alkylideneamino having up to 4 carbon atoms, represents in each case optionally halogen- or C₁-C₄-alkoxy-substituted alkyl, alkoxy, alkylamino, dialkylamino or alkanoylamino having in each case up to 6 carbon atoms in the alkyl groups, represents in each case optionally halogen-substituted alkenyl, alkinyl or alkenyloxy having in each case up to 6 carbon atoms in the alkenyl or alkinyl groups, represents in each case optionally halogen-substituted cycloalkyl, cycloalkylalkyl or cycloalkylamino having in each case 3 to 6 carbon atoms in the cycloalkyl groups and optionally up to 3 carbon atoms in the alkyl moiety, or represents in each case optionally halogen-, C₁-C₄-alkyl- or C₁-C₄-alkoxy-substituted phenyl or benzyl, or together with an adjacent radical R⁵ or R⁶ represents optionally halogen- or C₁-C₄-alkyl-substituted alkanediyl having 3 to 5 carbon atoms, or - in the case that two adjacent radicals R⁵ and R⁵ are located at a double bond - together with the adjacent radical R⁵ also represents a benzo grouping.

2. Compounds of the formula (I), according to Claim 1, **characterized in that**
m represents the numbers 0, 1 or 2,
n represents the numbers 0, 1 or 2,
A represents methylene, ethylidene (ethane-1,1-diyl) or dimethylene (ethane-1,2-diyl),
R¹ represents hydrogen, represents in each case optionally fluorine-, chlorine-, methoxy-, ethoxy-, n- or i-propoxy-, methylthio-, ethylthio-, n- or i-propylthio-, methylsulphinyl-, ethylsulphinyl-, n- or i-propylsulphinyl-, methylsulphonyl-, ethylsulphonyl-, n- or i-propylsulphonyl-substituted methyl, ethyl, n- or i-propyl, n-, i- or s-butyl, or represents methoxycarbonyl, ethoxycarbonyl, n- or i-propoxycarbonyl,
R² represents methyl, ethyl, n- or i-propyl, or together with R¹ represents methylene, ethane-1,1-diyl (ethylidene, -CH(CH₃)-), ethane-1,2-diyl (dimethylene, -CH₂CH₂-), propane-1,3-diyl (trimethylene, -CH₂CH₂CH₂₋), butane-1,4-diyl (tetramethylene, -CH₂CH₂CH₂CH₂-) or pentane-1,5-diyl (pentamethylene, -CH₂CH₂CH₂CH₂CH₂-), where in this case m represents 1 and R¹ and R² are located at the same carbon atom ("geminal") or at two adjacent carbon atoms ("vicinal"),
R³ represents hydrogen, nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents methylamino, ethylamino, n- or i-propylamino, dimethylamino, diethylamino, dimethylaminosulphonyl or diethylaminosulphonyl,
R⁴ represents nitro, cyano, carboxyl, carbamoyl, thiocarbamoyl, fluorine, chlorine, bromine, represents in each case optionally fluorine- and/or chlorine-, methoxy-, ethoxy-, n- or i-propoxy-, methylthio-, ethylthio-, n- or i-propylthio-, methylsulphinyl-, ethylsulphinyl-, methylsulphonyl- or ethylsulphonyl-substituted methyl, ethyl, n- or i-propyl, n-, i-, s- or t-butyl, represents in each case optionally fluorine- and/or chlorine-, methoxy-, ethoxy-, n- or i-propoxy-substituted methoxy, ethoxy, n- or i-propoxy, represents in each case optionally fluorine- and/or chlorine-substituted methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents methylamino, ethylamino, n- or i-propylamino, dimethylamino, diethylamino, dimethylaminosulphonyl or diethylaminosulphonyl, and
Z represents one of the heterocyclic groupings below 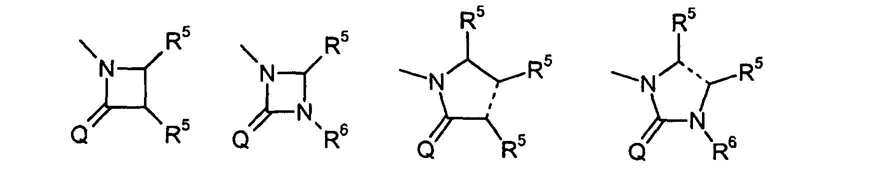 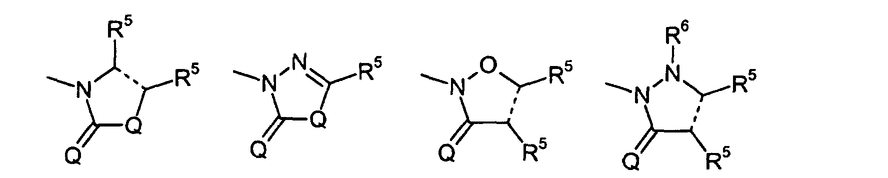 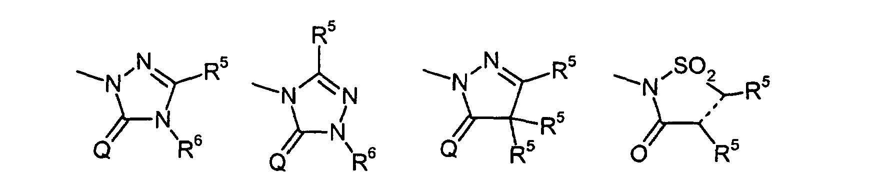 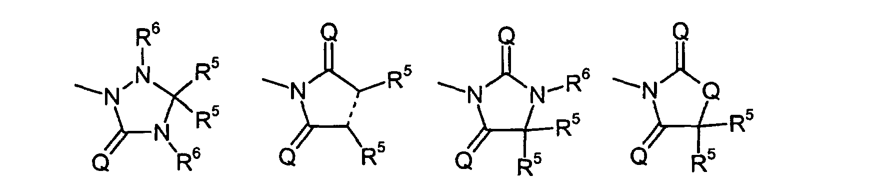 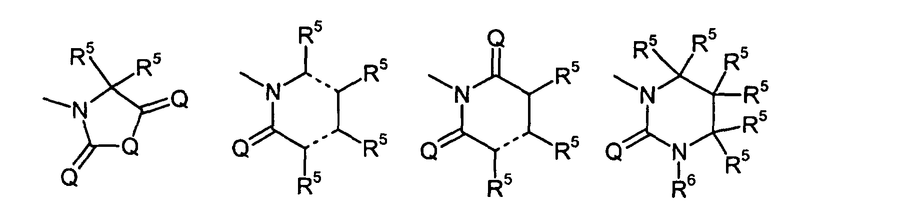 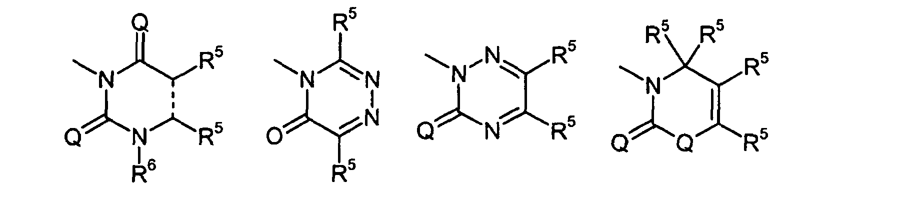 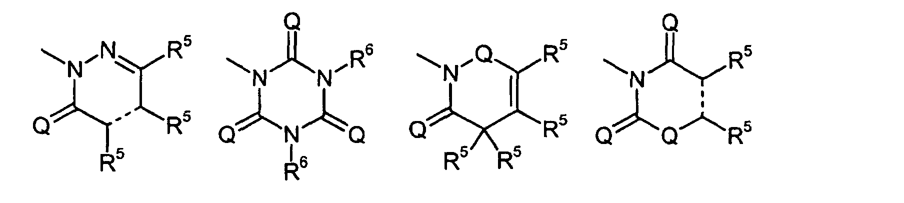 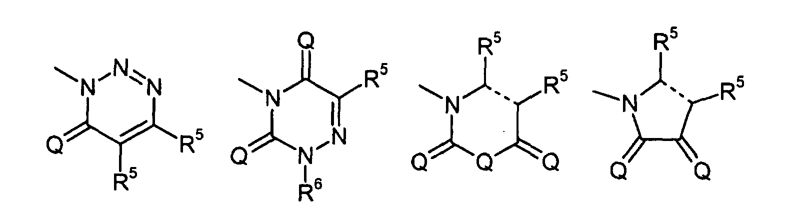 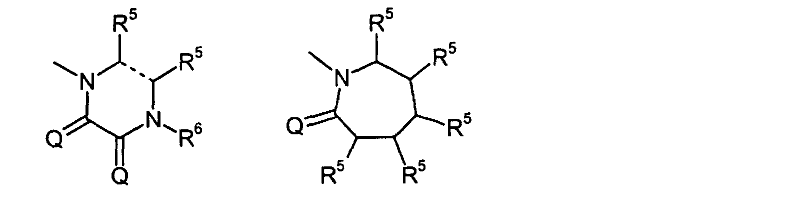
in which the bond drawn broken in each case denotes a single bond or a double bond,
Q represents oxygen or sulphur,
R⁵ represents hydrogen, hydroxyl, mercapto, cyano, fluorine, chlorine, bromine, iodine, represents in each case optionally fluorine-, chlorine-, methoxy-, ethoxy-, n- or i-propoxy-, n-, i-, s- or t-butoxy-, methylthio-, ethylthio-, n- or i-propylthio-, n-, i-, s- or t-butylthio-, methylsulphinyl-, ethylsulphinyl-, n- or i-propylsulphinyl-, methylsulphonyl-, ethylsulphonyl-, n- or i-propylsulphonyl-substituted methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, n-, i-, s- or t-butylthio, methylsulphinyl, ethylsulphinyl, n- or i-propylsulphinyl, methylsulphonyl, ethylsulphonyl, n- or i-propylsulphonyl, represents methylamino, ethylamino, n- or i-propylamino, n-, i-, s- or t-butylamino, dimethylamino, diethylamino, di-n-propylamino or di-i-propylamino, represents in each case optionally fluorine- and/or chlorine-substituted ethenyl, propenyl, butenenyl, ethinyl, propinyl, butinyl, propenyloxy, butenyloxy, propenylthio, butenylthio, propenylamino or butenylamino, represents in each case optionally fluorine- and/or chlorine-substituted cyclopropyl, cyclobutyl, cyclopentyl, cyclohexyl, cyclopropylmethyl, cyclobutylmethyl, cyclopentylmethyl, cyclohexylmethyl, cyclopropyloxy, cyclobutyloxy, cyclopentyloxy, cyclohexyloxy, cyclopropylthio, cyclobutylthio, cyclopentylthio, cyclohexylthio, cyclopropylamino, cyclobutylamino, cyclopentylamino or cyclohexylamino, or represents in each case optionally fluorine-, chlorine-, methyl-, ethyl-, n- or i-propyl-, n-, i-, s- or t-butyl-, methoxy-, ethoxy-, n- or i-propoxy-substituted phenyl, phenyloxy, phenylthio, phenylamino, benzyl, benzyloxy, benzylthio or benzylamino, and
R⁶ represents hydrogen, hydroxyl, amino, represents in each case optionally fluorine- and/or chlorine-, methoxy-, or ethoxy-substituted methyl, ethyl, n- or i-propyl, n-, i- or s-butyl, methoxy, ethoxy, n- or i-propoxy, methylamino, ethylamino or dimethylamino, represents in each case optionally fluorine- and/or chlorine-substituted ethenyl, propenyl, ethinyl, propinyl or propenyloxy, represents in each case optionally fluorine- and/or chlorine-substituted cyclopropyl, cyclobutyl, cyclopentyl, cyclohexyl, cyclopropylmethyl, cyclobutylmethyl, cyclopentylmethyl, cyclohexylmethyl, or represents in each case optionally fluorine-, chlorine-, methyl-, ethyl-, n- or i-propyl-, n-, i-, s- or t-butyl-, methoxy-, ethoxy-, n- or i-propoxy-substituted phenyl or benzyl, or together with an adjacent radical R⁵ or R⁶ represents in each case optionally methyl- and/or ethyl-substituted propane-1,3-diyl (trimethylene) or butane-1,4-diyl (tetramethylene), or - in the case that two adjacent radicals R⁵ and R⁵ are located at a double bond - together with the adjacent radical R⁵ also represents a benzo grouping.

3. Compounds of the general formula (IA), according to Claim 1 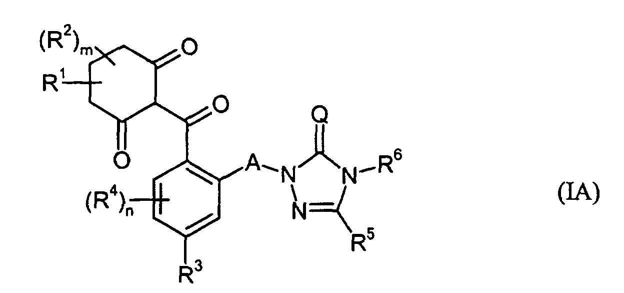 in which
m represents the numbers 0, 1 or 2,
n represents the numbers 0, 1 or 2,
A represents methylene,
Q represents oxygen or sulphur,
R¹ represents hydrogen, methyl, ethyl, n- or i-propyl,
R² represents methyl,
R³ represents hydrogen, nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁴ represents nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁵ represents methyl, ethyl, n- or i-propyl, trifluoromethyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents cyclopropyl, and
R⁶ represents methyl, ethyl, methoxy, ethoxy or cyclopropyl.

4. Compounds of the general formula (IB), according to Claim 1 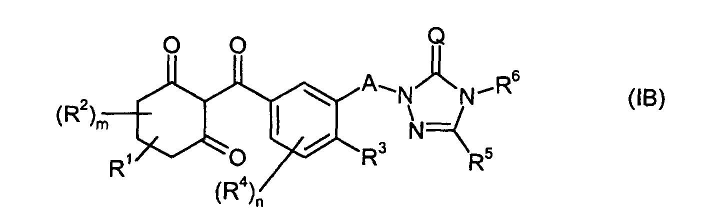 in which
m represents the numbers 0, 1 or 2,
n represents the numbers 0, 1 or 2,
A represents methylene,
Q represents oxygen or sulphur,
R¹ represents hydrogen, methyl, ethyl, n- or i-propyl,
R² represents methyl,
R³ represents hydrogen, nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethyl aminosulphonyl,
R⁴ represents nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁵ represents methyl, ethyl, n- or i-propyl, trifluoromethyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents cyclopropyl, and
R⁶ represents methyl, ethyl, methoxy, ethoxy or cyclopropyl.

5. Compounds of the general formula (IC), according to Claim 1 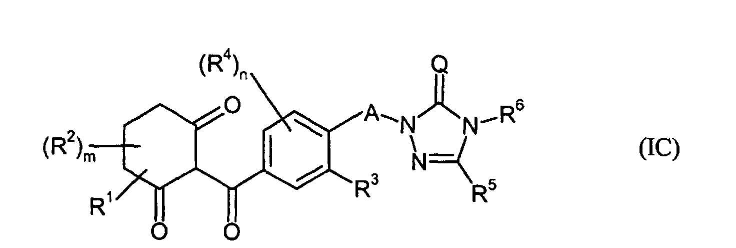 in which
m represents the numbers 0, 1 or 2,
n represents the numbers 0, 1 or 2,
A represents methylene,
Q represents oxygen or sulphur,
R¹ represents hydrogen, methyl, ethyl, n- or i-propyl,
R² represents methyl,
R³ represents hydrogen, nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁴ represents nitro, cyano, fluorine, chlorine, bromine, methyl, ethyl, trifluoromethyl, methoxymethyl, methylthiomethyl, methylsulphinylmethyl, methylsulphonylmethyl, methoxy, ethoxy, difluoromethoxy, trifluoromethoxy, methylthio, ethylthio, methylsulphinyl, ethylsulphinyl, methylsulphonyl, ethylsulphonyl or dimethylaminosulphonyl,
R⁵ represents methyl, ethyl, n- or i-propyl, trifluoromethyl, methoxy, ethoxy, n- or i-propoxy, methylthio, ethylthio, n- or i-propylthio, methylsulphinyl, ethylsulphinyl, n- or i-propylsulphinyl, methylsulphonyl, ethylsulphonyl, n- or i-propylsulphonyl, or represents cyclopropyl, and
R⁶ represents methyl, ethyl, methoxy, ethoxy or cyclopropyl.

6. Compounds of the formula (I), (IA), (IB), (IC) according to any of Claims 1 to 6, **characterized in that** the salts are the sodium, potassium, magnesium, calcium, ammonium, C₁-C₄-alkyl-ammonium, di-(C₁-C₄-alkyl)-ammonium, tri-(C₁-C₄-alkyl)-ammonium, tetra-(C₁-C₄-alkyl)-ammonium, tri-(C₁-C₄-alkyl)-sulphonium, C₅- or C₆-cycloalkyl-ammonium and di-(C₁-C₂-alkyl)-benzyl-ammonium salts.

7. Process for preparing compounds of the formula (I), (IA), (IB), (IC) according to any of Claims 1 to 5, **characterized in that** 1,3-cyclohexanedione or its derivatives of the general formula (II), 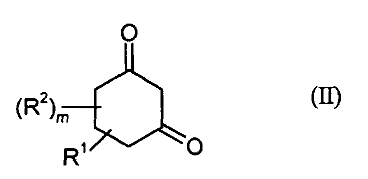 in which
m, R¹ and R² are each as defined in any of Claims 1 to 5,
are reacted with substituted benzoic acids of the general formula Formel (III), 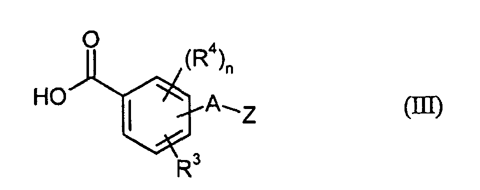 in which
n, A, R³, R⁴ and Z are each as defined in any of Claims 1 to 5,
in the presence of a dehydrating agent, if appropriate in the presence of one or more reaction auxiliaries and if appropriate in the presence of a diluent,
and, if appropriate, the compounds of the formula (I) obtained in this manner are subsequently subjected in a customary manner, within the scope of the definition of the substituents, to electrophilic or nucleophilic or oxidation or reduction reactions, or the compounds of the formula (I) are converted in a customary manner into salts.

8. Compounds of the general formula (III), 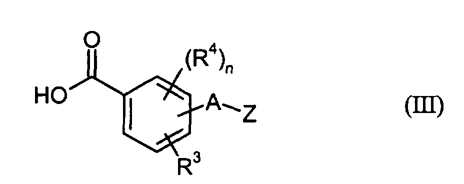 in which
n, A, R³, R⁴ and Z are each as defined in any of Claims 1 to 5.

9. Use of at least one compound of the formula (I), (IA), (IB), (IC) according to any of Claims 1 to 5 for controlling undesirable plants.

10. Herbicidal compositions, **characterized in that** they contain at least one compound of the formula (I), (IA), (IB), (IC) according to any of Claims 1 to 5 and customary extenders.

## Revendications

1. composés de formule générale (I) 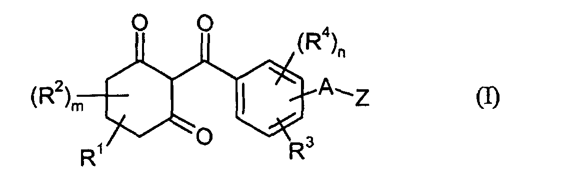 dans laquelle
m représente les nombres 0, 1 ou 2,
n représente les nombres 0, 1 ou 2,
A représente un reste alcanediyle (alkylène) ayant 1 à 4 atomes de carbone,
R¹ représente l'hydrogène, un reste alkyle ayant 1 à 6 atomes de carbone éventuellement substitué par un radical halogéno, alkoxy en C₁ à C₄, alkylthio en C₁ à C₄, alkylsulfinyle en C₁ à C₄ ou alkylsulfonyle en C₁ à C₄ ou un reste alkoxycarbonyle ayant jusqu'à 6 atomes de carbone,
R² représente un reste alkyle ayant 1 à 6 atomes de carbone éventuellement substitué par un halogène, ou forme conjointement avec R¹ un reste alcanediyle (alkylène) ayant 2 à 5 atomes de carbone, auquel cas m a la valeur 1 et R¹ et R² sont portés par le même atome de carbone ("géminés") ou par deux atomes de carbone contigus ("vicinaux"),
R³ représente l'hydrogène, un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et chacun étant éventuellement substitué par un radical halogéno, alkoxy en C₁ à C₄, alkylthio en C₁ à C₄, alkylsulfinyle en C₁ à C₄ ou alkylsulfonyle en C₁ à C₄, ou un reste alkylamino, dialkylamino ou dialkylaminosulfonyle ayant chacun jusqu'à 4 atomes de carbone dans les groupes alkyle,
R¹ représente un groupe nitro, cyano, carboxy, carbamoyle, thiocarbamoyle, un halogène, un reste alkyle, alkoxy, alkylthio, alkylsulfinyle ou alkylsulfonyle ayant chacun jusqu'à 4 atomes de carbone dans les groupes alkyle et chacun étant éventuellement substitué par un radical halogène, alkoxy en C₁ à C₄, alkylthio en C₁ à C₄, alkylsulfinyle en C₁ à C₄ ou alkoxysulfonyle en C₁ à C₄, ou un reste alkylamino, dialkylamino ou dialkylaminosulfonyle ayant jusqu'à 4 atomes de carbone dans les groupes alkyle, et
Z représente l'un des groupements hétérocycliques suivants 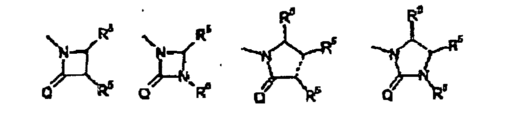 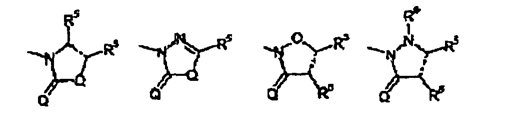 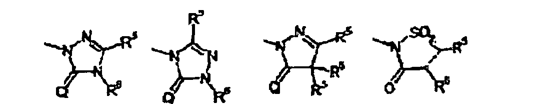 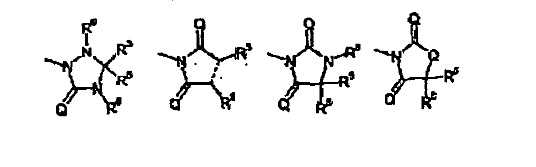 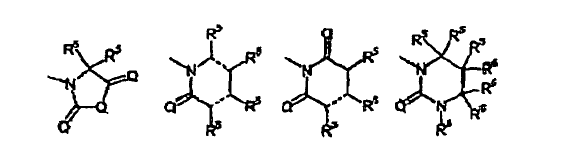 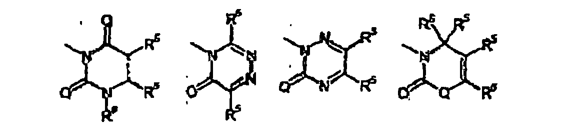 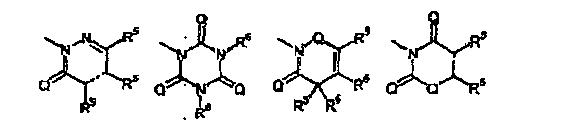 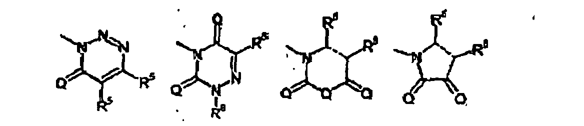 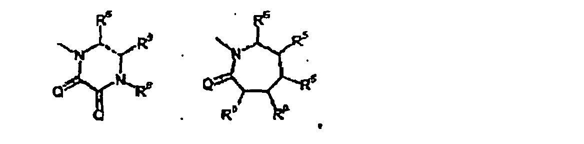
dans lesquels la liaison en traits interrompus représente à chaque fois une liaison simple ou une liaison double,
Q représente l'oxygène'ou le soufre,
R⁵ représente l'hydrogène, un groupe hydroxy, mercapto, cyano, un halogène, un reste alkyle, alkylcarbonyle, alkoxy, alkoxycarbonyle, alkylthio, alkylsulfinyle ou alkylsulfonyle ayant chacun jusqu'à 6 atomes de carbone dans les groupes alkyle et chacun étant éventuellement substitué par un radical halogène, alkoxy en C₁ à C₄, alkylthio en C₁ à C₄, alkylsulfinyle en C₁ à C₄ ou alkylsulfonyle en C₁ à C₄, un reste alkylamino ou dialkylamino ayant chacun jusqu'à 6 atomes de carbone dans les groupes alkyle et chacun étant éventuellement substitué par un radical halogèno, un reste alcényle, alcynyle, alcényloxy, alcénylthio ou alcénylamino ayant chacun jusqu'à 6 atomes de carbone dans les groupes alcényle et alcynyle et chacun étant éventuellement substitué par un radical halogène, un reste cycloalkyle, cycloalkylalkyle, cycloalkyloxy, cycloalkylthio ou cycloalkylamino ayant chacun 3 à 6 atomes de carbone dans les groupes cycloalkyle et le cas échéant jusqu'à 4 atomes de carbone dans la partie alkyle et chacun étant éventuellement; substitué par un radical halogène, ou un reste phényle, phényloxy, phénylthio, phénylamino, benzyle, benzyloxy, benzylthio ou benzylamino, chacun éventuellement substitué par un radical halogéno, alkyle en C₁ à C₄ ou alkoxy en C₁ à C₄, et
R⁶ représente l'hydrogène, un groupe hydroxy, amino, alkylidène-amino ayant jusqu'à 4 atomes de carbone, un reste alkyle, alkoxy, alkylamino, dialkylamino ou alcanoylamino ayant chacun jusqu'à 6 atomes de carbone dans les groupes alkyle et chacun étant éventuellement substitué par un radical halogèno ou alkoxy en C₁ à C₄, un reste alcényle, alcynyle ou alcényloxy ayant chacun jusqu' à 6 atomes de carbone dans les groupes alcényle et alcynyle et chacun étant éventuellement substitué par un halogène, un reste cycloalkyle, cycloalkylalkyle ou cycloalkylamino ayant chacun 3 à 6 atomes de carbone dans les groupes cycloalkyle et, le cas échéant, jusqu'à 3 atomes de carbone dans la partie alkyle et chacun étant éventuellement substitué par un radical halogéno, ou un reste phényle ou benzyle, chacun éventuellement substitué par un radical halogèno, alkyle en C₁ à C₄ ou alkoxy en C₁ à C₄ ou représente, conjointement avec un reste R⁵ ou R⁶ contigu un reste alcanediyle ayant 3 à 5 atomes de carbone éventuellement substitué par un radical halogèno ou alkyle en C₁ à C₄ ou représente aussi - au cas où deux restes R⁵ et R⁵ contigus sont adjacents à une double liaison - un groupement benzo conjointement avec le reste R⁵ contigu.

2. Composés de formule (I) suivant la revendication 1, **caractérisés en ce que**
m représente les nombres 0, 1, ou 2,
n représente les nombres 0, 1 ou 2,
A représente un reste méthylène, éthylidène (éthane-1,1-diyle) ou diméthylène (éthane-1,2-diyle),
R¹ représente l'hydrogène; un reste méthyle, éthyle, n-propyle, isopropyle, n-butyle, isobutyle ou sec.-butyle chacun éventuellement substitué par un radical fluoro, chloro, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle, ou un reste méthoxycarbonyle, éthoxycarbonyle, n-propoxycarbonyle ou isopropoxycarbonyle,
R² représente un reste méthyle, éthyle, n-propyle ou isopropyle ou forme conjointement avec R¹ un groupe méthylène, éthane-1,1-diyle (éthylidène, -CH(CH₃)-), éthane-1,2-diyle (diméthylène, -CH₂CH₂-), propane-1,3-diyle (triméthylène, -CH₂CH₂CH₂-), butane-1,4-diyle (tétraméthylène, -CH₂CH₂CH₂CH₂-) ou pentane-1,5-diyle (pentaméthylène, -CH₂CH₂CH₂CH₂CH₂-), auquel cas m est égal à 1 et R¹ et R² se trouvent sur le même atome de carbone ("géminés") ou sur deux atomes de carbone contigus ("vicinaux"),
R³ représente l'hydrogène, un groupe nitro, cyano, carboxy, carbamoyle, thiocarbamoyle, le fluor, le chlore, le brome, un reste méthyle, éthyle, n-propyle, isopropyle, n-butyle, isobutyle, sec.-butyle ou tertiobutyle chacun éventuellement substitué par un radical fluoro et/ou chloro, méthoxy, éthoxy, n-propoxy, isopropoxy, méthylthio, éthylyhio, n-propylthio, isopropylthio, méthylsulfinyle, éthylsulfinyle, méthylsulfonyle ou éthylsulfonyle, un reste méthoxy, éthoxy, n-propoxy ou isopropoxy chacun éventuellement substitué par un radical fluoro et/ou chloro, méthoxy, éthoxy, n-propoxy ou isopropoxy, un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle ou isopropylsulfonyle chacun éventuellement substitue par un radical fluoro et/ou chloro, ou un reste méthylamino, éthylamino, n-propylamino, isopropylamino; diméthylamino, diéthylamino, diméthylaminosulfonyle ou diéthylaminosulfonyle,
R⁴ représente un groupe nitro, cyano, carboxy, carbamoyle, thiocarbamoyle, le fluor, le chlore, le brome, un reste méthyle, éthyle, n-propyle, isopropyle, n-butyle, isobutyle, sec.-butyle ou tertiobutyle chacun éventuellement substitué par un radical fluoro et/ou chloro, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, un reste méthoxy, éthoxy, n-propoxy ou isopropoxy chacun éventuellement substitué par un radical fluoro et/ou chloro, méthoxy, éthoxy, n-propoxy ou isopropoxy, un reste méthylthio, éthylthio, n-propylthio, isopropylthio, méthylsulfinyle, éthylsulfinyle, n-propylsulfinyle, isopropylsulfinyle, méthylsulfonyle, éthylsulfonyle n-propylsulfonyle ou isopropylsulfonyle, chacun éventuellement substitué par un radical fluoro et/ou chloro, ou un reste méthylamino, éthylamino, n-propylamino, isopropylamino, diméthylamino, diéthylamino, diméthylaminosulfonyle ou diéthylaminosulfonyle, et
Z représente l'un des groupements hétérocycliques suivants 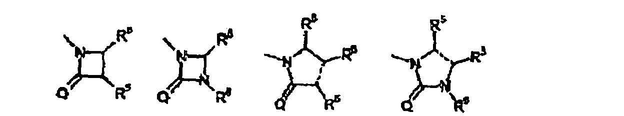 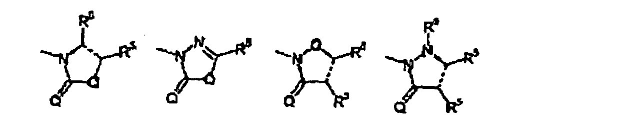 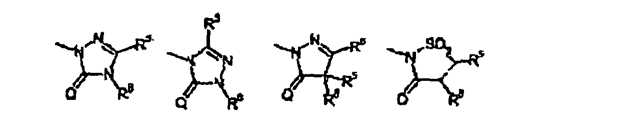 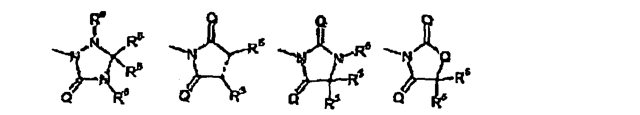 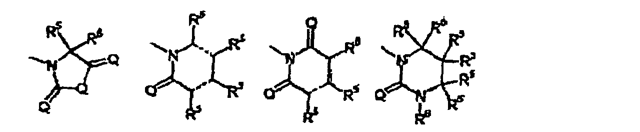 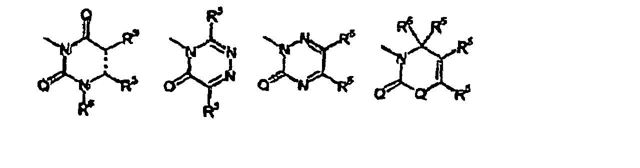 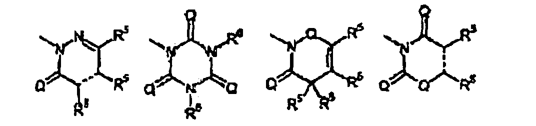 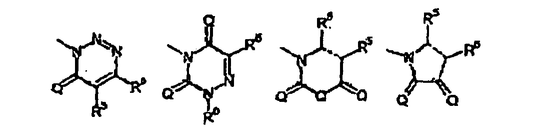 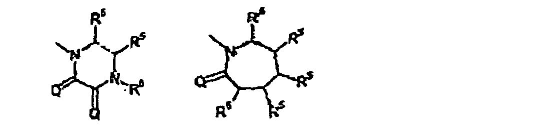
dans chacun desquels la liaison en traits interrompus représente à chaque fois une liaison simple ou une liaison double,
Q représente l'oxygène ou le soufre,
R⁵ représente l'hydrogène, un groupe hydroxy, mercapto, cyano, le fluor, le chlore, le brome, l'iode, un reste méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, n-butyl-thio, isobutylthio, sec.-butylthio, tertiobutylthio, méthylsulfinyle, éthylsulfinyle, n-propylsulfinyle, isopropylsulfinyle, méthylsulfonyle, éthylsulfonyle, n-propylsulfonyle ou isopropylsulfonyle, chacun éventuellement substitué par un radical fluoro, chloro, méthoxy, éthoxy, n-propoxy, isopropoxy, n-butoxy, isobutoxy, sec.-butoxy, tertiobutoxy, méthylthio, éthylthio, n-propylthio, isopropylthio, n-butylthio, isobutylthio, sec.-butylthio, tertiobutylthio, méthylsulfinyle, éthylsulfinyle, n-propylsulfinyle, isopropylsulfinyle, méthylsulfonyle, éthyl-sulfonyle, n-propylsulfonyle ou isopropylsulfonyle, un reste méthylamino, éthylamino, n-propylamino, isopropylamino, n-butylamino, isobutylamino, sec.-butylamino, tertiobutylamino, diméthylamino, diéthylamino, di-n-propylamino ou diisopropylamino, un reste éthényle, propényle, butényle, éthynyle, propynyle, butynyle, propényloxy, butényloxy, propénylthio, buténylthio, propénylamino ou buténylamino, chacun éventuellement substitué par un radical fluoro et/ou chloro, un reste cyclopropyle, cyclobutyle, cyclopentyle, cyclohexyle, cyclopropylméthyle, cyclobutylméthyle, cyclopentylméthyle, cyclohexylméthyle, cyclopropyloxy, cyclobutyloxy, cyclopentyloxy, cyclohexyloxy cyclopropylthio, cyclobutylthio, cyclopentylthio, cyclohexylthio, cyclopropylamino, cyclobutylamino, cyclopentylamino ou cyclohexylamino, chacun éventuellement substitué par un radical fluoro et/ou chloro, ou un radical phényle, phényloxy, phénylthio, phénylamino, benzyle, benzyloxy, benzylthio ou benzylamino chacun éventuellement substitué par un radical fluoro, chloro, méthyle, éthyle, n-propyle, isopropyle, n-butyle, isobutyle, sec.-butyle, tertiobutyle, méthoxy, éthoxy, n-propoxy ou isopropoxy, et
R⁶ représente l'hydrogène, un groupe hydroxy, amino, un reste méthyle, éthyle, n-propyle, isopropyle, n-butyle, isobutyle, sec.-butyle, méthoxy, éthoxy, n-propoxy, isopropoxy, méthylamino, éthylamino ou diméthylamino, chacun éventuellement substitué par un radical fluoro et/ou chloro, méthoxy ou éthoxy, un reste éthényle, propényle, éthynyle, propynyle ou propényloxy chacun éventuellement substitué par un radical fluoro et/ou (chloro, un reste cyclopropyle, cyclobutyle, cyclopentyle, cyclohexyle, cyclopropylméthyle, cyclobutylméthyle, cyclopentylméthyle, cyclohexylméthyle, chacun éventuellement substitué par un radical fluoro et/ou chloro, ou un reste phényle ou benzyle chacun éventuellement substitué par un radical fluoro, chloro, méthyle, éthyle, n-propyle, isopropyle, n-butyle, isobutyle, sec.-butyle, tertiobutyle, méthoxy, éthoxy, n-propoxy ou isopropoxy, ou forme conjointement avec un reste R⁵ ou R⁶ contigu un reste propane-1,3-diyle (triméthylène) ou butane-1,4-diyle (tétraméthylène) chacun éventuellement substitué par un radical méthyle et/ou éthyle, ou forme aussi - au cas où deux restes R⁵ et R⁵ contigus sont adjacents à une double liaison - un groupement benzo conjointement avec le reste R⁵ contigu.

3. Composés de formule générale (IA) suivant la revendication 1, 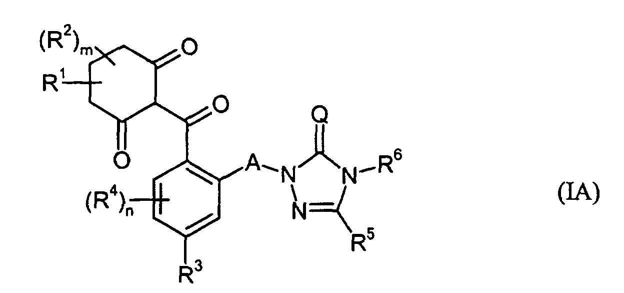 formule dans laquelle
m représente les nombres 0, 1 ou 2,
n représente les nombres 0, 1 ou 2,
A est un groupe méthylène,
Q représente l'oxygène ou le soufre,
R¹ représente l'hydrogène, un reste méthyle, éthyle, n-propyle ou isopropyle
R² est un reste méthyle,
R³ représente l'hydrogène, un groupe nitro, cyano, le fluor, le chlore, le brome, un reste méthyle, éthyle, trifluorométhyle, méthoxyméthyle, méthylthiométhyle, méthylsulfinylaméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁴ est un groupe nitro, cyano, le fluor, le chlore, le brome, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁵ est un reste méthyle, éthyle, n-propyle, isopropyle, trifluorométhyle, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle ou un reste cyclopropyle, et
R⁶ est un reste méthyle, éthyle, méthoxy, éthoxy ou cyclopropyle.

4. Composés de formule générale (IB) suivant la revendication 1, 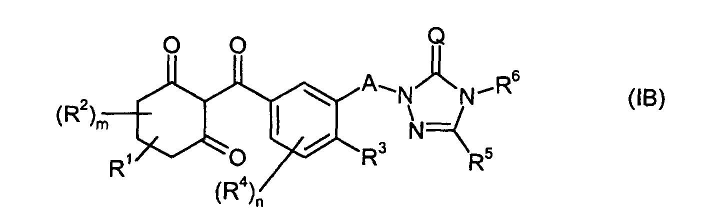 formule dans laquelle
m représente les nombres 0; 1 ou 2,
n représente les nombres 0, 1 ou 2,
A est un groupe méthylène,
Q représente l'oxygène ou le soufre,
R¹ représente l'hydrogène, un reste méthyle, éthyle, n-propyle ou isopropyle,
R² est un reste méthyle,
R³ représente l'hydrogène, un groupe nitro, cyano, le fluor, le chlore, le brome, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁴ représente un groupe nitro, cyano, le fluor, le chlore, le brome, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁵ représente un reste méthyle, éthyle, n-propyle, isopropyle, trifluorométhyle, méthoxy, éthoxy, n-propoxy, isopropoxy, iméthylthio, éthylthio, n-propylthio, isopropylthio, méthylsulfinyle, éthylsulfinyle, n-propylsulfinyle, isopropylsulfinyle, méthylsulfonyle, méthylsulfonyle, n-propylsulfonyle, isopropylsulfonyle ou un reste cyclopropyle, et
R⁶ est un reste méthyle, éthyle, méthoxy, éthoxy ou cyclopropyle.

5. Composés de formule générale (IC) suivant la revendication 1 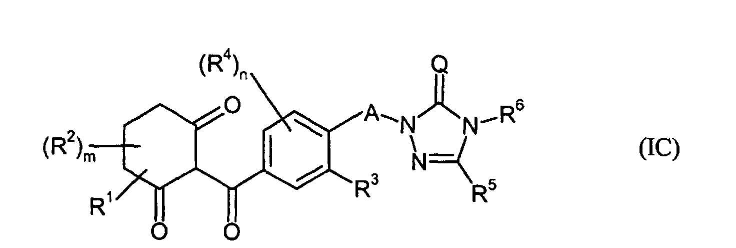 formule dans laquelle
m représente les nombres 0, 1 ou 2,
n représente les nombres 0, 1 ou 2,
A est un groupe méthylène,
Q représente l'oxygène ou le soufre,
R¹ représente l'hydrogène, un reste méthyle, éthyle, n-propyle ou isopropyle,
R² est un reste méthyle,
R³ représente l'hydrogène, un groupe nitro, cyano, le fluor, le chlore, le brome, un reste méthyle, éthyle, trifluorométhyle, méthoxyméthyle, méthylthiométhyle, méthylsulfinylméthyle, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁴ représente un groupe nitro, cyano, le fluor, le chlore, le brome, un reste méthyle, éthyle, trifluorométhyle, méthoxyméthyle, méthylthiométhyle, méthylsulfinylméthyl, méthylsulfonylméthyle, méthoxy, éthoxy, difluorométhoxy, trifluorométhoxy, méthylthio, éthylthio, méthylsulfinyle, éthylsulfinyle, méthylsulfonyle, éthylsulfonyle ou diméthylaminosulfonyle,
R⁵ est un reste méthyle, éthyle, n-propyle, isopropyle, trifluorométhyle, méthoxy, éthoxy, n-propoxy, isopropoxy, méthylthio, éthylthio, n-propylthio, isopropylthio, méthylsulfinyle, éthylsulfinyle, n-propylsulfinyle, isopropylsulfinyle, méthylsulfonyle, éthylsulfonyle, n-propylsulfonyle, isopropylsulfonyle ou un reste cyclopropyle, et
R⁶ est un reste méthyle, éthyle, méthoxy, éthoxy ou cyclopropyle.

6. Composés de formules (I), (IA), (IB), (IC) suivant l'une des revendications 1 à 5, **caractérisés en ce que** dans le cas de sels, il s'agit des sels de sodium, de potassium, de magnésium, de calcium, d'ammonium, d' (alkyle en C₁ à C₄)-ammonium, de di-(alkyle en C₁ à C₄)-ammonium, de tri- (alkyle en C₁ à C₄)-ammonium, de tétra- (alkyle en C₁ à C₄)-ammonium, de tri-(alkyle en C₁ à C₄)-sulfonium, de (cycloalkyle en C₅ ou C₆)-ammonium et de di-(alkyle en C₁ ou C₂)-benzylammonium.

7. Procédé de production de composés de formules (I), (IA), (IB), (IC) suivant l'une des revendications 1 à 5, caractérisé en de qu'on fait réagir la 1,3-cyclohexanedione ou ses dérivas de formule générale (II) 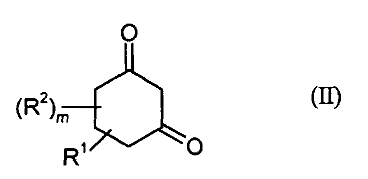 dans laquelle
m, R¹ et R² ont la définition indiquée dans l'une des revendications 1 à 5
avec des acides benzoïques substitués de formule générale (III) 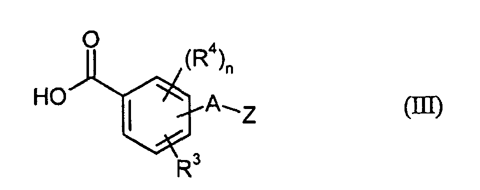 dans laquelle
n, A, R³, R⁴ et Z ont la définition indiquée dans l'une des revendications 1 à 5,
en présence d'un agent déshydratant, le cas échéant en présence d'un ou plusieurs auxiliaires de réaction et en présence éventuelle d'un diluant, après quoi on conduit éventuellement de manière usuelle sur les composés de formule (I) ainsi obtenus, dans le cadre de la définition des substituants, des réactions électrophiles ou nucléophiles, plus précisément d'oxydation ou de réduction ou bien on transforme les composés de formule (I) en sels d'une manière classique.

8. Composés de formule générale (III) 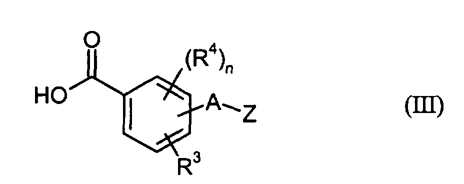 dans laquelle
n, A, R³, R⁴ et Z ont la définition indiquée dans l'une des revendications 1 à 5.

9. Utilisation d'au moins un composé des formules (I), (IA), (IB), (IC) suivant l'une des revendications 1 à 5 dans la lutte contre des plantes indésirables.

10. Compositions herbicides, **caractérisées par** une teneur en au moins un composé des formules (I), (IA), (IB), (IC) suivant l'une des revendications 1 à 5 et des diluants usuels.
